# EUROPÄISCHE PATENTSCHRIFT

(11) **EP 1 531 683 B1**
(45) Veröffentlichungstag und Bekanntmachung des Hinweises auf die Patenterteilung: **13.10.2010**
(21) Anmeldenummer: 03792350.5
(22) Anmeldetag: 18.08.2003
(51) Int. Cl.: A23K 1/14, A23K 1/16, C09B 61/00, C12N 15/82

(54) **VERWENDUNG VON ASTAXANTHINHALTIGEN PFLANZEN ODER PFLANZENTEILEN DER GATTUNG TAGETES ALS FUTTERMITTEL**
USE OF ASTAXANTHIN-CONTAINING PLANTS OR PARTS OF PLANTS OF THE GENUS TAGETES AS ANIMAL FEED
UTILISATION DE PLANTES OU DE PARTIES DE PLANTES CONTENANT DE L' ASTAXANTHINE DU GENRE TAGETES COMME PRODUIT DE FOURRAGE

(30) Priorität: 20.08.2002 DE 10238980; 20.08.2002 DE 10238978; 20.08.2002 DE 10238979; 13.11.2002 DE 10253112; 16.12.2002 DE 10258971
(43) Veröffentlichungstag der Anmeldung: 25.05.2005
(73) Patentinhaber: Sungene GmbH & Co. KGaA, 06466 Gatersleben (DE); BASF SE, 67056 Ludwigshafen (DE); BASF Plant Science GmbH, 67056 Ludwigshafen (DE)
(72) Erfinder: FLACHMANN, Ralf, 06484 Quedlinburg (DE); SAUER, Matt, 06484 Quedlinburg (DE); SCHOPFER, Christel Renate, 06484 Quedlinburg (DE); KLEBSATTEL, Martin, 06484 Quedlinburg (DE); PFEIFFER, Angelika-Maria, 67134 Birkenheide (DE); LUCK, Thomas, 67435 Neustadt (DE); VOESTE, Dirk, 67105 Schifferstadt (DE)
(74) Vertreter: Bieberbach, Andreas
(86) Internationale Anmeldenummer: PCT/EP2003/009109
(87) Internationale Veröffentlichungsnummer: WO 2004/017749

(56) Entgegenhaltungen:
- WO-A-00/32788
- WO-A-99/07867
- WO-A-03/077950
- WO-A-03/080849

### SEQUENCE LISTING

<110> SunGene GmbH Co. KGaA
<120> Verwendung von astaxanthinhaltigen Pflanzen oder Pflanzenteilen der Gattung Tagetes als Futtermittel
<130> PF 54148
<160> 142
<170> PatentIn version 3.1
<210> 1
   <211> 1771
   <212> DNA
   <213> Haematococcus pluvialis
<220>
   <221> CDS
   <222> (166)..(1155)
   <223>
<400> 1
   ggcacgagct tgcacgcaag tcagcgcgcg caagtcaaca cctgccggtc cacagcctca      60
   aataataaag agctcaagcg tttgtgcgcc tcgacgtggc cagtctgcac tgccttgaac     120
   ccgcgagtct cccgccgcac tgactgccat agcacagcta gacga atg cag cta gca     177
                                                     Met Gln Leu Ala
                                                     1
   gcg aca gta atg ttg gag cag ctt acc gga agc gct gag gca ctc aag        225
   Ala Thr Val Met Leu Glu Gln Leu Thr Gly Ser Ala Glu Ala Leu Lys
   5                   10                  15                  20
   gag aag gag aag gag gtt gca ggc agc tct gac gtg ttg cgt aca tgg        273
   Glu Lys Glu Lys Glu Val Ala Gly Ser Ser Asp Val Leu Arg Thr Trp
                   25                  30                  35
   gcg acc cag tac tcg ctt ccg tca gaa gag tca gac gcg gcc cgc ccg        321
   Ala Thr Gln Tyr Ser Leu Pro Ser Glu Glu Ser Asp Ala Ala Arg Pro
               40                  45                  50
   gga ctg aag aat gcc tac aag cca cca cct tcc gac aca aag ggc atc        369
   Gly Leu Lys Asn Ala Tyr Lys Pro Pro Pro Ser Asp Thr Lys Gly Ile
           55                  60                  65
   aca atg gcg cta cgt gtc atc ggc tcc tgg gcc gca gtg ttc ctc cac        417
   Thr Met Ala Leu Arg Val Ile Gly Ser Trp Ala Ala Val Phe Leu His
       70                  75                  80
   gcc att ttt caa atc aag ctt ccg acc tcc ttg gac cag ctg cac tgg        465
   Ala Ile Phe Gln Ile Lys Leu Pro Thr Ser Leu Asp Gln Leu His Trp
   85                  90                  95                  100
   ctg ccc gtg tca gat gcc aca gct cag ctg gtt agc ggc acg agc agc        513
   Leu Pro Val Ser Asp Ala Thr Ala Gln Leu Val Ser Gly Thr Ser Ser
                   105                 110                 115
   ctg ctc gac atc gtc gta gta ttc ttt gtc ctg gag ttc ctg tac aca        561
   Leu Leu Asp Ile Val Val Val Phe Phe Val Leu Glu Phe Leu Tyr Thr
               120                 125                 130
   ggc ctt ttt atc acc acg cat gat gct atg cat ggc acc atc gcc atg        609
   Gly Leu Phe Ile Thr Thr His Asp Ala Met His Gly Thr Ile Ala Met
           135                 140                 145
   aga aac agg cag ctt aat gac ttc ttg ggc aga gta tgc atc tcc ttg        657
   Arg Asn Arg Gln Leu Asn Asp Phe Leu Gly Arg Val Cys Ile Ser Leu
       150                 155                 160
   tac gcc tgg ttt gat tac aac atg ctg cac cgc aag cat tgg gag cac        705
   Tyr Ala Trp Phe Asp Tyr Asn Met Leu His Arg Lys His Trp Glu His
   165                 170                 175                 180
   cac aac cac act ggc gag gtg ggc aag gac cct gac ttc cac agg gga        753
   His Asn His Thr Gly Glu Val Gly Lys Asp Pro Asp Phe His Arg Gly
                   185                 190                 195
   aac cct ggc att gtg ccc tgg ttt gcc agc ttc atg tcc agc tac atg        801
   Asn Pro Gly Ile Val Pro Trp Phe Ala Ser Phe Met Ser Ser Tyr Met
               200                 205                 210
   tcg atg tgg cag ttt gcg cgc ctc gca tgg tgg acg gtg gtc atg cag        849
   Ser Met Trp Gln Phe Ala Arg Leu Ala Trp Trp Thr Val Val Met Gln
           215                 220                 225
   ctg ctg ggt gcg cca atg gcg aac ctg ctg gtg ttc atg gcg gcc gcg      897
   Leu Leu Gly Ala Pro Met Ala Asn Leu Leu Val Phe Met Ala Ala Ala
       230                 235                 240
   ccc atc ctg tcc gcc ttc cgc ttg ttc tac ttt ggc acg tac atg ccc      945
   Pro Ile Leu Ser Ala Phe Arg Leu Phe Tyr Phe Gly Thr Tyr Met Pro
   245                 250                 255                 260
   cac aag cct gag cct ggc gcc gcg tca ggc tct tca cca gcc gtc atg      993
   His Lys Pro Glu Pro Gly Ala Ala Ser Gly Ser Ser Pro Ala Val Met
                   265                 270                 275
   aac tgg tgg aag tcg cgc act agc cag gcg tcc gac ctg gtc agc ttt     1041
   Asn Trp Trp Lys Ser Arg Thr Ser Gln Ala Ser Asp Leu Val Ser Phe
               280                 285                 290
   ctg acc tgc tac cac ttc gac ctg cac tgg gag cac cac cgc tgg ccc     1089
   Leu Thr Cys Tyr His Phe Asp Leu His Trp Glu His His Arg Trp Pro
           295                 300                 305
   ttc gcc ccc tgg tgg gag ctg ccc aac tgc cgc cgc ctg tct ggc cga     1137
   Phe Ala Pro Trp Trp Glu Leu Pro Asn Cys Arg Arg Leu Ser Gly Arg
       310                 315                 320
   ggt ctg gtt cct gcc tag ctggacacac tgcagtgggc cctgctgcca            1185
   Gly Leu Val Pro Ala
   325
   gctgggcatg caggttgtgg caggactggg tgaggtgaaa agctgcaggc gctgctgccg   1245
   gacacgctgc atgggctacc ctgtgtagct gccgccacta ggggaggggg tttgtagctg   1305
   tcgagcttgc cccatggatg aagctgtgta gtggtgcagg gagtacaccc acaggccaac   1365
   acccttgcag gagatgtctt gcgtcgggag gagtgttggg cagtgtagat gctatgattg   1425
   tatcttaatg ctgaagcctt taggggagcg acacttagtg ctgggcaggc aacgccctgc   1485
   aaggtgcagg cacaagctag gctggacgag gactcggtgg caggcaggtg aagaggtgcg   1545
   ggagggtggt gccacaccca ctgggcaaga ccatgctgca atgctggcgg tgtggcagtg   1605
   agagctgcgt gattaactgg gctatggatt gtttgagcag tctcacttat tctttgatat   1665
   agatactggt caggcaggtc aggagagtga gtatgaacaa gttgagaggt ggtgcgctgc   1725
   ccctgcgctt atgaagctgt aacaataaag tggttcaaaa aaaaaa                  1771
<210> 2
   <211> 329
   <212> PRT
   <213> Haematococcus pluvialis
<400> 2
   Met Gln Leu Ala Ala Thr Val Met Leu Glu Gln Leu Thr Gly Ser Ala
   1               5                   10                  15
   Glu Ala Leu Lys Glu Lys Glu Lys Glu Val Ala Gly Ser Ser Asp Val
               20                  25                  30
   Leu Arg Thr Trp Ala Thr Gln Tyr Ser Leu Pro Ser Glu Glu Ser Asp
           35                  40                  45
   Ala Ala Arg Pro Gly Leu Lys Asn Ala Tyr Lys Pro Pro Pro Ser Asp
       50                  55                  60
   Thr Lys Gly Ile Thr Met Ala Leu Arg Val Ile Gly Ser Trp Ala Ala
   65                  70                  75                  80
   Val Phe Leu His Ala Ile Phe Gln Ile Lys Leu Pro Thr Ser Leu Asp
                   85                  90                  95
   Gln Leu His Trp Leu Pro Val Ser Asp Ala Thr Ala Gln Leu Val Ser
               100                 105                 110
   Gly Thr Ser Ser Leu Leu Asp Ile Val Val Val Phe Phe Val Leu Glu
           115                 120                 125
   Phe Leu Tyr Thr Gly Leu Phe Ile Thr Thr His Asp Ala Met His Gly
       130                 135                 140
   Thr Ile Ala Met Arg Asn Arg Gln Leu Asn Asp Phe Leu Gly Arg Val
   145                 150                 155                 160
   Cys Ile Ser Leu Tyr Ala Trp Phe Asp Tyr Asn Met Leu His Arg Lys
                   165                 170                 175
   His Trp Glu His His Asn His Thr Gly Glu Val Gly Lys Asp Pro Asp
   180 185 190
   Phe His Arg Gly Asn Pro Gly Ile Val Pro Trp Phe Ala Ser Phe Met
   195 200 205
   Ser Ser Tyr Met Ser Met Trp Gln Phe Ala Arg Leu Ala Trp Trp Thr
   210 215 220
   Val Val Met Gln Leu Leu Gly Ala Pro Met Ala Asn Leu Leu Val Phe
   225 230 235 240
   Met Ala Ala Ala Pro Ile Leu Ser Ala Phe Arg Leu Phe Tyr Phe Gly
   245 250 255
   Thr Tyr Met Pro His Lys Pro Glu Pro Gly Ala Ala Ser Gly Ser Ser
   260 265 270
   Pro Ala Val Met Asn Trp Trp Lys Ser Arg Thr Ser Gln Ala Ser Asp
   275 280 285
   Leu Val Ser Phe Leu Thr Cys Tyr His Phe Asp Leu His Trp Glu His
   290 295 300
   His Arg Trp Pro Phe Ala Pro Trp Trp Glu Leu Pro Asn Cys Arg Arg
   305 310 315 320
   Leu Ser Gly Arg Gly Leu Val Pro Ala
   325
<210> 3
   <211> 1662
   <212> DNA
   <213> Haematococcus pluvialis
<220>
   <221> CDS
   <222> (168)..(1130)
   <223>
<400> 3
   cggggcaact caagaaattc aacagctgca agcgcgcccc agcctcacag cgccaagtga      60
   gctatcgacg tggttgtgag cgctcgacgt ggtccactga cgggcctgtg agcctctgcg     120
   ctccgtcctc tgccaaatct cgcgtcgggg cctgcctaag tcgaaga atg cac gtc       176
                                                        Met His Val
                                                        1
   gca tcg gca cta atg gtc gag cag aaa ggc agt gag gca gct gct tcc       224
   Ala Ser Ala Leu Met Val Glu Gln Lys Gly Ser Glu Ala Ala Ala Ser
       5                   10                  15
   agc cca gac gtc ttg aga gcg tgg gcg aca cag tat cac atg cca tcc       272
   Ser Pro Asp Val Leu Arg Ala Trp Ala Thr Gln Tyr His Met Pro Ser
   20                  25                  30                  35
   gag tcg tca gac gca gct cgt cct gcg cta aag cac gcc tac aaa cct       320
   Glu Ser Ser Asp Ala Ala Arg Pro Ala Leu Lys His Ala Tyr Lys Pro
                   40                  45                  50
   cca gca tct gac gcc aag ggc atc acg atg gcg ctg acc atc att ggc       368
   Pro Ala Ser Asp Ala Lys Gly Ile Thr Met Ala Leu Thr Ile Ile Gly
               55                  60                  65
   acc tgg acc gca gtg ttt tta cac gca ata ttt caa atc agg cta ccg       416
   Thr Trp Thr Ala Val Phe Leu His Ala Ile Phe Gln Ile Arg Leu Pro
           70                  75                  80
   aca tcc atg gac cag ctt cac tgg ttg cct gtg tcc gaa gcc aca gcc       464
   Thr Ser Met Asp Gln Leu His Trp Leu Pro Val Ser Glu Ala Thr Ala
       85                  90                  95
   cag ctt ttg ggc gga agc agc agc cta ctg cac atc gct gca gtc ttc       512
   Gln Leu Leu Gly Gly Ser Ser Ser Leu Leu His Ile Ala Ala Val Phe
   100                 105                 110                 115
   att gta ctt gag ttc ctg tac act ggt cta ttc atc acc aca cat gac       560
   Ile Val Leu Glu Phe Leu Tyr Thr Gly Leu Phe Ile Thr Thr His Asp
                   120                 125                 130
   gca atg cat ggc acc ata gct ttg agg cac agg cag ctc aat gat ctc       608
   Ala Met His Gly Thr Ile Ala Leu Arg His Arg Gln Leu Asn Asp Leu
               135                 140                 145
   ctt ggc aac atc tgc ata tca ctg tac gcc tgg ttt gac tac agc atg       656
   Leu Gly Asn Ile Cys Ile Ser Leu Tyr Ala Trp Phe Asp Tyr Ser Met
           150                 155                 160
   ctg cat cgc aag cac tgg gag cac cac aac cat act ggc gaa gtg ggg      704
   Leu His Arg Lys His Trp Glu His His Asn His Thr Gly Glu Val Gly
       165                 170                 175
   aaa gac cct gac ttc cac aag gga aat ccc ggc ctt gtc ccc tgg ttc      752
   Lys Asp Pro Asp Phe His Lys Gly Asn Pro Gly Leu Val Pro Trp Phe
   180                 185                 190                 195
   gcc agc ttc atg tcc agc tac atg tcc ctg tgg cag ttt gcc cgg ctg      800
   Ala Ser Phe Met Ser Ser Tyr Met Ser Leu Trp Gln Phe Ala Arg Leu
                   200                 205                 210
   gca tgg tgg gca gtg gtg atg caa atg ctg ggg gcg ccc atg gca aat      848
   Ala Trp Trp Ala Val Val Met Gln Met Leu Gly Ala Pro Met Ala Asn
               215                 220                 225
   ctc cta gtc ttc atg gct gca gcc cca atc ttg tca gca ttc cgc ctc      896
   Leu Leu Val Phe Met Ala Ala Ala Pro Ile Leu Ser Ala Phe Arg Leu
           230                 235                 240
   ttc tac ttc ggc act tac ctg cca cac aag cct gag cca ggc cct gca      944
   Phe Tyr Phe Gly Thr Tyr Leu Pro His Lys Pro Glu Pro Gly Pro Ala
       245                 250                 255
   gca ggc tct cag gtg atg gcc tgg ttc agg gcc aag aca agt gag gca      992
   Ala Gly Ser Gln Val Met Ala Trp Phe Arg Ala Lys Thr Ser Glu Ala
   260                 265                 270                 275
   tct gat gtg atg agt ttc ctg aca tgc tac cac ttt gac ctg cac tgg     1040
   Ser Asp Val Met Ser Phe Leu Thr Cys Tyr His Phe Asp Leu His Trp
                   280                 285                 290
   gag cac cac agg tgg ccc ttt gcc ccc tgg tgg cag ctg ccc cac tgc     1088
   Glu His His Arg Trp Pro Phe Ala Pro Trp Trp Gln Leu Pro His Cys
               295                 300                 305
   cgc cgc ctg tcc ggg cgt ggc ctg gtg cct gcc ttg gca tga             1130
   Arg Arg Leu Ser Gly Arg Gly Leu Val Pro Ala Leu Ala
           310                 315                 320
   cctggtccct ccgctggtga cccagcgtct gcacaagagt gtcatgctac agggtgctgc   1190
   ggccagtggc agcgcagtgc actctcagcc tgtatggggc taccgctgtg ccactgagca   1250
   ctgggcatgc cactgagcac tgggcgtgct actgagcaat gggcgtgcta ctgagcaatg   1310
   ggcgtgctac tgacaatggg cgtgctactg gggtctggca gtggctagga tggagtttga    1370
   tgcattcagt agcggtggcc aacgtcatgt ggatggtgga agtgctgagg ggtttaggca   1430
   gccggcattt gagagggcta agttataaat cgcatgctgc tcatgcgcac atatctgcac   1490
   acagccaggg aaatcccttc gagagtgatt atgggacact tgtattggtt tcgtgctatt   1550
   gttttattca gcagcagtac ttagtgaggg tgagagcagg gtggtgagag tggagtgagt 1610
   gagtatgaac ctggtcagcg aggtgaacag cctgtaatga atgactctgt ct 1662
<210> 4
   <211> 320
   <212> PRT
   <213> Haematococcus pluvialis
<400> 4
   Met His Val Ala Ser Ala Leu Met Val Glu Gln Lys Gly Ser Glu Ala
   1 5 10 15
   Ala Ala Ser Ser Pro Asp Val Leu Arg Ala Trp Ala Thr Gln Tyr His
   20 25 30
   Met Pro Ser Glu Ser Ser Asp Ala Ala Arg Pro Ala Leu Lys His Ala
   35 40 45
   Tyr Lys Pro Pro Ala Ser Asp Ala Lys Gly Ile Thr Met Ala Leu Thr
   50 55 60
   Ile Ile Gly Thr Trp Thr Ala Val Phe Leu His Ala Ile Phe Gln Ile
   65 70 75 80
   Arg Leu Pro Thr Ser Met Asp Gln Leu His Trp Leu Pro Val Ser Glu
   85 90 95
   Ala Thr Ala Gln Leu Leu Gly Gly Ser Ser Ser Leu Leu His Ile Ala
   100 105 110
   Ala Val Phe Ile Val Leu Glu Phe Leu Tyr Thr Gly Leu Phe Ile Thr
   115 120 125
   Thr His Asp Ala Met His Gly Thr Ile Ala Leu Arg His Arg Gln Leu
   130 135 140
   Asn Asp Leu Leu Gly Asn Ile Cys Ile Ser Leu Tyr Ala Trp Phe Asp
   145                 150                 155                 160
   Tyr Ser Met Leu His Arg Lys His Trp Glu His His Asn His Thr Gly
                   165                 170                 175
   Glu Val Gly Lys Asp Pro Asp Phe His Lys Gly Asn Pro Gly Leu Val
               180                 185                 190
   Pro Trp Phe Ala Ser Phe Met Ser Ser Tyr Met Ser Leu Trp Gln Phe
           195                 200                 205
   Ala Arg Leu Ala Trp Trp Ala Val Val Met Gln Met Leu Gly Ala Pro
       210                 215                 220
   Met Ala Asn Leu Leu Val Phe Met Ala Ala Ala Pro Ile Leu Ser Ala
   225                 230                 235                 240
   Phe Arg Leu Phe Tyr Phe Gly Thr Tyr Leu Pro His Lys Pro Glu Pro
                   245                 250                 255
   Gly Pro Ala Ala Gly Ser Gln Val Met Ala Trp Phe Arg Ala Lys Thr
               260                 265                 270
   Ser Glu Ala Ser Asp Val Met Ser Phe Leu Thr Cys Tyr His Phe Asp
           275                 280                 285
   Leu His Trp Glu His His Arg Trp Pro Phe Ala Pro Trp Trp Gln Leu
       290                 295                 300
   Pro His Cys Arg Arg Leu Ser Gly Arg Gly Leu Val Pro Ala Leu Ala
   305                 310                 315                 320
<210> 5
   <211> 729
   <212> DNA
   <213> Agrobacterium aurantiacum
<220>
   <221> CDS
   <222> (1)..(729)
   <223>
<400> 5
   atg agc gca cat gcc ctg ccc aag gca gat ctg acc gcc acc agc ctg        48
   Met Ser Ala His Ala Leu Pro Lys Ala Asp Leu Thr Ala Thr Ser Leu
   1               5                   10                  15
   atc gtc tcg ggc ggc atc atc gcc gct tgg ctg gcc ctg cat gtg cat        96
   Ile Val Ser Gly Gly Ile Ile Ala Ala Trp Leu Ala Leu His Val His
               20                  25                  30
   gcg ctg tgg ttt ctg gac gca gcg gcg cat ccc atc ctg gcg atc gca       144
   Ala Leu Trp Phe Leu Asp Ala Ala Ala His Pro Ile Leu Ala Ile Ala
           35                  40                  45
   aat ttc ctg ggg ctg acc tgg ctg tcg gtc gga ttg ttc atc atc gcg       192
   Asn Phe Leu Gly Leu Thr Trp Leu Ser Val Gly Leu Phe Ile Ile Ala
       50                  55                  60
   cat gac gcg atg cac ggg tcg gtg gtg ccg ggg cgt ccg cgc gcc aat       240
   His Asp Ala Met His Gly Ser Val Val Pro Gly Arg Pro Arg Ala Asn
   65                  70                  75                  80
   gcg gcg atg ggc cag ctt gtc ctg tgg ctg tat gcc gga ttt tcg tgg       288
   Ala Ala Met Gly Gln Leu Val Leu Trp Leu Tyr Ala Gly Phe Ser Trp
                   85                  90                  95
   cgc aag atg atc gtc aag cac atg gcc cat cac cgc cat gcc gga acc       336
   Arg Lys Met Ile Val Lys His Met Ala His His Arg His Ala Gly Thr
               100                 105                 110
   gac gac gac ccc gat ttc gac cat ggc ggc ccg gtc cgc tgg tac gcc       384
   Asp Asp Asp Pro Asp Phe Asp His Gly Gly Pro Val Arg Trp Tyr Ala
           115                 120                 125
   cgc ttc atc ggc acc tat ttc ggc tgg cgc gag ggg ctg ctg ctg ccc       432
   Arg Phe Ile Gly Thr Tyr Phe Gly Trp Arg Glu Gly Leu Leu Leu Pro
       130                 135                 140
   gtc atc gtg acg gtc tat gcg ctg atc ctt ggg gat cgc tgg atg tac       480
   Val Ile Val Thr Val Tyr Ala Leu Ile Leu Gly Asp Arg Trp Met Tyr
   145                 150                 155                 160
   gtg gtc ttc tgg ccg ctg ccg tcg atc ctg gcg tcg atc cag ctg ttc       528
   Val Val Phe Trp Pro Leu Pro Ser Ile Leu Ala Ser Ile Gln Leu Phe
                   165                 170                 175
   gtg ttc ggc acc tgg ctg ccg cac cgc ccc ggc cac gac gcg ttc ccg      576
   Val Phe Gly Thr Trp Leu Pro His Arg Pro Gly His Asp Ala Phe Pro
               180                 185                 190
   gac cgc cac aat gcg cgg tcg tcg cgg atc agc gac ccc gtg tcg ctg      624
   Asp Arg His Asn Ala Arg Ser Ser Arg Ile Ser Asp Pro Val Ser Leu
           195                 200                 205
   ctg acc tgc ttt cac ttt ggc ggt tat cat cac gaa cac cac ctg cac      672
   Leu Thr Cys Phe His Phe Gly Gly Tyr His His Glu His His Leu His
       210                 215                 220
   ccg acg gtg ccg tgg tgg cgc ctg ccc agc acc cgc acc aag ggg gac      720
   Pro Thr Val Pro Trp Trp Arg Leu Pro Ser Thr Arg Thr Lys Gly Asp
   225                 230                 235                 240
   acc gca tga                                                          729
   Thr Ala
<210> 6
   <211> 242
   <212> PRT
   <213> Agrobacterium aurantiacum
<400> 6
   Met Ser Ala His Ala Leu Pro Lys Ala Asp Leu Thr Ala Thr Ser Leu
   1               5                   10                  15
   Ile Val Ser Gly Gly Ile Ile Ala Ala Trp Leu Ala Leu His Val His
               20                  25                  30
   Ala Leu Trp Phe Leu Asp Ala Ala Ala His Pro Ile Leu Ala Ile Ala
           35                  40                  45
   Asn Phe Leu Gly Leu Thr Trp Leu Ser Val Gly Leu Phe Ile Ile Ala
       50                  55                  60
   His Asp Ala Met His Gly Ser Val Val Pro Gly Arg Pro Arg Ala Asn
   65                  70                  75                  80
   Ala Ala Met Gly Gln Leu Val Leu Trp Leu Tyr Ala Gly Phe Ser Trp
                   85                  90                  95
   Arg Lys Met Ile Val Lys His Met Ala His His Arg His Ala Gly Thr
               100                 105                 110
   Asp Asp Asp Pro Asp Phe Asp His Gly Gly Pro Val Arg Trp Tyr Ala
           115                 120                 125
   Arg Phe Ile Gly Thr Tyr Phe Gly Trp Arg Glu Gly Leu Leu Leu Pro
       130                 135                 140
   Val Ile Val Thr Val Tyr Ala Leu Ile Leu Gly Asp Arg Trp Met Tyr
   145                 150                 155                 160
   Val Val Phe Trp Pro Leu Pro Ser Ile Leu Ala Ser Ile Gln Leu Phe
                   165                 170                 175
   Val Phe Gly Thr Trp Leu Pro His Arg Pro Gly His Asp Ala Phe Pro
               180                 185                 190
   Asp Arg His Asn Ala Arg Ser Ser Arg Ile Ser Asp Pro Val Ser Leu
           195                 200                 205
   Leu Thr Cys Phe His Phe Gly Gly Tyr His His Glu His His Leu His
       210                 215                 220
   Pro Thr Val Pro Trp Trp Arg Leu Pro Ser Thr Arg Thr Lys Gly Asp
   225                 230                 235                 240
   Thr Ala
<210> 7
   <211> 1631
   <212> DNA
   <213> Alcaligenes sp.
<220>
   <221> CDS
   <222> (99)..(827)
   <223>
<400> 7
   ctgcaggccg ggcccggtgg ccaatggtcg caaccggcag gactggaaca ggacggcggg      60
   ccggtctagg ctgtcgccct acgcagcagg agtttcgg atg tcc gga cgg aag cct     116
                                             Met Ser Gly Arg Lys Pro
                                             1               5
   ggc aca act ggc gac acg atc gtc aat ctc ggt ctg acc gcc gcg atc      164
   Gly Thr Thr Gly Asp Thr Ile Val Asn Leu Gly Leu Thr Ala Ala Ile
               10                  15                  20
   ctg ctg tgc tgg ctg gtc ctg cac gcc ttt acg cta tgg ttg cta gat      212
   Leu Leu Cys Trp Leu Val Leu His Ala Phe Thr Leu Trp Leu Leu Asp
           25                  30                  35
   gcg gcc gcg cat ccg ctg ctt gcc gtg ctg tgc ctg gct ggg ctg acc      260
   Ala Ala Ala His Pro Leu Leu Ala Val Leu Cys Leu Ala Gly Leu Thr
       40                  45                  50
   tgg ctg tcg gtc ggg ctg ttc atc atc gcg cat gac gca atg cac ggg      308
   Trp Leu Ser Val Gly Leu Phe Ile Ile Ala His Asp Ala Met His Gly
   55                  60                  65                  70
   tcc gtg gtg ccg ggg cgg ccg cgc gcc aat gcg gcg atc ggg caa ctg      356
   Ser Val Val Pro Gly Arg Pro Arg Ala Asn Ala Ala Ile Gly Gln Leu
                   75                  80                  85
   gcg ctg tgg ctc tat gcg ggg ttc tcg tgg ccc aag ctg atc gcc aag      404
   Ala Leu Trp Leu Tyr Ala Gly Phe Ser Trp Pro Lys Leu Ile Ala Lys
               90                  95                  100
   cac atg acg cat cac cgg cac gcc ggc acc gac aac gat ccc gat ttc      452
   His Met Thr His His Arg His Ala Gly Thr Asp Asn Asp Pro Asp Phe
           105                 110                 115
   ggt cac gga ggg ccc gtg cgc tgg tac ggc agc ttc gtc tcc acc tat      500
   Gly His Gly Gly Pro Val Arg Trp Tyr Gly Ser Phe Val Ser Thr Tyr
       120                 125                 130
   ttc ggc tgg cga gag gga ctg ctg cta ccg gtg atc gtc acc acc tat      548
   Phe Gly Trp Arg Glu Gly Leu Leu Leu Pro Val Ile Val Thr Thr Tyr
   135                 140                 145                 150
   gcg ctg atc ctg ggc gat cgc tgg atg tat gtc atc ttc tgg ccg gtc      596
   Ala Leu Ile Leu Gly Asp Arg Trp Met Tyr Val Ile Phe Trp Pro Val
                   155                 160                 165
   ccg gcc gtt ctg gcg tcg atc cag att ttc gtc ttc gga act tgg ctg      644
   Pro Ala Val Leu Ala Ser Ile Gln Ile Phe Val Phe Gly Thr Trp Leu
               170                 175                 180
   ccc cac cgc ccg gga cat gac gat ttt ccc gac cgg cac aac gcg agg      692
   Pro His Arg Pro Gly His Asp Asp Phe Pro Asp Arg His Asn Ala Arg
           185                 190                 195
   tcg acc ggc atc ggc gac ccg ttg tca cta ctg acc tgc ttc cat ttc      740
   Ser Thr Gly Ile Gly Asp Pro Leu Ser Leu Leu Thr Cys Phe His Phe
       200                 205                 210
   ggc ggc tat cac cac gaa cat cac ctg cat ccg cat gtg ccg tgg tgg      788
   Gly Gly Tyr His His Glu His His Leu His Pro His Val Pro Trp Trp
   215                 220                 225                 230
   cgc ctg cct cgt aca cgc aag acc gga ggc cgc gca tga cgcaattcct       837
   Arg Leu Pro Arg Thr Arg Lys Thr Gly Gly Arg Ala
                   235                 240
   cattgtcgtg gcgacagtcc tcgtgatgga gctgaccgcc tattccgtcc accgctggat    897
   tatgcacggc cccctaggct ggggctggca caagtcccat cacgaagagc acgaccacgc    957
   gttggagaag aacgacctct acggcgtcgt cttcgcggtg ctggcgacga tcctcttcac   1017
   cgtgggcgcc tattggtggc cggtgctgtg gtggatcgcc ctgggcatga cggtctatgg   1077
   gttgatctat ttcatcctgc acgacgggct tgtgcatcaa cgctggccgt ttcggtatat   1137
   tccgcggcgg ggctatttcc gcaggctcta ccaagctcat cgcctgcacc acgcggtcga   1197
   ggggcgggac cactgcgtca gcttcggctt catctatgcc ccacccgtgg acaagctgaa   1257
   gcaggatctg aagcggtcgg gtgtcctgcg cccccaggac gagcgtccgt cgtgatctct   1317
   gatcccggcg tggccgcatg aaatccgacg tgctgctggc aggggccggc cttgccaacg   1377
   gactgatcgc gctggcgatc cgcaaggcgc ggcccgacct tcgcgtgctg ctgctggacc   1437
   gtgcggcggg cgcctcggac gggcatactt ggtcctgcca cgacaccgat ttggcgccgc   1497
   actggctgga ccgcctgaag ccgatcaggc gtggcgactg gcccgatcag gaggtgcggt   1557
   tcccagacca ttcgcgaagg ctccgggccg gatatggctc gatcgacggg cgggggctga   1617
   tgcgtgcggt gacc                                                     1631
<210> 8
   <211> 242
   <212> PRT
   <213> Alcaligenes sp.
<400> 8
   Met Ser Gly Arg Lys Pro Gly Thr Thr Gly Asp Thr Ile Val Asn Leu
   1               5                   10                  15
   Gly Leu Thr Ala Ala Ile Leu Leu Cys Trp Leu Val Leu His Ala Phe
               20                  25                  30
   Thr Leu Trp Leu Leu Asp Ala Ala Ala His Pro Leu Leu Ala Val Leu
           35                  40                  45
   Cys Leu Ala Gly Leu Thr Trp Leu Ser Val Gly Leu Phe Ile Ile Ala
       50                  55                  60
   His Asp Ala Met His Gly Ser Val Val Pro Gly Arg Pro Arg Ala Asn
   65                  70                  75                  80
   Ala Ala Ile Gly Gln Leu Ala Leu Trp Leu Tyr Ala Gly Phe Ser Trp
                   85                  90                  95
   Pro Lys Leu Ile Ala Lys His Met Thr His His Arg His Ala Gly Thr
               100                 105                 110
   Asp Asn Asp Pro Asp Phe Gly His Gly Gly Pro Val Arg Trp Tyr Gly
           115                 120                 125
   Ser Phe Val Ser Thr Tyr Phe Gly Trp Arg Glu Gly Leu Leu Leu Pro
       130                 135                 140
   Val Ile Val Thr Thr Tyr Ala Leu Ile Leu Gly Asp Arg Trp Met Tyr
   145                 150                 155                 160
   Val Ile Phe Trp Pro Val Pro Ala Val Leu Ala Ser Ile Gln Ile Phe
                   165                 170                 175
   Val Phe Gly Thr Trp Leu Pro His Arg Pro Gly His Asp Asp Phe Pro
   180 185 190
   Asp Arg His Asn Ala Arg Ser Thr Gly Ile Gly Asp Pro Leu Ser Leu
   195 200 205
   Leu Thr Cys Phe His Phe Gly Gly Tyr His His Glu His His Leu His
   210 215 220
   Pro His Val Pro Trp Trp Arg Leu Pro Arg Thr Arg Lys Thr Gly Gly
   225 230 235 240
   Arg Ala
<400> 9
<210> 10
   <211> 242
   <212> PRT
   <213> Paracoccus marcusii
<400> 10
   Met Ser Ala His Ala Leu Pro Lys Ala Asp Leu Thr Ala Thr Ser Leu
   1               5                   10                  15
   Ile Val Ser Gly Gly Ile Ile Ala Ala Trp Leu Ala Leu His Val His
               20                  25                  30
   Ala Leu Trp Phe Leu Asp Ala Ala Ala His Pro Ile Leu Ala Val Ala
           35                  40                  45
   Asn Phe Leu Gly Leu Thr Trp Leu Ser Val Gly Leu Phe Ile Ile Ala
       50                  55                  60
   His Asp Ala Met His Gly Ser Val Val Pro Gly Arg Pro Arg Ala Asn
   65                  70                  75                  80
   Ala Ala Met Gly Gln Leu Val Leu Trp Leu Tyr Ala Gly Phe Ser Trp
                   85                  90                  95
   Arg Lys Met Ile Val Lys His Met Ala His His Arg His Ala Gly Thr
               100                 105                 110
   Asp Asp Asp Pro Asp Phe Asp His Gly Gly Pro Val Arg Trp Tyr Ala
           115                 120                 125
   Arg Phe Ile Gly Thr Tyr Phe Gly Trp Arg Glu Gly Leu Leu Leu Pro
       130                 135                 140
   Val Ile Val Thr Val Tyr Ala Leu Ile Leu Gly Asp Arg Trp Met Tyr
   145                 150                 155                 160
   Val Val Phe Trp Pro Leu Pro Ser Ile Leu Ala Ser Ile Gln Leu Phe
                   165                 170                 175
   Val Phe Gly Thr Trp Leu Pro His Arg Pro Gly His Asp Ala Phe Pro
               180                 185                 190
   Asp Arg His Asn Ala Arg Ser Ser Arg Ile Ser Asp Pro Val Ser Leu
           195                 200                 205
   Leu Thr Cys Phe His Phe Gly Gly Tyr His His Glu His His Leu His
       210                 215                 220
   Pro Thr Val Pro Trp Trp Arg Leu Pro Ser Thr Arg Thr Lys Gly Asp
   225                 230                 235                 240
   Thr Ala
<210> 11
   <211> 1629
   <212> DNA
   <213> Synechococcus sp.
<220>
   <221> CDS
   <222> (1)..(1629)
   <223>
<400> 11
   atg atc acc acc gat gtt gtc att att ggg gcg ggg cac aat ggc tta        48
   Met Ile Thr Thr Asp Val Val Ile Ile Gly Ala Gly His Asn Gly Leu
   1               5                   10                  15
   gtc tgt gca gcc tat ttg ctc caa cgg ggc ttg ggg gtg acg tta cta        96
   Val Cys Ala Ala Tyr Leu Leu Gln Arg Gly Leu Gly Val Thr Leu Leu
               20                  25                  30
   gaa aag cgg gaa gta cca ggg ggg gcg gcc acc aca gaa gct ctc atg       144
   Glu Lys Arg Glu Val Pro Gly Gly Ala Ala Thr Thr Glu Ala Leu Met
           35                  40                  45
   ccg gag cta tcc ccc cag ttt cgc ttt aac cgc tgt gcc att gac cac       192
   Pro Glu Leu Ser Pro Gln Phe Arg Phe Asn Arg Cys Ala Ile Asp His
       50                  55                  60
   gaa ttt atc ttt ctg ggg ccg gtg ttg cag gag cta aat tta gcc cag      240
   Glu Phe Ile Phe Leu Gly Pro Val Leu Gln Glu Leu Asn Leu Ala Gln
   65                  70                  75                  80
   tat ggt ttg gaa tat tta ttt tgt gac ccc agt gtt ttt tgt ccg ggg      288
   Tyr Gly Leu Glu Tyr Leu Phe Cys Asp Pro Ser Val Phe Cys Pro Gly
                   85                  90                  95
   ctg gat ggc caa gct ttt atg agc tac cgt tcc cta gaa aaa acc tgt      336
   Leu Asp Gly Gln Ala Phe Met Ser Tyr Arg Ser Leu Glu Lys Thr Cys
               100                 105                 110
   gcc cac att gcc acc tat agc ccc cga gat gcg gaa aaa tat cgg caa      384
   Ala His Ile Ala Thr Tyr Ser Pro Arg Asp Ala Glu Lys Tyr Arg Gln
           115                 120                 125
   ttt gtc aat tat tgg acg gat ttg ctc aac gct gtc cag cct gct ttt      432
   Phe Val Asn Tyr Trp Thr Asp Leu Leu Asn Ala Val Gln Pro Ala Phe
       130                 135                 140
   aat gct ccg ccc cag gct tta cta gat tta gcc ctg aac tat ggt tgg      480
   Asn Ala Pro Pro Gln Ala Leu Leu Asp Leu Ala Leu Asn Tyr Gly Trp
   145                 150                 155                 160
   gaa aac tta aaa tcc gtg ctg gcg atc gcc ggg tcg aaa acc aag gcg      528
   Glu Asn Leu Lys Ser Val Leu Ala Ile Ala Gly Ser Lys Thr Lys Ala
                   165                 170                 175
   ttg gat ttt atc cgc act atg atc ggc tcc ccg gaa gat gtg ctc aat      576
   Leu Asp Phe Ile Arg Thr Met Ile Gly Ser Pro Glu Asp Val Leu Asn
               180                 185                 190
   gaa tgg ttc gac agc gaa cgg gtt aaa gct cct tta gct aga cta tgt      624
   Glu Trp Phe Asp Ser Glu Arg Val Lys Ala Pro Leu Ala Arg Leu Cys
           195                 200                 205
   tcg gaa att ggc gct ccc cca tcc caa aag ggt agt agc tcc ggc atg      672
   Ser Glu Ile Gly Ala Pro Pro Ser Gln Lys Gly Ser Ser Ser Gly Met
       210                 215                 220
   atg atg gtg gcc atg cgg cat ttg gag gga att gcc aga cca aaa gga      720
   Met Met Val Ala Met Arg His Leu Glu Gly Ile Ala Arg Pro Lys Gly
   225                 230                 235                 240
   ggc act gga gcc ctc aca gaa gcc ttg gtg aag tta gtg caa gcc caa      768
   Gly Thr Gly Ala Leu Thr Glu Ala Leu Val Lys Leu Val Gln Ala Gln
                   245                 250                 255
   ggg gga aaa atc ctc act gac caa acc gtc aaa cgg gta ttg gtg gaa      816
   Gly Gly Lys Ile Leu Thr Asp Gln Thr Val Lys Arg Val Leu Val Glu
               260                 265                 270
   aac aac cag gcg atc ggg gtg gag gta gct aac gga gaa cag tac cgg      864
   Asn Asn Gln Ala Ile Gly Val Glu Val Ala Asn Gly Glu Gln Tyr Arg
               275                 280                 285
   gcc aaa aaa ggc gtg att tct aac atc gat gcc cgc cgt tta ttt ttg      912
   Ala Lys Lys Gly Val Ile Ser Asn Ile Asp Ala Arg Arg Leu Phe Leu
           290                 295                 300
   caa ttg gtg gaa ccg ggg gcc cta gcc aag gtg aat caa aac cta ggg      960
   Gln Leu Val Glu Pro Gly Ala Leu Ala Lys Val Asn Gln Asn Leu Gly
   305                 310                 315                 320
   gaa cga ctg gaa cgg cgc act gtg aac aat aac gaa gcc att tta aaa     1008
   Glu Arg Leu Glu Arg Arg Thr Val Asn Asn Asn Glu Ala Ile Leu Lys
                   325                 330                 335
   atc gat tgt gcc ctc tcc ggt tta ccc cac ttc act gcc atg gcc ggg     1056
   Ile Asp Cys Ala Leu Ser Gly Leu Pro His Phe Thr Ala Met Ala Gly
               340                 345                 350
   ccg gag gat cta acg gga act att ttg att gcc gac tcg gta cgc cat     1104
   Pro Glu Asp Leu Thr Gly Thr Ile Leu Ile Ala Asp Ser Val Arg His
           355                 360                 365
   gtc gag gaa gcc cac gcc ctc att gcc ttg ggg caa att ccc gat gct     1152
   Val Glu Glu Ala His Ala Leu Ile Ala Leu Gly Gln Ile Pro Asp Ala
       370                 375                 380
   aat ccg tct tta tat ttg gat att ccc act gta ttg gac ccc acc atg     1200
   Asn Pro Ser Leu Tyr Leu Asp Ile Pro Thr Val Leu Asp Pro Thr Met
   385                 390                 395                 400
   gcc ccc cct ggg cag cac acc ctc tgg atc gaa ttt ttt gcc ccc tac     1248
   Ala Pro Pro Gly Gln His Thr Leu Trp Ile Glu Phe Phe Ala Pro Tyr
                   405                 410                 415
   cgc atc gcc ggg ttg gaa ggg aca ggg tta atg ggc aca ggt tgg acc     1296
   Arg Ile Ala Gly Leu Glu Gly Thr Gly Leu Met Gly Thr Gly Trp Thr
               420                 425                 430
   gat gag tta aag gaa aaa gtg gcg gat cgg gtg att gat aaa tta acg     1344
   Asp Glu Leu Lys Glu Lys Val Ala Asp Arg Val Ile Asp Lys Leu Thr
           435                 440                 445
   gac tat gcc cct aac cta aaa tct ctg atc att ggt cgc cga gtg gaa     1392
   Asp Tyr Ala Pro Asn Leu Lys Ser Leu Ile Ile Gly Arg Arg Val Glu
       450                 455                 460
   agt ccc gcc gaa ctg gcc caa cgg ctg gga agt tac aac ggc aat gtc     1440
   Ser Pro Ala Glu Leu Ala Gln Arg Leu Gly Ser Tyr Asn Gly Asn Val
   465                 470                 475                 480
   tat cat ctg gat atg agt ttg gac caa atg atg ttc ctc cgg cct cta     1488
   Tyr His Leu Asp Met Ser Leu Asp Gln Met Met Phe Leu Arg Pro Leu
                   485                 490                 495
   ccg gaa att gcc aac tac caa acc ccc atc aaa aat ctt tac tta aca      1536
   Pro Glu Ile Ala Asn Tyr Gln Thr Pro Ile Lys Asn Leu Tyr Leu Thr
               500                 505                 510
   ggg gcg ggt acc cat ccc ggt ggc tcc ata tca ggt atg ccc ggt aga      1584
   Gly Ala Gly Thr His Pro Gly Gly Ser Ile Ser Gly Met Pro Gly Arg
           515                 520                 525
   aat tgc gct cgg gtc ttt tta aaa caa caa cgt cgt ttt tgg taa          1629
   Asn Cys Ala Arg Val Phe Leu Lys Gln Gln Arg Arg Phe Trp
       530                 535                 540
<210> 12
   <211> 542
   <212> PRT
   <213> Synechococcus sp.
<400> 12
   Met Ile Thr Thr Asp Val Val Ile Ile Gly Ala Gly His Asn Gly Leu
   1               5                   10                  15
   Val Cys Ala Ala Tyr Leu Leu Gln Arg Gly Leu Gly Val Thr Leu Leu
               20                  25                  30
   Glu Lys Arg Glu Val Pro Gly Gly Ala Ala Thr Thr Glu Ala Leu Met
           35                  40                  45
   Pro Glu Leu Ser Pro Gln Phe Arg Phe Asn Arg Cys Ala Ile Asp His
       50                  55                  60
   Glu Phe Ile Phe Leu Gly Pro Val Leu Gln Glu Leu Asn Leu Ala Gln
   65                  70                  75                  80
   Tyr Gly Leu Glu Tyr Leu Phe Cys Asp Pro Ser Val Phe Cys Pro Gly
                   85                  90                  95
   Leu Asp Gly Gln Ala Phe Met Ser Tyr Arg Ser Leu Glu Lys Thr Cys
               100                 105                 110
   Ala His Ile Ala Thr Tyr Ser Pro Arg Asp Ala Glu Lys Tyr Arg Gln
   115 120 125
   Phe Val Asn Tyr Trp Thr Asp Leu Leu Asn Ala Val Gln Pro Ala Phe
   130 135 140
   Asn Ala Pro Pro Gln Ala Leu Leu Asp Leu Ala Leu Asn Tyr Gly Trp
   145 150 155 160
   Glu Asn Leu Lys Ser Val Leu Ala Ile Ala Gly Ser Lys Thr Lys Ala
   165 170 175
   Leu Asp Phe Ile Arg Thr Met Ile Gly Ser Pro Glu Asp Val Leu Asn
   180 185 190
   Glu Trp Phe Asp Ser Glu Arg Val Lys Ala Pro Leu Ala Arg Leu Cys
   195 200 205
   Ser Glu Ile Gly Ala Pro Pro Ser Gln Lys Gly Ser Ser Ser Gly Met
   210 215 220
   Met Met Val Ala Met Arg His Leu Glu Gly Ile Ala Arg Pro Lys Gly
   225 230 235 240
   Gly Thr Gly Ala Leu Thr Glu Ala Leu Val Lys Leu Val Gln Ala Gln
   245 250 255
   Gly Gly Lys Ile Leu Thr Asp Gln Thr Val Lys Arg Val Leu Val Glu
   260 265 270
   Asn Asn Gln Ala Ile Gly Val Glu Val Ala Asn Gly Glu Gln Tyr Arg
   275 280 285
   Ala Lys Lys Gly Val Ile Ser Asn Ile Asp Ala Arg Arg Leu Phe Leu
   290 295 300
   Gln Leu Val Glu Pro Gly Ala Leu Ala Lys Val Asn Gln Asn Leu Gly
   305 310 315 320
   Glu Arg Leu Glu Arg Arg Thr Val Asn Asn Asn Glu Ala Ile Leu Lys
                   325                 330                 335
   Ile Asp Cys Ala Leu Ser Gly Leu Pro His Phe Thr Ala Met Ala Gly
               340                 345                 350
   Pro Glu Asp Leu Thr Gly Thr Ile Leu Ile Ala Asp Ser Val Arg His
           355                 360                 365
   Val Glu Glu Ala His Ala Leu Ile Ala Leu Gly Gln Ile Pro Asp Ala
       370                 375                 380
   Asn Pro Ser Leu Tyr Leu Asp Ile Pro Thr Val Leu Asp Pro Thr Met
   385                 390                 395                 400
   Ala Pro Pro Gly Gln His Thr Leu Trp Ile Glu Phe Phe Ala Pro Tyr
                   405                 410                 415
   Arg Ile Ala Gly Leu Glu Gly Thr Gly Leu Met Gly Thr Gly Trp Thr
               420                 425                 430
   Asp Glu Leu Lys Glu Lys Val Ala Asp Arg Val Ile Asp Lys Leu Thr
           435                 440                 445
   Asp Tyr Ala Pro Asn Leu Lys Ser Leu Ile Ile Gly Arg Arg Val Glu
       450                 455                 460
   Ser Pro Ala Glu Leu Ala Gln Arg Leu Gly Ser Tyr Asn Gly Asn Val
   465                 470                 475                 480
   Tyr His Leu Asp Met Ser Leu Asp Gln Met Met Phe Leu Arg Pro Leu
                   485                 490                 495
   Pro Glu Ile Ala Asn Tyr Gln Thr Pro Ile Lys Asn Leu Tyr Leu Thr
               500                 505                 510
   Gly Ala Gly Thr His Pro Gly Gly Ser Ile Ser Gly Met Pro Gly Arg
           515                 520                 525
   Asn Cys Ala Arg Val Phe Leu Lys Gln Gln Arg Arg Phe Trp
       530                 535                 540
<210> 13
   <211> 776
   <212> DNA
   <213> Bradyrhizobium sp.
<220>
   <221> CDS
   <222> (1)..(774)
   <223>
<400> 13
   atg cat gca gca acc gcc aag gct act gag ttc ggg gcc tct cgg cgc       48
   Met His Ala Ala Thr Ala Lys Ala Thr Glu Phe Gly Ala Ser Arg Arg
   1               5                   10                  15
   gac gat gcg agg cag cgc cgc gtc ggt ctc acg ctg gcc gcg gtc atc       96
   Asp Asp Ala Arg Gln Arg Arg Val Gly Leu Thr Leu Ala Ala Val Ile
               20                  25                  30
   atc gcc gcc tgg ctg gtg ctg cat gtc ggt ctg atg ttc ttc tgg ccg      144
   Ile Ala Ala Trp Leu Val Leu His Val Gly Leu Met Phe Phe Trp Pro
           35                  40                  45
   ctg acc ctt cac agc ctg ctg ccg gct ttg cct ctg gtg gtg ctg cag      192
   Leu Thr Leu His Ser Leu Leu Pro Ala Leu Pro Leu Val Val Leu Gln
       50                  55                  60
   acc tgg ctc tat gta ggc ctg ttc atc atc gcg cat gac tgc atg cac      240
   Thr Trp Leu Tyr Val Gly Leu Phe Ile Ile Ala His Asp Cys Met His
   65                  70                  75                  80
   ggc tcg ctg gtg ccg ttc aag ccg cag gtc aac cgc cgt atc gga cag      288
   Gly Ser Leu Val Pro Phe Lys Pro Gln Val Asn Arg Arg Ile Gly Gln
                   85                  90                  95
   ctc tgc ctg ttc ctc tat gcc ggg ttc tcc ttc gac gct ctc aat gtc      336
   Leu Cys Leu Phe Leu Tyr Ala Gly Phe Ser Phe Asp Ala Leu Asn Val
               100                 105                 110
   gag cac cac aag cat cac cgc cat ccc ggc acg gcc gag gat ccc gat      384
   Glu His His Lys His His Arg His Pro Gly Thr Ala Glu Asp Pro Asp
           115                 120                 125
   ttc gac gag gtg ccg ccg cac ggc ttc tgg cac tgg ttc gcc agc ttt      432
   Phe Asp Glu Val Pro Pro His Gly Phe Trp His Trp Phe Ala Ser Phe
       130                 135                 140
   ttc ctg cac tat ttc ggc tgg aag cag gtc gcg atc atc gca gcc gtc      480
   Phe Leu His Tyr Phe Gly Trp Lys Gln Val Ala Ile Ile Ala Ala Val
   145                 150                 155                 160
   tcg ctg gtt tat cag ctc gtc ttc gcc gtt ccc ttg cag aac atc ctg      528
   Ser Leu Val Tyr Gln Leu Val Phe Ala Val Pro Leu Gln Asn Ile Leu
                   165                 170                 175
   ctg ttc tgg gcg ctg ccc ggg ctg ctg tcg gcg ctg cag ctg ttc acc      576
   Leu Phe Trp Ala Leu Pro Gly Leu Leu Ser Ala Leu Gln Leu Phe Thr
               180                 185                 190
   ttc ggc acc tat ctg ccg cac aag ccg gcc acg cag ccc ttc gcc gat      624
   Phe Gly Thr Tyr Leu Pro His Lys Pro Ala Thr Gln Pro Phe Ala Asp
           195                 200                 205
   cgc cac aac gcg cgg acg agc gaa ttt ccc gcg tgg ctg tcg ctg ctg      672
   Arg His Asn Ala Arg Thr Ser Glu Phe Pro Ala Trp Leu Ser Leu Leu
       210                 215                 220
   acc tgc ttc cac ttc ggc ttt cat cac gag cat cat ctg cat ccc gat      720
   Thr Cys Phe His Phe Gly Phe His His Glu His His Leu His Pro Asp
   225                 230                 235                 240
   gcg ccg tgg tgg cgg ctg ccg gag atc aag cgg cgg gcc ctg gaa agg      768
   Ala Pro Trp Trp Arg Leu Pro Glu Ile Lys Arg Arg Ala Leu Glu Arg
                   245                 250                 255
   cgt gac ta                                                           776
   Arg Asp
<210> 14
   <211> 258
   <212> PRT
   <213> Bradyrhizobium sp.
<400> 14
   Met His Ala Ala Thr Ala Lys Ala Thr Glu Phe Gly Ala Ser Arg Arg
   1               5                   10                  15
   Asp Asp Ala Arg Gln Arg Arg Val Gly Leu Thr Leu Ala Ala Val Ile
               20                  25                  30
   Ile Ala Ala Trp Leu Val Leu His Val Gly Leu Met Phe Phe Trp Pro
           35                  40                  45
   Leu Thr Leu His Ser Leu Leu Pro Ala Leu Pro Leu Val Val Leu Gln
       50                  55                  60
   Thr Trp Leu Tyr Val Gly Leu Phe Ile Ile Ala His Asp Cys Met His
   65                  70                  75                  80
   Gly Ser Leu Val Pro Phe Lys Pro Gln Val Asn Arg Arg Ile Gly Gln
                   85                  90                  95
   Leu Cys Leu Phe Leu Tyr Ala Gly Phe Ser Phe Asp Ala Leu Asn Val
               100                 105                 110
   Glu His His Lys His His Arg His Pro Gly Thr Ala Glu Asp Pro Asp
           115                 120                 125
   Phe Asp Glu Val Pro Pro His Gly Phe Trp His Trp Phe Ala Ser Phe
       130                 135                 140
   Phe Leu His Tyr Phe Gly Trp Lys Gln Val Ala Ile Ile Ala Ala Val
   145                 150                 155                 160
   Ser Leu Val Tyr Gln Leu Val Phe Ala Val Pro Leu Gln Asn Ile Leu
                   165                 170                 175
   Leu Phe Trp Ala Leu Pro Gly Leu Leu Ser Ala Leu Gln Leu Phe Thr
               180                 185                 190
   Phe Gly Thr Tyr Leu Pro His Lys Pro Ala Thr Gln Pro Phe Ala Asp
           195                 200                 205
   Arg His Asn Ala Arg Thr Ser Glu Phe Pro Ala Trp Leu Ser Leu Leu
       210                 215                 220
   Thr Cys Phe His Phe Gly Phe His His Glu His His Leu His Pro Asp
   225                 230                 235                 240
   Ala Pro Trp Trp Arg Leu Pro Glu Ile Lys Arg Arg Ala Leu Glu Arg
                   245                 250                 255
   Arg Asp
<210> 15
   <211> 777
   <212> DNA
   <213> Nostoc sp.
<220>
   <221> CDS
   <222> (1)..(777)
   <223>
<400> 15
   atg gtt cag tgt caa cca tca tct ctg cat tca gaa aaa ctg gtg tta       48
   Met Val Gln Cys Gln Pro Ser Ser Leu His Ser Glu Lys Leu Val Leu
   1               5                   10                  15
   ttg tca tcg aca atc aga gat gat aaa aat att aat aag ggt ata ttt       96
   Leu Ser Ser Thr Ile Arg Asp Asp Lys Asn Ile Asn Lys Gly Ile Phe
               20                  25                  30
   att gcc tgc ttt atc tta ttt tta tgg gca att agt tta atc tta tta      144
   Ile Ala Cys Phe Ile Leu Phe Leu Trp Ala Ile Ser Leu Ile Leu Leu
           35                  40                  45
   ctc tca ata gat aca tcc ata att cat aag agc tta tta ggt ata gcc      192
   Leu Ser Ile Asp Thr Ser Ile Ile His Lys Ser Leu Leu Gly Ile Ala
       50                  55                  60
   atg ctt tgg cag acc ttc tta tat aca ggt tta ttt att act gct cat      240
   Met Leu Trp Gln Thr Phe Leu Tyr Thr Gly Leu Phe Ile Thr Ala His
   65                  70                  75                  80
   gat gcc atg cac ggc gta gtt tat ccc aaa aat ccc aga ata aat aat      288
   Asp Ala Met His Gly Val Val Tyr Pro Lys Asn Pro Arg Ile Asn Asn
                   85                  90                  95
   ttt ata ggt aag ctc act cta atc ttg tat gga cta ctc cct tat aaa      336
   Phe Ile Gly Lys Leu Thr Leu Ile Leu Tyr Gly Leu Leu Pro Tyr Lys
               100                 105                 110
   gat tta ttg aaa aaa cat tgg tta cac cac gga cat cct ggt act gat      384
   Asp Leu Leu Lys Lys His Trp Leu His His Gly His Pro Gly Thr Asp
           115                 120                 125
   tta gac cct gat tat tac aat ggt cat ccc caa aac ttc ttt ctt tgg      432
   Leu Asp Pro Asp Tyr Tyr Asn Gly His Pro Gln Asn Phe Phe Leu Trp
       130                 135                 140
   tat cta cat ttt atg aag tct tat tgg cga tgg acg caa att ttc gga      480
   Tyr Leu His Phe Met Lys Ser Tyr Trp Arg Trp Thr Gln Ile Phe Gly
   145                 150                 155                 160
   tta gtg atg att ttt cat gga ctt aaa aat ctg gtg cat ata cca gaa      528
   Leu Val Met Ile Phe His Gly Leu Lys Asn Leu Val His Ile Pro Glu
                   165                 170                 175
   aat aat tta att ata ttt tgg atg ata cct tct att tta agt tca gta      576
   Asn Asn Leu Ile Ile Phe Trp Met Ile Pro Ser Ile Leu Ser Ser Val
               180                 185                 190
   caa cta ttt tat ttt ggt aca ttt ttg cct cat aaa aag cta gaa ggt      624
   Gln Leu Phe Tyr Phe Gly Thr Phe Leu Pro His Lys Lys Leu Glu Gly
           195                 200                 205
   ggt tat act aac ccc cat tgt gcg cgc agt atc cca tta cct ctt ttt      672
   Gly Tyr Thr Asn Pro His Cys Ala Arg Ser Ile Pro Leu Pro Leu Phe
       210                 215                 220
   tgg tct ttt gtt act tgt tat cac ttc ggc tac cac aag gaa cat cac      720
   Trp Ser Phe Val Thr Cys Tyr His Phe Gly Tyr His Lys Glu His His
   225                 230                 235                 240
   gaa tac cct caa ctt cct tgg tgg aaa tta cct gaa gct cac aaa ata      768
   Glu Tyr Pro Gln Leu Pro Trp Trp Lys Leu Pro Glu Ala His Lys Ile
                   245                 250                 255
   tct tta taa                                                          777
   Ser Leu
<210> 16
   <211> 258
   <212> PRT
   <213> Nostoc sp.
<400> 16
   Met Val Gln Cys Gln Pro Ser Ser Leu His Ser Glu Lys Leu Val Leu
   1 5 10 15
   Leu Ser Ser Thr Ile Arg Asp Asp Lys Asn Ile Asn Lys Gly Ile Phe
   20 25 30
   Ile Ala Cys Phe Ile Leu Phe Leu Trp Ala Ile Ser Leu Ile Leu Leu
   35 40 45
   Leu Ser Ile Asp Thr Ser Ile Ile His Lys Ser Leu Leu Gly Ile Ala
   50 55 60
   Met Leu Trp Gln Thr Phe Leu Tyr Thr Gly Leu Phe Ile Thr Ala His
   65 70 75 80
   Asp Ala Met His Gly Val Val Tyr Pro Lys Asn Pro Arg Ile Asn Asn
   85 90 95
   Phe Ile Gly Lys Leu Thr Leu Ile Leu Tyr Gly Leu Leu Pro Tyr Lys
   100 105 110
   Asp Leu Leu Lys Lys His Trp Leu His His Gly His Pro Gly Thr Asp
   115 120 125
   Leu Asp Pro Asp Tyr Tyr Asn Gly His Pro Gln Asn Phe Phe Leu Trp
   130 135 140
   Tyr Leu His Phe Met Lys Ser Tyr Trp Arg Trp Thr Gln Ile Phe Gly
   145 150 155 160
   Leu Val Met Ile Phe His Gly Leu Lys Asn Leu Val His Ile Pro Glu
   165 170 175
   Asn Asn Leu Ile Ile Phe Trp Met Ile Pro Ser Ile Leu Ser Ser Val
   180 185 190
   Gln Leu Phe Tyr Phe Gly Thr Phe Leu Pro His Lys Lys Leu Glu Gly
   195 200 205
   Gly Tyr Thr Asn Pro His Cys Ala Arg Ser Ile Pro Leu Pro Leu Phe
   210 215 220
   Trp Ser Phe Val Thr Cys Tyr His Phe Gly Tyr His Lys Glu His His
   225 230 235 240
   Glu Tyr Pro Gln Leu Pro Trp Trp Lys Leu Pro Glu Ala His Lys Ile
   245 250 255
   Ser Leu
<210> 17
   <211> 1608
   <212> DNA
   <213> Haematococcus pluvialis
<220>
   <221> CDS
   <222> (3)..(971)
   <223>
<400> 17
   ct aca ttt cac aag ccc gtg agc ggt gca agc gct ctg ccc cac atc 47
   Thr Phe His Lys Pro Val Ser Gly Ala Ser Ala Leu Pro His Ile
   1 5 10 15
   ggc cca cct cct cat ctc cat cgg tca ttt gct gct acc acg atg ctg 95
   Gly Pro Pro Pro His Leu His Arg Ser Phe Ala Ala Thr Thr Met Leu
   20 25 30
   tcg aag ctg cag tca atc agc gtc aag gcc cgc cgc gtt gaa cta gcc 143
   Ser Lys Leu Gln Ser Ile Ser Val Lys Ala Arg Arg Val Glu Leu Ala
   35 40 45
   cgc gac atc acg cgg ccc aaa gtc tgc ctg cat gct cag cgg tgc tcg 191
   Arg Asp Ile Thr Arg Pro Lys Val Cys Leu His Ala Gln Arg Cys Ser
   50 55 60
   tta gtt cgg ctg cga gtg gca gca cca cag aca gag gag gcg ctg gga      239
   Leu Val Arg Leu Arg Val Ala Ala Pro Gln Thr Glu Glu Ala Leu Gly
       65                  70                  75
   acc gtg cag gct gcc ggc gcg ggc gat gag cac agc gcc gat gta gca      287
   Thr Val Gln Ala Ala Gly Ala Gly Asp Glu His Ser Ala Asp Val Ala
   80                  85                  90                  95
   ctc cag cag ctt gac cgg gct atc gca gag cgt cgt gcc cgg cgc aaa      335
   Leu Gln Gln Leu Asp Arg Ala Ile Ala Glu Arg Arg Ala Arg Arg Lys
                   100                 105                 110
   cgg gag cag ctg tca tac cag gct gcc gcc att gca gca tca att ggc      383
   Arg Glu Gln Leu Ser Tyr Gln Ala Ala Ala Ile Ala Ala Ser Ile Gly
               115                 120                 125
   gtg tca ggc att gcc atc ttc gcc acc tac ctg aga ttt gcc atg cac      431
   Val Ser Gly Ile Ala Ile Phe Ala Thr Tyr Leu Arg Phe Ala Met His
           130                 135                 140
   atg acc gtg ggc ggc gca gtg cca tgg ggt gaa gtg gct ggc act ctc      479
   Met Thr Val Gly Gly Ala Val Pro Trp Gly Glu Val Ala Gly Thr Leu
       145                 150                 155
   ctc ttg gtg gtt ggt ggc gcg ctc ggc atg gag atg tat gcc cgc tat      527
   Leu Leu Val Val Gly Gly Ala Leu Gly Met Glu Met Tyr Ala Arg Tyr
   160                 165                 170                 175
   gca cac aaa gcc atc tgg cat gag tcg cct ctg ggc tgg ctg ctg cac      575
   Ala His Lys Ala Ile Trp His Glu Ser Pro Leu Gly Trp Leu Leu His
                   180                 185                 190
   aag agc cac cac aca cct cgc act gga ccc ttt gaa gcc aac gac ttg      623
   Lys Ser His His Thr Pro Arg Thr Gly Pro Phe Glu Ala Asn Asp Leu
               195                 200                 205
   ttt gca atc atc aat gga ctg ccc gcc atg ctc ctg tgt acc ttt ggc      671
   Phe Ala Ile Ile Asn Gly Leu Pro Ala Met Leu Leu Cys Thr Phe Gly
           210                 215                 220
   ttc tgg ctg ccc aac gtc ctg ggg gcg gcc tgc ttt gga gcg ggg ctg      719
   Phe Trp Leu Pro Asn Val Leu Gly Ala Ala Cys Phe Gly Ala Gly Leu
       225                 230                 235
   ggc atc acg cta tac ggc atg gca tat atg ttt gta cac gat ggc ctg      767
   Gly Ile Thr Leu Tyr Gly Met Ala Tyr Met Phe Val His Asp Gly Leu
   240                 245                 250                 255
   gtg cac agg cgc ttt ccc acc ggg ccc atc gct ggc ctg ccc tac atg      815
   Val His Arg Arg Phe Pro Thr Gly Pro Ile Ala Gly Leu Pro Tyr Met
                   260                 265                 270
   aag cgc ctg aca gtg gcc cac cag cta cac cac agc ggc aag tac ggt      863
   Lys Arg Leu Thr Val Ala His Gln Leu His His Ser Gly Lys Tyr Gly
               275                 280                 285
   ggc gcg ccc tgg ggt atg ttc ttg ggt cca cag gag ctg cag cac att      911
   Gly Ala Pro Trp Gly Met Phe Leu Gly Pro Gln Glu Leu Gln His Ile
           290                 295                 300
   cca ggt gcg gcg gag gag gtg gag cga ctg gtc ctg gaa ctg gac tgg      959
   Pro Gly Ala Ala Glu Glu Val Glu Arg Leu Val Leu Glu Leu Asp Trp
       305                 310                 315
   tcc aag cgg tag ggtgcggaac caggcacgct ggtttcacac ctcatgcctg         1011
   Ser Lys Arg
   320
   tgataaggtg tggctagagc gatgcgtgtg agacgggtat gtcacggtcg actggtctga   1071
   tggccaatgg catcggccat gtctggtcat cacgggctgg ttgcctgggt gaaggtgatg   1131
   cacatcatca tgtgcggttg gaggggctgg cacagtgtgg gctgaactgg agcagttgtc   1191
   caggctggcg ttgaatcagt gagggtttgt gattggcggt tgtgaagcaa tgactccgcc   1251
   catattctat ttgtgggagc tgagatgatg gcatgcttgg gatgtgcatg gatcatggta   1311
   gtgcagcaaa ctatattcac ctagggctgt tggtaggatc aggtgaggcc ttgcacattg   1371
   catgatgtac tcgtcatggt gtgttggtga gaggatggat gtggatggat gtgtattctc   1431
   agacgtagac cttgactgga ggcttgatcg agagagtggg ccgtattctt tgagagggga   1491
   ggctcgtgcc agaaatggtg agtggatgac tgtgacgctg tacattgcag gcaggtgaga   1551
   tgcactgtct cgattgtaaa atacattcag atgcaaaaaa aaaaaaaaaa aaaaaaa      1608
<210> 18
   <211> 322
   <212> PRT
   <213> Haematococcus pluvialis
<400> 18
   Thr Phe His Lys Pro Val Ser Gly Ala Ser Ala Leu Pro His Ile Gly
   1               5                   10                  15
   Pro Pro Pro His Leu His Arg Ser Phe Ala Ala Thr Thr Met Leu Ser
               20                  25                  30
   Lys Leu Gln Ser Ile Ser Val Lys Ala Arg Arg Val Glu Leu Ala Arg
   35 40 45
   Asp Ile Thr Arg Pro Lys Val Cys Leu His Ala Gln Arg Cys Ser Leu
   50 55 60
   Val Arg Leu Arg Val Ala Ala Pro Gln Thr Glu Glu Ala Leu Gly Thr
   65 70 75 80
   Val Gln Ala Ala Gly Ala Gly Asp Glu His Ser Ala Asp Val Ala Leu
   85 90 95
   Gln Gln Leu Asp Arg Ala Ile Ala Glu Arg Arg Ala Arg Arg Lys Arg
   100 105 110
   Glu Gln Leu Ser Tyr Gln Ala Ala Ala Ile Ala Ala Ser Ile Gly Val
   115 120 125
   Ser Gly Ile Ala Ile Phe Ala Thr Tyr Leu Arg Phe Ala Met His Met
   130 135 140
   Thr Val Gly Gly Ala Val Pro Trp Gly Glu Val Ala Gly Thr Leu Leu
   145 150 155 160
   Leu Val Val Gly Gly Ala Leu Gly Met Glu Met Tyr Ala Arg Tyr Ala
   165 170 175
   His Lys Ala Ile Trp His Glu Ser Pro Leu Gly Trp Leu Leu His Lys
   180 185 190
   Ser His His Thr Pro Arg Thr Gly Pro Phe Glu Ala Asn Asp Leu Phe
   195 200 205
   Ala Ile Ile Asn Gly Leu Pro Ala Met Leu Leu Cys Thr Phe Gly Phe
   210 215 220
   Trp Leu Pro Asn Val Leu Gly Ala Ala Cys Phe Gly Ala Gly Leu Gly
   225 230 235 240
   Ile Thr Leu Tyr Gly Met Ala Tyr Met Phe Val His Asp Gly Leu Val
                   245                 250                 255
   His Arg Arg Phe Pro Thr Gly Pro Ile Ala Gly Leu Pro Tyr Met Lys
               260                 265                 270
   Arg Leu Thr Val Ala His Gln Leu His His Ser Gly Lys Tyr Gly Gly
           275                 280                 285
   Ala Pro Trp Gly Met Phe Leu Gly Pro Gln Glu Leu Gln His Ile Pro
       290                 295                 300
   Gly Ala Ala Glu Glu Val Glu Arg Leu Val Leu Glu Leu Asp Trp Ser
   305                 310                 315                 320
   Lys Arg
<210> 19
   <211> 1503
   <212> DNA
   <213> Tomate
<220>
   <221> CDS
   <222> (1)..(1503)
   <223>
<400> 19
   atg gat act ttg ttg aaa acc cca aat aac ctt gaa ttt ctg aac cca        48
   Met Asp Thr Leu Leu Lys Thr Pro Asn Asn Leu Glu Phe Leu Asn Pro
   1               5                   10                  15
   cat cat ggt ttt gct gtt aaa gct agt acc ttt aga tct gag aag cat        96
   His His Gly Phe Ala Val Lys Ala Ser Thr Phe Arg Ser Glu Lys His
               20                  25                  30
   cat aat ttt ggt tct agg aag ttt tgt gaa act ttg ggt aga agt gtt       144
   His Asn Phe Gly Ser Arg Lys Phe Cys Glu Thr Leu Gly Arg Ser Val
           35                  40                  45
   tgt gtt aag ggt agt agt agt gct ctt tta gag ctt gta cct gag acc      192
   Cys Val Lys Gly Ser Ser Ser Ala Leu Leu Glu Leu Val Pro Glu Thr
       50                  55                  60
   aaa aag gag aat ctt gat ttt gag ctt cct atg tat gac cct tca aaa      240
   Lys Lys Glu Asn Leu Asp Phe Glu Leu Pro Met Tyr Asp Pro Ser Lys
   65                  70                  75                  80
   ggg gtt gtt gtg gat ctt gct gtg gtt ggt ggt ggc cct gca gga ctt      288
   Gly Val Val Val Asp Leu Ala Val Val Gly Gly Gly Pro Ala Gly Leu
                   85                  90                  95
   gct gtt gca cag caa gtt tct gaa gca gga ctc tct gtt tgt tca att      336
   Ala Val Ala Gln Gln Val Ser Glu Ala Gly Leu Ser Val Cys Ser Ile
               100                 105                 110
   gat ccg aat cct aaa ttg ata tgg cct aat aac tat ggt gtt tgg gtg      384
   Asp Pro Asn Pro Lys Leu Ile Trp Pro Asn Asn Tyr Gly Val Trp Val
           115                 120                 125
   gat gaa ttt gag gct atg gac ttg tta gat tgt cta gat gct acc tgg      432
   Asp Glu Phe Glu Ala Met Asp Leu Leu Asp Cys Leu Asp Ala Thr Trp
       130                 135                 140
   tct ggt gca gca gtg tac att gat gat aat acg gct aaa gat ctt cat      480
   Ser Gly Ala Ala Val Tyr Ile Asp Asp Asn Thr Ala Lys Asp Leu His
   145                 150                 155                 160
   aga cct tat gga agg gtt aac cgg aaa cag ctg aaa tcg aaa atg atg      528
   Arg Pro Tyr Gly Arg Val Asn Arg Lys Gln Leu Lys Ser Lys Met Met
                   165                 170                 175
   cag aaa tgt ata atg aat ggt gtt aaa ttc cac caa gcc aaa gtt ata      576
   Gln Lys Cys Ile Met Asn Gly Val Lys Phe His Gln Ala Lys Val Ile
               180                 185                 190
   aag gtg att cat gag gaa tcg aaa tcc atg ttg ata tgc aat gat ggt      624
   Lys Val Ile His Glu Glu Ser Lys Ser Met Leu Ile Cys Asn Asp Gly
           195                 200                 205
   att act att cag gca acg gtg gtg ctc gat gca act ggc ttc tct aga      672
   Ile Thr Ile Gln Ala Thr Val Val Leu Asp Ala Thr Gly Phe Ser Arg
       210                 215                 220
   tct ctt gtt cag tat gat aag cct tat aac ccc ggg tat caa gtt gct      720
   Ser Leu Val Gln Tyr Asp Lys Pro Tyr Asn Pro Gly Tyr Gln Val Ala
   225                 230                 235                 240
   tat ggc att ttg gct gaa gtg gaa gag cac ccc ttt gat gta aac aag      768
   Tyr Gly Ile Leu Ala Glu Val Glu Glu His Pro Phe Asp Val Asn Lys
                   245                 250                 255
   atg gtt ttc atg gat tgg cga gat tct cat ttg aag aac aat act gat      816
   Met Val Phe Met Asp Trp Arg Asp Ser His Leu Lys Asn Asn Thr Asp
               260                 265                 270
   ctc aag gag aga aat agt aga ata cca act ttt ctt tat gca atg cca      864
   Leu Lys Glu Arg Asn Ser Arg Ile Pro Thr Phe Leu Tyr Ala Met Pro
           275                 280                 285
   ttt tca tcc aac agg ata ttt ctt gaa gaa aca tca ctc gta gct cgt      912
   Phe Ser Ser Asn Arg Ile Phe Leu Glu Glu Thr Ser Leu Val Ala Arg
       290                 295                 300
   cct ggc ttg cgt ata gat gat att caa gaa cga atg gtg gct cgt tta      960
   Pro Gly Leu Arg Ile Asp Asp Ile Gln Glu Arg Met Val Ala Arg Leu
   305                 310                 315                 320
   aac cat ttg ggg ata aaa gtg aag agc att gaa gaa gat gaa cat tgt     1008
   Asn His Leu Gly Ile Lys Val Lys Ser Ile Glu Glu Asp Glu His Cys
                   325                 330                 335
   cta ata cca atg ggt ggt cca ctt cca gta tta cct cag aga gtc gtt     1056
   Leu Ile Pro Met Gly Gly Pro Leu Pro Val Leu Pro Gln Arg Val Val
               340                 345                 350
   gga atc ggt ggt aca gct ggc atg gtt cat cca tcc acc ggt tat atg     1104
   Gly Ile Gly Gly Thr Ala Gly Met Val His Pro Ser Thr Gly Tyr Met
           355                 360                 365
   gtg gca agg aca cta gct gcg gct cct gtt gtt gcc aat gcc ata att     1152
   Val Ala Arg Thr Leu Ala Ala Ala Pro Val Val Ala Asn Ala Ile Ile
       370                 375                 380
   caa tac ctc ggt tct gaa aga agt cat tcg ggt aat gaa tta tcc aca     1200
   Gln Tyr Leu Gly Ser Glu Arg Ser His Ser Gly Asn Glu Leu Ser Thr
   385                 390                 395                 400
   gct gtt tgg aaa gat ttg tgg cct ata gag agg aga cgt caa aga gag     1248
   Ala Val Trp Lys Asp Leu Trp Pro Ile Glu Arg Arg Arg Gln Arg Glu
                   405                 410                 415
   ttc ttc tgc ttc ggt atg gat att ctt ctg aag ctt gat tta cct gct     1296
   Phe Phe Cys Phe Gly Met Asp Ile Leu Leu Lys Leu Asp Leu Pro Ala
               420                 425                 430
   aca aga agg ttc ttt gat gca ttc ttt gac tta gaa cct cgt tat tgg     1344
   Thr Arg Arg Phe Phe Asp Ala Phe Phe Asp Leu Glu Pro Arg Tyr Trp
           435                 440                 445
   cat ggc ttc tta tcg tct cga ttg ttt cta cct gaa ctc ata gtt ttt     1392
   His Gly Phe Leu Ser Ser Arg Leu Phe Leu Pro Glu Leu Ile Val Phe
       450                 455                 460
   ggg ctg tct cta ttc tct cat gct tca aat act tct aga ttt gag ata     1440
   Gly Leu Ser Leu Phe Ser His Ala Ser Asn Thr Ser Arg Phe Glu Ile
   465 470 475 480
   atg aca aag gga act gtt cca tta gta aat atg atc aac aat ttg tta 1488
   Met Thr Lys Gly Thr Val Pro Leu Val Asn Met Ile Asn Asn Leu Leu
   485 490 495
   cag gat aaa gaa tga 1503
   Gln Asp Lys Glu
   500
<210> 20
   <211> 500
   <212> PRT
   <213> Tomate
<400> 20
   Met Asp Thr Leu Leu Lys Thr Pro Asn Asn Leu Glu Phe Leu Asn Pro
   1 5 10 15
   His His Gly Phe Ala Val Lys Ala Ser Thr Phe Arg Ser Glu Lys His
   20 25 30
   His Asn Phe Gly Ser Arg Lys Phe Cys Glu Thr Leu Gly Arg Ser Val
   35 40 45
   Cys Val Lys Gly Ser Ser Ser Ala Leu Leu Glu Leu Val Pro Glu Thr
   50 55 60
   Lys Lys Glu Asn Leu Asp Phe Glu Leu Pro Met Tyr Asp Pro Ser Lys
   65 70 75 80
   Gly Val Val Val Asp Leu Ala Val Val Gly Gly Gly Pro Ala Gly Leu
   85 90 95
   Ala Val Ala Gln Gln Val Ser Glu Ala Gly Leu Ser Val Cys Ser Ile
   100 105 110
   Asp Pro Asn Pro Lys Leu Ile Trp Pro Asn Asn Tyr Gly Val Trp Val
   115 120 125
   Asp Glu Phe Glu Ala Met Asp Leu Leu Asp Cys Leu Asp Ala Thr Trp
   130 135 140
   Ser Gly Ala Ala Val Tyr Ile Asp Asp Asn Thr Ala Lys Asp Leu His
   145 150 155 160
   Arg Pro Tyr Gly Arg Val Asn Arg Lys Gln Leu Lys Ser Lys Met Met
   165 170 175
   Gln Lys Cys Ile Met Asn Gly Val Lys Phe His Gln Ala Lys Val Ile
   180 185 190
   Lys Val Ile His Glu Glu Ser Lys Ser Met Leu Ile Cys Asn Asp Gly
   195 200 205
   Ile Thr Ile Gln Ala Thr Val Val Leu Asp Ala Thr Gly Phe Ser Arg
   210 215 220
   Ser Leu Val Gln Tyr Asp Lys Pro Tyr Asn Pro Gly Tyr Gln Val Ala
   225 230 235 240
   Tyr Gly Ile Leu Ala Glu Val Glu Glu His Pro Phe Asp Val Asn Lys
   245 250 255
   Met Val Phe Met Asp Trp Arg Asp Ser His Leu Lys Asn Asn Thr Asp
   260 265 270
   Leu Lys Glu Arg Asn Ser Arg Ile Pro Thr Phe Leu Tyr Ala Met Pro
   275 280 285
   Phe Ser Ser Asn Arg Ile Phe Leu Glu Glu Thr Ser Leu Val Ala Arg
   290 295 300
   Pro Gly Leu Arg Ile Asp Asp Ile Gln Glu Arg Met Val Ala Arg Leu
   305 310 315 320
   Asn His Leu Gly Ile Lys Val Lys Ser Ile Glu Glu Asp Glu His Cys
   325 330 335
   Leu Ile Pro Met Gly Gly Pro Leu Pro Val Leu Pro Gln Arg Val Val
   340 345 350
   Gly Ile Gly Gly Thr Ala Gly Met Val His Pro Ser Thr Gly Tyr Met
   355 360 365
   Val Ala Arg Thr Leu Ala Ala Ala Pro Val Val Ala Asn Ala Ile Ile
   370 375 380
   Gln Tyr Leu Gly Ser Glu Arg Ser His Ser Gly Asn Glu Leu Ser Thr
   385 390 395 400
   Ala Val Trp Lys Asp Leu Trp Pro Ile Glu Arg Arg Arg Gln Arg Glu
   405 410 415
   Phe Phe Cys Phe Gly Met Asp Ile Leu Leu Lys Leu Asp Leu Pro Ala
   420 425 430
   Thr Arg Arg Phe Phe Asp Ala Phe Phe Asp Leu Glu Pro Arg Tyr Trp
   435 440 445
   His Gly Phe Leu Ser Ser Arg Leu Phe Leu Pro Glu Leu Ile Val Phe
   450 455 460
   Gly Leu Ser Leu Phe Ser His Ala Ser Asn Thr Ser Arg Phe Glu Ile
   465 470 475 480
   Met Thr Lys Gly Thr Val Pro Leu Val Asn Met Ile Asn Asn Leu Leu
   485 490 495
   Gln Asp Lys Glu
   500
<210> 21
   <211> 195
   <212> DNA
   <213> Kartoffel
<220>
   <221> Intron
   <222> (1)..(195)
   <223>
<400> 21
   tacgtaagtt tctgcttcta cctttgatat atatataata attatcatta attagtagta      60
   atataatatt tcaaatattt ttttcaaaat aaaagaatgt agtatatagc aattgctttt     120
   ctgtagttta taagtgtgta tattttaatt tataactttt ctaatatatg accaaaattt     180
   gttgatgtgc agctg                                                      195
<210> 22
   <211> 1155
   <212> DNA
   <213> Haematococcus pluvialis
<220>
   <221> CDS
   <222> (6)..(995)
   <223>
<400> 22 <210> 23
   <211> 329
   <212> PRT
   <213> Haematococcus pluvialis
<400> 23
<210> 24
   <211> 1111
   <212> DNA
   <213> Haematococcus pluvialis
<220>
   <221> CDS
   <222> (4)..(951)
   <223>
<400> 24
   tgc atg cta gag gca ctc aag gag aag gag aag gag gtt gca ggc agc        48
       Met Leu Glu Ala Leu Lys Glu Lys Glu Lys Glu Val Ala Gly Ser
       1               5                   10                  15
   tct gac gtg ttg cgt aca tgg gcg acc cag tac tcg ctt ccg tca gaa        96
   Ser Asp Val Leu Arg Thr Trp Ala Thr Gln Tyr Ser Leu Pro Ser Glu
                   20                  25                  30
   gag tca gac gcg gcc cgc ccg gga ctg aag aat gcc tac aag cca cca       144
   Glu Ser Asp Ala Ala Arg Pro Gly Leu Lys Asn Ala Tyr Lys Pro Pro
               35                  40                  45
   cct tcc gac aca aag ggc atc aca atg gcg cta gct gtc atc ggc tcc       192
   Pro Ser Asp Thr Lys Gly Ile Thr Met Ala Leu Ala Val Ile Gly Ser
           50                  55                  60
   tgg gcc gca gtg ttc ctc cac gcc att ttt caa atc aag ctt ccg acc       240
   Trp Ala Ala Val Phe Leu His Ala Ile Phe Gln Ile Lys Leu Pro Thr
       65                  70                  75
   tcc ttg gac cag ctg cac tgg ctg ccc gtg tca gat gcc aca gct cag       288
   Ser Leu Asp Gln Leu His Trp Leu Pro Val Ser Asp Ala Thr Ala Gln
   80                  85                  90                  95
   ctg gtt agc ggc agc agc agc ctg ctg cac atc gtc gta gta ttc ttt      336
   Leu Val Ser Gly Ser Ser Ser Leu Leu His Ile Val Val Val Phe Phe
                   100                 105                 110
   gtc ctg gag ttc ctg tac aca ggc ctt ttt atc acc acg cat gat gct      384
   Val Leu Glu Phe Leu Tyr Thr Gly Leu Phe Ile Thr Thr His Asp Ala
               115                 120                 125
   atg cat ggc acc atc gcc atg aga aac agg cag ctt aat gac ttc ttg      432
   Met His Gly Thr Ile Ala Met Arg Asn Arg Gln Leu Asn Asp Phe Leu
           130                 135                 140
   ggc aga gta tgc atc tcc ttg tac gcc tgg ttt gat tac aac atg ctg      480
   Gly Arg Val Cys Ile Ser Leu Tyr Ala Trp Phe Asp Tyr Asn Met Leu
       145                 150                 155
   cac cgc aag cat tgg gag cac cac aac cac act ggc gag gtg ggc aag      528
   His Arg Lys His Trp Glu His His Asn His Thr Gly Glu Val Gly Lys
   160                 165                 170                 175
   gac cct gac ttc cac agg gga aac cct ggc att gtg ccc tgg ttt gcc      576
   Asp Pro Asp Phe His Arg Gly Asn Pro Gly Ile Val Pro Trp Phe Ala
                   180                 185                 190
   agc ttc atg tcc agc tac atg tcg atg tgg cag ttt gcg cgc ctc gca      624
   Ser Phe Met Ser Ser Tyr Met Ser Met Trp Gln Phe Ala Arg Leu Ala
               195                 200                 205
   tgg tgg acg gtg gtc atg cag ctg ctg ggt gcg cca atg gcg aac ctg      672
   Trp Trp Thr Val Val Met Gln Leu Leu Gly Ala Pro Met Ala Asn Leu
           210                 215                 220
   ctg gtg ttc atg gcg gcc gcg ccc atc ctg tcc gcc ttc cgc ttg ttc      720
   Leu Val Phe Met Ala Ala Ala Pro Ile Leu Ser Ala Phe Arg Leu Phe
       225                 230                 235
   tac ttt ggc acg tac atg ccc cac aag cct gag cct ggc gcc gcg tca      768
   Tyr Phe Gly Thr Tyr Met Pro His Lys Pro Glu Pro Gly Ala Ala Ser
   240                 245                 250                 255
   ggc tct tca cca gcc gtc atg aac tgg tgg aag tcg cgc act agc cag      816
   Gly Ser Ser Pro Ala Val Met Asn Trp Trp Lys Ser Arg Thr Ser Gln
                   260                 265                 270
   gcg tcc gac ctg gtc agc ttt ctg acc tgc tac cac ttc gac ctg cac      864
   Ala Ser Asp Leu Val Ser Phe Leu Thr Cys Tyr His Phe Asp Leu His
               275                 280                 285
   tgg gag cac cac cgc tgg ccc ttc gcc ccc tgg tgg gag ctg ccc aac      912
   Trp Glu His His Arg Trp Pro Phe Ala Pro Trp Trp Glu Leu Pro Asn
           290                 295                 300
   tgc cgc cgc ctg tct ggc cga ggt ctg gtt cct gcc tag ctggacacac       961
   Cys Arg Arg Leu Ser Gly Arg Gly Leu Val Pro Ala
       305                 310                 315
   tgcagtgggc cctgctgcca gctgggcatg caggttgtgg caggactggg tgaggtgaaa   1021
   agctgcaggc gctgctgccg gacacgttgc atgggctacc ctgtgtagct gccgccacta   1081
   ggggaggggg tttgtagctg tcgagcttgc                                    1111
<210> 25
   <211> 315
   <212> PRT
   <213> Haematococcus pluvialis
<400> 25
   Met Leu Glu Ala Leu Lys Glu Lys Glu Lys Glu Val Ala Gly Ser Ser
   1               5                   10                  15
   Asp Val Leu Arg Thr Trp Ala Thr Gln Tyr Ser Leu Pro Ser Glu Glu
               20                  25                  30
   Ser Asp Ala Ala Arg Pro Gly Leu Lys Asn Ala Tyr Lys Pro Pro Pro
           35                  40                  45
   Ser Asp Thr Lys Gly Ile Thr Met Ala Leu Ala Val Ile Gly Ser Trp
       50                  55                  60
   Ala Ala Val Phe Leu His Ala Ile Phe Gln Ile Lys Leu Pro Thr Ser
   65                  70                  75                  80
   Leu Asp Gln Leu His Trp Leu Pro Val Ser Asp Ala Thr Ala Gln Leu
                   85                  90                  95
   Val Ser Gly Ser Ser Ser Leu Leu His Ile Val Val Val Phe Phe Val
               100                 105                 110
   Leu Glu Phe Leu Tyr Thr Gly Leu Phe Ile Thr Thr His Asp Ala Met
           115                 120                 125
   His Gly Thr Ile Ala Met Arg Asn Arg Gln Leu Asn Asp Phe Leu Gly
       130                 135                 140
   Arg Val Cys Ile Ser Leu Tyr Ala Trp Phe Asp Tyr Asn Met Leu His
   145                 150                 155                 160
   Arg Lys His Trp Glu His His Asn His Thr Gly Glu Val Gly Lys Asp
                   165                 170                 175
   Pro Asp Phe His Arg Gly Asn Pro Gly Ile Val Pro Trp Phe Ala Ser
               180                 185                 190
   Phe Met Ser Ser Tyr Met Ser Met Trp Gln Phe Ala Arg Leu Ala Trp
           195                 200                 205
   Trp Thr Val Val Met Gln Leu Leu Gly Ala Pro Met Ala Asn Leu Leu
       210                 215                 220
   Val Phe Met Ala Ala Ala Pro Ile Leu Ser Ala Phe Arg Leu Phe Tyr
   225                 230                 235                 240
   Phe Gly Thr Tyr Met Pro His Lys Pro Glu Pro Gly Ala Ala Ser Gly
                   245                 250                 255
   Ser Ser Pro Ala Val Met Asn Trp Trp Lys Ser Arg Thr Ser Gln Ala
               260                 265                 270
   Ser Asp Leu Val Ser Phe Leu Thr Cys Tyr His Phe Asp Leu His Trp
           275                 280                 285
   Glu His His Arg Trp Pro Phe Ala Pro Trp Trp Glu Leu Pro Asn Cys
       290                 295                 300
   Arg Arg Leu Ser Gly Arg Gly Leu Val Pro Ala
   305                 310                 315
<210> 26
   <211> 1031
   <212> DNA
   <213> Haematococcus pluvialis
<220>
   <221> CDS
   <222> (6)..(1031)
   <223>
<400> 26
   gaagc atg cag cta gca gcg aca gta atg ttg gag cag ctt acc gga agc      50
         Met Gln Leu Ala Ala Thr Val Met Leu Glu Gln Leu Thr Gly Ser
         1               5                   10                  15
   gct gag gca ctc aag gag aag gag aag gag gtt gca ggc agc tct gac        98
   Ala Glu Ala Leu Lys Glu Lys Glu Lys Glu Val Ala Gly Ser Ser Asp
                   20                  25                  30
   gtg ttg cgt aca tgg gcg acc cag tac tcg ctt ccg tca gag gag tca       146
   Val Leu Arg Thr Trp Ala Thr Gln Tyr Ser Leu Pro Ser Glu Glu Ser
               35                  40                  45
   gac gcg gcc cgc ccg gga ctg aag aat gcc tac aag cca cca cct tcc       194
   Asp Ala Ala Arg Pro Gly Leu Lys Asn Ala Tyr Lys Pro Pro Pro Ser
           50                  55                  60
   gac aca aag ggc atc aca atg gcg cta gct gtc atc ggc tcc tgg gct       242
   Asp Thr Lys Gly Ile Thr Met Ala Leu Ala Val Ile Gly Ser Trp Ala
       65                  70                  75
   gca gtg ttc ctc cac gcc att ttt caa atc aag ctt ccg acc tcc ttg       290
   Ala Val Phe Leu His Ala Ile Phe Gln Ile Lys Leu Pro Thr Ser Leu
   80                  85                  90                  95
   gac cag ctg cac tgg ctg ccc gtg tca gat gcc aca gct cag ctg gtt       338
   Asp Gln Leu His Trp Leu Pro Val Ser Asp Ala Thr Ala Gln Leu Val
                   100                 105                 110
   agc ggc agc agc agc ctg ctg cac atc gtc gta gta ttc ttt gtc ctg       386
   Ser Gly Ser Ser Ser Leu Leu His Ile Val Val Val Phe Phe Val Leu
               115                 120                 125
   gag ttc ctg tac aca ggc ctt ttt atc acc acg cat gat gct atg cat       434
   Glu Phe Leu Tyr Thr Gly Leu Phe Ile Thr Thr His Asp Ala Met His
           130                 135                 140
   ggc acc atc gcc atg aga aac agg cag ctt aat gac ttc ttg ggc aga       482
   Gly Thr Ile Ala Met Arg Asn Arg Gln Leu Asn Asp Phe Leu Gly Arg
       145                 150                 155
   gta tgc atc tcc ttg tac gcc tgg ttt gat tac aac atg ctg cac cgc       530
   Val Cys Ile Ser Leu Tyr Ala Trp Phe Asp Tyr Asn Met Leu His Arg
   160                 165                 170                 175
   aag cat tgg gag cac cac aac cac act ggc gag gtg ggc aag gac cct       578
   Lys His Trp Glu His His Asn His Thr Gly Glu Val Gly Lys Asp Pro
                   180                 185                 190
   gac ttc cac agg gga aac cct ggc att gtg ccc tgg ttt gcc agc ttc       626
   Asp Phe His Arg Gly Asn Pro Gly Ile Val Pro Trp Phe Ala Ser Phe
               195                 200                 205
   atg tcc agc tac atg tcg atg tgg cag ttt gcg cgc ctc gca tgg tgg       674
   Met Ser Ser Tyr Met Ser Met Trp Gln Phe Ala Arg Leu Ala Trp Trp
           210                 215                 220
   acg gtg gtc atg cag ctg ctg ggt gcg cca atg gcg aac ctg ctg gtg       722
   Thr Val Val Met Gln Leu Leu Gly Ala Pro Met Ala Asn Leu Leu Val
       225                 230                 235
   ttc atg gcg gcc gcg ccc atc ctg tcc gcc ttc cgc ttg ttc tac ttt       770
   Phe Met Ala Ala Ala Pro Ile Leu Ser Ala Phe Arg Leu Phe Tyr Phe
   240                 245                 250                 255
   ggc acg tac atg ccc cac aag cct gag cct ggc gcc gcg tca ggc tct       818
   Gly Thr Tyr Met Pro His Lys Pro Glu Pro Gly Ala Ala Ser Gly Ser
                   260                 265                 270
   tca cca gcc gtc atg aac tgg tgg aag tcg cgc act agc cag gcg tcc       866
   Ser Pro Ala Val Met Asn Trp Trp Lys Ser Arg Thr Ser Gln Ala Ser
               275                 280                 285
   gac ctg gtc agc ttt ctg acc tgc tac cac ttc gac ctg cac tgg gag       914
   Asp Leu Val Ser Phe Leu Thr Cys Tyr His Phe Asp Leu His Trp Glu
           290                 295                 300
   cac cac cgc tgg ccc ttt gcc ccc tgg tgg gag ctg ccc aac tgc cgc       962
   His His Arg Trp Pro Phe Ala Pro Trp Trp Glu Leu Pro Asn Cys Arg
       305                 310                 315
   cgc ctg tct ggc cga ggt ctg gtt cct gcc gag caa aaa ctc atc tca      1010
   Arg Leu Ser Gly Arg Gly Leu Val Pro Ala Glu Gln Lys Leu Ile Ser
   320                 325                 330                 335
   gaa gag gat ctg aat agc tag                                          1031
   Glu Glu Asp Leu Asn Ser
                   340
<210> 27
   <211> 341
   <212> PRT
   <213> Haematococcus pluvialis
<400> 27
   Met Gln Leu Ala Ala Thr Val Met Leu Glu Gln Leu Thr Gly Ser Ala
   1               5                   10                  15
   Glu Ala Leu Lys Glu Lys Glu Lys Glu Val Ala Gly Ser Ser Asp Val
               20                  25                  30
   Leu Arg Thr Trp Ala Thr Gln Tyr Ser Leu Pro Ser Glu Glu Ser Asp
           35                  40                  45
   Ala Ala Arg Pro Gly Leu Lys Asn Ala Tyr Lys Pro Pro Pro Ser Asp
       50                  55                  60
   Thr Lys Gly Ile Thr Met Ala Leu Ala Val Ile Gly Ser Trp Ala Ala
   65                  70                  75                  80
   Val Phe Leu His Ala Ile Phe Gln Ile Lys Leu Pro Thr Ser Leu Asp
                   85                  90                  95
   Gln Leu His Trp Leu Pro Val Ser Asp Ala Thr Ala Gln Leu Val Ser
               100                 105                 110
   Gly Ser Ser Ser Leu Leu His Ile Val Val Val Phe Phe Val Leu Glu
           115                 120                 125
   Phe Leu Tyr Thr Gly Leu Phe Ile Thr Thr His Asp Ala Met His Gly
       130                 135                 140
   Thr Ile Ala Met Arg Asn Arg Gln Leu Asn Asp Phe Leu Gly Arg Val
   145                 150                 155                 160
   Cys Ile Ser Leu Tyr Ala Trp Phe Asp Tyr Asn Met Leu His Arg Lys
                   165                 170                 175
   His Trp Glu His His Asn His Thr Gly Glu Val Gly Lys Asp Pro Asp
   180 185 190
   Phe His Arg Gly Asn Pro Gly Ile Val Pro Trp Phe Ala Ser Phe Met
   195 200 205
   Ser Ser Tyr Met Ser Met Trp Gln Phe Ala Arg Leu Ala Trp Trp Thr
   210 215 220
   Val Val Met Gln Leu Leu Gly Ala Pro Met Ala Asn Leu Leu Val Phe
   225 230 235 240
   Met Ala Ala Ala Pro Ile Leu Ser Ala Phe Arg Leu Phe Tyr Phe Gly
   245 250 255
   Thr Tyr Met Pro His Lys Pro Glu Pro Gly Ala Ala Ser Gly Ser Ser
   260 265 270
   Pro Ala Val Met Asn Trp Trp Lys Ser Arg Thr Ser Gln Ala Ser Asp
   275 280 285
   Leu Val Ser Phe Leu Thr Cys Tyr His Phe Asp Leu His Trp Glu His
   290 295 300
   His Arg Trp Pro Phe Ala Pro Trp Trp Glu Leu Pro Asn Cys Arg Arg
   305 310 315 320
   Leu Ser Gly Arg Gly Leu Val Pro Ala Glu Gln Lys Leu Ile Ser Glu
   325 330 335
   Glu Asp Leu Asn Ser
   340
<210> 28
   <211> 777
   <212> DNA
   <213> Arabidopsis thaliana
<220>
   <221> promoter
   <222> (1)..(777)
   <223>
<400> 28
   gagctcactc actgatttcc attgcttgaa aattgatgat gaactaagat caatccatgt      60
   tagtttcaaa acaacagtaa ctgtggccaa cttagttttg aaacaacact aactggtcga     120
   agcaaaaaga aaaaagagtt tcatcatata tctgatttga tggactgttt ggagttagga     180
   ccaaacatta tctacaaaca aagacttttc tcctaacttg tgattccttc ttaaacccta     240
   ggggtaatat tctattttcc aaggatcttt agttaaaggc aaatccggga aattattgta     300
   atcatttggg gaaacatata aaagatttga gttagatgga agtgacgatt aatccaaaca     360
   tatatatctc tttcttctta tttcccaaat taacagacaa aagtagaata ttggctttta     420
   acaccaatat aaaaacttgc ttcacaccta aacacttttg tttactttag ggtaagtgca     480
   aaaagccaac caaatccacc tgcactgatt tgacgtttac aaacgccgtt aagtcgatgt     540
   ccgttgattt aaacagtgtc ttgtaattaa aaaaatcagt ttacataaat ggaaaattta     600
   tcacttagtt ttcatcaact tctgaactta cctttcatgg attaggcaat actttccatt     660
   tttagtaact caagtggacc ctttacttct tcaactccat ctctctcttt ctatttcact     720
   tctttcttct cattatatct cttgtcctct ccaccaaatc tcttcaacaa aaagctt        777
<210> 29
   <211> 22
   <212> DNA
   <213> kuenstlich
<220>
   <221> primer_bind
   <222> (1)..(22)
   <223>
<400> 29
   gcaagctcga cagctacaaa cc 22
<210> 30
   <211> 24
   <212> DNA
   <213> kuenstlich
<220>
   <221> primer_bind
   <222> (1)..(24)
   <223>
<400> 30
   gaagcatgca gctagcagcg acag 24
<210> 31
   <211> 30
   <212> DNA
   <213> kuenstlich
<220>
   <221> primer_bind
   <222> (1)..(30)
   <223>
<400> 31
   tgcatgctag aggcactcaa ggagaaggag 30
<210> 32
   <211> 59
   <212> DNA
   <213> kuenstlich
<220>
   <221> primer_bind
   <222> (1)..(59)
   <223>
<400> 32
   ctagctattc agatcctctt ctgagatgag tttttgctcg gcaggaacca gacctcggc 59
<210> 33
   <211> 28
   <212> DNA
   <213> kuenstlich
<220>
   <221> primer_bind
   <222> (1)..(28)
   <223>
<400> 33
   gagctcactc actgatttcc attgcttg 28
<210> 34
   <211> 37
   <212> DNA
   <213> kuenstlich
<220>
   <221> primer_bind
   <222> (1)..(37)
   <223>
<400> 34
   cgccgttaag tcgatgtccg ttgatttaaa cagtgtc 37
<210> 35
   <211> 34
   <212> DNA
   <213> kuenstlich
<220>
   <221> primer_bind
   <222> (1)..(34)
   <223>
<400> 35
   atcaacggac atcgacttaa cggcgtttgt aaac 34
<210> 36
   <211> 25
   <212> DNA
   <213> kuenstlich
<220>
   <221> primer_bind
   <222> (1)..(25)
   <223>
<400> 36
   taagcttttt gttgaagaga tttgg 25
<210> 37
   <211> 212
   <212> DNA
   <213> Kuenstliche Sequenz
<220>
   <221> Intron
   <222> (1)..(212)
   <223>
<400> 37
   gtcgactacg taagtttctg cttctacctt tgatatatat ataataatta tcattaatta 60
   gtagtaatat aatatttcaa atattttttt caaaataaaa gaatgtagta tatagcaatt 120
   gcttttctgt agtttataag tgtgtatatt ttaatttata acttttctaa tatatgacca 180
   aaatttgttg atgtgcaggt atcaccggat cc 212
<210> 38
   <211> 1830
   <212> DNA
   <213> Tagetes erecta
<220> <221> CDS <222> (141)..(1691) <223>
<400> 38
   ggcacgaggc aaagcaaagg ttgtttgttg ttgttgttga gagacactcc aatccaaaca      60
   gatacaaggc gtgactggat atttctctct cgttcctaac aacagcaacg aagaagaaaa     120
   agaatcatta ctaacaatca atg agt atg aga gct gga cac atg acg gca aca     173
                         Met Ser Met Arg Ala Gly His Met Thr Ala Thr
                         1               5                   10
   atg gcg gct ttt aca tgc cct agg ttt atg act agc atc aga tac acg       221
   Met Ala Ala Phe Thr Cys Pro Arg Phe Met Thr Ser Ile Arg Tyr Thr
               15                  20                  25
   aag caa att aag tgc aac gct gct aaa agc cag cta gtc gtt aaa caa       269
   Lys Gln Ile Lys Cys Asn Ala Ala Lys Ser Gln Leu Val Val Lys Gln
           30                  35                  40
   gag att gag gag gaa gaa gat tat gtg aaa gcc ggt gga tcg gag ctg       317
   Glu Ile Glu Glu Glu Glu Asp Tyr Val Lys Ala Gly Gly Ser Glu Leu
       45                  50                  55
   ctt ttt gtt caa atg caa cag aat aag tcc atg gat gca cag tct agc       365
   Leu Phe Val Gln Met Gln Gln Asn Lys Ser Met Asp Ala Gln Ser Ser
   60                  65                  70                  75
   cta tcc caa aag ctc cca agg gta cca ata gga gga gga gga gac agt       413
   Leu Ser Gln Lys Leu Pro Arg Val Pro Ile Gly Gly Gly Gly Asp Ser
                   80                  85                  90
   aac tgt ata ctg gat ttg gtt gta att ggt tgt ggt cct gct ggc ctt       461
   Asn Cys Ile Leu Asp Leu Val Val Ile Gly Cys Gly Pro Ala Gly Leu
               95                  100                 105
   gct ctt gct gga gaa tca gcc aag cta ggc ttg aat gtc gca ctt atc       509
   Ala Leu Ala Gly Glu Ser Ala Lys Leu Gly Leu Asn Val Ala Leu Ile
           110                 115                 120
   ggc cct gat ctt cct ttt aca aat aac tat ggt gtt tgg gag gat gaa       557
   Gly Pro Asp Leu Pro Phe Thr Asn Asn Tyr Gly Val Trp Glu Asp Glu
       125                 130                 135
   ttt ata ggt ctt gga ctt gag ggc tgt att gaa cat gtt tgg cga gat       605
   Phe Ile Gly Leu Gly Leu Glu Gly Cys Ile Glu His Val Trp Arg Asp
   140                 145                 150                 155
   act gta gta tat ctt gat gac aac gat ccc att ctc ata ggt cgt gcc      653
   Thr Val Val Tyr Leu Asp Asp Asn Asp Pro Ile Leu Ile Gly Arg Ala
                   160                 165                 170
   tat gga cga gtt agt cgt gat tta ctt cac gag gag ttg ttg act agg      701
   Tyr Gly Arg Val Ser Arg Asp Leu Leu His Glu Glu Leu Leu Thr Arg
               175                 180                 185
   tgc atg gag tca ggc gtt tca tat ctg agc tcc aaa gtg gaa cgg att      749
   Cys Met Glu Ser Gly Val Ser Tyr Leu Ser Ser Lys Val Glu Arg Ile
           190                 195                 200
   act gaa gct cca aat ggc cta agt ctc ata gag tgt gaa ggc aat atc      797
   Thr Glu Ala Pro Asn Gly Leu Ser Leu Ile Glu Cys Glu Gly Asn Ile
       205                 210                 215
   aca att cca tgc agg ctt gct act gtc gct tct gga gca gct tct gga      845
   Thr Ile Pro Cys Arg Leu Ala Thr Val Ala Ser Gly Ala Ala Ser Gly
   220                 225                 230                 235
   aaa ctt ttg cag tat gaa ctt ggc ggt ccc cgt gtt tgc gtt caa aca      893
   Lys Leu Leu Gln Tyr Glu Leu Gly Gly Pro Arg Val Cys Val Gln Thr
                   240                 245                 250
   gct tat ggt ata gag gtt gag gtt gaa agc ata ccc tat gat cca agc      941
   Ala Tyr Gly Ile Glu Val Glu Val Glu Ser Ile Pro Tyr Asp Pro Ser
               255                 260                 265
   cta atg gtt ttc atg gat tat aga gac tac acc aaa cat aaa tct caa      989
   Leu Met Val Phe Met Asp Tyr Arg Asp Tyr Thr Lys His Lys Ser Gln
           270                 275                 280
   tca cta gaa gca caa tat cca aca ttt ttg tat gtc atg cca atg tct     1037
   Ser Leu Glu Ala Gln Tyr Pro Thr Phe Leu Tyr Val Met Pro Met Ser
       285                 290                 295
   cca act aaa gta ttc ttt gag gaa act tgt ttg gct tca aaa gag gcc     1085
   Pro Thr Lys Val Phe Phe Glu Glu Thr Cys Leu Ala Ser Lys Glu Ala
   300                 305                 310                 315
   atg cct ttt gag tta ttg aag aca aaa ctc atg tca aga tta aag act     1133
   Met Pro Phe Glu Leu Leu Lys Thr Lys Leu Met Ser Arg Leu Lys Thr
                   320                 325                 330
   atg ggg atc cga ata acc aaa act tat gaa gag gaa tgg tca tat att     1181
   Met Gly Ile Arg Ile Thr Lys Thr Tyr Glu Glu Glu Trp Ser Tyr Ile
               335                 340                 345
   cca gta ggt gga tcc tta cca aat acc gag caa aag aac ctt gca ttt     1229
   Pro Val Gly Gly Ser Leu Pro Asn Thr Glu Gln Lys Asn Leu Ala Phe
           350                 355                 360
   ggt gct gct gct agc atg gtg cat cca gcc aca gga tat tcg gtt gta     1277
   Gly Ala Ala Ala Ser Met Val His Pro Ala Thr Gly Tyr Ser Val Val
       365                 370                 375
   aga tca ctg tca gaa gct cct aat tat gca gca gta att gca aag att      1325
   Arg Ser Leu Ser Glu Ala Pro Asn Tyr Ala Ala Val Ile Ala Lys Ile
   380                 385                 390                 395
   tta ggg aaa gga aat tca aaa cag atg ctt gat cat gga aga tac aca      1373
   Leu Gly Lys Gly Asn Ser Lys Gln Met Leu Asp His Gly Arg Tyr Thr
                   400                 405                 410
   acc aac atc tca aag caa gct tgg gaa aca ctt tgg ccc ctt gaa agg      1421
   Thr Asn Ile Ser Lys Gln Ala Trp Glu Thr Leu Trp Pro Leu Glu Arg
               415                 420                 425
   aaa aga cag aga gca ttc ttt ctc ttt gga tta gca ctg att gtc cag      1469
   Lys Arg Gln Arg Ala Phe Phe Leu Phe Gly Leu Ala Leu Ile Val Gln
           430                 435                 440
   atg gat att gag ggg acc cgc aca ttc ttc cgg act ttc ttc cgc ttg      1517
   Met Asp Ile Glu Gly Thr Arg Thr Phe Phe Arg Thr Phe Phe Arg Leu
       445                 450                 455
   ccc aca tgg atg tgg tgg ggg ttt ctt gga tct tcg tta tca tca act      1565
   Pro Thr Trp Met Trp Trp Gly Phe Leu Gly Ser Ser Leu Ser Ser Thr
   460                 465                 470                 475
   gac ttg ata ata ttt gcg ttt tac atg ttt atc ata gca ccg cat agc      1613
   Asp Leu Ile Ile Phe Ala Phe Tyr Met Phe Ile Ile Ala Pro His Ser
                   480                 485                 490
   ctg aga atg ggt ctg gtt aga cat ttg ctt tct gac ccg aca gga gga      1661
   Leu Arg Met Gly Leu Val Arg His Leu Leu Ser Asp Pro Thr Gly Gly
               495                 500                 505
   aca atg tta aaa gcg tat ctc acg ata taa ataactctag tcgcgatcag        1711
   Thr Met Leu Lys Ala Tyr Leu Thr Ile
           510                 515
   tttagattat aggcacatct tgcatatata tatgtataaa ccttatgtgt gctgtatcct    1771
   tacatcaaca cagtcattaa ttgtatttct tggggtaatg ctgatgaagt attttctgg     1830
<210> 39
   <211> 516
   <212> PRT
   <213> Tagetes erecta <400> 39
<210> 40
   <211> 445
   <212> DNA
   <213> Tagetes erecta
<220>
   <221> Sense Fragment
   <222> (1)..(445)
   <223>
<400> 40
   aagcttgcac gaggcaaagc aaaggttgtt tgttgttgtt gttgagagac actccaatcc 60
   aaacagatac aaggcgtgac tggatatttc tctctcgttc ctaacaacag caacgaagaa 120
   gaaaaagaat cattactaac aatcaatgag tatgagagct ggacacatga cggcaacaat 180
   ggcggctttt acatgcccta ggtttatgac tagcatcaga tacacgaagc aaattaagtg 240
   caacgctgct aaaagccagc tagtcgttaa acaagagatt gaggaggaag aagattatgt 300
   gaaagccggt ggatcggagc tgctttttgt tcaaatgcaa cagaataagt ccatggatgc 360
   acagtctagc ctatcccaaa agctcccaag ggtaccaata ggaggaggag gagacagtaa 420
   ctgtatactg gatttggttg tcgac 445
<210> 41
   <211> 446
   <212> DNA
   <213> Tagetes erecta
<220>
   <221> Antisense Fragment
   <222> (1)..(446)
   <223>
<400> 41
   gaattcgcac gaggcaaagc aaaggttgtt tgttgttgtt gttgagagac actccaatcc 60
   aaacagatac aaggcgtgac tggatatttc tctctcgttc ctaacaacag caacgaagaa 120
   gaaaaagaat cattactaac aatcaatgag tatgagagct ggacacatga cggcaacaat 180
   ggcggctttt acatgcccta ggtttatgac tagcatcaga tacacgaagc aaattaagtg 240
   caacgctgct aaaagccagc tagtcgttaa acaagagatt gaggaggaag aagattatgt 300
   gaaagccggt ggatcggagc tgctttttgt tcaaatgcaa cagaataagt ccatggatgc 360
   acagtctagc ctatcccaaa agctcccaag ggtaccaata ggaggaggag gagacagtaa 420
   ctgtatactg gatttggttg gatcct 446
<210> 42
   <211> 393
   <212> DNA
   <213> Tagetes erecta
<220>
   <221> Sense Fragment
   <222> (1)..(393)
   <223>
<400> 42
   aagctttgga ttagcactga ttgtccagat ggatattgag gggacccgca cattcttccg 60
   gactttcttc cgcttgccca catggatgtg gtgggggttt cttggatctt cgttatcatc 120
   aactgacttg ataatatttg cgttttacat gtttatcata gcaccgcata gcctgagaat 180
   gggtctggtt agacatttgc tttctgaccc gacaggagga acaatgttaa aagcgtatct 240
   cacgatataa ataactctag tcgcgatcag tttagattat aggcacatct tgcatatata 300
   tatgtataaa ccttatgtgt gctgtatcct tacatcaaca cagtcattaa ttgtatttct 360
   tggggtaatg ctgatgaagt attttctgtc gac 393
<210> 43
   <211> 397
   <212> DNA
   <213> Tagetes erecta
<220>
   <221> Antisense Fragment
   <222> (1)..(397)
   <223>
<400> 43
   gaattctctt tggattagca ctgattgtcc agatggatat tgaggggacc cgcacattct 60
   tccggacttt cttccgcttg cccacatgga tgtggtgggg gtttcttgga tcttcgttat 120
   catcaactga cttgataata tttgcgtttt acatgtttat catagcaccg catagcctga 180
   gaatgggtct ggttagacat ttgctttctg acccgacagg aggaacaatg ttaaaagcgt 240
   atctcacgat ataaataact ctagtcgcga tcagtttaga ttataggcac atcttgcata 300
   tatatatgta taaaccttat gtgtgctgta tccttacatc aacacagtca ttaattgtat 360
   ttcttggggt aatgctgatg aagtattttc tggatcc 397
<210> 44
   <211> 1537
   <212> DNA
   <213> -
<220>
   <221> promoter
   <222> (1)..(1537)
   <223>
<400> 44
   gagctctaca aattagggtt actttattca ttttcatcca ttctctttat tgttaaattt     60
   tgtacattta ttcaataata ttatatgttt attacaaatt ctcactttct tattcatacc    120
   tattcactca agcctttacc atcttccttt tctatttcaa tactatttct acttcatttt    180
   tcacgttttt aacatctttc tttatttctt gtccacttcg tttagggatg cctaatgtcc    240
   caaatttcat ctctcgtagt aacacaaaac caatgtaatg ctacttctct ctacattttt    300
   aatacaaata aagtgaaaca aaatatctat aaataaacaa atatatatat tttgttagac    360
   gctgtctcaa cccatcaatt aaaaaatttt gttatatttc tactttacct actaaatttg    420
   tttctcatat ttacctttta acccccacaa aaaaaaatta taaaaaagaa agaaaaaagc    480
   taaaccctat ttaaatagct aactataaga tcttaaaatt atcctcatca gtgtatagtt    540
   taattggtta ttaacttata acattatata tctatgacat atactctctc ctagctattt    600
   ctcacatttt ttaacttaag aaaatagtca taacatagtc taaaattcaa acatccacat    660
   gctctaattt gattaacaaa aagttagaaa tatttattta aataaaaaag actaataaat    720
   atataaaatg aatgttcata cgcagaccca tttagagatg agtatgcttt cacatgctga    780
   gattattttc aaaactaagg ttgtagcaat attaaatcaa taaaattatt ataaataaca    840
   aaattaacct gctcgtgttt gctgtatatg ggaggctaca aaataaatta aactaaagat    900
   gattatgttt tagacatttt ttctatctgt attagtttat acatattaat tcaggagctg    960
   cacaacccaa ttctattttc gttccttggt ggctgggttt ctcacaaggt tcaatagtca   1020
   atattaggtt ttattggact tttaatagta tcaaacaaat ctatgtgtga acttaaaaat   1080
   tgtattaaat atttagggta acctgttgcc gtttttagaa taatgtttct tcttaataca   1140
   cgaaagcgta ttgtgtattc attcatttgg cgcctcacat gcttcggttg gctcgcttta   1200
   gtctctgcct tctttgtata ttgtactccc cctcttccta tgccacgtgt tctgagctta   1260
   acaagccacg ttgcgtgcca ttgccaaaca agtcatttta acttcacaag gtccgatttg   1320
   acctccaaaa caacgacaag tttccgaaca gtcgcgaaga tcaagggtat aatcgtcttt   1380
   ttgaattcta tttctcttta tttaatagtc cctctcgtgt gatagttttt aaaagatttt   1440
   taaaacgtag ctgctgttta agtaaatccc agtccttcag tttgtgcttt tgtgtgtttt   1500
   gtttctctga tttacggaat ttggaaataa taagctt                             1537
<210> 45
   <211> 734
   <212> DNA
   <213> kuenstliche Sequenz
<220>
   <221> variation
   <222> (1)..(739)
   <223>
<400> 45
   ctaacaatca atgagtagag agctggacac atgacggcaa caatggcggc ttttacatgc     60
   cctaggttta tgactagcat cagatacacg aagcaaatta agtgcaacgc tgctaaaagc 120
   cagctagtcg ttaaacaaga gattgaggag gaagaagatt atgtgaaagc cggtggatcg 180
   gagctgcttt ttgttcaaat gcaacagaat aagtccatgg atgcacagtc tagcctatcc 240
   caaaaggtca ctccagactt aattgcttat aaataaataa atatgttttt taggaataat 300
   gatatttaga tagattagct atcacctgtg ctgtggtgtg cagctcccaa gggtcttacc 360
   gatagtaaaa tcgttagtta tgattaatac ttgggaggtg ggggattata ggctttgttg 420
   tgagaatgtt gagaaagagg tttgacaaat cggtgtttga atgaggttaa atggagttta 480
   attaaaataa agagaagaga aagattaaga gggtgatggg gatattaaag acggscaata 540
   tagtgatgcc acgtagaaaa aggtaagtga aaacatacaa cgtggcttta aaagatggct 600
   tggctgctaa tcaactcaac tcaactcata tcctatccat tcaaattcaa ttcaattcta 660
   ttgaatgcaa agcaaagcaa aggttgtttg ttgttgttgt tgagagacac tccaatccaa 720
   acagatacaa ggcg 734
<210> 46
   <211> 280
   <212> DNA
   <213> kuenstliche Sequenz
<220>
   <221> variation
   <222> (1)..(280)
   <223>
<400> 46
   gtcgagtatg gagttcaatt aaaataaaga gaagaraaag attaagaggg tgatggggat 60
   attaaagacg gccaatrtag tgatgccacg taagaaaaag gtaagtgaaa acatacaacg 120
   tggctttaaa agatggcttg gctgctaatc aactcaactc aactcatatc ctatccattc 180
   aaattcaatt caattctatt gaatgcaaag caaagcaaag caaaggttgt ttgttgttgt 240
   tgttgagaga cactccaatc caaacagata caaggcgtga 280
<210> 47
   <211> 358
   <212> DNA
   <213> Tagetes erecta
<220>
   <221> Sense Promotor
   <222> (1)..(358)
   <223>
<400> 47
   aagcttaccg atagtaaaat cgttagttat gattaatact tgggaggtgg gggattatag      60
   gctttgttgt gagaatgttg agaaagaggt ttgacaaatc ggtgtttgaa tgaggttaaa     120
   tggagtttaa ttaaaataaa gagaagagaa agattaagag ggtgatgggg atattaaaga     180
   cggccaatat agtgatgcca cgtagaaaaa ggtaagtgaa aacatacaac gtggctttaa     240
   aagatggctt ggctgctaat caactcaact caactcatat cctatccatt caaattcaat     300
   tcaattctat tgaatgcaaa gcaaagcaaa gcaaaggttg tttgttgttg ttgtcgac       358
<210> 48
   <211> 361
   <212> DNA
   <213> Tagetes erecta
<220>
   <221> Antisense Promotor
   <222> (1)..(361)
   <223>
<400> 48
   ctcgagctta ccgatagtaa aatcgttagt tatgattaat acttgggagg tgggggatta      60
   taggctttgt tgtgagaatg ttgagaaaga ggtttgacaa atcggtgttt gaatgaggtt     120
   aaatggagtt taattaaaat aaagagaaga gaaagattaa gagggtgatg gggatattaa     180
   agacggccaa tatagtgatg ccacgtagaa aaaggtaagt gaaaacatac aacgtggctt     240
   taaaagatgg cttggctgct aatcaactca actcaactca tatcctatcc attcaaattc     300
   aattcaattc tattgaatgc aaagcaaagc aaagcaaagg ttgtttgttg ttgttggatc     360
   c                                                                     361
<210> 49
   <211> 28
   <212> DNA
   <213> kuenstliche Sequenz
<220>
   <221> Primer
   <222> (1)..(28)
   <223>
<400> 49
   gagctcactc actgatttcc attgcttg 28
<210> 50
   <211> 37
   <212> DNA
   <213> kuenstliche Sequenz
<220>
   <221> Primer
   <222> (1)..(37)
   <223>
<400> 50
   cgccgttaag tcgatgtccg ttgatttaaa cagtgtc 37
<210> 51
   <211> 34
   <212> DNA
   <213> kuenstliche Sequenz
<220>
   <221> Primer
   <222> (1)..(34)
   <223>
<400> 51
   atcaacggac atcgacttaa cggcgtttgt aaac 34
<210> 52
   <211> 25
   <212> DNA
   <213> kuenstliche Sequenz
<220>
   <221> Primer
   <222> (1)..(25)
   <223>
<400> 52
   taagcttttt gttgaagaga tttgg 25
<210> 53
   <211> 23
   <212> DNA
   <213> kuenstliche Sequenz
<220>
   <221> Primer
   <222> (1)..(23)
   <223>
<400> 53
   gaaaatactt catcagcatt acc 23
<210> 54
   <211> 28
   <212> DNA
   <213> kuenstliche Sequenz
<220>
   <221> Primer
   <222> (1)..(28)
   <223>
<400> 54
   gtcgactacg taagtttctg cttctacc 28
<210> 55
   <211> 26
   <212> DNA
   <213> kuenstliche Sequenz
<220>
   <221> Primer
   <222> (1)..(26)
   <223>
<400> 55
   ggatccggtg atacctgcac atcaac 26
<210> 56
   <211> 28
   <212> DNA
   <213> kuenstliche Sequenz
<220>
   <221> Primer
   <222> (1)..(28)
   <223>
<400> 56
   aagcttgcac gaggcaaagc aaaggttg 28
<210> 57
   <211> 29
   <212> DNA
   <213> kuenstliche Sequenz
<220>
   <221> Primer
   <222> (1)..(29)
   <223>
<400> 57
   gtcgacaacc aaatccagta tacagttac 29
<210> 58
   <211> 30
   <212> DNA
   <213> kuenstliche Sequenz
<220>
   <221> Primer
   <222> (1)..(30)
   <223>
<400> 58
   aggatccaac caaatccagt atacagttac 30
<210> 59
   <211> 28
   <212> DNA
   <213> kuenstliche Sequenz
<220>
   <221> Primer
   <222> (1)..(28)
   <223>
<400> 59
   gaattcgcac gaggcaaagc aaaggttg 28
<210> 60
   <211> 25
   <212> DNA
   <213> kuenstliche Sequenz
<220>
   <221> Primer
   <222> (1)..(25)
   <223>
<400> 60
   aagctttgga ttagcactga ttgtc 25
<210> 61
   <211> 29
   <212> DNA
   <213> kuenstliche Sequenz
<220>
   <221> Primer
   <222> (1)..(29)
   <223>
<400> 61
   gtcgacagaa aatacttcat cagcattac 29
<210> 62
   <211> 29
   <212> DNA
   <213> kuenstliche Sequenz
<220>
   <221> Primer
   <222> (1)..(29)
   <223>
<400> 62
   ggatccagaa aatacttcat cagcattac 29
<210> 63
   <211> 27
   <212> DNA
   <213> kuenstliche Sequenz
<220>
   <221> Primer
   <222> (1)..(27)
   <223>
<400> 63
   gaattctctt tggattagca ctgattg 27
<210> 64
   <211> 23
   <212> DNA
   <213> kuenstliche Sequenz
<220>
   <221> Primer
   <222> (1)..(23)
   <223>
<400> 64
   cgccttgtat ctgtttggat tgg 23
<210> 65
   <211> 24
   <212> DNA
   <213> kuenstliche Sequenz
<220>
   <221> Primer
   <222> (1)..(24)
   <223>
<400> 65
   ctaacaatca atgagtatga gagc 24
<210> 66
   <211> 26
   <212> DNA
   <213> kuenstliche Sequenz
<220>
   <221> Primer
   <222> (1)..(26)
   <223>
<400> 66
   agagcaaggc cagcaggacc acaacc 26
<210> 67
   <211> 26
   <212> DNA
   <213> kuenstliche Sequenz
<220>
   <221> Primer
   <222> (1)..(26)
   <223>
<400> 67
   ccttgggagc ttttgggata ggctag 26
<210> 68
   <211> 26
   <212> DNA
   <213> kuenstliche Sequenz
<220>
   <221> Primer
   <222> (1)..(26)
   <223>
<400> 68
   tcacgccttg tatctgtttg gattgg 26
<210> 69
   <211> 15
   <212> DNA
   <213> kuenstliche Sequenz
<220>
   <221> Primer
   <222> (1)..(15)
   <223>
<400> 69
   gtcgagtatg gagtt 15
<210> 70
   <211> 28
   <212> DNA
   <213> kuenstliche Sequenz
<220>
   <221> Primer
   <222> (1)..(28)
   <223>
<400> 70
   aagcttaccg atagtaaaat cgttagtt 28
<210> 71
   <211> 31
   <212> DNA
   <213> kuenstliche Sequenz
<220>
   <221> Primer
   <222> (1)..(31)
   <223>
<400> 71
   ctcgagctta ccgatagtaa aatcgttagt t 31
<210> 72
   <211> 28
   <212> DNA
   <213> kuenstliche Sequenz
<400> 72
   gtcgacaaca acaacaaaca acctttgc 28
<210> 73
   <211> 28
   <212> DNA
   <213> kuenstliche Sequenz
<220>
   <221> Primer
   <222> (1)..(28)
   <223>
<400> 73
   ggatccaaca acaacaaaca acctttgc 28
<210> 74
   <211> 28
   <212> DNA
   <213> kuenstliche Sequenz
<220>
   <221> Primer
   <222> (1)..(28)
   <223>
<400> 74
   gtcgactttt tgttgaagag atttggtg 28
<210> 75
   <211> 28
   <212> DNA
   <213> kuenstliche Sequenz
<220>
   <221> Primer
   <222> (1)..(28)
   <223>
<400> 75
   ctcgagactc actgatttcc attgcttg 28
<210> 76
   <211> 22
   <212> DNA
   <213> kuenstliche Sequenz
<220>
   <221> Primer
   <222> (1)..(22)
   <223>
<400> 76
   gagctctaca aattagggtt ac 22
<210> 77
   <211> 23
   <212> DNA
   <213> kuenstliche Sequenz
<220>
   <221> Primer
   <222> (1)..(23)
   <223>
<400> 77
   aagcttatta tttccaaatt ccg 23
<210> 78
   <211> 50
   <212> DNA
   <213> kuenstliche Sequenz
<220>
   <221> Primer
   <222> (1)..(50)
   <223>
<400> 78
   aagctttgca attcatacag aagtgagaaa aatgcagcta gcagcgacag 50
<210> 79
   <211> 1062
   <212> DNA
   <213> Haematococcus pluvialis
<220>
   <221> CDS
   <222> (32)..(1021)
   <223>
<400> 79
   aagctttgca attcatacag aagtgagaaa a atg cag cta gca gcg aca gta        52
                                      Met Gln Leu Ala Ala Thr Val
                                      1               5
   atg ttg gag cag ctt acc gga agc gct gag gca ctc aag gag aag gag       100
   Met Leu Glu Gln Leu Thr Gly Ser Ala Glu Ala Leu Lys Glu Lys Glu
           10                  15                  20
   aag gag gtt gca ggc agc tct gac gtg ttg cgt aca tgg gcg acc cag      148
   Lys Glu Val Ala Gly Ser Ser Asp Val Leu Arg Thr Trp Ala Thr Gln
       25                  30                  35
   tac tcg ctt ccg tca gag gag tca gac gcg gcc cgc ccg gga ctg aag      196
   Tyr Ser Leu Pro Ser Glu Glu Ser Asp Ala Ala Arg Pro Gly Leu Lys
   40                  45                  50                  55
   aat gcc tac aag cca cca cct tcc gac aca aag ggc atc aca atg gcg      244
   Asn Ala Tyr Lys Pro Pro Pro Ser Asp Thr Lys Gly Ile Thr Met Ala
                   60                  65                  70
   cta gct gtc atc ggc tcc tgg gcc gca gtg ttc ctc cac gcc att ttt      292
   Leu Ala Val Ile Gly Ser Trp Ala Ala Val Phe Leu His Ala Ile Phe
               75                  80                  85
   caa atc aag ctt ccg acc tcc ttg gac cag ctg cac tgg ctg ccc gtg      340
   Gln Ile Lys Leu Pro Thr Ser Leu Asp Gln Leu His Trp Leu Pro Val
           90                  95                  100
   tca gat gcc aca gct cag ctg gtt agc ggc agc agc agc ctg ctg cac      388
   Ser Asp Ala Thr Ala Gln Leu Val Ser Gly Ser Ser Ser Leu Leu His
       105                 110                 115
   atc gtc gta gta ttc ttt gtc ctg gag ttc ctg tac aca ggc ctt ttt      436
   Ile Val Val Val Phe Phe Val Leu Glu Phe Leu Tyr Thr Gly Leu Phe
   120                 125                 130                 135
   atc acc acg cat gat gct atg cat ggc acc atc gcc atg aga aac agg      484
   Ile Thr Thr His Asp Ala Met His Gly Thr Ile Ala Met Arg Asn Arg
                   140                 145                 150
   cag ctt aat gac ttc ttg ggc aga gta tgc atc tcc ttg tac gcc tgg      532
   Gln Leu Asn Asp Phe Leu Gly Arg Val Cys Ile Ser Leu Tyr Ala Trp
               155                 160                 165
   ttt gat tac aac atg ctg cac cgc aag cat tgg gag cac cac aac cac      580
   Phe Asp Tyr Asn Met Leu His Arg Lys His Trp Glu His His Asn His
           170                 175                 180
   act ggc gag gtg ggc aag gac cct gac ttc cac agg gga aac cct ggc      628
   Thr Gly Glu Val Gly Lys Asp Pro Asp Phe His Arg Gly Asn Pro Gly
       185                 190                 195
   att gtg ccc tgg ttt gcc agc ttc atg tcc agc tac atg tcg atg tgg      676
   Ile Val Pro Trp Phe Ala Ser Phe Met Ser Ser Tyr Met Ser Met Trp
   200                 205                 210                 215
   cag ttt gcg cgc ctc gca tgg tgg acg gtg gtc atg cag ctg ctg ggt      724
   Gln Phe Ala Arg Leu Ala Trp Trp Thr Val Val Met Gln Leu Leu Gly
                   220                 225                 230
   gcg cca atg gcg aac ctg ctg gtg ttc atg gcg gcc gcg ccc atc ctg        772
   Ala Pro Met Ala Asn Leu Leu Val Phe Met Ala Ala Ala Pro Ile Leu
               235                 240                 245
   tcc gcc ttc cgc ttg ttc tac ttt ggc acg tac atg ccc cac aag cct        820
   Ser Ala Phe Arg Leu Phe Tyr Phe Gly Thr Tyr Met Pro His Lys Pro
           250                 255                 260
   gag cct ggc gcc gcg tca ggc tct tca cca gcc gtc atg aac tgg tgg        868
   Glu Pro Gly Ala Ala Ser Gly Ser Ser Pro Ala Val Met Asn Trp Trp
       265                 270                 275
   aag tcg cgc act agc cag gcg tcc gac ctg gtc agc ttt ctg acc tgc        916
   Lys Ser Arg Thr Ser Gln Ala Ser Asp Leu Val Ser Phe Leu Thr Cys
   280                 285                 290                 295
   tac cac ttc gac ctg cac tgg gag cac cac cgc tgg ccc ttt gcc ccc        964
   Tyr His Phe Asp Leu His Trp Glu His His Arg Trp Pro Phe Ala Pro
                   300                 305                 310
   tgg tgg gag ctg ccc aac tgc cgc cgc ctg tct ggc cga ggt ctg gtt       1012
   Trp Trp Glu Leu Pro Asn Cys Arg Arg Leu Ser Gly Arg Gly Leu Val
               315                 320                 325
   cct gcc tag ctggacacac tgcagtgggc cctgctgcca gctgggcatg c             1062
   Pro Ala
<210> 80
   <211> 329
   <212> PRT
   <213> Haematococcus pluvialis
<400> 80
   Met Gln Leu Ala Ala Thr Val Met Leu Glu Gln Leu Thr Gly Ser Ala
   1               5                   10                  15
   Glu Ala Leu Lys Glu Lys Glu Lys Glu Val Ala Gly Ser Ser Asp Val
               20                  25                  30
   Leu Arg Thr Trp Ala Thr Gln Tyr Ser Leu Pro Ser Glu Glu Ser Asp
           35                  40                  45
   Ala Ala Arg Pro Gly Leu Lys Asn Ala Tyr Lys Pro Pro Pro Ser Asp
       50                  55                  60
   Thr Lys Gly Ile Thr Met Ala Leu Ala Val Ile Gly Ser Trp Ala Ala
   65                  70                  75                  80
   Val Phe Leu His Ala Ile Phe Gln Ile Lys Leu Pro Thr Ser Leu Asp
                   85                  90                  95
   Gln Leu His Trp Leu Pro Val Ser Asp Ala Thr Ala Gln Leu Val Ser
               100                 105                 110
   Gly Ser Ser Ser Leu Leu His Ile Val Val Val Phe Phe Val Leu Glu
           115                 120                 125
   Phe Leu Tyr Thr Gly Leu Phe Ile Thr Thr His Asp Ala Met His Gly
       130                 135                 140
   Thr Ile Ala Met Arg Asn Arg Gln Leu Asn Asp Phe Leu Gly Arg Val
   145                 150                 155                 160
   Cys Ile Ser Leu Tyr Ala Trp Phe Asp Tyr Asn Met Leu His Arg Lys
                   165                 170                 175
   His Trp Glu His His Asn His Thr Gly Glu Val Gly Lys Asp Pro Asp
               180                 185                 190
   Phe His Arg Gly Asn Pro Gly Ile Val Pro Trp Phe Ala Ser Phe Met
           195                 200                 205
   Ser Ser Tyr Met Ser Met Trp Gln Phe Ala Arg Leu Ala Trp Trp Thr
       210                 215                 220
   Val Val Met Gln Leu Leu Gly Ala Pro Met Ala Asn Leu Leu Val Phe
   225                 230                 235                 240
   Met Ala Ala Ala Pro Ile Leu Ser Ala Phe Arg Leu Phe Tyr Phe Gly
                   245                 250                 255
   Thr Tyr Met Pro His Lys Pro Glu Pro Gly Ala Ala Ser Gly Ser Ser
               260                 265                 270
   Pro Ala Val Met Asn Trp Trp Lys Ser Arg Thr Ser Gln Ala Ser Asp
   275 280 285
   Leu Val Ser Phe Leu Thr Cys Tyr His Phe Asp Leu His Trp Glu His
   290 295 300
   His Arg Trp Pro Phe Ala Pro Trp Trp Glu Leu Pro Asn Cys Arg Arg
   305 310 315 320
   Leu Ser Gly Arg Gly Leu Val Pro Ala
   325
<210> 81
   <211> 789
   <212> DNA
   <213> Nostoc punctiforme
<220>
   <221> CDS
   <222> (1)..(789)
   <223>
<400> 81
   ttg aat ttt tgt gat aaa cca gtt agc tat tat gtt gca ata gag caa 48
   Leu Asn Phe Cys Asp Lys Pro Val Ser Tyr Tyr Val Ala Ile Glu Gln
   1 5 10 15
   tta agt gct aaa gaa gat act gtt tgg ggg ctg gtg att gtc ata gta 96
   Leu Ser Ala Lys Glu Asp Thr Val Trp Gly Leu Val Ile Val Ile Val
   20 25 30
   att att agt ctt tgg gta gct agt ttg gct ttt tta cta gct att aat 144
   Ile Ile Ser Leu Trp Val Ala Ser Leu Ala Phe Leu Leu Ala Ile Asn
   35 40 45
   tat gcc aaa gtc cca att tgg ttg ata cct att gca ata gtt tgg caa 192
   Tyr Ala Lys Val Pro Ile Trp Leu Ile Pro Ile Ala Ile Val Trp Gln
   50 55 60
   atg ttc ctt tat aca ggg cta ttt att act gca cat gat gct atg cat      240
   Met Phe Leu Tyr Thr Gly Leu Phe Ile Thr Ala His Asp Ala Met His
   65                  70                  75                  80
   ggg tca gtt tat cgt aaa aat ccc aaa att aat aat ttt atc ggt tca      288
   Gly Ser Val Tyr Arg Lys Asn Pro Lys Ile Asn Asn Phe Ile Gly Ser
                   85                  90                  95
   cta gct gta gcg ctt tac gct gtg ttt cca tat caa cag atg tta aag      336
   Leu Ala Val Ala Leu Tyr Ala Val Phe Pro Tyr Gln Gln Met Leu Lys
               100                 105                 110
   aat cat tgc tta cat cat cgt cat cct gct agc gaa gtt gac cca gat      384
   Asn His Cys Leu His His Arg His Pro Ala Ser Glu Val Asp Pro Asp
           115                 120                 125
   ttt cat gat ggt aag aga aca aac gct att ttc tgg tat ctc cat ttc      432
   Phe His Asp Gly Lys Arg Thr Asn Ala Ile Phe Trp Tyr Leu His Phe
       130                 135                 140
   atg ata gaa tac tcc agt tgg caa cag tta ata gta cta act atc cta      480
   Met Ile Glu Tyr Ser Ser Trp Gln Gln Leu Ile Val Leu Thr Ile Leu
   145                 150                 155                 160
   ttt aat tta gct aaa tac gtt ttg cac atc cat caa ata aat ctc atc      528
   Phe Asn Leu Ala Lys Tyr Val Leu His Ile His Gln Ile Asn Leu Ile
                   165                 170                 175
   tta ttt tgg agt att cct cca att tta agt tcc att caa ctg ttt tat      576
   Leu Phe Trp Ser Ile Pro Pro Ile Leu Ser Ser Ile Gln Leu Phe Tyr
               180                 185                 190
   ttc gga aca ttt ttg cct cat cga gaa ccc aag aaa gga tat gtt tat      624
   Phe Gly Thr Phe Leu Pro His Arg Glu Pro Lys Lys Gly Tyr Val Tyr
           195                 200                 205
   ccc cat tgc agc caa aca ata aaa ttg cca act ttt ttg tca ttt atc      672
   Pro His Cys Ser Gln Thr Ile Lys Leu Pro Thr Phe Leu Ser Phe Ile
       210                 215                 220
   gct tgc tac cac ttt ggt tat cat gaa gaa cat cat gag tat ccc cat      720
   Ala Cys Tyr His Phe Gly Tyr His Glu Glu His His Glu Tyr Pro His
   225                 230                 235                 240
   gta cct tgg tgg caa ctt cca tct gta tat aag cag aga gta ttc aac      768
   Val Pro Trp Trp Gln Leu Pro Ser Val Tyr Lys Gln Arg Val Phe Asn
                   245                 250                 255
   aat tca gta acc aat tcg taa                                          789
   Asn Ser Val Thr Asn Ser
               260
<210> 82
   <211> 262
   <212> PRT
   <213> Nostoc punctiforme
<400> 82
   Leu Asn Phe Cys Asp Lys Pro Val Ser Tyr Tyr Val Ala Ile Glu Gln
   1               5                   10                  15
   Leu Ser Ala Lys Glu Asp Thr Val Trp Gly Leu Val Ile Val Ile Val
               20                  25                  30
   Ile Ile Ser Leu Trp Val Ala Ser Leu Ala Phe Leu Leu Ala Ile Asn
           35                  40                  45
   Tyr Ala Lys Val Pro Ile Trp Leu Ile Pro Ile Ala Ile Val Trp Gln
       50                  55                  60
   Met Phe Leu Tyr Thr Gly Leu Phe Ile Thr Ala His Asp Ala Met His
   65                  70                  75                  80
   Gly Ser Val Tyr Arg Lys Asn Pro Lys Ile Asn Asn Phe Ile Gly Ser
                   85                  90                  95
   Leu Ala Val Ala Leu Tyr Ala Val Phe Pro Tyr Gln Gln Met Leu Lys
               100                 105                 110
   Asn His Cys Leu His His Arg His Pro Ala Ser Glu Val Asp Pro Asp
           115                 120                 125
   Phe His Asp Gly Lys Arg Thr Asn Ala Ile Phe Trp Tyr Leu His Phe
       130                 135                 140
   Met Ile Glu Tyr Ser Ser Trp Gln Gln Leu Ile Val Leu Thr Ile Leu
   145                 150                 155                 160
   Phe Asn Leu Ala Lys Tyr Val Leu His Ile His Gln Ile Asn Leu Ile
                   165                 170                 175
   Leu Phe Trp Ser Ile Pro Pro Ile Leu Ser Ser Ile Gln Leu Phe Tyr
               180                 185                 190
   Phe Gly Thr Phe Leu Pro His Arg Glu Pro Lys Lys Gly Tyr Val Tyr
           195                 200                 205
   Pro His Cys Ser Gln Thr Ile Lys Leu Pro Thr Phe Leu Ser Phe Ile
       210                 215                 220
   Ala Cys Tyr His Phe Gly Tyr His Glu Glu His His Glu Tyr Pro His
   225                 230                 235                 240
   Val Pro Trp Trp Gln Leu Pro Ser Val Tyr Lys Gln Arg Val Phe Asn
                   245                 250                 255
   Asn Ser Val Thr Asn Ser
               260
<210> 83
   <211> 762
   <212> DNA
   <213> Nostoc punctiforme
<220>
   <221> CDS
   <222> (1)..(762)
   <223>
<400> 83
   gtg atc cag tta gaa caa cca ctc agt cat caa gca aaa ctg act cca      48
   Val Ile Gln Leu Glu Gln Pro Leu Ser His Gln Ala Lys Leu Thr Pro
   1               5                   10                  15
   gta ctg aga agt aaa tct cag ttt aag ggg ctt ttc att gct att gtc      96
   Val Leu Arg Ser Lys Ser Gln Phe Lys Gly Leu Phe Ile Ala Ile Val
               20                  25                  30
   att gtt agc gca tgg gtc att agc ctg agt tta tta ctt tcc ctt gac      144
   Ile Val Ser Ala Trp Val Ile Ser Leu Ser Leu Leu Leu Ser Leu Asp
           35                  40                  45
   atc tca aag cta aaa ttt tgg atg tta ttg cct gtt ata cta tgg caa      192
   Ile Ser Lys Leu Lys Phe Trp Met Leu Leu Pro Val Ile Leu Trp Gln
       50                  55                  60
   aca ttt tta tat acg gga tta ttt att aca tct cat gat gcc atg cat      240
   Thr Phe Leu Tyr Thr Gly Leu Phe Ile Thr Ser His Asp Ala Met His
   65                  70                  75                  80
   ggc gta gta ttt ccc caa aac acc aag att aat cat ttg att gga aca      288
   Gly Val Val Phe Pro Gln Asn Thr Lys Ile Asn His Leu Ile Gly Thr
                   85                  90                  95
   ttg acc cta tcc ctt tat ggt ctt tta cca tat caa aaa cta ttg aaa      336
   Leu Thr Leu Ser Leu Tyr Gly Leu Leu Pro Tyr Gln Lys Leu Leu Lys
               100                 105                 110
   aaa cat tgg tta cac cac cac aat cca gca agc tca ata gac ccg gat      384
   Lys His Trp Leu His His His Asn Pro Ala Ser Ser Ile Asp Pro Asp
           115                 120                 125
   ttt cac aat ggt aaa cac caa agt ttc ttt gct tgg tat ttt cat ttt      432
   Phe His Asn Gly Lys His Gln Ser Phe Phe Ala Trp Tyr Phe His Phe
       130                 135                 140
   atg aaa ggt tac tgg agt tgg ggg caa ata att gcg ttg act att att      480
   Met Lys Gly Tyr Trp Ser Trp Gly Gln Ile Ile Ala Leu Thr Ile Ile
   145                 150                 155                 160
   tat aac ttt gct aaa tac ata ctc cat atc cca agt gat aat cta act      528
   Tyr Asn Phe Ala Lys Tyr Ile Leu His Ile Pro Ser Asp Asn Leu Thr
                   165                 170                 175
   tac ttt tgg gtg cta ccc tcg ctt tta agt tca tta caa tta ttc tat      576
   Tyr Phe Trp Val Leu Pro Ser Leu Leu Ser Ser Leu Gln Leu Phe Tyr
               180                 185                 190
   ttt ggt act ttt tta ccc cat agt gaa cca ata ggg ggt tat gtt cag      624
   Phe Gly Thr Phe Leu Pro His Ser Glu Pro Ile Gly Gly Tyr Val Gln
           195                 200                 205
   cct cat tgt gcc caa aca att agc cgt cct att tgg tgg tca ttt atc      672
   Pro His Cys Ala Gln Thr Ile Ser Arg Pro Ile Trp Trp Ser Phe Ile
       210                 215                 220
   acg tgc tat cat ttt ggc tac cac gag gaa cat cac gaa tat cct cat      720
   Thr Cys Tyr His Phe Gly Tyr His Glu Glu His His Glu Tyr Pro His
   225                 230                 235                 240
   att tct tgg tgg cag tta cca gaa att tac aaa gca aaa tag              762
   Ile Ser Trp Trp Gln Leu Pro Glu Ile Tyr Lys Ala Lys
   245 250
<210> 84
   <211> 253
   <212> PRT
   <213> Nostoc punctiforme
<400> 84
   Val Ile Gln Leu Glu Gln Pro Leu Ser His Gln Ala Lys Leu Thr Pro
   1 5 10 15
   Val Leu Arg Ser Lys Ser Gln Phe Lys Gly Leu Phe Ile Ala Ile Val
   20 25 30
   Ile Val Ser Ala Trp Val Ile Ser Leu Ser Leu Leu Leu Ser Leu Asp
   35 40 45
   Ile Ser Lys Leu Lys Phe Trp Met Leu Leu Pro Val Ile Leu Trp Gln
   50 55 60
   Thr Phe Leu Tyr Thr Gly Leu Phe Ile Thr Ser His Asp Ala Met His
   65 70 75 80
   Gly Val Val Phe Pro Gln Asn Thr Lys Ile Asn His Leu Ile Gly Thr
   85 90 95
   Leu Thr Leu Ser Leu Tyr Gly Leu Leu Pro Tyr Gln Lys Leu Leu Lys
   100 105 110
   Lys His Trp Leu His His His Asn Pro Ala Ser Ser Ile Asp Pro Asp
   115 120 125
   Phe His Asn Gly Lys His Gln Ser Phe Phe Ala Trp Tyr Phe His Phe
   130 135 140
   Met Lys Gly Tyr Trp Ser Trp Gly Gln Ile Ile Ala Leu Thr Ile Ile
   145 150 155 160
   Tyr Asn Phe Ala Lys Tyr Ile Leu His Ile Pro Ser Asp Asn Leu Thr
   165 170 175
   Tyr Phe Trp Val Leu Pro Ser Leu Leu Ser Ser Leu Gln Leu Phe Tyr
   180 185 190
   Phe Gly Thr Phe Leu Pro His Ser Glu Pro Ile Gly Gly Tyr Val Gln
   195 200 205
   Pro His Cys Ala Gln Thr Ile Ser Arg Pro Ile Trp Trp Ser Phe Ile
   210 215 220
   Thr Cys Tyr His Phe Gly Tyr His Glu Glu His His Glu Tyr Pro His
   225 230 235 240
   Ile Ser Trp Trp Gln Leu Pro Glu Ile Tyr Lys Ala Lys
   245 250
<210> 85
   <211> 804
   <212> DNA
   <213> Synechococcus WH8102
<220>
   <221> CDS
   <222> (1)..(804)
   <223>
<400> 85
   atg aaa acg aca aga tct att tcg tgg cca tcg act tgc tgg cat cac 48
   Met Lys Thr Thr Arg Ser Ile Ser Trp Pro Ser Thr Cys Trp His His
   1 5 10 15
   cag ccg agt tgc tca agc tgg gtg gca aat gag ttc agc cct cag gcc 96
   Gln Pro Ser Cys Ser Ser Trp Val Ala Asn Glu Phe Ser Pro Gln Ala
   20 25 30
   ctc aaa ggg ttg gct ctg gct ggt ctg att gga tca gcc tgg ctg ctc        144
   Leu Lys Gly Leu Ala Leu Ala Gly Leu Ile Gly Ser Ala Trp Leu Leu
           35                  40                  45
   tcc ctg ggc ctg agc tac acc ctg cca ctt gat cag acg cct ggg ctg        192
   Ser Leu Gly Leu Ser Tyr Thr Leu Pro Leu Asp Gln Thr Pro Gly Leu
       50                  55                  60
   ttg att ggc agc ttg att ctg ctc aga gca ttt ctg cac acc ggg ctg        240
   Leu Ile Gly Ser Leu Ile Leu Leu Arg Ala Phe Leu His Thr Gly Leu
   65                  70                  75                  80
   ttc atc gtt gcc cac gat tcc atg cac gcc agt ctg gtt ccg ggt cat        288
   Phe Ile Val Ala His Asp Ser Met His Ala Ser Leu Val Pro Gly His
                   85                  90                  95
   ccc gga ttg aac cgc tgg atc ggc aaa gtg tat ttg ttg gtg tat gca        336
   Pro Gly Leu Asn Arg Trp Ile Gly Lys Val Tyr Leu Leu Val Tyr Ala
               100                 105                 110
   ggc ttg tct tat gag cgt tgt tcc cgc aac cac aga cgt cat cac ctg        384
   Gly Leu Ser Tyr Glu Arg Cys Ser Arg Asn His Arg Arg His His Leu
           115                 120                 125
   gca ccg gag acg ttc cag gat cct gac tac caa cgt tgc acc aat aac        432
   Ala Pro Glu Thr Phe Gln Asp Pro Asp Tyr Gln Arg Cys Thr Asn Asn
       130                 135                 140
   aac atc cta gat tgg tat gtt cac ttc atg ggc aac tat ctg ggc atg        480
   Asn Ile Leu Asp Trp Tyr Val His Phe Met Gly Asn Tyr Leu Gly Met
   145                 150                 155                 160
   cgg caa ctg tta aat cta agc tgt ctt tgg ctg gcg cta atc att ctc        528
   Arg Gln Leu Leu Asn Leu Ser Cys Leu Trp Leu Ala Leu Ile Ile Leu
                   165                 170                 175
   aac ggt tct gat ctc cct gct cag atc atg cat ctg ctg ttg ttc agc        576
   Asn Gly Ser Asp Leu Pro Ala Gln Ile Met His Leu Leu Leu Phe Ser
               180                 185                 190
   gtt ctg ccg ttg atc atc agt tcc tgt caa ttg ttt cta gtg gga acc        624
   Val Leu Pro Leu Ile Ile Ser Ser Cys Gln Leu Phe Leu Val Gly Thr
           195                 200                 205
   tgg tta ccc cac cga cgt ggg gcc acg aca cga ccg ggc gtg aca acg        672
   Trp Leu Pro His Arg Arg Gly Ala Thr Thr Arg Pro Gly Val Thr Thr
       210                 215                 220
   cgc agc ctg gct ttg cat cca gcc ctc tct ttc gca gct tgt tac aac        720
   Arg Ser Leu Ala Leu His Pro Ala Leu Ser Phe Ala Ala Cys Tyr Asn
   225                 230                 235                 240
   ttt ggc tat cat cgt gaa cat cat gaa tcg cct tcc aca ccc tgg ttt        768
   Phe Gly Tyr His Arg Glu His His Glu Ser Pro Ser Thr Pro Trp Phe
                   245                 250                 255
   cag ctg cca caa ctt cga aat gaa tca ttc act tga                         804
   Gln Leu Pro Gln Leu Arg Asn Glu Ser Phe Thr
               260                 265
<210> 86
   <211> 267
   <212> PRT
   <213> Synechococcus WH8102
<400> 86
   Met Lys Thr Thr Arg Ser Ile Ser Trp Pro Ser Thr Cys Trp His His
   1               5                   10                  15
   Gln Pro Ser Cys Ser Ser Trp Val Ala Asn Glu Phe Ser Pro Gln Ala
               20                  25                  30
   Leu Lys Gly Leu Ala Leu Ala Gly Leu Ile Gly Ser Ala Trp Leu Leu
           35                  40                  45
   Ser Leu Gly Leu Ser Tyr Thr Leu Pro Leu Asp Gln Thr Pro Gly Leu
       50                  55                  60
   Leu Ile Gly Ser Leu Ile Leu Leu Arg Ala Phe Leu His Thr Gly Leu
   65                  70                  75                  80
   Phe Ile Val Ala His Asp Ser Met His Ala Ser Leu Val Pro Gly His
                   85                  90                  95
   Pro Gly Leu Asn Arg Trp Ile Gly Lys Val Tyr Leu Leu Val Tyr Ala
               100                 105                 110
   Gly Leu Ser Tyr Glu Arg Cys Ser Arg Asn His Arg Arg His His Leu
           115                 120                 125
   Ala Pro Glu Thr Phe Gln Asp Pro Asp Tyr Gln Arg Cys Thr Asn Asn
       130                 135                 140
   Asn Ile Leu Asp Trp Tyr Val His Phe Met Gly Asn Tyr Leu Gly Met
   145                 150                 155                 160
   Arg Gln Leu Leu Asn Leu Ser Cys Leu Trp Leu Ala Leu Ile Ile Leu
                   165                 170                 175
   Asn Gly Ser Asp Leu Pro Ala Gln Ile Met His Leu Leu Leu Phe Ser
               180                 185                 190
   Val Leu Pro Leu Ile Ile Ser Ser Cys Gln Leu Phe Leu Val Gly Thr
           195                 200                 205
   Trp Leu Pro His Arg Arg Gly Ala Thr Thr Arg Pro Gly Val Thr Thr
       210                 215                 220
   Arg Ser Leu Ala Leu His Pro Ala Leu Ser Phe Ala Ala Cys Tyr Asn
   225                 230                 235                 240
   Phe Gly Tyr His Arg Glu His His Glu Ser Pro Ser Thr Pro Trp Phe
                   245                 250                 255
   Gln Leu Pro Gln Leu Arg Asn Glu Ser Phe Thr
               260                 265
<210> 87
   <211> 33
   <212> DNA
   <213> Künstliche Sequenz
<220>
   <221> primer_bind
   <222> (1)..(33)
   <223>
<400> 87
   gcatgctcta gaccttataa agatattttg tga 33
<210> 88
   <211> 33
   <212> DNA
   <213> Künstliche Sequenz
<220>
   <221> primer_bind
   <222> (1)..(33)
   <223>
<400> 88
   gcatgcatct agaaatggtt cagtgtcaac cat 33
<210> 89
   <211> 805
   <212> DNA
   <213> Nostoc sp. Strain PCC7120
<220>
   <221> variation
   <222> (1)..(805)
   <223>
<400> 89
   gcatgcatct agaaatggtt cagtgtcaac catcatctct gcattcagaa aaactggtgt 60
   tattgtcatc gacaatcaga gatgataaaa atattaataa gggtatattt attgcctgct 120
   ttatcttatt tttatgggca attagtttaa tcttattact ctcaatagat acatccataa 180
   ttcataagag cttattaggt atagccatgc tttggcagac cttcttatat acaggtttat 240
   ttattactgc tcatgatgcc atgcacggcg tagtttatcc caaaaatccc agaataaata 300
   attttatagg taagctcact ctaatcttgt atggactact cccttataaa gatttattga 360
   aaaaacattg gttacaccac ggacatcctg gtactgattt agaccctgat tattacaatg 420
   gtcatcccca aaacttcttt ctttggtatc tacattttat gaagtcttat tggcgatgga 480
   cgcaaatttt cggattagtg atgatttttc atggacttaa aaatctggtg catataccag 540
   aaaataattt aattatattt tggatgatac cttctatttt aagttcagta caactatttt 600
   attttggtac atttttgcct cataaaaagc tagaaggtgg ttatactaac ccccattgtg 660
   cgcgcagtat cccattacct cttttttggt cttttgttac ttgttatcac ttcggctacc 720
   acaaggaaca tcacgaatac cctcaacttc cttggtggaa attacctgaa gctcacaaaa 780
   tatctttata aggtctagag catgc 805
<210> 90
<211> 35 <212> DNA <213> Künstliche Sequenz
<220>
   <221> primer_bind
   <222> (1)..(35)
   <223>
<400> 90
   gagctcttca ttatttcgat tttgatttcg tgacc 35
<210> 91
   <211> 44
   <212> DNA
   <213> Künstliche Sequenz
<220>
   <221> primer_bind
   <222> (1)..(44)
   <223>
<400> 91
   aagcttgagc tcggttgatc agaagaagaa gaagaagatg aact 44
<210> 92
   <211> 653
   <212> DNA
   <213> Arabidopsis thaliana
<220>
   <221> promoter
   <222> (1)..(653)
   <223>
<400> 92
   gagctcttca ttatttcgat tttgatttcg tgaccagcga acgcagaata ccttgttgtg 60
   taatacttta cccgtgtaaa tcaaaaacaa aaaggctttt gagctttttg tagttgaatt 120
   tctctggctg atcttttctg tacagattca tatatctgca gagacgatat cattgattat 180
   ttgagcttct tttgaactat ttcgtgtaat ttgggatgag agctctatgt atgtgtgtaa 240
   actttgaaga caacaagaaa ggtaacaagt gagggaggga tgactccatg tcaaaataga 300
   tgtcataaga ggcccatcaa taagtgcttg agcccattag ctagcccagt aactaccaga 360
   ttgtgagatg gatgtgtgaa cagttttttt tttgatgtag gactgaaatg tgaacaacag 420
   gcgcatgaaa ggctaaatta ggacaatgat aagcagaaat aacttatcct ctctaacact 480
   tggcctcaca ttgcccttca cacaatccac acacatccaa tcacaacctc atcatatatc 540
   tcccgctaat cttttttctt ttgatctttt tttttttgct tattattttt ttgactttga 600
   tctcccatca gttcatcttc ttcttcttct tctgatcaac cgagctcaag ctt 653
<210> 93
   <211> 28
   <212> DNA
   <213> Künstliche Sequenz
<220>
   <221> primer_bind
   <222> (1)..(28)
   <223>
<400> 93
   gagctcactc actgatttcc attgcttg 28
<210> 94
   <211> 30
   <212> DNA
   <213> Künstliche Sequenz
<220>
   <221> primer_bind
   <222> (1)..(30)
   <223>
<400> 94
   aagcttgagc tctttgttga agagatttgg 30
<210> 95
   <211> 37
   <212> DNA
   <213> Künstliche Sequenz
<220>
   <221> primer_bind
   <222> (1)..(37)
   <223>
<400> 95
   cgccgttaag tcgatgtccg ttgatttaaa cagtgtc 37
<210> 96
   <211> 34
   <212> DNA
   <213> Künstliche Sequenz
<220>
   <221> primer_bind
   <222> (1)..(34)
   <223>
<400> 96
   atcaacggac atcgacttaa cggcgtttgt aaac 34
<210> 97
   <211> 831
   <212> DNA
   <213> Haematococcus pluvialis
<220>
   <221> CDS
   <222> (1)..(831)
   <223>
<400> 97
   atg cca tcc gag tcg tca gac gca gct cgt cct gtg ttg aag cac gcc      48
   Met Pro Ser Glu Ser Ser Asp Ala Ala Arg Pro Val Leu Lys His Ala
   1               5                   10                  15
   tat aaa cct cca gca tct gac gcc aag ggc atc act atg gcg ctg acc      96
   Tyr Lys Pro Pro Ala Ser Asp Ala Lys Gly Ile Thr Met Ala Leu Thr
               20                  25                  30
   atc att ggc acc tgg acc gca gtg ttt tta cac gca ata ttc caa atc     144
   Ile Ile Gly Thr Trp Thr Ala Val Phe Leu His Ala Ile Phe Gln Ile
           35                  40                  45
   agg cta ccg aca tcc atg gac cag ctt cac tgg ttg cct gtg tcc gaa     192
   Arg Leu Pro Thr Ser Met Asp Gln Leu His Trp Leu Pro Val Ser Glu
       50                  55                  60
   gcc aca gcc cag ctg ttg ggc gga agc agc agc cta ttg cac atc gcc     240
   Ala Thr Ala Gln Leu Leu Gly Gly Ser Ser Ser Leu Leu His Ile Ala
   65                  70                  75                  80
   gca gtc ttc att gta ctt gag ttt ctg tac act ggt cta ttc atc acc     288
   Ala Val Phe Ile Val Leu Glu Phe Leu Tyr Thr Gly Leu Phe Ile Thr
                   85                  90                  95
   acg cat gat gca atg cat ggc acc ata gct ttg agg aac agg cag ctc     336
   Thr His Asp Ala Met His Gly Thr Ile Ala Leu Arg Asn Arg Gln Leu
               100                 105                 110
   aat gat ctc ctt ggc aac atc tgc ata tca ctg tac gcc tgg ttt gac     384
   Asn Asp Leu Leu Gly Asn Ile Cys Ile Ser Leu Tyr Ala Trp Phe Asp
           115                 120                 125
   tac agc atg cac tgg gag cac cac aac cat act ggc gaa gtg ggg aaa     432
   Tyr Ser Met His Trp Glu His His Asn His Thr Gly Glu Val Gly Lys
       130                 135                 140
   gac cct gac ttc cac aaa gga aat cct ggc ctt gtc ccc tgg ttc gcc     480
   Asp Pro Asp Phe His Lys Gly Asn Pro Gly Leu Val Pro Trp Phe Ala
   145                 150                 155                 160
   agc ttc atg tcc agc tac atg tcc ctg tgg cag ttt gcc cgg ctg gca      528
   Ser Phe Met Ser Ser Tyr Met Ser Leu Trp Gln Phe Ala Arg Leu Ala
                   165                 170                 175
   tgg tgg gca gtg gtg atg caa acg ttg ggg gcc ccc atg gcg aat ctc      576
   Trp Trp Ala Val Val Met Gln Thr Leu Gly Ala Pro Met Ala Asn Leu
               180                 185                 190
   cta gtc ttc atg gct gca gcc cca atc ttg tca gca ttc cgc ctc ttc      624
   Leu Val Phe Met Ala Ala Ala Pro Ile Leu Ser Ala Phe Arg Leu Phe
           195                 200                 205
   tac ttc ggc act tac ctg cca cac aag cct gag cca ggc cct gca gca      672
   Tyr Phe Gly Thr Tyr Leu Pro His Lys Pro Glu Pro Gly Pro Ala Ala
       210                 215                 220
   ggc tct cag gtc atg tct tgg ttc agg gcc aag aca agt gag gca tct      720
   Gly Ser Gln Val Met Ser Trp Phe Arg Ala Lys Thr Ser Glu Ala Ser
   225                 230                 235                 240
   gat gtg atg agc ttc ctg aca tgc tac cac ttt gac ctg ttt gcc ccc      768
   Asp Val Met Ser Phe Leu Thr Cys Tyr His Phe Asp Leu Phe Ala Pro
                   245                 250                 255
   tgg tgg cag ctg ccc cac tgc cgc cgc ctg tct ggg cgt ggc ctg gtg      816
   Trp Trp Gln Leu Pro His Cys Arg Arg Leu Ser Gly Arg Gly Leu Val
               260                 265                 270
   cct gcc ttg gca tga                                                  831
   Pro Ala Leu Ala
           275
<210> 98
   <211> 276
   <212> PRT
   <213> Haematococcus pluvialis
<400> 98
   Met Pro Ser Glu Ser Ser Asp Ala Ala Arg Pro Val Leu Lys His Ala
   1               5                   10                  15
   Tyr Lys Pro Pro Ala Ser Asp Ala Lys Gly Ile Thr Met Ala Leu Thr
               20                  25                  30
   Ile Ile Gly Thr Trp Thr Ala Val Phe Leu His Ala Ile Phe Gln Ile
           35                  40                  45
   Arg Leu Pro Thr Ser Met Asp Gln Leu His Trp Leu Pro Val Ser Glu
       50                  55                  60
   Ala Thr Ala Gln Leu Leu Gly Gly Ser Ser Ser Leu Leu His Ile Ala
   65                  70                  75                  80
   Ala Val Phe Ile Val Leu Glu Phe Leu Tyr Thr Gly Leu Phe Ile Thr
                   85                  90                  95
   Thr His Asp Ala Met His Gly Thr Ile Ala Leu Arg Asn Arg Gln Leu
               100                 105                 110
   Asn Asp Leu Leu Gly Asn Ile Cys Ile Ser Leu Tyr Ala Trp Phe Asp
           115                 120                 125
   Tyr Ser Met His Trp Glu His His Asn His Thr Gly Glu Val Gly Lys
       130                 135                 140
   Asp Pro Asp Phe His Lys Gly Asn Pro Gly Leu Val Pro Trp Phe Ala
   145                 150                 155                 160
   Ser Phe Met Ser Ser Tyr Met Ser Leu Trp Gln Phe Ala Arg Leu Ala
                   165                 170                 175
   Trp Trp Ala Val Val Met Gln Thr Leu Gly Ala Pro Met Ala Asn Leu
               180                 185                 190
   Leu Val Phe Met Ala Ala Ala Pro Ile Leu Ser Ala Phe Arg Leu Phe
           195                 200                 205
   Tyr Phe Gly Thr Tyr Leu Pro His Lys Pro Glu Pro Gly Pro Ala Ala
       210                 215                 220
   Gly Ser Gln Val Met Ser Trp Phe Arg Ala Lys Thr Ser Glu Ala Ser
   225                 230                 235                 240
   Asp Val Met Ser Phe Leu Thr Cys Tyr His Phe Asp Leu Phe Ala Pro
   245 250 255
   Trp Trp Gln Leu Pro His Cys Arg Arg Leu Ser Gly Arg Gly Leu Val
   260 265 270
   Pro Ala Leu Ala
   275
<210> 99
   <211> 729
   <212> DNA
   <213> Paracoccus sp. MBIC1143
<220>
   <221> CDS
   <222> (1)..(729)
   <223>
<400> 99
   atg agc gca cat gcc ctg ccc aag gca gat ctg acc gcc acc agc ctg 48
   Met Ser Ala His Ala Leu Pro Lys Ala Asp Leu Thr Ala Thr Ser Leu
   1 5 10 15
   atc gtc tcg ggc ggc atc atc gcc gct tgg ctg gcc ctg cat gtg cat 96
   Ile Val Ser Gly Gly Ile Ile Ala Ala Trp Leu Ala Leu His Val His
   20 25 30
   gcg ctg tgg ttt ctg gac gca gcg gcg cat ccc atc ctg gcg atc gca 144
   Ala Leu Trp Phe Leu Asp Ala Ala Ala His Pro Ile Leu Ala Ile Ala
   35 40 45
   aat ttc ctg ggg ctg acc tgg ctg tcg gtc gga ttg ttc atc atc gcg 192
   Asn Phe Leu Gly Leu Thr Trp Leu Ser Val Gly Leu Phe Ile Ile Ala
   50 55 60
   cat gac gcg atg cac ggg tcg gtg gtg ccg ggg cgt ccg cgc gcc aat 240
   His Asp Ala Met His Gly Ser Val Val Pro Gly Arg Pro Arg Ala Asn
   65 70 75 80
   gcg gcg atg ggc cag ctt gtc ctg tgg ctg tat gcc gga ttt tcg tgg 288
   Ala Ala Met Gly Gln Leu Val Leu Trp Leu Tyr Ala Gly Phe Ser Trp
                   85                  90                  95
   cgc aag atg atc gtc aag cac atg gcc cat cac cgc cat gcc gga acc      336
   Arg Lys Met Ile Val Lys His Met Ala His His Arg His Ala Gly Thr
               100                 105                 110
   gac gac gac ccc gat ttc gac cat ggc ggc ccg gtc cgc tgg tac gcc      384
   Asp Asp Asp Pro Asp Phe Asp His Gly Gly Pro Val Arg Trp Tyr Ala
           115                 120                 125
   cgc ttc atc ggc acc tat ttc ggc tgg cgc gag ggg ctg ctg ctg ccc      432
   Arg Phe Ile Gly Thr Tyr Phe Gly Trp Arg Glu Gly Leu Leu Leu Pro
       130                 135                 140
   gtc atc gtg acg gtc tat gcg ctg atc ctt ggg gat cgc tgg atg tac      480
   Val Ile Val Thr Val Tyr Ala Leu Ile Leu Gly Asp Arg Trp Met Tyr
   145                 150                 155                 160
   gtg gtc ttc tgg ccg ctg ccg tcg atc ctg gcg tcg atc cag ctg ttc      528
   Val Val Phe Trp Pro Leu Pro Ser Ile Leu Ala Ser Ile Gln Leu Phe
                   165                 170                 175
   gtg ttc ggc acc tgg ctg ccg cac cgc ccc ggc cac gac gcg ttc ccg      576
   Val Phe Gly Thr Trp Leu Pro His Arg Pro Gly His Asp Ala Phe Pro
               180                 185                 190
   gac cgc cac aat gcg cgg tcg tcg cgg atc agc gac ccc gtg tcg ctg      624
   Asp Arg His Asn Ala Arg Ser Ser Arg Ile Ser Asp Pro Val Ser Leu
           195                 200                 205
   ctg acc tgc ttt cac ttt ggc ggt tat cat cac gaa cac cac ctg cac      672
   Leu Thr Cys Phe His Phe Gly Gly Tyr His His Glu His His Leu His
       210                 215                 220
   ccg acg gtg ccg tgg tgg cgc ctg ccc agc acc cgc acc aag ggg gac      720
   Pro Thr Val Pro Trp Trp Arg Leu Pro Ser Thr Arg Thr Lys Gly Asp
   225                 230                 235                 240
   acc gca tga                                                          729
   Thr Ala
<210> 100
   <211> 242
   <212> PRT
   <213> Paracoccus sp. MBIC1143
<400> 100
   Met Ser Ala His Ala Leu Pro Lys Ala Asp Leu Thr Ala Thr Ser Leu
   1               5                   10                  15
   Ile Val Ser Gly Gly Ile Ile Ala Ala Trp Leu Ala Leu His Val His
               20                  25                  30
   Ala Leu Trp Phe Leu Asp Ala Ala Ala His Pro Ile Leu Ala Ile Ala
           35                  40                  45
   Asn Phe Leu Gly Leu Thr Trp Leu Ser Val Gly Leu Phe Ile Ile Ala
       50                  55                  60
   His Asp Ala Met His Gly Ser Val Val Pro Gly Arg Pro Arg Ala Asn
   65                  70                  75                  80
   Ala Ala Met Gly Gln Leu Val Leu Trp Leu Tyr Ala Gly Phe Ser Trp
                   85                  90                  95
   Arg Lys Met Ile Val Lys His Met Ala His His Arg His Ala Gly Thr
               100                 105                 110
   Asp Asp Asp Pro Asp Phe Asp His Gly Gly Pro Val Arg Trp Tyr Ala
           115                 120                 125
   Arg Phe Ile Gly Thr Tyr Phe Gly Trp Arg Glu Gly Leu Leu Leu Pro
       130                 135                 140
   Val Ile Val Thr Val Tyr Ala Leu Ile Leu Gly Asp Arg Trp Met Tyr
   145                 150                 155                 160
   Val Val Phe Trp Pro Leu Pro Ser Ile Leu Ala Ser Ile Gln Leu Phe
                   165                 170                 175
   Val Phe Gly Thr Trp Leu Pro His Arg Pro Gly His Asp Ala Phe Pro
               180                 185                 190
   Asp Arg His Asn Ala Arg Ser Ser Arg Ile Ser Asp Pro Val Ser Leu
           195                 200                 205
   Leu Thr Cys Phe His Phe Gly Gly Tyr His His Glu His His Leu His
       210                 215                 220
   Pro Thr Val Pro Trp Trp Arg Leu Pro Ser Thr Arg Thr Lys Gly Asp
   225                 230                 235                 240
   Thr Ala
<210> 101
   <211> 735
   <212> DNA
   <213> Brevundimonas aurantiaca
<220>
   <221> CDS
   <222> (1)..(735)
   <223>
<400> 101
   atg acc gcc gcc gtc gcc gag cca cgc acc gtc ccg cgc cag acc tgg        48
   Met Thr Ala Ala Val Ala Glu Pro Arg Thr Val Pro Arg Gln Thr Trp
   1               5                   10                  15
   atc ggt ctg acc ctg gcg gga atg atc gtg gcg gga tgg gcg gtt ctg        96
   Ile Gly Leu Thr Leu Ala Gly Met Ile Val Ala Gly Trp Ala Val Leu
               20                  25                  30
   cat gtc tac ggc gtc tat ttt cac cga tgg ggg ccg ttg acc ctg gtg       144
   His Val Tyr Gly Val Tyr Phe His Arg Trp Gly Pro Leu Thr Leu Val
           35                  40                  45
   atc gcc ccg gcg atc gtg gcg gtc cag acc tgg ttg tcg gtc ggc ctt       192
   Ile Ala Pro Ala Ile Val Ala Val Gln Thr Trp Leu Ser Val Gly Leu
       50                  55                  60
   ttc atc gtc gcc cat gac gcc atg tac ggc tcc ctg gcg ccg gga cgg       240
   Phe Ile Val Ala His Asp Ala Met Tyr Gly Ser Leu Ala Pro Gly Arg
   65                  70                  75                  80
   ccg cgg ctg aac gcc gca gtc ggc cgg ctg acc ctg ggg ctc tat gcg       288
   Pro Arg Leu Asn Ala Ala Val Gly Arg Leu Thr Leu Gly Leu Tyr Ala
                   85                  90                  95
   ggc ttc cgc ttc gat cgg ctg aag acg gcg cac cac gcc cac cac gcc      336
   Gly Phe Arg Phe Asp Arg Leu Lys Thr Ala His His Ala His His Ala
               100                 105                 110
   gcg ccc ggc acg gcc gac gac ccg gat ttt cac gcc ccg gcg ccc cgc      384
   Ala Pro Gly Thr Ala Asp Asp Pro Asp Phe His Ala Pro Ala Pro Arg
           115                 120                 125
   gcc ttc ctt ccc tgg ttc ctg aac ttc ttt cgc acc tat ttc ggc tgg      432
   Ala Phe Leu Pro Trp Phe Leu Asn Phe Phe Arg Thr Tyr Phe Gly Trp
       130                 135                 140
   cgc gag atg gcg gtc ctg acc gcc ctg gtc ctg atc gcc ctc ttc ggc      480
   Arg Glu Met Ala Val Leu Thr Ala Leu Val Leu Ile Ala Leu Phe Gly
   145                 150                 155                 160
   ctg ggg gcg cgg ccg gcc aat ctc ctg acc ttc tgg gcc gcg ccg gcc      528
   Leu Gly Ala Arg Pro Ala Asn Leu Leu Thr Phe Trp Ala Ala Pro Ala
                   165                 170                 175
   ctg ctt tca gcg ctt cag ctc ttc acc ttc ggc acc tgg ctg ccg cac      576
   Leu Leu Ser Ala Leu Gln Leu Phe Thr Phe Gly Thr Trp Leu Pro His
               180                 185                 190
   cgc cac acc gac cag ccg ttc gcc gac gcg cac cac gcc cgc agc agc      624
   Arg His Thr Asp Gln Pro Phe Ala Asp Ala His His Ala Arg Ser Ser
           195                 200                 205
   ggc tac ggc ccc gtg ctt tcc ctg ctc acc tgt ttc cac ttc ggc cgc      672
   Gly Tyr Gly Pro Val Leu Ser Leu Leu Thr Cys Phe His Phe Gly Arg
       210                 215                 220
   cac cac gaa cac cat ctg agc ccc tgg cgg ccc tgg tgg cgt ctg tgg      720
   His His Glu His His Leu Ser Pro Trp Arg Pro Trp Trp Arg Leu Trp
   225                 230                 235                 240
   cgc ggc gag tct tga                                                  735
   Arg Gly Glu Ser
<210> 102
   <211> 244
   <212> PRT
   <213> Brevundimonas aurantiaca
<400> 102
   Met Thr Ala Ala Val Ala Glu Pro Arg Thr Val Pro Arg Gln Thr Trp
   1               5                   10                  15
   Ile Gly Leu Thr Leu Ala Gly Met Ile Val Ala Gly Trp Ala Val Leu
               20                  25                  30
   His Val Tyr Gly Val Tyr Phe His Arg Trp Gly Pro Leu Thr Leu Val
           35                  40                  45
   Ile Ala Pro Ala Ile Val Ala Val Gln Thr Trp Leu Ser Val Gly Leu
       50                  55                  60
   Phe Ile Val Ala His Asp Ala Met Tyr Gly Ser Leu Ala Pro Gly Arg
   65                  70                  75                  80
   Pro Arg Leu Asn Ala Ala Val Gly Arg Leu Thr Leu Gly Leu Tyr Ala
                   85                  90                  95
   Gly Phe Arg Phe Asp Arg Leu Lys Thr Ala His His Ala His His Ala
               100                 105                 110
   Ala Pro Gly Thr Ala Asp Asp Pro Asp Phe His Ala Pro Ala Pro Arg
           115                 120                 125
   Ala Phe Leu Pro Trp Phe Leu Asn Phe Phe Arg Thr Tyr Phe Gly Trp
       130                 135                 140
   Arg Glu Met Ala Val Leu Thr Ala Leu Val Leu Ile Ala Leu Phe Gly
   145                 150                 155                 160
   Leu Gly Ala Arg Pro Ala Asn Leu Leu Thr Phe Trp Ala Ala Pro Ala
                   165                 170                 175
   Leu Leu Ser Ala Leu Gln Leu Phe Thr Phe Gly Thr Trp Leu Pro His
               180                 185                 190
   Arg His Thr Asp Gln Pro Phe Ala Asp Ala His His Ala Arg Ser Ser
           195                 200                 205
   Gly Tyr Gly Pro Val Leu Ser Leu Leu Thr Cys Phe His Phe Gly Arg
   210 215 220
   His His Glu His His Leu Ser Pro Trp Arg Pro Trp Trp Arg Leu Trp
   225 230 235 240
   Arg Gly Glu Ser
<210> 103
   <211> 690
   <212> DNA
   <213> Nodularia spumigena NSOR10
<220>
   <221> CDS
   <222> (1)..(690)
   <223>
<400> 103
   atg gcg atc gcc att att agt ata tgg gct atc agc cta ggt ttg tta 48
   Met Ala Ile Ala Ile Ile Ser Ile Trp Ala Ile Ser Leu Gly Leu Leu
   1 5 10 15
   ctt tat att gat ata tcc caa ttc aag ttt tgg atg ttg tta ccg ctc 96
   Leu Tyr Ile Asp Ile Ser Gln Phe Lys Phe Trp Met Leu Leu Pro Leu
   20 25 30
   ata ttt tgg caa aca ttt tta tat acg gga tta ttt att aca gct cat 144
   Ile Phe Trp Gln Thr Phe Leu Tyr Thr Gly Leu Phe Ile Thr Ala His
   35 40 45
   gat gcc atg cat ggg gta gtt ttt ccc aaa aat ccc aaa atc aac cat 192
   Asp Ala Met His Gly Val Val Phe Pro Lys Asn Pro Lys Ile Asn His
   50 55 60
   ttc att ggc tca ttg tgc ctg ttt ctt tat ggt ctt tta cct tat caa 240
   Phe Ile Gly Ser Leu Cys Leu Phe Leu Tyr Gly Leu Leu Pro Tyr Gln
   65 70 75 80
   aaa ctt tta aaa aag cat tgg cta cat cac cat aat cca gcc agt gaa 288
   Lys Leu Leu Lys Lys His Trp Leu His His His Asn Pro Ala Ser Glu
                   85                  90                  95
   aca gat cca gat ttt cac aac ggg aag cag aaa aac ttt ttt gct tgg      336
   Thr Asp Pro Asp Phe His Asn Gly Lys Gln Lys Asn Phe Phe Ala Trp
               100                 105                 110
   tat tta tat ttt atg aag cgt tac tgg agt tgg tta caa att atc aca      384
   Tyr Leu Tyr Phe Met Lys Arg Tyr Trp Ser Trp Leu Gln Ile Ile Thr
           115                 120                 125
   tta atg att att tat aac tta cta aaa tat ata tgg cat ttt cca gag      432
   Leu Met Ile Ile Tyr Asn Leu Leu Lys Tyr Ile Trp His Phe Pro Glu
       130                 135                 140
   gat aat atg act tat ttt tgg gta gtt ccc tca att tta agt tct tta      480
   Asp Asn Met Thr Tyr Phe Trp Val Val Pro Ser Ile Leu Ser Ser Leu
   145                 150                 155                 160
   caa tta ttt tat ttt gga act ttt cta ccc cac agt gag cct gta gaa      528
   Gln Leu Phe Tyr Phe Gly Thr Phe Leu Pro His Ser Glu Pro Val Glu
                   165                 170                 175
   ggt tat aaa gag cct cat cgt tcc caa act att agc cgt ccc att tgg      576
   Gly Tyr Lys Glu Pro His Arg Ser Gln Thr Ile Ser Arg Pro Ile Trp
               180                 185                 190
   tgg tca ttt ata act tgt tac cat ttt ggt tat cat tac gaa cat cat      624
   Trp Ser Phe Ile Thr Cys Tyr His Phe Gly Tyr His Tyr Glu His His
           195                 200                 205
   gaa tac ccc cat gtt cct tgg tgg caa tta cca gaa att tat aaa atg      672
   Glu Tyr Pro His Val Pro Trp Trp Gln Leu Pro Glu Ile Tyr Lys Met
       210                 215                 220
   tct aaa tca aat ttg tga                                              690
   Ser Lys Ser Asn Leu
   225
<210> 104
   <211> 229
   <212> PRT
   <213> Nodularia spumigena NSOR10
<400> 104
   Met Ala Ile Ala Ile Ile Ser Ile Trp Ala Ile Ser Leu Gly Leu Leu
   1               5                   10                  15
   Leu Tyr Ile Asp Ile Ser Gln Phe Lys Phe Trp Met Leu Leu Pro Leu
   20 25 30
   Ile Phe Trp Gln Thr Phe Leu Tyr Thr Gly Leu Phe Ile Thr Ala His
   35 40 45
   Asp Ala Met His Gly Val Val Phe Pro Lys Asn Pro Lys Ile Asn His
   50 55 60
   Phe Ile Gly Ser Leu Cys Leu Phe Leu Tyr Gly Leu Leu Pro Tyr Gln
   65 70 75 80
   Lys Leu Leu Lys Lys His Trp Leu His His His Asn Pro Ala Ser Glu
   85 90 95
   Thr Asp Pro Asp Phe His Asn Gly Lys Gln Lys Asn Phe Phe Ala Trp
   100 105 110
   Tyr Leu Tyr Phe Met Lys Arg Tyr Trp Ser Trp Leu Gln Ile Ile Thr
   115 120 125
   Leu Met Ile Ile Tyr Asn Leu Leu Lys Tyr Ile Trp His Phe Pro Glu
   130 135 140
   Asp Asn Met Thr Tyr Phe Trp Val Val Pro Ser Ile Leu Ser Ser Leu
   145 150 155 160
   Gln Leu Phe Tyr Phe Gly Thr Phe Leu Pro His Ser Glu Pro Val Glu
   165 170 175
   Gly Tyr Lys Glu Pro His Arg Ser Gln Thr Ile Ser Arg Pro Ile Trp
   180 185 190
   Trp Ser Phe Ile Thr Cys Tyr His Phe Gly Tyr His Tyr Glu His His
   195 200 205
   Glu Tyr Pro His Val Pro Trp Trp Gln Leu Pro Glu Ile Tyr Lys Met
   210 215 220
   Ser Lys Ser Asn Leu
   225
<210> 105
   <211> 1536
   <212> DNA
   <213> Deinococcus radiodurans R1
<220>
   <221> CDS
   <222> (1)..(1536)
   <223>
<400> 105
   atg ccg gat tac gac ctg atc gtc atg ggc gcg ggc cac aac gcg ctg       48
   Met Pro Asp Tyr Asp Leu Ile Val Met Gly Ala Gly His Asn Ala Leu
   1               5                   10                  15
   gtg act gct gcc tac gcc gcc cgg gcg ggc ctg aaa gtc ggc gtg ttc       96
   Val Thr Ala Ala Tyr Ala Ala Arg Ala Gly Leu Lys Val Gly Val Phe
               20                  25                  30
   gag cgg cgg cac ctc gtc ggc ggg gcg gtc agc acc gag gag gtc gtg      144
   Glu Arg Arg His Leu Val Gly Gly Ala Val Ser Thr Glu Glu Val Val
           35                  40                  45
   ccc ggt tac cgc ttc gac tac ggc ggc agc gcc cac atc ctg att cgg      192
   Pro Gly Tyr Arg Phe Asp Tyr Gly Gly Ser Ala His Ile Leu Ile Arg
       50                  55                  60
   atg acg ccc atc gtg cgc gaa ctc gaa ctc acg cgg cac ggg ctg cat      240
   Met Thr Pro Ile Val Arg Glu Leu Glu Leu Thr Arg His Gly Leu His
   65                  70                  75                  80
   tac ctc gaa gtg gac cct atg ttt cac gct tcc gac ggt gaa acg ccc      288
   Tyr Leu Glu Val Asp Pro Met Phe His Ala Ser Asp Gly Glu Thr Pro
                   85                  90                  95
   tgg ttc att cac cgc gac gcc ggg cgg acc atc cgc gaa ctg gac gaa      336
   Trp Phe Ile His Arg Asp Ala Gly Arg Thr Ile Arg Glu Leu Asp Glu
               100                 105                 110
   aag ttt ccc ggg cag ggc gac gcc tac ggg cgc ttt ctc gac gat tgg      384
   Lys Phe Pro Gly Gln Gly Asp Ala Tyr Gly Arg Phe Leu Asp Asp Trp
           115                 120                 125
   aca ccc ttc gcg cgc gcc gtg gcc gac ctg ttc aac tcg gcg ccg ggg      432
   Thr Pro Phe Ala Arg Ala Val Ala Asp Leu Phe Asn Ser Ala Pro Gly
       130                 135                 140
   ccg ctc gac ctg ggc aaa atg gtg atg cgc agc ggc cag ggc aag gac      480
   Pro Leu Asp Leu Gly Lys Met Val Met Arg Ser Gly Gln Gly Lys Asp
   145                 150                 155                 160
   tgg aac gag cag ctc ccg cgc atc ctg cgg ccc tac ggc gac gtg gcg      528
   Trp Asn Glu Gln Leu Pro Arg Ile Leu Arg Pro Tyr Gly Asp Val Ala
                   165                 170                 175
   cgc gag tac ttc agc gag gag cgc gtg cgg gct ccc ctg acc tgg atg      576
   Arg Glu Tyr Phe Ser Glu Glu Arg Val Arg Ala Pro Leu Thr Trp Met
               180                 185                 190
   gcg gcc cag agc ggc ccc cca ccc tcg gac ccg ctg agc gcg ccc ttt      624
   Ala Ala Gln Ser Gly Pro Pro Pro Ser Asp Pro Leu Ser Ala Pro Phe
           195                 200                 205
   ttg ctg tgg cac ccg ctc tac cac gaa ggc ggc gtg gcg cgg ccc aaa      672
   Leu Leu Trp His Pro Leu Tyr His Glu Gly Gly Val Ala Arg Pro Lys
       210                 215                 220
   ggc ggc agc ggc ggc ctg acc aaa gcc ctg cgc cgg gcc acc gag gcc      720
   Gly Gly Ser Gly Gly Leu Thr Lys Ala Leu Arg Arg Ala Thr Glu Ala
   225                 230                 235                 240
   gaa ggc ggc gag gtc ttc acc gac gcg ccg gtc aag gaa att ctg gtc      768
   Glu Gly Gly Glu Val Phe Thr Asp Ala Pro Val Lys Glu Ile Leu Val
                   245                 250                 255
   aag gac ggc aag gcg cag ggc atc cgg ctg gaa agc ggc gag acg tac      816
   Lys Asp Gly Lys Ala Gln Gly Ile Arg Leu Glu Ser Gly Glu Thr Tyr
               260                 265                 270
   acc gcc cgc gcc gtc gtg tcg ggc gtc cac atc ctg acc act gcg aat      864
   Thr Ala Arg Ala Val Val Ser Gly Val His Ile Leu Thr Thr Ala Asn
           275                 280                 285
   gcc ctg ccc gcc gaa tat gtc cct agc gcc gcc agg aat gtg cgc gtg      912
   Ala Leu Pro Ala Glu Tyr Val Pro Ser Ala Ala Arg Asn Val Arg Val
       290                 295                 300
   ggc aac ggc ttc ggc atg att ttg cgc ctc gcc ctc agt gaa aaa gtc      960
   Gly Asn Gly Phe Gly Met Ile Leu Arg Leu Ala Leu Ser Glu Lys Val
   305                 310                 315                 320
   aaa tac cgt cac cac acc gag ccc gac tca cgc atc ggc ctg gga ttg     1008
   Lys Tyr Arg His His Thr Glu Pro Asp Ser Arg Ile Gly Leu Gly Leu
                   325                 330                 335
   ctg atc aaa aac gag cgg caa atc atg cag ggc tac ggc gaa tac ctc      1056
   Leu Ile Lys Asn Glu Arg Gln Ile Met Gln Gly Tyr Gly Glu Tyr Leu
               340                 345                 350
   gcc ggg cag ccc acc acc gac ccg ccc ctc gtc gcc atg agc ttc agc      1104
   Ala Gly Gln Pro Thr Thr Asp Pro Pro Leu Val Ala Met Ser Phe Ser
           355                 360                 365
   gcg gtg gac gac tcg ctc gcc cca ccg aac ggc gac gtg ttg tgg ctg      1152
   Ala Val Asp Asp Ser Leu Ala Pro Pro Asn Gly Asp Val Leu Trp Leu
       370                 375                 380
   tgg gcg cag tac tac ccc ttc gag ctc gcc acc ggg agc tgg gaa acg      1200
   Trp Ala Gln Tyr Tyr Pro Phe Glu Leu Ala Thr Gly Ser Trp Glu Thr
   385                 390                 395                 400
   cgc acc gcc gaa gcg cgg gag aac atc ctg cgg gcc ttt gag cac tac      1248
   Arg Thr Ala Glu Ala Arg Glu Asn Ile Leu Arg Ala Phe Glu His Tyr
                   405                 410                 415
   gcg ccg ggc acc cgc gac acg att gtg ggc gaa ctc gtg cag acg ccg      1296
   Ala Pro Gly Thr Arg Asp Thr Ile Val Gly Glu Leu Val Gln Thr Pro
               420                 425                 430
   cag tgg ctg gaa acc aac ctc ggc ctg cac cgg ggc aac gtg atg cac      1344
   Gln Trp Leu Glu Thr Asn Leu Gly Leu His Arg Gly Asn Val Met His
           435                 440                 445
   ctg gaa atg tcc ttc gac cag atg ttc tcc ttc cgc ccc tgg ctg aaa      1392
   Leu Glu Met Ser Phe Asp Gln Met Phe Ser Phe Arg Pro Trp Leu Lys
       450                 455                 460
   gcg agc cag tac cgc tgg ccg ggc gtg cag ggg ctg tac ctc acc ggc      1440
   Ala Ser Gln Tyr Arg Trp Pro Gly Val Gln Gly Leu Tyr Leu Thr Gly
   465                 470                 475                 480
   gcc agc acc cac ccc ggc gga ggc atc atg ggc gcc tcg gga cgc aac      1488
   Ala Ser Thr His Pro Gly Gly Gly Ile Met Gly Ala Ser Gly Arg Asn
                   485                 490                 495
   gcg gcg cgg gtc atc gtg aag gac ctg acg cgg agg cgc tgg aaa tga      1536
   Ala Ala Arg Val Ile Val Lys Asp Leu Thr Arg Arg Arg Trp Lys
               500                 505                 510
<210> 106
   <211> 511
   <212> PRT
   <213> Deinococcus radiodurans R1
<400> 106
   Met Pro Asp Tyr Asp Leu Ile Val Met Gly Ala Gly His Asn Ala Leu
   1               5                   10                  15
   Val Thr Ala Ala Tyr Ala Ala Arg Ala Gly Leu Lys Val Gly Val Phe
               20                  25                  30
   Glu Arg Arg His Leu Val Gly Gly Ala Val Ser Thr Glu Glu Val Val
           35                  40                  45
   Pro Gly Tyr Arg Phe Asp Tyr Gly Gly Ser Ala His Ile Leu Ile Arg
       50                  55                  60
   Met Thr Pro Ile Val Arg Glu Leu Glu Leu Thr Arg His Gly Leu His
   65                  70                  75                  80
   Tyr Leu Glu Val Asp Pro Met Phe His Ala Ser Asp Gly Glu Thr Pro
                   85                  90                  95
   Trp Phe Ile His Arg Asp Ala Gly Arg Thr Ile Arg Glu Leu Asp Glu
               100                 105                 110
   Lys Phe Pro Gly Gln Gly Asp Ala Tyr Gly Arg Phe Leu Asp Asp Trp
           115                 120                 125
   Thr Pro Phe Ala Arg Ala Val Ala Asp Leu Phe Asn Ser Ala Pro Gly
       130                 135                 140
   Pro Leu Asp Leu Gly Lys Met Val Met Arg Ser Gly Gln Gly Lys Asp
   145                 150                 155                 160
   Trp Asn Glu Gln Leu Pro Arg Ile Leu Arg Pro Tyr Gly Asp Val Ala
                   165                 170                 175
   Arg Glu Tyr Phe Ser Glu Glu Arg Val Arg Ala Pro Leu Thr Trp Met
               180                 185                 190
   Ala Ala Gln Ser Gly Pro Pro Pro Ser Asp Pro Leu Ser Ala Pro Phe
           195                 200                 205
   Leu Leu Trp His Pro Leu Tyr His Glu Gly Gly Val Ala Arg Pro Lys
       210                 215                 220
   Gly Gly Ser Gly Gly Leu Thr Lys Ala Leu Arg Arg Ala Thr Glu Ala
   225                 230                 235                 240
   Glu Gly Gly Glu Val Phe Thr Asp Ala Pro Val Lys Glu Ile Leu Val
                   245                 250                 255
   Lys Asp Gly Lys Ala Gln Gly Ile Arg Leu Glu Ser Gly Glu Thr Tyr
               260                 265                 270
   Thr Ala Arg Ala Val Val Ser Gly Val His Ile Leu Thr Thr Ala Asn
           275                 280                 285
   Ala Leu Pro Ala Glu Tyr Val Pro Ser Ala Ala Arg Asn Val Arg Val
       290                 295                 300
   Gly Asn Gly Phe Gly Met Ile Leu Arg Leu Ala Leu Ser Glu Lys Val
   305                 310                 315                 320
   Lys Tyr Arg His His Thr Glu Pro Asp Ser Arg Ile Gly Leu Gly Leu
                   325                 330                 335
   Leu Ile Lys Asn Glu Arg Gln Ile Met Gln Gly Tyr Gly Glu Tyr Leu
               340                 345                 350
   Ala Gly Gln Pro Thr Thr Asp Pro Pro Leu Val Ala Met Ser Phe Ser
           355                 360                 365
   Ala Val Asp Asp Ser Leu Ala Pro Pro Asn Gly Asp Val Leu Trp Leu
       370                 375                 380
   Trp Ala Gln Tyr Tyr Pro Phe Glu Leu Ala Thr Gly Ser Trp Glu Thr
   385                 390                 395                 400
   Arg Thr Ala Glu Ala Arg Glu Asn Ile Leu Arg Ala Phe Glu His Tyr
                   405                 410                 415
   Ala Pro Gly Thr Arg Asp Thr Ile Val Gly Glu Leu Val Gln Thr Pro
               420                 425                 430
   Gln Trp Leu Glu Thr Asn Leu Gly Leu His Arg Gly Asn Val Met His
           435                 440                 445
   Leu Glu Met Ser Phe Asp Gln Met Phe Ser Phe Arg Pro Trp Leu Lys
       450                 455                 460
   Ala Ser Gln Tyr Arg Trp Pro Gly Val Gln Gly Leu Tyr Leu Thr Gly
   465                 470                 475                 480
   Ala Ser Thr His Pro Gly Gly Gly Ile Met Gly Ala Ser Gly Arg Asn
                   485                 490                 495
   Ala Ala Arg Val Ile Val Lys Asp Leu Thr Arg Arg Arg Trp Lys
               500                 505                 510
<210> 107
   <211> 1666
   <212> DNA
   <213> Lycopersicon esculentum
<220>
   <221> CDS
   <222> (1)..(1494)
   <223>
<400> 107
   atg gaa gct ctt ctc aag cct ttt cca tct ctt tta ctt tcc tct cct      48
   Met Glu Ala Leu Leu Lys Pro Phe Pro Ser Leu Leu Leu Ser Ser Pro
   1               5                   10                  15
   aca ccc cat agg tct att ttc caa caa aat ccc tct ttt cta agt ccc      96
   Thr Pro His Arg Ser Ile Phe Gln Gln Asn Pro Ser Phe Leu Ser Pro
               20                  25                  30
   acc acc aaa aaa aaa tca aga aaa tgt ctt ctt aga aac aaa agt agt     144
   Thr Thr Lys Lys Lys Ser Arg Lys Cys Leu Leu Arg Asn Lys Ser Ser
           35                  40                  45
   aaa ctt ttt tgt agc ttt ctt gat tta gca ccc aca tca aag cca gag        192
   Lys Leu Phe Cys Ser Phe Leu Asp Leu Ala Pro Thr Ser Lys Pro Glu
       50                  55                  60
   tct tta gat gtt aac atc tca tgg gtt gat cct aat tcg aat cgg gct        240
   Ser Leu Asp Val Asn Ile Ser Trp Val Asp Pro Asn Ser Asn Arg Ala
   65                  70                  75                  80
   caa ttc gac gtg atc att atc gga gct ggc cct gct ggg ctc agg cta        288
   Gln Phe Asp Val Ile Ile Ile Gly Ala Gly Pro Ala Gly Leu Arg Leu
                   85                  90                  95
   gct gaa caa gtt tct aaa tat ggt att aag gta tgt tgt gtt gac cct        336
   Ala Glu Gln Val Ser Lys Tyr Gly Ile Lys Val Cys Cys Val Asp Pro
               100                 105                 110
   tca cca ctc tcc atg tgg cca aat aat tat ggt gtt tgg gtt gat gag        384
   Ser Pro Leu Ser Met Trp Pro Asn Asn Tyr Gly Val Trp Val Asp Glu
           115                 120                 125
   ttt gag aat tta gga ctg gaa aat tgt tta gat cat aaa tgg cct atg        432
   Phe Glu Asn Leu Gly Leu Glu Asn Cys Leu Asp His Lys Trp Pro Met
       130                 135                 140
   act tgt gtg cat ata aat gat aac aaa act aag tat ttg gga aga cca        480
   Thr Cys Val His Ile Asn Asp Asn Lys Thr Lys Tyr Leu Gly Arg Pro
   145                 150                 155                 160
   tat ggt aga gtt agt aga aag aag ctg aag ttg aaa ttg ttg aat agt        528
   Tyr Gly Arg Val Ser Arg Lys Lys Leu Lys Leu Lys Leu Leu Asn Ser
                   165                 170                 175
   tgt gtt gag aac aga gtg aag ttt tat aaa gct aag gtt tgg aaa gtg        576
   Cys Val Glu Asn Arg Val Lys Phe Tyr Lys Ala Lys Val Trp Lys Val
               180                 185                 190
   gaa cat gaa gaa ttt gag tct tca att gtt tgt gat gat ggt aag aag        624
   Glu His Glu Glu Phe Glu Ser Ser Ile Val Cys Asp Asp Gly Lys Lys
           195                 200                 205
   ata aga ggt agt ttg gtt gtg gat gca agt ggt ttt gct agt gat ttt        672
   Ile Arg Gly Ser Leu Val Val Asp Ala Ser Gly Phe Ala Ser Asp Phe
       210                 215                 220
   ata gag tat gac agg cca aga aac cat ggt tat caa att gct cat ggg        720
   Ile Glu Tyr Asp Arg Pro Arg Asn His Gly Tyr Gln Ile Ala His Gly
   225                 230                 235                 240
   gtt tta gta gaa gtt gat aat cat cca ttt gat ttg gat aaa atg gtg        768
   Val Leu Val Glu Val Asp Asn His Pro Phe Asp Leu Asp Lys Met Val
                   245                 250                 255
   ctt atg gat tgg agg gat tct cat ttg ggt aat gag cca tat tta agg       816
   Leu Met Asp Trp Arg Asp Ser His Leu Gly Asn Glu Pro Tyr Leu Arg
               260                 265                 270
   gtg aat aat gct aaa gaa cca aca ttc ttg tat gca atg cca ttt gat       864
   Val Asn Asn Ala Lys Glu Pro Thr Phe Leu Tyr Ala Met Pro Phe Asp
           275                 280                 285
   aga gat ttg gtt ttc ttg gaa gag act tct ttg gtg agt cgt cct gtt       912
   Arg Asp Leu Val Phe Leu Glu Glu Thr Ser Leu Val Ser Arg Pro Val
       290                 295                 300
   tta tcg tat atg gaa gta aaa aga agg atg gtg gca aga tta agg cat       960
   Leu Ser Tyr Met Glu Val Lys Arg Arg Met Val Ala Arg Leu Arg His
   305                 310                 315                 320
   ttg ggg atc aaa gtg aaa agt gtt att gag gaa gag aaa tgt gtg atc      1008
   Leu Gly Ile Lys Val Lys Ser Val Ile Glu Glu Glu Lys Cys Val Ile
                   325                 330                 335
   cct atg gga gga cca ctt ccg cgg att cct caa aat gtt atg gct att      1056
   Pro Met Gly Gly Pro Leu Pro Arg Ile Pro Gln Asn Val Met Ala Ile
               340                 345                 350
   ggt ggg aat tca ggg ata gtt cat cca tca aca ggg tac atg gtg gct      1104
   Gly Gly Asn Ser Gly Ile Val His Pro Ser Thr Gly Tyr Met Val Ala
           355                 360                 365
   agg agc atg gct tta gca cca gta cta gct gaa gcc atc gtc gag ggg      1152
   Arg Ser Met Ala Leu Ala Pro Val Leu Ala Glu Ala Ile Val Glu Gly
       370                 375                 380
   ctt ggc tca aca aga atg ata aga ggg tct caa ctt tac cat aga gtt      1200
   Leu Gly Ser Thr Arg Met Ile Arg Gly Ser Gln Leu Tyr His Arg Val
   385                 390                 395                 400
   tgg aat ggt ttg tgg cct ttg gat aga aga tgt gtt aga gaa tgt tat      1248
   Trp Asn Gly Leu Trp Pro Leu Asp Arg Arg Cys Val Arg Glu Cys Tyr
                   405                 410                 415
   tca ttt ggg atg gag aca ttg ttg aag ctt gat ttg aaa ggg act agg      1296
   Ser Phe Gly Met Glu Thr Leu Leu Lys Leu Asp Leu Lys Gly Thr Arg
               420                 425                 430
   aga ttg ttt gac gct ttc ttt gat ctt gat cct aaa tac tgg caa ggg      1344
   Arg Leu Phe Asp Ala Phe Phe Asp Leu Asp Pro Lys Tyr Trp Gln Gly
           435                 440                 445
   ttc ctt tct tca aga ttg tct gtc aaa gaa ctt ggt tta ctc agc ttg      1392
   Phe Leu Ser Ser Arg Leu Ser Val Lys Glu Leu Gly Leu Leu Ser Leu
       450                 455                 460
   tgt ctt ttc gga cat ggc tca aac atg act agg ttg gat att gtt aca      1440
   Cys Leu Phe Gly His Gly Ser Asn Met Thr Arg Leu Asp Ile Val Thr
   465                 470                 475                 480
   aaa tgt cct ctt cct ttg gtt aga ctg att ggc aat cta gca ata gag      1488
   Lys Cys Pro Leu Pro Leu Val Arg Leu Ile Gly Asn Leu Ala Ile Glu
                   485                 490                 495
   agc ctt tgaatgtgaa aagtttgaat cattttcttc attttaattt ctttgattat      1544
   Ser Leu
   tttcatattt tctcaattgc aaaagtgaga taagagctac atactgtcaa caaataaact   1604
   actattggaa agttaaaata tgtgtttgtt gtatgttatt ctaatggaat ggattttgta   1664
   aa                                                                   1666
<210> 108
   <211> 498
   <212> PRT
   <213> Lycopersicon esculentum
<400> 108
   Met Glu Ala Leu Leu Lys Pro Phe Pro Ser Leu Leu Leu Ser Ser Pro
   1               5                   10                  15
   Thr Pro His Arg Ser Ile Phe Gln Gln Asn Pro Ser Phe Leu Ser Pro
               20                  25                  30
   Thr Thr Lys Lys Lys Ser Arg Lys Cys Leu Leu Arg Asn Lys Ser Ser
           35                  40                  45
   Lys Leu Phe Cys Ser Phe Leu Asp Leu Ala Pro Thr Ser Lys Pro Glu
       50                  55                  60
   Ser Leu Asp Val Asn Ile Ser Trp Val Asp Pro Asn Ser Asn Arg Ala
   65                  70                  75                  80
   Gln Phe Asp Val Ile Ile Ile Gly Ala Gly Pro Ala Gly Leu Arg Leu
                   85                  90                  95
   Ala Glu Gln Val Ser Lys Tyr Gly Ile Lys Val Cys Cys Val Asp Pro
   100 105 110
   Ser Pro Leu Ser Met Trp Pro Asn Asn Tyr Gly Val Trp Val Asp Glu
   115 120 125
   Phe Glu Asn Leu Gly Leu Glu Asn Cys Leu Asp His Lys Trp Pro Met
   130 135 140
   Thr Cys Val His Ile Asn Asp Asn Lys Thr Lys Tyr Leu Gly Arg Pro
   145 150 155 160
   Tyr Gly Arg Val Ser Arg Lys Lys Leu Lys Leu Lys Leu Leu Asn Ser
   165 170 175
   Cys Val Glu Asn Arg Val Lys Phe Tyr Lys Ala Lys Val Trp Lys Val
   180 185 190
   Glu His Glu Glu Phe Glu Ser Ser Ile Val Cys Asp Asp Gly Lys Lys
   195 200 205
   Ile Arg Gly Ser Leu Val Val Asp Ala Ser Gly Phe Ala Ser Asp Phe
   210 215 220
   Ile Glu Tyr Asp Arg Pro Arg Asn His Gly Tyr Gln Ile Ala His Gly
   225 230 235 240
   Val Leu Val Glu Val Asp Asn His Pro Phe Asp Leu Asp Lys Met Val
   245 250 255
   Leu Met Asp Trp Arg Asp Ser His Leu Gly Asn Glu Pro Tyr Leu Arg
   260 265 270
   Val Asn Asn Ala Lys Glu Pro Thr Phe Leu Tyr Ala Met Pro Phe Asp
   275 280 285
   Arg Asp Leu Val Phe Leu Glu Glu Thr Ser Leu Val Ser Arg Pro Val
   290 295 300
   Leu Ser Tyr Met Glu Val Lys Arg Arg Met Val Ala Arg Leu Arg His
   305 310 315 320
   Leu Gly Ile Lys Val Lys Ser Val Ile Glu Glu Glu Lys Cys Val Ile
   325 330 335
   Pro Met Gly Gly Pro Leu Pro Arg Ile Pro Gln Asn Val Met Ala Ile
   340 345 350
   Gly Gly Asn Ser Gly Ile Val His Pro Ser Thr Gly Tyr Met Val Ala
   355 360 365
   Arg Ser Met Ala Leu Ala Pro Val Leu Ala Glu Ala Ile Val Glu Gly
   370 375 380
   Leu Gly Ser Thr Arg Met Ile Arg Gly Ser Gln Leu Tyr His Arg Val
   385 390 395 400
   Trp Asn Gly Leu Trp Pro Leu Asp Arg Arg Cys Val Arg Glu Cys Tyr
   405 410 415
   Ser Phe Gly Met Glu Thr Leu Leu Lys Leu Asp Leu Lys Gly Thr Arg
   420 425 430
   Arg Leu Phe Asp Ala Phe Phe Asp Leu Asp Pro Lys Tyr Trp Gln Gly
   435 440 445
   Phe Leu Ser Ser Arg Leu Ser Val Lys Glu Leu Gly Leu Leu Ser Leu
   450 455 460
   Cys Leu Phe Gly His Gly Ser Asn Met Thr Arg Leu Asp Ile Val Thr
   465 470 475 480
   Lys Cys Pro Leu Pro Leu Val Arg Leu Ile Gly Asn Leu Ala Ile Glu
   485 490 495
   Ser Leu
<210> 109
   <211> 1125
   <212> DNA
   <213> Lycopersicon esculentum
<220>
   <221> CDS
   <222> (20)..(946)
   <223>
<400> 109
   ttggtcatct ccacaatca atg gct gcc gcc gcc aga atc tcc gcc tcc tct      52
                        Met Ala Ala Ala Ala Arg Ile Ser Ala Ser Ser
                        1               5                   10
   acc tca cga act ttt tat ttc cgt cat tca ccg ttt ctt ggc cca aaa      100
   Thr Ser Arg Thr Phe Tyr Phe Arg His Ser Pro Phe Leu Gly Pro Lys
               15                  20                  25
   cct act tcg aca acc tca cat gtt tct cca atc tct cct ttt tct ctt      148
   Pro Thr Ser Thr Thr Ser His Val Ser Pro Ile Ser Pro Phe Ser Leu
           30                  35                  40
   aat cta ggc cca att ttg agg tct aga aga aaa ccc agt ttc act gtt      196
   Asn Leu Gly Pro Ile Leu Arg Ser Arg Arg Lys Pro Ser Phe Thr Val
       45                  50                  55
   tgc ttt gtt ctc gag gat gag aag ctg aaa cct caa ttt gac gat gag      244
   Cys Phe Val Leu Glu Asp Glu Lys Leu Lys Pro Gln Phe Asp Asp Glu
   60                  65                  70                  75
   gct gag gat ttt gaa aag aag att gag gaa cag atc tta gct act cgc      292
   Ala Glu Asp Phe Glu Lys Lys Ile Glu Glu Gln Ile Leu Ala Thr Arg
                   80                  85                  90
   ttg gcg gag aaa ctg gct agg aag aaa tcg gag agg ttt act tat ctt      340
   Leu Ala Glu Lys Leu Ala Arg Lys Lys Ser Glu Arg Phe Thr Tyr Leu
               95                  100                 105
   gtg gct gct ata atg tct agt ttt ggg att act tct atg gct gtt atg      388
   Val Ala Ala Ile Met Ser Ser Phe Gly Ile Thr Ser Met Ala Val Met
           110                 115                 120
   gct gtt tat tac aga ttt tcg tgg caa atg gag gga gga gaa gtt cct      436
   Ala Val Tyr Tyr Arg Phe Ser Trp Gln Met Glu Gly Gly Glu Val Pro
       125                 130                 135
   gta acc gaa atg ttg ggt aca ttt gct ctc tct gtt ggt gct gct gta      484
   Val Thr Glu Met Leu Gly Thr Phe Ala Leu Ser Val Gly Ala Ala Val
   140                 145                 150                 155
   gga atg gag ttt tgg gcg aga tgg gca cac aaa gca ctg tgg cat gct      532
   Gly Met Glu Phe Trp Ala Arg Trp Ala His Lys Ala Leu Trp His Ala
                   160                 165                 170
   tca cta tgg cac atg cat gag tca cac cac aaa cca aga gaa gga cct      580
   Ser Leu Trp His Met His Glu Ser His His Lys Pro Arg Glu Gly Pro
               175                 180                 185
   ttt gag ctg aac gac gtt ttc gcc ata aca aac gct gtt cca gca ata      628
   Phe Glu Leu Asn Asp Val Phe Ala Ile Thr Asn Ala Val Pro Ala Ile
           190                 195                 200
   gcc ctc ctc aac tat ggt ttc ttc cat aaa ggc ctc att gcc gga cta      676
   Ala Leu Leu Asn Tyr Gly Phe Phe His Lys Gly Leu Ile Ala Gly Leu
       205                 210                 215
   tgc ttc ggt gct ggg cta ggg atc aca gta ttt gga atg gca tac atg      724
   Cys Phe Gly Ala Gly Leu Gly Ile Thr Val Phe Gly Met Ala Tyr Met
   220                 225                 230                 235
   ttt gtt cac gat ggt ttg gtt cac aag aga ttc cca gtt gga cct gta      772
   Phe Val His Asp Gly Leu Val His Lys Arg Phe Pro Val Gly Pro Val
                   240                 245                 250
   gcc aat gta cct tat ctt agg aag gtg gct gct gct cat tcg ctt cat      820
   Ala Asn Val Pro Tyr Leu Arg Lys Val Ala Ala Ala His Ser Leu His
               255                 260                 265
   cac tca gag aag ttc aat ggt gtc cca tat ggc ttg ttc ttc gga cct      868
   His Ser Glu Lys Phe Asn Gly Val Pro Tyr Gly Leu Phe Phe Gly Pro
           270                 275                 280
   aag gaa ctg gaa gaa gta gga ggg acg gaa gag ttg gaa aag gaa gtg      916
   Lys Glu Leu Glu Glu Val Gly Gly Thr Glu Glu Leu Glu Lys Glu Val
       285                 290                 295
   ata cga agg acg aga ctt tcg aaa gga tca tgaacgattg ttcataaaca        966
   Ile Arg Arg Thr Arg Leu Ser Lys Gly Ser
   300                 305
   tagaatgtca ttttacactt cttatcaatg aggaagggtg atttttgatg tatttgatag   1026
   tagagaaaaa tgtagctctc ttgatgaaat gaatttgtat ttatgtaggc tcttcttatt   1086
   cagtaagatt ttttcttttt tttgatctcg tgccgaatt                          1125
<210> 110
   <211> 309
   <212> PRT
   <213> Lycopersicon esculentum
<400> 110
   Met Ala Ala Ala Ala Arg Ile Ser Ala Ser Ser Thr Ser Arg Thr Phe
   1               5                   10                  15
   Tyr Phe Arg His Ser Pro Phe Leu Gly Pro Lys Pro Thr Ser Thr Thr
               20                  25                  30
   Ser His Val Ser Pro Ile Ser Pro Phe Ser Leu Asn Leu Gly Pro Ile
           35                  40                  45
   Leu Arg Ser Arg Arg Lys Pro Ser Phe Thr Val Cys Phe Val Leu Glu
       50                  55                  60
   Asp Glu Lys Leu Lys Pro Gln Phe Asp Asp Glu Ala Glu Asp Phe Glu
   65                  70                  75                  80
   Lys Lys Ile Glu Glu Gln Ile Leu Ala Thr Arg Leu Ala Glu Lys Leu
                   85                  90                  95
   Ala Arg Lys Lys Ser Glu Arg Phe Thr Tyr Leu Val Ala Ala Ile Met
               100                 105                 110
   Ser Ser Phe Gly Ile Thr Ser Met Ala Val Met Ala Val Tyr Tyr Arg
           115                 120                 125
   Phe Ser Trp Gln Met Glu Gly Gly Glu Val Pro Val Thr Glu Met Leu
       130                 135                 140
   Gly Thr Phe Ala Leu Ser Val Gly Ala Ala Val Gly Met Glu Phe Trp
   145                 150                 155                 160
   Ala Arg Trp Ala His Lys Ala Leu Trp His Ala Ser Leu Trp His Met
                   165                 170                 175
   His Glu Ser His His Lys Pro Arg Glu Gly Pro Phe Glu Leu Asn Asp
               180                 185                 190
   Val Phe Ala Ile Thr Asn Ala Val Pro Ala Ile Ala Leu Leu Asn Tyr
   195 200 205
   Gly Phe Phe His Lys Gly Leu Ile Ala Gly Leu Cys Phe Gly Ala Gly
   210 215 220
   Leu Gly Ile Thr Val Phe Gly Met Ala Tyr Met Phe Val His Asp Gly
   225 230 235 240
   Leu Val His Lys Arg Phe Pro Val Gly Pro Val Ala Asn Val Pro Tyr
   245 250 255
   Leu Arg Lys Val Ala Ala Ala His Ser Leu His His Ser Glu Lys Phe
   260 265 270
   Asn Gly Val Pro Tyr Gly Leu Phe Phe Gly Pro Lys Glu Leu Glu Glu
   275 280 285
   Val Gly Gly Thr Glu Glu Leu Glu Lys Glu Val Ile Arg Arg Thr Arg
   290 295 300
   Leu Ser Lys Gly Ser
   305
<210> 111
   <211> 1779
   <212> DNA
   <213> Arabidopsis thaliana
<220>
   <221> CDS
   <222> (1)..(1779)
   <223>
<400> 111
   atg gat ctc cgt cgg agg cct cct aaa cca ccg gtt acc aac aac aac      48
   Met Asp Leu Arg Arg Arg Pro Pro Lys Pro Pro Val Thr Asn Asn Asn
   1               5                   10                  15
   aac tcc aac gga tct ttc cgt tct tat cag cct cgc act tcc gat gac      96
   Asn Ser Asn Gly Ser Phe Arg Ser Tyr Gln Pro Arg Thr Ser Asp Asp
               20                  25                  30
   gat cat cgt cgc cgg gct aca aca att gct cct cca ccg aaa gca tcc      144
   Asp His Arg Arg Arg Ala Thr Thr Ile Ala Pro Pro Pro Lys Ala Ser
           35                  40                  45
   gac gcg ctt cct ctt ccg tta tat ctc aca aac gcc gtt ttc ttc acg      192
   Asp Ala Leu Pro Leu Pro Leu Tyr Leu Thr Asn Ala Val Phe Phe Thr
       50                  55                  60
   ctc ttc ttc tcc gtc gcg tat tac ctc ctc cac cgg tgg cgt gac aag      240
   Leu Phe Phe Ser Val Ala Tyr Tyr Leu Leu His Arg Trp Arg Asp Lys
   65                  70                  75                  80
   atc cgt tac aat acg cct ctt cac gtc gtc act atc aca gaa ctc ggc      288
   Ile Arg Tyr Asn Thr Pro Leu His Val Val Thr Ile Thr Glu Leu Gly
                   85                  90                  95
   gcc att att gct ctc atc gct tcg ttt atc tat ctc cta ggg ttt ttt      336
   Ala Ile Ile Ala Leu Ile Ala Ser Phe Ile Tyr Leu Leu Gly Phe Phe
               100                 105                 110
   ggt att gac ttt gtt cag tca ttt atc tca cgt gcc tct ggt gat gct      384
   Gly Ile Asp Phe Val Gln Ser Phe Ile Ser Arg Ala Ser Gly Asp Ala
           115                 120                 125
   tgg gat ctc gcc gat acg atc gat gat gat gac cac cgc ctt gtc acg      432
   Trp Asp Leu Ala Asp Thr Ile Asp Asp Asp Asp His Arg Leu Val Thr
       130                 135                 140
   tgc tct cca ccg act ccg atc gtt tcc gtt gct aaa tta cct aat ccg      480
   Cys Ser Pro Pro Thr Pro Ile Val Ser Val Ala Lys Leu Pro Asn Pro
   145                 150                 155                 160
   gaa cct att gtt acc gaa tcg ctt cct gag gaa gac gag gag att gtg      528
   Glu Pro Ile Val Thr Glu Ser Leu Pro Glu Glu Asp Glu Glu Ile Val
                   165                 170                 175
   aaa tcg gtt atc gac gga gtt att cca tcg tac tcg ctt gaa tct cgt      576
   Lys Ser Val Ile Asp Gly Val Ile Pro Ser Tyr Ser Leu Glu Ser Arg
               180                 185                 190
   ctc ggt gat tgc aaa aga gcg gcg tcg att cgt cgt gag gcg ttg cag      624
   Leu Gly Asp Cys Lys Arg Ala Ala Ser Ile Arg Arg Glu Ala Leu Gln
           195                 200                 205
   aga gtc acc ggg aga tcg att gaa ggg tta ccg ttg gat gga ttt gat      672
   Arg Val Thr Gly Arg Ser Ile Glu Gly Leu Pro Leu Asp Gly Phe Asp
       210                 215                 220
   tat gaa tcg att ttg ggg caa tgc tgt gag atg cct gtt gga tac att      720
   Tyr Glu Ser Ile Leu Gly Gln Cys Cys Glu Met Pro Val Gly Tyr Ile
   225                 230                 235                 240
   cag att cct gtt ggg att gct ggt cca ttg ttg ctt gat ggt tat gag      768
   Gln Ile Pro Val Gly Ile Ala Gly Pro Leu Leu Leu Asp Gly Tyr Glu
                   245                 250                 255
   tac tct gtt cct atg gct aca acc gaa ggt tgt ttg gtt gct agc act      816
   Tyr Ser Val Pro Met Ala Thr Thr Glu Gly Cys Leu Val Ala Ser Thr
               260                 265                 270
   aac aga ggc tgc aag gct atg ttt atc tct ggt ggc gcc acc agt acc      864
   Asn Arg Gly Cys Lys Ala Met Phe Ile Ser Gly Gly Ala Thr Ser Thr
           275                 280                 285
   gtt ctt aag gac ggt atg acc cga gca cct gtt gtt cgg ttc gct tcg      912
   Val Leu Lys Asp Gly Met Thr Arg Ala Pro Val Val Arg Phe Ala Ser
       290                 295                 300
   gcg aga cga gct tcg gag ctt aag ttt ttc ttg gag aat cca gag aac      960
   Ala Arg Arg Ala Ser Glu Leu Lys Phe Phe Leu Glu Asn Pro Glu Asn
   305                 310                 315                 320
   ttt gat act ttg gca gta gtc ttc aac agg tcg agt aga ttt gca aga     1008
   Phe Asp Thr Leu Ala Val Val Phe Asn Arg Ser Ser Arg Phe Ala Arg
                   325                 330                 335
   ctg caa agt gtt aaa tgc aca atc gcg ggg aag aat gct tat gta agg     1056
   Leu Gln Ser Val Lys Cys Thr Ile Ala Gly Lys Asn Ala Tyr Val Arg
               340                 345                 350
   ttc tgt tgt agt act ggt gat gct atg ggg atg aat atg gtt tct aaa     1104
   Phe Cys Cys Ser Thr Gly Asp Ala Met Gly Met Asn Met Val Ser Lys
           355                 360                 365
   ggt gtg cag aat gtt ctt gag tat ctt acc gat gat ttc cct gac atg     1152
   Gly Val Gln Asn Val Leu Glu Tyr Leu Thr Asp Asp Phe Pro Asp Met
       370                 375                 380
   gat gtg att gga atc tct ggt aac ttc tgt tcg gac aag aaa cct gct     1200
   Asp Val Ile Gly Ile Ser Gly Asn Phe Cys Ser Asp Lys Lys Pro Ala
   385                 390                 395                 400
   gct gtg aac tgg att gag gga cgt ggt aaa tca gtt gtt tgc gag gct     1248
   Ala Val Asn Trp Ile Glu Gly Arg Gly Lys Ser Val Val Cys Glu Ala
                   405                 410                 415
   gta atc aga gga gag atc gtg aac aag gtc ttg aaa acg agc gtg gct     1296
   Val Ile Arg Gly Glu Ile Val Asn Lys Val Leu Lys Thr Ser Val Ala
               420                 425                 430
   gct tta gtc gag ctc aac atg ctc aag aac cta gct ggc tct gct gtt      1344
   Ala Leu Val Glu Leu Asn Met Leu Lys Asn Leu Ala Gly Ser Ala Val
           435                 440                 445
   gca ggc tct cta ggt gga ttc aac gct cat gcc agt aac ata gtg tct      1392
   Ala Gly Ser Leu Gly Gly Phe Asn Ala His Ala Ser Asn Ile Val Ser
       450                 455                 460
   gct gta ttc ata gct act ggc caa gat cca gct caa aac gtg gag agt      1440
   Ala Val Phe Ile Ala Thr Gly Gln Asp Pro Ala Gln Asn Val Glu Ser
   465                 470                 475                 480
   tct caa tgc atc acc atg atg gaa gct att aat gac ggc aaa gat atc      1488
   Ser Gln Cys Ile Thr Met Met Glu Ala Ile Asn Asp Gly Lys Asp Ile
                   485                 490                 495
   cat atc tca gtc act atg cca tct atc gag gtg ggg aca gtg gga gga      1536
   His Ile Ser Val Thr Met Pro Ser Ile Glu Val Gly Thr Val Gly Gly
               500                 505                 510
   gga aca cag ctt gca tct caa tca gcg tgt tta aac ctg ctc gga gtt      1584
   Gly Thr Gln Leu Ala Ser Gln Ser Ala Cys Leu Asn Leu Leu Gly Val
           515                 520                 525
   aaa gga gca agc aca gag tcg ccg gga atg aac gca agg agg cta gcg      1632
   Lys Gly Ala Ser Thr Glu Ser Pro Gly Met Asn Ala Arg Arg Leu Ala
       530                 535                 540
   acg atc gta gcc gga gca gtt tta gct gga gag tta tct tta atg tca      1680
   Thr Ile Val Ala Gly Ala Val Leu Ala Gly Glu Leu Ser Leu Met Ser
   545                 550                 555                 560
   gca att gca gct gga cag ctt gtg aga agt cac atg aaa tac aat aga      1728
   Ala Ile Ala Ala Gly Gln Leu Val Arg Ser His Met Lys Tyr Asn Arg
                   565                 570                 575
   tcc agc cga gac atc tct gga gca acg aca acg aca aca aca aca aca      1776
   Ser Ser Arg Asp Ile Ser Gly Ala Thr Thr Thr Thr Thr Thr Thr Thr
               580                 585                 590
   tga                                                                  1779
<210> 112
   <211> 592
   <212> PRT
   <213> Arabidopsis thaliana
<400> 112
   Met Asp Leu Arg Arg Arg Pro Pro Lys Pro Pro Val Thr Asn Asn Asn
   1               5                   10                  15
   Asn Ser Asn Gly Ser Phe Arg Ser Tyr Gln Pro Arg Thr Ser Asp Asp
               20                  25                  30
   Asp His Arg Arg Arg Ala Thr Thr Ile Ala Pro Pro Pro Lys Ala Ser
           35                  40                  45
   Asp Ala Leu Pro Leu Pro Leu Tyr Leu Thr Asn Ala Val Phe Phe Thr
       50                  55                  60
   Leu Phe Phe Ser Val Ala Tyr Tyr Leu Leu His Arg Trp Arg Asp Lys
   65                  70                  75                  80
   Ile Arg Tyr Asn Thr Pro Leu His Val Val Thr Ile Thr Glu Leu Gly
                   85                  90                  95
   Ala Ile Ile Ala Leu Ile Ala Ser Phe Ile Tyr Leu Leu Gly Phe Phe
               100                 105                 110
   Gly Ile Asp Phe Val Gln Ser Phe Ile Ser Arg Ala Ser Gly Asp Ala
           115                 120                 125
   Trp Asp Leu Ala Asp Thr Ile Asp Asp Asp Asp His Arg Leu Val Thr
       130                 135                 140
   Cys Ser Pro Pro Thr Pro Ile Val Ser Val Ala Lys Leu Pro Asn Pro
   145                 150                 155                 160
   Glu Pro Ile Val Thr Glu Ser Leu Pro Glu Glu Asp Glu Glu Ile Val
                   165                 170                 175
   Lys Ser Val Ile Asp Gly Val Ile Pro Ser Tyr Ser Leu Glu Ser Arg
               180                 185                 190
   Leu Gly Asp Cys Lys Arg Ala Ala Ser Ile Arg Arg Glu Ala Leu Gln
           195                 200                 205
   Arg Val Thr Gly Arg Ser Ile Glu Gly Leu Pro Leu Asp Gly Phe Asp
       210                 215                 220
   Tyr Glu Ser Ile Leu Gly Gln Cys Cys Glu Met Pro Val Gly Tyr Ile
   225                 230                 235                 240
   Gln Ile Pro Val Gly Ile Ala Gly Pro Leu Leu Leu Asp Gly Tyr Glu
                   245                 250                 255
   Tyr Ser Val Pro Met Ala Thr Thr Glu Gly Cys Leu Val Ala Ser Thr
               260                 265                 270
   Asn Arg Gly Cys Lys Ala Met Phe Ile Ser Gly Gly Ala Thr Ser Thr
           275                 280                 285
   Val Leu Lys Asp Gly Met Thr Arg Ala Pro Val Val Arg Phe Ala Ser
       290                 295                 300
   Ala Arg Arg Ala Ser Glu Leu Lys Phe Phe Leu Glu Asn Pro Glu Asn
   305                 310                 315                 320
   Phe Asp Thr Leu Ala Val Val Phe Asn Arg Ser Ser Arg Phe Ala Arg
                   325                 330                 335
   Leu Gln Ser Val Lys Cys Thr Ile Ala Gly Lys Asn Ala Tyr Val Arg
               340                 345                 350
   Phe Cys Cys Ser Thr Gly Asp Ala Met Gly Met Asn Met Val Ser Lys
           355                 360                 365
   Gly Val Gln Asn Val Leu Glu Tyr Leu Thr Asp Asp Phe Pro Asp Met
       370                 375                 380
   Asp Val Ile Gly Ile Ser Gly Asn Phe Cys Ser Asp Lys Lys Pro Ala
   385                 390                 395                 400
   Ala Val Asn Trp Ile Glu Gly Arg Gly Lys Ser Val Val Cys Glu Ala
                   405                 410                 415
   Val Ile Arg Gly Glu Ile Val Asn Lys Val Leu Lys Thr Ser Val Ala
               420                 425                 430
   Ala Leu Val Glu Leu Asn Met Leu Lys Asn Leu Ala Gly Ser Ala Val
           435                 440                 445
   Ala Gly Ser Leu Gly Gly Phe Asn Ala His Ala Ser Asn Ile Val Ser
       450                 455                 460
   Ala Val Phe Ile Ala Thr Gly Gln Asp Pro Ala Gln Asn Val Glu Ser
   465                 470                 475                 480
   Ser Gln Cys Ile Thr Met Met Glu Ala Ile Asn Asp Gly Lys Asp Ile
                   485                 490                 495
   His Ile Ser Val Thr Met Pro Ser Ile Glu Val Gly Thr Val Gly Gly
               500                 505                 510
   Gly Thr Gln Leu Ala Ser Gln Ser Ala Cys Leu Asn Leu Leu Gly Val
           515                 520                 525
   Lys Gly Ala Ser Thr Glu Ser Pro Gly Met Asn Ala Arg Arg Leu Ala
       530                 535                 540
   Thr Ile Val Ala Gly Ala Val Leu Ala Gly Glu Leu Ser Leu Met Ser
   545                 550                 555                 560
   Ala Ile Ala Ala Gly Gln Leu Val Arg Ser His Met Lys Tyr Asn Arg
                   565                 570                 575
   Ser Ser Arg Asp Ile Ser Gly Ala Thr Thr Thr Thr Thr Thr Thr Thr
               580                 585                 590
<210> 113
   <211> 1401
   <212> DNA
   <213> Arabidopsis thaliana ISPH
<220>
   <221> CDS
   <222> (1)..(1401)
   <223>
<400> 113
   atg gct gtt gcg ctc caa ttc agc cga tta tgc gtt cga ccg gat act       48
   Met Ala Val Ala Leu Gln Phe Ser Arg Leu Cys Val Arg Pro Asp Thr
   1               5                   10                  15
   ttc gtg cgg gag aat cat ctc tct gga tcc gga tct ctc cgc cgc cgg       96
   Phe Val Arg Glu Asn His Leu Ser Gly Ser Gly Ser Leu Arg Arg Arg
               20                  25                  30
   aaa gct tta tca gtc cgg tgc tcg tct ggc gat gag aac gct cct tcg      144
   Lys Ala Leu Ser Val Arg Cys Ser Ser Gly Asp Glu Asn Ala Pro Ser
           35                  40                  45
   cca tcg gtg gtg atg gac tcc gat ttc gac gcc aag gtg ttc cgt aag      192
   Pro Ser Val Val Met Asp Ser Asp Phe Asp Ala Lys Val Phe Arg Lys
       50                  55                  60
   aac ttg acg aga agc gat aat tac aat cgt aaa ggg ttc ggt cat aag      240
   Asn Leu Thr Arg Ser Asp Asn Tyr Asn Arg Lys Gly Phe Gly His Lys
   65                  70                  75                  80
   gag gag aca ctc aag ctc atg aat cga gag tac acc agt gat ata ttg      288
   Glu Glu Thr Leu Lys Leu Met Asn Arg Glu Tyr Thr Ser Asp Ile Leu
                   85                  90                  95
   gag aca ctg aaa aca aat ggg tat act tat tct tgg gga gat gtt act      336
   Glu Thr Leu Lys Thr Asn Gly Tyr Thr Tyr Ser Trp Gly Asp Val Thr
               100                 105                 110
   gtg aaa ctc gct aaa gca tat ggt ttt tgc tgg ggt gtt gag cgt gct      384
   Val Lys Leu Ala Lys Ala Tyr Gly Phe Cys Trp Gly Val Glu Arg Ala
           115                 120                 125
   gtt cag att gca tat gaa gca cga aag cag ttt cca gag gag agg ctt      432
   Val Gln Ile Ala Tyr Glu Ala Arg Lys Gln Phe Pro Glu Glu Arg Leu
       130                 135                 140
   tgg att act aac gaa atc att cat aac ccg acc gtc aat aag agg ttg      480
   Trp Ile Thr Asn Glu Ile Ile His Asn Pro Thr Val Asn Lys Arg Leu
   145                 150                 155                 160
   gaa gat atg gat gtt aaa att att ccg gtt gag gat tca aag aaa cag      528
   Glu Asp Met Asp Val Lys Ile Ile Pro Val Glu Asp Ser Lys Lys Gln
                   165                 170                 175
   ttt gat gta gta gag aaa gat gat gtg gtt atc ctt cct gcg ttt gga      576
   Phe Asp Val Val Glu Lys Asp Asp Val Val Ile Leu Pro Ala Phe Gly
               180                 185                 190
   gct ggt gtt gac gag atg tat gtt ctt aat gat aaa aag gtg caa att      624
   Ala Gly Val Asp Glu Met Tyr Val Leu Asn Asp Lys Lys Val Gln Ile
           195                 200                 205
   gtt gac acg act tgt cct tgg gtg aca aag gtc tgg aac acg gtt gag      672
   Val Asp Thr Thr Cys Pro Trp Val Thr Lys Val Trp Asn Thr Val Glu
       210                 215                 220
   aag cac aag aag ggg gaa tac aca tca gta atc cat ggt aaa tat aat      720
   Lys His Lys Lys Gly Glu Tyr Thr Ser Val Ile His Gly Lys Tyr Asn
   225                 230                 235                 240
   cat gaa gag acg att gca act gcg tct ttt gca gga aag tac atc att      768
   His Glu Glu Thr Ile Ala Thr Ala Ser Phe Ala Gly Lys Tyr Ile Ile
                   245                 250                 255
   gta aag aac atg aaa gag gca aat tac gtt tgt gat tac att ctc ggt      816
   Val Lys Asn Met Lys Glu Ala Asn Tyr Val Cys Asp Tyr Ile Leu Gly
               260                 265                 270
   ggc caa tac gat gga tct agc tcc aca aaa gag gag ttc atg gag aaa      864
   Gly Gln Tyr Asp Gly Ser Ser Ser Thr Lys Glu Glu Phe Met Glu Lys
           275                 280                 285
   ttc aaa tac gca att tcg aag ggt ttc gat ccc gac aat gac ctt gtc      912
   Phe Lys Tyr Ala Ile Ser Lys Gly Phe Asp Pro Asp Asn Asp Leu Val
       290                 295                 300
   aaa gtt ggt att gca aac caa aca acg atg cta aag gga gaa aca gag      960
   Lys Val Gly Ile Ala Asn Gln Thr Thr Met Leu Lys Gly Glu Thr Glu
   305                 310                 315                 320
   gag ata gga aga tta ctc gag aca aca atg atg cgc aag tat gga gtg     1008
   Glu Ile Gly Arg Leu Leu Glu Thr Thr Met Met Arg Lys Tyr Gly Val
                   325                 330                 335
   gaa aat gta agc gga cat ttc atc agc ttc aac aca ata tgc gac gct     1056
   Glu Asn Val Ser Gly His Phe Ile Ser Phe Asn Thr Ile Cys Asp Ala
               340                 345                 350
   act caa gag cga caa gac gca atc tat gag cta gtg gaa gag aag att     1104
   Thr Gln Glu Arg Gln Asp Ala Ile Tyr Glu Leu Val Glu Glu Lys Ile
           355                 360                 365
   gac ctc atg cta gtg gtt ggc gga tgg aat tca agt aac acc tct cac     1152
   Asp Leu Met Leu Val Val Gly Gly Trp Asn Ser Ser Asn Thr Ser His
       370                 375                 380
   ctt cag gaa atc tca gag gca cgg gga atc cca tct tac tgg atc gat     1200
   Leu Gln Glu Ile Ser Glu Ala Arg Gly Ile Pro Ser Tyr Trp Ile Asp
   385                 390                 395                 400
   agt gag aaa cgg ata gga cct ggg aat aaa ata gcc tat aag ctc cac      1248
   Ser Glu Lys Arg Ile Gly Pro Gly Asn Lys Ile Ala Tyr Lys Leu His
                   405                 410                 415
   tat gga gaa ctg gtc gag aag gaa aac ttt ctc cca aag gga cca ata      1296
   Tyr Gly Glu Leu Val Glu Lys Glu Asn Phe Leu Pro Lys Gly Pro Ile
               420                 425                 430
   aca atc ggt gtg aca tca ggt gca tca acc ccg gat aag gtc gtg gaa      1344
   Thr Ile Gly Val Thr Ser Gly Ala Ser Thr Pro Asp Lys Val Val Glu
           435                 440                 445
   gat gct ttg gtg aag gtg ttc gac att aaa cgt gaa gag tta ttg cag      1392
   Asp Ala Leu Val Lys Val Phe Asp Ile Lys Arg Glu Glu Leu Leu Gln
       450                 455                 460
   ctg gct tga                                                          1401
   Leu Ala
   465
<210> 114
   <211> 466
   <212> PRT
   <213> Arabidopsis thaliana ISPH
<400> 114
   Met Ala Val Ala Leu Gln Phe Ser Arg Leu Cys Val Arg Pro Asp Thr
   1               5                   10                  15
   Phe Val Arg Glu Asn His Leu Ser Gly Ser Gly Ser Leu Arg Arg Arg
               20                  25                  30
   Lys Ala Leu Ser Val Arg Cys Ser Ser Gly Asp Glu Asn Ala Pro Ser
           35                  40                  45
   Pro Ser Val Val Met Asp Ser Asp Phe Asp Ala Lys Val Phe Arg Lys
       50                  55                  60
   Asn Leu Thr Arg Ser Asp Asn Tyr Asn Arg Lys Gly Phe Gly His Lys
   65                  70                  75                  80
   Glu Glu Thr Leu Lys Leu Met Asn Arg Glu Tyr Thr Ser Asp Ile Leu
   85 90 95
   Glu Thr Leu Lys Thr Asn Gly Tyr Thr Tyr Ser Trp Gly Asp Val Thr
   100 105 110
   Val Lys Leu Ala Lys Ala Tyr Gly Phe Cys Trp Gly Val Glu Arg Ala
   115 120 125
   Val Gln Ile Ala Tyr Glu Ala Arg Lys Gln Phe Pro Glu Glu Arg Leu
   130 135 140
   Trp Ile Thr Asn Glu Ile Ile His Asn Pro Thr Val Asn Lys Arg Leu
   145 150 155 160
   Glu Asp Met Asp Val Lys Ile Ile Pro Val Glu Asp Ser Lys Lys Gln
   165 170 175
   Phe Asp Val Val Glu Lys Asp Asp Val Val Ile Leu Pro Ala Phe Gly
   180 185 190
   Ala Gly Val Asp Glu Met Tyr Val Leu Asn Asp Lys Lys Val Gln Ile
   195 200 205
   Val Asp Thr Thr Cys Pro Trp Val Thr Lys Val Trp Asn Thr Val Glu
   210 215 220
   Lys His Lys Lys Gly Glu Tyr Thr Ser Val Ile His Gly Lys Tyr Asn
   225 230 235 240
   His Glu Glu Thr Ile Ala Thr Ala Ser Phe Ala Gly Lys Tyr Ile Ile
   245 250 255
   Val Lys Asn Met Lys Glu Ala Asn Tyr Val Cys Asp Tyr Ile Leu Gly
   260 265 270
   Gly Gln Tyr Asp Gly Ser Ser Ser Thr Lys Glu Glu Phe Met Glu Lys
   275 280 285
   Phe Lys Tyr Ala Ile Ser Lys Gly Phe Asp Pro Asp Asn Asp Leu Val
   290 295 300
   Lys Val Gly Ile Ala Asn Gln Thr Thr Met Leu Lys Gly Glu Thr Glu
   305 310 315 320
   Glu Ile Gly Arg Leu Leu Glu Thr Thr Met Met Arg Lys Tyr Gly Val
   325 330 335
   Glu Asn Val Ser Gly His Phe Ile Ser Phe Asn Thr Ile Cys Asp Ala
   340 345 350
   Thr Gln Glu Arg Gln Asp Ala Ile Tyr Glu Leu Val Glu Glu Lys Ile
   355 360 365
   Asp Leu Met Leu Val Val Gly Gly Trp Asn Ser Ser Asn Thr Ser His
   370 375 380
   Leu Gln Glu Ile Ser Glu Ala Arg Gly Ile Pro Ser Tyr Trp Ile Asp
   385 390 395 400
   Ser Glu Lys Arg Ile Gly Pro Gly Asn Lys Ile Ala Tyr Lys Leu His
   405 410 415
   Tyr Gly Glu Leu Val Glu Lys Glu Asn Phe Leu Pro Lys Gly Pro Ile
   420 425 430
   Thr Ile Gly Val Thr Ser Gly Ala Ser Thr Pro Asp Lys Val Val Glu
   435 440 445
   Asp Ala Leu Val Lys Val Phe Asp Ile Lys Arg Glu Glu Leu Leu Gln
   450 455 460
   Leu Ala
   465
<210> 115
   <211> 2160
   <212> DNA
   <213> Lycopersicon esculentum
<220>
   <221> CDS
   <222> (1)..(2160)
   <223>
<400> 115
   atg gct ttg tgt gct tat gca ttt cct ggg att ttg aac agg act ggt      48
   Met Ala Leu Cys Ala Tyr Ala Phe Pro Gly Ile Leu Asn Arg Thr Gly
   1               5                   10                  15
   gtg gtt tca gat tct tct aag gca acc cct ttg ttc tct gga tgg att      96
   Val Val Ser Asp Ser Ser Lys Ala Thr Pro Leu Phe Ser Gly Trp Ile
               20                  25                  30
   cat gga aca gat ctg cag ttt ttg ttc caa cac aag ctt act cat gag     144
   His Gly Thr Asp Leu Gln Phe Leu Phe Gln His Lys Leu Thr His Glu
           35                  40                  45
   gtc aag aaa agg tca cgt gtg gtt cag gct tcc tta tca gaa tct gga     192
   Val Lys Lys Arg Ser Arg Val Val Gln Ala Ser Leu Ser Glu Ser Gly
       50                  55                  60
   gaa tac tac aca cag aga ccg cca acg cct att ttg gac act gtg aac     240
   Glu Tyr Tyr Thr Gln Arg Pro Pro Thr Pro Ile Leu Asp Thr Val Asn
   65                  70                  75                  80
   tat ccc att cat atg aaa aat ctg tct ctg aag gaa ctt aaa caa cta     288
   Tyr Pro Ile His Met Lys Asn Leu Ser Leu Lys Glu Leu Lys Gln Leu
                   85                  90                  95
   gca gat gaa cta agg tca gat aca att ttc aat gta tca aag act ggg     336
   Ala Asp Glu Leu Arg Ser Asp Thr Ile Phe Asn Val Ser Lys Thr Gly
               100                 105                 110
   ggt cac ctt ggc tca agt ctt ggt gtt gtt gag ctg act gtt gct ctt     384
   Gly His Leu Gly Ser Ser Leu Gly Val Val Glu Leu Thr Val Ala Leu
           115                 120                 125
   cat tat gtc ttc aat gca ccg caa gat agg att ctc tgg gat gtt ggt     432
   His Tyr Val Phe Asn Ala Pro Gln Asp Arg Ile Leu Trp Asp Val Gly
       130                 135                 140
   cat cag tct tat cct cac aaa atc ttg act ggt aga agg gac aag atg     480
   His Gln Ser Tyr Pro His Lys Ile Leu Thr Gly Arg Arg Asp Lys Met
   145                 150                 155                 160
   tcg aca tta agg cag aca gat ggt ctt gca gga ttt act aag cga tcg     528
   Ser Thr Leu Arg Gln Thr Asp Gly Leu Ala Gly Phe Thr Lys Arg Ser
                   165                 170                 175
   gag agt gaa tat gat tgc ttt ggc acc ggc cac agt tcc acc acc atc      576
   Glu Ser Glu Tyr Asp Cys Phe Gly Thr Gly His Ser Ser Thr Thr Ile
               180                 185                 190
   tca gca ggc cta ggg atg gct gtt ggt aga gat cta aaa gga aga aac      624
   Ser Ala Gly Leu Gly Met Ala Val Gly Arg Asp Leu Lys Gly Arg Asn
           195                 200                 205
   aac aat gtt att gcc gta ata ggt gat ggt gcc atg aca gca ggt caa      672
   Asn Asn Val Ile Ala Val Ile Gly Asp Gly Ala Met Thr Ala Gly Gln
       210                 215                 220
   gct tat gaa gcc atg aat aat gct ggt tac ctg gac tct gac atg att      720
   Ala Tyr Glu Ala Met Asn Asn Ala Gly Tyr Leu Asp Ser Asp Met Ile
   225                 230                 235                 240
   gtt atc tta aac gac aat aga caa gtt tct tta cct act gct act ctg      768
   Val Ile Leu Asn Asp Asn Arg Gln Val Ser Leu Pro Thr Ala Thr Leu
                   245                 250                 255
   gat ggg cca gtt gct cct gtt gga gct cta agt agt gct ttg agc agg      816
   Asp Gly Pro Val Ala Pro Val Gly Ala Leu Ser Ser Ala Leu Ser Arg
               260                 265                 270
   tta cag tct aat agg cct ctc aga gaa cta aga gaa gtc gca aag gga      864
   Leu Gln Ser Asn Arg Pro Leu Arg Glu Leu Arg Glu Val Ala Lys Gly
           275                 280                 285
   gtt act aag cag att ggt ggt cct atg cat gag ctt gct gca aaa gtt      912
   Val Thr Lys Gln Ile Gly Gly Pro Met His Glu Leu Ala Ala Lys Val
       290                 295                 300
   gat gaa tat gct cgt ggc atg att agt ggt tct gga tca aca ttg ttt      960
   Asp Glu Tyr Ala Arg Gly Met Ile Ser Gly Ser Gly Ser Thr Leu Phe
   305                 310                 315                 320
   gaa gaa ctt gga ctt tac tat att ggt cct gtg gat ggt cac aac att     1008
   Glu Glu Leu Gly Leu Tyr Tyr Ile Gly Pro Val Asp Gly His Asn Ile
                   325                 330                 335
   gat gat cta att gcg att ctc aaa gag gtt aga agt act aaa aca aca     1056
   Asp Asp Leu Ile Ala Ile Leu Lys Glu Val Arg Ser Thr Lys Thr Thr
               340                 345                 350
   ggt cca gta ctg atc cat gtt gtc act gag aaa ggc aga ggt tat cca     1104
   Gly Pro Val Leu Ile His Val Val Thr Glu Lys Gly Arg Gly Tyr Pro
           355                 360                 365
   tat gct gag aga gct gca gat aag tat cat gga gtt gcc aag ttt gat     1152
   Tyr Ala Glu Arg Ala Ala Asp Lys Tyr His Gly Val Ala Lys Phe Asp
       370                 375                 380
   cca gca aca gga aag caa ttc aaa gcc agt gcc aag aca cag tcc tat      1200
   Pro Ala Thr Gly Lys Gln Phe Lys Ala Ser Ala Lys Thr Gln Ser Tyr
   385                 390                 395                 400
   aca aca tat ttt gcc gag gct tta att gca gaa gca gaa gca gat aaa      1248
   Thr Thr Tyr Phe Ala Glu Ala Leu Ile Ala Glu Ala Glu Ala Asp Lys
                   405                 410                 415
   gac att gtt gca atc cat gct gcc atg ggg ggt ggg acc gga atg aac      1296
   Asp Ile Val Ala Ile His Ala Ala Met Gly Gly Gly Thr Gly Met Asn
               420                 425                 430
   ctt ttc cat cgt cgc ttc cca aca agg tgt ttt gat gtt gga ata gca      1344
   Leu Phe His Arg Arg Phe Pro Thr Arg Cys Phe Asp Val Gly Ile Ala
           435                 440                 445
   gaa caa cat gca gta acc ttt gct gct gga ttg gct tgt gaa ggc att      1392
   Glu Gln His Ala Val Thr Phe Ala Ala Gly Leu Ala Cys Glu Gly Ile
       450                 455                 460
   aaa cct ttc tgt gca atc tat tcg tct ttc atg cag agg gct tat gac      1440
   Lys Pro Phe Cys Ala Ile Tyr Ser Ser Phe Met Gln Arg Ala Tyr Asp
   465                 470                 475                 480
   cag gta gtg cat gac gtt gat ttg caa aag ctg ccc gtg agg ttt gca      1488
   Gln Val Val His Asp Val Asp Leu Gln Lys Leu Pro Val Arg Phe Ala
                   485                 490                 495
   atg gac aga gca ggt ctt gtt gga gca gat ggt cca aca cat tgt ggt      1536
   Met Asp Arg Ala Gly Leu Val Gly Ala Asp Gly Pro Thr His Cys Gly
               500                 505                 510
   gca ttt gat gtt act tac atg gca tgt ctt cct aac atg gtt gta atg      1584
   Ala Phe Asp Val Thr Tyr Met Ala Cys Leu Pro Asn Met Val Val Met
           515                 520                 525
   gct cct tct gat gaa gcg gag cta ttt cac atg gta gca act gct gcc      1632
   Ala Pro Ser Asp Glu Ala Glu Leu Phe His Met Val Ala Thr Ala Ala
       530                 535                 540
   gcc att gat gac aga cca agt tgt ttt aga tac cca aga gga aat ggg      1680
   Ala Ile Asp Asp Arg Pro Ser Cys Phe Arg Tyr Pro Arg Gly Asn Gly
   545                 550                 555                 560
   atc ggt gta gag ctt ccg gct gga aac aaa gga att cct ctt gag gtt      1728
   Ile Gly Val Glu Leu Pro Ala Gly Asn Lys Gly Ile Pro Leu Glu Val
                   565                 570                 575
   ggt aaa ggt agg ata ttg att gag ggg gag aga gtg gct cta ttg gga      1776
   Gly Lys Gly Arg Ile Leu Ile Glu Gly Glu Arg Val Ala Leu Leu Gly
               580                 585                 590
   tat ggc tca gca gtg cag aac tgt ttg gat gct gct att gtg cta gaa      1824
   Tyr Gly Ser Ala Val Gln Asn Cys Leu Asp Ala Ala Ile Val Leu Glu
           595                 600                 605
   tcc cgc ggc tta caa gta aca gtt gca gat gca cgt ttc tgc aaa cca      1872
   Ser Arg Gly Leu Gln Val Thr Val Ala Asp Ala Arg Phe Cys Lys Pro
       610                 615                 620
   ctg gac cat gcc ctc ata agg agc ctt gca aaa tca cat gaa gtg cta      1920
   Leu Asp His Ala Leu Ile Arg Ser Leu Ala Lys Ser His Glu Val Leu
   625                 630                 635                 640
   atc act gtc gaa gaa gga tca att gga ggt ttt gga tct cat gtt gtt      1968
   Ile Thr Val Glu Glu Gly Ser Ile Gly Gly Phe Gly Ser His Val Val
                   645                 650                 655
   cag ttc atg gcc tta gat ggg ctt ctt gat ggc aag ttg aag tgg aga      2016
   Gln Phe Met Ala Leu Asp Gly Leu Leu Asp Gly Lys Leu Lys Trp Arg
               660                 665                 670
   cca ata gtt ctt cct gat cga tac att gac cat gga tct cct gtt gat      2064
   Pro Ile Val Leu Pro Asp Arg Tyr Ile Asp His Gly Ser Pro Val Asp
           675                 680                 685
   cag ttg gcg gaa gct ggc cta aca cca tct cac att gca gca aca gta      2112
   Gln Leu Ala Glu Ala Gly Leu Thr Pro Ser His Ile Ala Ala Thr Val
       690                 695                 700
   ttt aac ata ctt gga caa acc aga gag gct cta gag gtc atg aca taa      2160
   Phe Asn Ile Leu Gly Gln Thr Arg Glu Ala Leu Glu Val Met Thr
   705                 710                 715
<210> 116
   <211> 719
   <212> PRT
   <213> Lycopersicon esculentum
<400> 116
   Met Ala Leu Cys Ala Tyr Ala Phe Pro Gly Ile Leu Asn Arg Thr Gly
   1               5                   10                  15
   Val Val Ser Asp Ser Ser Lys Ala Thr Pro Leu Phe Ser Gly Trp Ile
               20                  25                  30
   His Gly Thr Asp Leu Gln Phe Leu Phe Gln His Lys Leu Thr His Glu
           35                  40                  45
   Val Lys Lys Arg Ser Arg Val Val Gln Ala Ser Leu Ser Glu Ser Gly
       50                  55                  60
   Glu Tyr Tyr Thr Gln Arg Pro Pro Thr Pro Ile Leu Asp Thr Val Asn
   65                  70                  75                  80
   Tyr Pro Ile His Met Lys Asn Leu Ser Leu Lys Glu Leu Lys Gln Leu
                   85                  90                  95
   Ala Asp Glu Leu Arg Ser Asp Thr Ile Phe Asn Val Ser Lys Thr Gly
               100                 105                 110
   Gly His Leu Gly Ser Ser Leu Gly Val Val Glu Leu Thr Val Ala Leu
           115                 120                 125
   His Tyr Val Phe Asn Ala Pro Gln Asp Arg Ile Leu Trp Asp Val Gly
       130                 135                 140
   His Gln Ser Tyr Pro His Lys Ile Leu Thr Gly Arg Arg Asp Lys Met
   145                 150                 155                 160
   Ser Thr Leu Arg Gln Thr Asp Gly Leu Ala Gly Phe Thr Lys Arg Ser
                   165                 170                 175
   Glu Ser Glu Tyr Asp Cys Phe Gly Thr Gly His Ser Ser Thr Thr Ile
               180                 185                 190
   Ser Ala Gly Leu Gly Met Ala Val Gly Arg Asp Leu Lys Gly Arg Asn
           195                 200                 205
   Asn Asn Val Ile Ala Val Ile Gly Asp Gly Ala Met Thr Ala Gly Gln
       210                 215                 220
   Ala Tyr Glu Ala Met Asn Asn Ala Gly Tyr Leu Asp Ser Asp Met Ile
   225                 230                 235                 240
   Val Ile Leu Asn Asp Asn Arg Gln Val Ser Leu Pro Thr Ala Thr Leu
                   245                 250                 255
   Asp Gly Pro Val Ala Pro Val Gly Ala Leu Ser Ser Ala Leu Ser Arg
   260 265 270
   Leu Gln Ser Asn Arg Pro Leu Arg Glu Leu Arg Glu Val Ala Lys Gly
   275 280 285
   Val Thr Lys Gln Ile Gly Gly Pro Met His Glu Leu Ala Ala Lys Val
   290 295 300
   Asp Glu Tyr Ala Arg Gly Met Ile Ser Gly Ser Gly Ser Thr Leu Phe
   305 310 315 320
   Glu Glu Leu Gly Leu Tyr Tyr Ile Gly Pro Val Asp Gly His Asn Ile
   325 330 335
   Asp Asp Leu Ile Ala Ile Leu Lys Glu Val Arg Ser Thr Lys Thr Thr
   340 345 350
   Gly Pro Val Leu Ile His Val Val Thr Glu Lys Gly Arg Gly Tyr Pro
   355 360 365
   Tyr Ala Glu Arg Ala Ala Asp Lys Tyr His Gly Val Ala Lys Phe Asp
   370 375 380
   Pro Ala Thr Gly Lys Gln Phe Lys Ala Ser Ala Lys Thr Gln Ser Tyr
   385 390 395 400
   Thr Thr Tyr Phe Ala Glu Ala Leu Ile Ala Glu Ala Glu Ala Asp Lys
   405 410 415
   Asp Ile Val Ala Ile His Ala Ala Met Gly Gly Gly Thr Gly Met Asn
   420 425 430
   Leu Phe His Arg Arg Phe Pro Thr Arg Cys Phe Asp Val Gly Ile Ala
   435 440 445
   Glu Gln His Ala Val Thr Phe Ala Ala Gly Leu Ala Cys Glu Gly Ile
   450 455 460
   Lys Pro Phe Cys Ala Ile Tyr Ser Ser Phe Met Gln Arg Ala Tyr Asp
   465 470 475 480
   Gln Val Val His Asp Val Asp Leu Gln Lys Leu Pro Val Arg Phe Ala
   485 490 495
   Met Asp Arg Ala Gly Leu Val Gly Ala Asp Gly Pro Thr His Cys Gly
   500 505 510
   Ala Phe Asp Val Thr Tyr Met Ala Cys Leu Pro Asn Met Val Val Met
   515 520 525
   Ala Pro Ser Asp Glu Ala Glu Leu Phe His Met Val Ala Thr Ala Ala
   530 535 540
   Ala Ile Asp Asp Arg Pro Ser Cys Phe Arg Tyr Pro Arg Gly Asn Gly
   545 550 555 560
   Ile Gly Val Glu Leu Pro Ala Gly Asn Lys Gly Ile Pro Leu Glu Val
   565 570 575
   Gly Lys Gly Arg Ile Leu Ile Glu Gly Glu Arg Val Ala Leu Leu Gly
   580 585 590
   Tyr Gly Ser Ala Val Gln Asn Cys Leu Asp Ala Ala Ile Val Leu Glu
   595 600 605
   Ser Arg Gly Leu Gln Val Thr Val Ala Asp Ala Arg Phe Cys Lys Pro
   610 615 620
   Leu Asp His Ala Leu Ile Arg Ser Leu Ala Lys Ser His Glu Val Leu
   625 630 635 640
   Ile Thr Val Glu Glu Gly Ser Ile Gly Gly Phe Gly Ser His Val Val
   645 650 655
   Gln Phe Met Ala Leu Asp Gly Leu Leu Asp Gly Lys Leu Lys Trp Arg
   660 665 670
   Pro Ile Val Leu Pro Asp Arg Tyr Ile Asp His Gly Ser Pro Val Asp
   675 680 685
   Gln Leu Ala Glu Ala Gly Leu Thr Pro Ser His Ile Ala Ala Thr Val
       690                 695                 700
   Phe Asn Ile Leu Gly Gln Thr Arg Glu Ala Leu Glu Val Met Thr
   705                 710                 715
<210> 117
   <211> 1434
   <212> DNA
   <213> Arabidopsis thaliana
<220>
   <221> CDS
   <222> (1)..(1434)
   <223>
<400> 117
   atg atg aca tta aac tca cta tct cca gct gaa tcc aaa gct att tct      48
   Met Met Thr Leu Asn Ser Leu Ser Pro Ala Glu Ser Lys Ala Ile Ser
   1               5                   10                  15
   ttc ttg gat acc tcc agg ttc aat cca atc cct aaa ctc tca ggt ggg      96
   Phe Leu Asp Thr Ser Arg Phe Asn Pro Ile Pro Lys Leu Ser Gly Gly
               20                  25                  30
   ttt agt ttg agg agg agg aat caa ggg aga ggt ttt gga aaa ggt gtt      144
   Phe Ser Leu Arg Arg Arg Asn Gln Gly Arg Gly Phe Gly Lys Gly Val
           35                  40                  45
   aag tgt tca gtg aaa gtg cag cag caa caa caa cct cct cca gca tgg      192
   Lys Cys Ser Val Lys Val Gln Gln Gln Gln Gln Pro Pro Pro Ala Trp
       50                  55                  60
   cct ggg aga gct gtc cct gag gcg cct cgt caa tct tgg gat gga cca      240
   Pro Gly Arg Ala Val Pro Glu Ala Pro Arg Gln Ser Trp Asp Gly Pro
   65                  70                  75                  80
   aaa ccc atc tct atc gtt gga tct act ggt tct att ggc act cag aca      288
   Lys Pro Ile Ser Ile Val Gly Ser Thr Gly Ser Ile Gly Thr Gln Thr
                   85                  90                  95
   ttg gat att gtg gct gag aat cct gac aaa ttc aga gtt gtg gct cta      336
   Leu Asp Ile Val Ala Glu Asn Pro Asp Lys Phe Arg Val Val Ala Leu
               100                 105                 110
   gct gct ggt tcg aat gtt act cta ctt gct gat cag gta agg aga ttt      384
   Ala Ala Gly Ser Asn Val Thr Leu Leu Ala Asp Gln Val Arg Arg Phe
           115                 120                 125
   aag cct gca ttg gtt gct gtt aga aac gag tca ctg att aat gag ctt      432
   Lys Pro Ala Leu Val Ala Val Arg Asn Glu Ser Leu Ile Asn Glu Leu
       130                 135                 140
   aaa gag gct tta gct gat ttg gac tat aaa ctc gag att att cca gga      480
   Lys Glu Ala Leu Ala Asp Leu Asp Tyr Lys Leu Glu Ile Ile Pro Gly
   145                 150                 155                 160
   gag caa gga gtg att gag gtt gcc cga cat cct gaa gct gta acc gtt      528
   Glu Gln Gly Val Ile Glu Val Ala Arg His Pro Glu Ala Val Thr Val
                   165                 170                 175
   gtt acc gga ata gta ggt tgt gcg gga cta aag cct acg gtt gct gca      576
   Val Thr Gly Ile Val Gly Cys Ala Gly Leu Lys Pro Thr Val Ala Ala
               180                 185                 190
   att gaa gca gga aag gac att gct ctt gca aac aaa gag aca tta atc      624
   Ile Glu Ala Gly Lys Asp Ile Ala Leu Ala Asn Lys Glu Thr Leu Ile
           195                 200                 205
   gca ggt ggt cct ttc gtg ctt ccg ctt gcc aac aaa cat aat gta aag      672
   Ala Gly Gly Pro Phe Val Leu Pro Leu Ala Asn Lys His Asn Val Lys
       210                 215                 220
   att ctt ccg gca gat tca gaa cat tct gcc ata ttt cag tgt att caa      720
   Ile Leu Pro Ala Asp Ser Glu His Ser Ala Ile Phe Gln Cys Ile Gln
   225                 230                 235                 240
   ggt ttg cct gaa ggc gct ctg cgc aag ata atc ttg act gca tct ggt      768
   Gly Leu Pro Glu Gly Ala Leu Arg Lys Ile Ile Leu Thr Ala Ser Gly
                   245                 250                 255
   gga gct ttt agg gat tgg cct gtc gaa aag cta aag gaa gtt aaa gta      816
   Gly Ala Phe Arg Asp Trp Pro Val Glu Lys Leu Lys Glu Val Lys Val
               260                 265                 270
   gcg gat gcg ttg aag cat cca aac tgg aac atg gga aag aaa atc act      864
   Ala Asp Ala Leu Lys His Pro Asn Trp Asn Met Gly Lys Lys Ile Thr
           275                 280                 285
   gtg gac tct gct acg ctt ttc aac aag ggt ctt gag gtc att gaa gcg      912
   Val Asp Ser Ala Thr Leu Phe Asn Lys Gly Leu Glu Val Ile Glu Ala
       290                 295                 300
   cat tat ttg ttt gga gct gag tat gac gat ata gag att gtc att cat      960
   His Tyr Leu Phe Gly Ala Glu Tyr Asp Asp Ile Glu Ile Val Ile His
   305                 310                 315                 320
   ccg caa agt atc ata cat tcc atg att gaa aca cag gat tca tct gtg      1008
   Pro Gln Ser Ile Ile His Ser Met Ile Glu Thr Gln Asp Ser Ser Val
                   325                 330                 335
   ctt gct caa ttg ggt tgg cct gat atg cgt tta ccg att ctc tac acc      1056
   Leu Ala Gln Leu Gly Trp Pro Asp Met Arg Leu Pro Ile Leu Tyr Thr
               340                 345                 350
   atg tca tgg ccc gat aga gtt cct tgt tct gaa gta act tgg cca aga      1104
   Met Ser Trp Pro Asp Arg Val Pro Cys Ser Glu Val Thr Trp Pro Arg
           355                 360                 365
   ctt gac ctt tgc aaa ctc ggt tca ttg act ttc aag aaa cca gac aat      1152
   Leu Asp Leu Cys Lys Leu Gly Ser Leu Thr Phe Lys Lys Pro Asp Asn
       370                 375                 380
   gtg aaa tac cca tcc atg gat ctt gct tat gct gct gga cga gct gga      1200
   Val Lys Tyr Pro Ser Met Asp Leu Ala Tyr Ala Ala Gly Arg Ala Gly
   385                 390                 395                 400
   ggc aca atg act gga gtt ctc agc gcc gcc aat gag aaa gct gtt gaa      1248
   Gly Thr Met Thr Gly Val Leu Ser Ala Ala Asn Glu Lys Ala Val Glu
                   405                 410                 415
   atg ttc att gat gaa aag ata agc tat ttg gat atc ttc aag gtt gtg      1296
   Met Phe Ile Asp Glu Lys Ile Ser Tyr Leu Asp Ile Phe Lys Val Val
               420                 425                 430
   gaa tta aca tgc gat aaa cat cga aac gag ttg gta aca tca ccg tct      1344
   Glu Leu Thr Cys Asp Lys His Arg Asn Glu Leu Val Thr Ser Pro Ser
           435                 440                 445
   ctt gaa gag att gtt cac tat gac ttg tgg gca cgt gaa tat gcc gcg      1392
   Leu Glu Glu Ile Val His Tyr Asp Leu Trp Ala Arg Glu Tyr Ala Ala
       450                 455                 460
   aat gtg cag ctt tct tct ggt gct agg cca gtt cat gca tga              1434
   Asn Val Gln Leu Ser Ser Gly Ala Arg Pro Val His Ala
   465                 470                 475
<210> 118
   <211> 477
   <212> PRT
   <213> Arabidopsis thaliana
<400> 118
   Met Met Thr Leu Asn Ser Leu Ser Pro Ala Glu Ser Lys Ala Ile Ser
   1               5                   10                  15
   Phe Leu Asp Thr Ser Arg Phe Asn Pro Ile Pro Lys Leu Ser Gly Gly
               20                  25                  30
   Phe Ser Leu Arg Arg Arg Asn Gln Gly Arg Gly Phe Gly Lys Gly Val
           35                  40                  45
   Lys Cys Ser Val Lys Val Gln Gln Gln Gln Gln Pro Pro Pro Ala Trp
       50                  55                  60
   Pro Gly Arg Ala Val Pro Glu Ala Pro Arg Gln Ser Trp Asp Gly Pro
   65                  70                  75                  80
   Lys Pro Ile Ser Ile Val Gly Ser Thr Gly Ser Ile Gly Thr Gln Thr
                   85                  90                  95
   Leu Asp Ile Val Ala Glu Asn Pro Asp Lys Phe Arg Val Val Ala Leu
               100                 105                 110
   Ala Ala Gly Ser Asn Val Thr Leu Leu Ala Asp Gln Val Arg Arg Phe
           115                 120                 125
   Lys Pro Ala Leu Val Ala Val Arg Asn Glu Ser Leu Ile Asn Glu Leu
       130                 135                 140
   Lys Glu Ala Leu Ala Asp Leu Asp Tyr Lys Leu Glu Ile Ile Pro Gly
   145                 150                 155                 160
   Glu Gln Gly Val Ile Glu Val Ala Arg His Pro Glu Ala Val Thr Val
                   165                 170                 175
   Val Thr Gly Ile Val Gly Cys Ala Gly Leu Lys Pro Thr Val Ala Ala
               180                 185                 190
   Ile Glu Ala Gly Lys Asp Ile Ala Leu Ala Asn Lys Glu Thr Leu Ile
           195                 200                 205
   Ala Gly Gly Pro Phe Val Leu Pro Leu Ala Asn Lys His Asn Val Lys
       210                 215                 220
   Ile Leu Pro Ala Asp Ser Glu His Ser Ala Ile Phe Gln Cys Ile Gln
   225                 230                 235                 240
   Gly Leu Pro Glu Gly Ala Leu Arg Lys Ile Ile Leu Thr Ala Ser Gly
                   245                 250                 255
   Gly Ala Phe Arg Asp Trp Pro Val Glu Lys Leu Lys Glu Val Lys Val
               260                 265                 270
   Ala Asp Ala Leu Lys His Pro Asn Trp Asn Met Gly Lys Lys Ile Thr
           275                 280                 285
   Val Asp Ser Ala Thr Leu Phe Asn Lys Gly Leu Glu Val Ile Glu Ala
       290                 295                 300
   His Tyr Leu Phe Gly Ala Glu Tyr Asp Asp Ile Glu Ile Val Ile His
   305                 310                 315                 320
   Pro Gln Ser Ile Ile His Ser Met Ile Glu Thr Gln Asp Ser Ser Val
                   325                 330                 335
   Leu Ala Gln Leu Gly Trp Pro Asp Met Arg Leu Pro Ile Leu Tyr Thr
               340                 345                 350
   Met Ser Trp Pro Asp Arg Val Pro Cys Ser Glu Val Thr Trp Pro Arg
           355                 360                 365
   Leu Asp Leu Cys Lys Leu Gly Ser Leu Thr Phe Lys Lys Pro Asp Asn
       370                 375                 380
   Val Lys Tyr Pro Ser Met Asp Leu Ala Tyr Ala Ala Gly Arg Ala Gly
   385                 390                 395                 400
   Gly Thr Met Thr Gly Val Leu Ser Ala Ala Asn Glu Lys Ala Val Glu
                   405                 410                 415
   Met Phe Ile Asp Glu Lys Ile Ser Tyr Leu Asp Ile Phe Lys Val Val
               420                 425                 430
   Glu Leu Thr Cys Asp Lys His Arg Asn Glu Leu Val Thr Ser Pro Ser
   435 440 445
   Leu Glu Glu Ile Val His Tyr Asp Leu Trp Ala Arg Glu Tyr Ala Ala
   450 455 460
   Asn Val Gln Leu Ser Ser Gly Ala Arg Pro Val His Ala
   465 470 475
<210> 119
   <211> 884
   <212> DNA
   <213> Adonis palaestina clone ApIPI28
<220>
   <221> CDS
   <222> (180)..(884)
   <223>
<400> 119
   cgtcgatcag gattaatcct ttatatagta tcttctccac caccactaaa acattatcag 60
   cttcgtgttc ttctcccgct gttcatcttc agcagcgttg tcgtactctt tctatttctt 120
   cttccatcac taacagtcct cgccgagggt tgaatcggct gttcgcctca acgtcgact 179
   atg ggt gaa gtc gct gat gct ggt atg gat gcc gtc cag aag cgg ctt 227
   Met Gly Glu Val Ala Asp Ala Gly Met Asp Ala Val Gln Lys Arg Leu
   1 5 10 15
   atg ttc gac gat gaa tgt att ttg gtg gat gag aat gac aag gtc gtc 275
   Met Phe Asp Asp Glu Cys Ile Leu Val Asp Glu Asn Asp Lys Val Val
   20 25 30
   gga cat gat tcc aaa tac aac tgt cat ttg atg gaa aag ata gag gca 323
   Gly His Asp Ser Lys Tyr Asn Cys His Leu Met Glu Lys Ile Glu Ala
   35 40 45
   gaa aac ttg ctt cac aga gcc ttc agt gtt ttc tta ttc aac tca aaa 371
   Glu Asn Leu Leu His Arg Ala Phe Ser Val Phe Leu Phe Asn Ser Lys
   50 55 60
   tac gag ttg ctt ctt cag caa cga tct gca acg aag gta aca ttc ccg      419
   Tyr Glu Leu Leu Leu Gln Gln Arg Ser Ala Thr Lys Val Thr Phe Pro
   65                  70                  75                  80
   ctc gta tgg aca aac acc tgt tgc agc cat ccc ctc ttc cgt gat tcc      467
   Leu Val Trp Thr Asn Thr Cys Cys Ser His Pro Leu Phe Arg Asp Ser
                   85                  90                  95
   gaa ctc ata gaa gaa aat ttt ctc ggg gta cga aac gct gca caa agg      515
   Glu Leu Ile Glu Glu Asn Phe Leu Gly Val Arg Asn Ala Ala Gln Arg
               100                 105                 110
   aag ctt tta gac gag cta ggc att cca gct gaa gac gta cca gtt gat      563
   Lys Leu Leu Asp Glu Leu Gly Ile Pro Ala Glu Asp Val Pro Val Asp
           115                 120                 125
   gaa ttc act cct ctt ggt cgc att ctt tac aaa gct cca tct gac gga      611
   Glu Phe Thr Pro Leu Gly Arg Ile Leu Tyr Lys Ala Pro Ser Asp Gly
       130                 135                 140
   aaa tgg gga gag cac gaa ctg gac tat ctt ctg ttt att gtc cga gat      659
   Lys Trp Gly Glu His Glu Leu Asp Tyr Leu Leu Phe Ile Val Arg Asp
   145                 150                 155                 160
   gtg aaa tac gat cca aac cca gat gaa gtt gct gac gct aag tac gtt      707
   Val Lys Tyr Asp Pro Asn Pro Asp Glu Val Ala Asp Ala Lys Tyr Val
                   165                 170                 175
   aat cgc gag gag ttg aaa gag ata ctg aga aaa gct gat gca ggt gaa      755
   Asn Arg Glu Glu Leu Lys Glu Ile Leu Arg Lys Ala Asp Ala Gly Glu
               180                 185                 190
   gag gga ata aag ttg tct cct tgg ttt aga ttg gtt gtg gat aac ttt      803
   Glu Gly Ile Lys Leu Ser Pro Trp Phe Arg Leu Val Val Asp Asn Phe
           195                 200                 205
   ttg ttc aag tgg tgg gat cat gta gag gag ggg aag att aag gac gtc      851
   Leu Phe Lys Trp Trp Asp His Val Glu Glu Gly Lys Ile Lys Asp Val
       210                 215                 220
   gcc gac atg aaa act atc cac aag ttg act taa                          884
   Ala Asp Met Lys Thr Ile His Lys Leu Thr
   225                 230
<210> 120
   <211> 234
   <212> PRT
   <213> Adonis palaestina clone ApIPI28
<400> 120
   Met Gly Glu Val Ala Asp Ala Gly Met Asp Ala Val Gln Lys Arg Leu
   1               5                   10                  15
   Met Phe Asp Asp Glu Cys Ile Leu Val Asp Glu Asn Asp Lys Val Val
               20                  25                  30
   Gly His Asp Ser Lys Tyr Asn Cys His Leu Met Glu Lys Ile Glu Ala
           35                  40                  45
   Glu Asn Leu Leu His Arg Ala Phe Ser Val Phe Leu Phe Asn Ser Lys
       50                  55                  60
   Tyr Glu Leu Leu Leu Gln Gln Arg Ser Ala Thr Lys Val Thr Phe Pro
   65                  70                  75                  80
   Leu Val Trp Thr Asn Thr Cys Cys Ser His Pro Leu Phe Arg Asp Ser
                   85                  90                  95
   Glu Leu Ile Glu Glu Asn Phe Leu Gly Val Arg Asn Ala Ala Gln Arg
               100                 105                 110
   Lys Leu Leu Asp Glu Leu Gly Ile Pro Ala Glu Asp Val Pro Val Asp
           115                 120                 125
   Glu Phe Thr Pro Leu Gly Arg Ile Leu Tyr Lys Ala Pro Ser Asp Gly
       130                 135                 140
   Lys Trp Gly Glu His Glu Leu Asp Tyr Leu Leu Phe Ile Val Arg Asp
   145                 150                 155                 160
   Val Lys Tyr Asp Pro Asn Pro Asp Glu Val Ala Asp Ala Lys Tyr Val
                   165                 170                 175
   Asn Arg Glu Glu Leu Lys Glu Ile Leu Arg Lys Ala Asp Ala Gly Glu
               180                 185                 190
   Glu Gly Ile Lys Leu Ser Pro Trp Phe Arg Leu Val Val Asp Asn Phe
           195                 200                 205
   Leu Phe Lys Trp Trp Asp His Val Glu Glu Gly Lys Ile Lys Asp Val
       210                 215                 220
   Ala Asp Met Lys Thr Ile His Lys Leu Thr
   225                 230
<210> 121
   <211> 1402
   <212> DNA
   <213> Arabidopsis thaliana
<220>
   <221> CDS
   <222> (52)..(1317)
   <223>
<400> 121
   aagtctttgc ctctttggtt tactttcctc tgttttcgat ccatttagaa a atg tta       57
                                                             Met Leu
                                                             1
   ttc acg agg agt gtt gct cgg att tct tct aag ttt ctg aga aac cgt      105
   Phe Thr Arg Ser Val Ala Arg Ile Ser Ser Lys Phe Leu Arg Asn Arg
           5                   10                  15
   agc ttc tat ggc tcc tct caa tct ctc gcc tct cat cgg ttc gca atc      153
   Ser Phe Tyr Gly Ser Ser Gln Ser Leu Ala Ser His Arg Phe Ala Ile
       20                  25                  30
   att ccc gat cag ggt cac tct tgt tct gac tct cca cac aag ggt tac      201
   Ile Pro Asp Gln Gly His Ser Cys Ser Asp Ser Pro His Lys Gly Tyr
   35                  40                  45                  50
   gtt tgc aga aca act tat tca ttg aaa tct ccg gtt ttt ggt gga ttt      249
   Val Cys Arg Thr Thr Tyr Ser Leu Lys Ser Pro Val Phe Gly Gly Phe
                   55                  60                  65
   agt cat caa ctc tat cac cag agt agc tcc ttg gtt gag gag gag ctt      297
   Ser His Gln Leu Tyr His Gln Ser Ser Ser Leu Val Glu Glu Glu Leu
               70                  75                  80
   gac cca ttt tcg ctt gtt gcc gat gag ctg tca ctt ctt agt aat aag      345
   Asp Pro Phe Ser Leu Val Ala Asp Glu Leu Ser Leu Leu Ser Asn Lys
           85                  90                  95
   ttg aga gag atg gta ctt gcc gag gtt cca aag ctt gcc tct gct gct      393
   Leu Arg Glu Met Val Leu Ala Glu Val Pro Lys Leu Ala Ser Ala Ala
       100                 105                 110
   gag tac ttc ttc aaa agg ggt gtg caa gga aaa cag ttt cgt tca act      441
   Glu Tyr Phe Phe Lys Arg Gly Val Gln Gly Lys Gln Phe Arg Ser Thr
   115                 120                 125                 130
   att ttg ctg ctg atg gcg aca gct ctg gat gta cga gtt cca gaa gca      489
   Ile Leu Leu Leu Met Ala Thr Ala Leu Asp Val Arg Val Pro Glu Ala
                   135                 140                 145
   ttg att ggg gaa tca aca gat ata gtc aca tca gaa tta cgc gta agg      537
   Leu Ile Gly Glu Ser Thr Asp Ile Val Thr Ser Glu Leu Arg Val Arg
               150                 155                 160
   caa cgg ggt att gct gaa atc act gaa atg ata cac gtc gca agt cta      585
   Gln Arg Gly Ile Ala Glu Ile Thr Glu Met Ile His Val Ala Ser Leu
           165                 170                 175
   ctg cac gat gat gtc ttg gat gat gcc gat aca agg cgt ggt gtt ggt      633
   Leu His Asp Asp Val Leu Asp Asp Ala Asp Thr Arg Arg Gly Val Gly
       180                 185                 190
   tcc tta aat gtt gta atg ggt aac aag atg tcg gta tta gca gga gac      681
   Ser Leu Asn Val Val Met Gly Asn Lys Met Ser Val Leu Ala Gly Asp
   195                 200                 205                 210
   ttc ttg ctc tcc cgg gct tgt ggg gct ctc gct gct tta aag aac aca      729
   Phe Leu Leu Ser Arg Ala Cys Gly Ala Leu Ala Ala Leu Lys Asn Thr
                   215                 220                 225
   gag gtt gta gca tta ctt gca act gct gta gaa cat ctt gtt acc ggt      777
   Glu Val Val Ala Leu Leu Ala Thr Ala Val Glu His Leu Val Thr Gly
               230                 235                 240
   gaa acc atg gag ata act agt tca acc gag cag cgt tat agt atg gac      825
   Glu Thr Met Glu Ile Thr Ser Ser Thr Glu Gln Arg Tyr Ser Met Asp
           245                 250                 255
   tac tac atg cag aag aca tat tat aag aca gca tcg cta atc tct aac      873
   Tyr Tyr Met Gln Lys Thr Tyr Tyr Lys Thr Ala Ser Leu Ile Ser Asn
       260                 265                 270
   agc tgc aaa gct gtt gcc gtt ctc act gga caa aca gca gaa gtt gcc      921
   Ser Cys Lys Ala Val Ala Val Leu Thr Gly Gln Thr Ala Glu Val Ala
   275                 280                 285                 290
   gtg tta gct ttt gag tat ggg agg aat ctg ggt tta gca ttc caa tta      969
   Val Leu Ala Phe Glu Tyr Gly Arg Asn Leu Gly Leu Ala Phe Gln Leu
                   295                 300                 305
   ata gac gac att ctt gat ttc acg ggc aca tct gcc tct ctc gga aag      1017
   Ile Asp Asp Ile Leu Asp Phe Thr Gly Thr Ser Ala Ser Leu Gly Lys
               310                 315                 320
   gga tcg ttg tca gat att cgc cat gga gtc ata aca gcc cca atc ctc      1065
   Gly Ser Leu Ser Asp Ile Arg His Gly Val Ile Thr Ala Pro Ile Leu
           325                 330                 335
   ttt gcc atg gaa gag ttt cct caa cta cgc gaa gtt gtt gat caa gtt      1113
   Phe Ala Met Glu Glu Phe Pro Gln Leu Arg Glu Val Val Asp Gln Val
       340                 345                 350
   gaa aaa gat cct agg aat gtt gac att gct tta gag tat ctt ggg aag      1161
   Glu Lys Asp Pro Arg Asn Val Asp Ile Ala Leu Glu Tyr Leu Gly Lys
   355                 360                 365                 370
   agc aag gga ata cag agg gca aga gaa tta gcc atg gaa cat gcg aat      1209
   Ser Lys Gly Ile Gln Arg Ala Arg Glu Leu Ala Met Glu His Ala Asn
                   375                 380                 385
   cta gca gca gct gca atc ggg tct cta cct gaa aca gac aat gaa gat      1257
   Leu Ala Ala Ala Ala Ile Gly Ser Leu Pro Glu Thr Asp Asn Glu Asp
               390                 395                 400
   gtc aaa aga tcg agg cgg gca ctt att gac ttg acc cat aga gtc atc      1305
   Val Lys Arg Ser Arg Arg Ala Leu Ile Asp Leu Thr His Arg Val Ile
           405                 410                 415
   acc aga aac aag tgagattaag taatgtttct ctctatacac caaaacattc          1357
   Thr Arg Asn Lys
       420
   ctcatttcat ttgtaggatt ttgttggtcc aattcgtttc acgaa                    1402
<210> 122
   <211> 422
   <212> PRT
   <213> Arabidopsis thaliana
<400> 122
   Met Leu Phe Thr Arg Ser Val Ala Arg Ile Ser Ser Lys Phe Leu Arg
   1               5                   10                  15
   Asn Arg Ser Phe Tyr Gly Ser Ser Gln Ser Leu Ala Ser His Arg Phe
               20                  25                  30
   Ala Ile Ile Pro Asp Gln Gly His Ser Cys Ser Asp Ser Pro His Lys
           35                  40                  45
   Gly Tyr Val Cys Arg Thr Thr Tyr Ser Leu Lys Ser Pro Val Phe Gly
       50                  55                  60
   Gly Phe Ser His Gln Leu Tyr His Gln Ser Ser Ser Leu Val Glu Glu
   65                  70                  75                  80
   Glu Leu Asp Pro Phe Ser Leu Val Ala Asp Glu Leu Ser Leu Leu Ser
                   85                  90                  95
   Asn Lys Leu Arg Glu Met Val Leu Ala Glu Val Pro Lys Leu Ala Ser
               100                 105                 110
   Ala Ala Glu Tyr Phe Phe Lys Arg Gly Val Gln Gly Lys Gln Phe Arg
           115                 120                 125
   Ser Thr Ile Leu Leu Leu Met Ala Thr Ala Leu Asp Val Arg Val Pro
       130                 135                 140
   Glu Ala Leu Ile Gly Glu Ser Thr Asp Ile Val Thr Ser Glu Leu Arg
   145                 150                 155                 160
   Val Arg Gln Arg Gly Ile Ala Glu Ile Thr Glu Met Ile His Val Ala
                   165                 170                 175
   Ser Leu Leu His Asp Asp Val Leu Asp Asp Ala Asp Thr Arg Arg Gly
               180                 185                 190
   Val Gly Ser Leu Asn Val Val Met Gly Asn Lys Met Ser Val Leu Ala
           195                 200                 205
   Gly Asp Phe Leu Leu Ser Arg Ala Cys Gly Ala Leu Ala Ala Leu Lys
       210                 215                 220
   Asn Thr Glu Val Val Ala Leu Leu Ala Thr Ala Val Glu His Leu Val
   225                 230                 235                 240
   Thr Gly Glu Thr Met Glu Ile Thr Ser Ser Thr Glu Gln Arg Tyr Ser
                   245                 250                 255
   Met Asp Tyr Tyr Met Gln Lys Thr Tyr Tyr Lys Thr Ala Ser Leu Ile
               260                 265                 270
   Ser Asn Ser Cys Lys Ala Val Ala Val Leu Thr Gly Gln Thr Ala Glu
           275                 280                 285
   Val Ala Val Leu Ala Phe Glu Tyr Gly Arg Asn Leu Gly Leu Ala Phe
       290                 295                 300
   Gln Leu Ile Asp Asp Ile Leu Asp Phe Thr Gly Thr Ser Ala Ser Leu
   305                 310                 315                 320
   Gly Lys Gly Ser Leu Ser Asp Ile Arg His Gly Val Ile Thr Ala Pro
                   325                 330                 335
   Ile Leu Phe Ala Met Glu Glu Phe Pro Gln Leu Arg Glu Val Val Asp
               340                 345                 350
   Gln Val Glu Lys Asp Pro Arg Asn Val Asp Ile Ala Leu Glu Tyr Leu
           355                 360                 365
   Gly Lys Ser Lys Gly Ile Gln Arg Ala Arg Glu Leu Ala Met Glu His
       370                 375                 380
   Ala Asn Leu Ala Ala Ala Ala Ile Gly Ser Leu Pro Glu Thr Asp Asn
   385                 390                 395                 400
   Glu Asp Val Lys Arg Ser Arg Arg Ala Leu Ile Asp Leu Thr His Arg
                   405                 410                 415
   Val Ile Thr Arg Asn Lys
               420
<210> 123
   <211> 1155
   <212> DNA
   <213> Arabidopsis thaliana
<220>
   <221> CDS
   <222> (1)..(1155)
   <223>
<400> 123
   atg agt gtg agt tgt tgt tgt agg aat ctg ggc aag aca ata aaa aag       48
   Met Ser Val Ser Cys Cys Cys Arg Asn Leu Gly Lys Thr Ile Lys Lys
   1               5                   10                  15
   gca ata cct tca cat cat ttg cat ctg aga agt ctt ggt ggg agt ctc       96
   Ala Ile Pro Ser His His Leu His Leu Arg Ser Leu Gly Gly Ser Leu
               20                  25                  30
   tat cgt cgt cgt atc caa agc tct tca atg gag acc gat ctc aag tca      144
   Tyr Arg Arg Arg Ile Gln Ser Ser Ser Met Glu Thr Asp Leu Lys Ser
           35                  40                  45
   acc ttt ctc aac gtt tat tct gtt ctc aag tct gac ctt ctt cat gac      192
   Thr Phe Leu Asn Val Tyr Ser Val Leu Lys Ser Asp Leu Leu His Asp
       50                  55                  60
   cct tcc ttc gaa ttc acc aat gaa tct cgt ctc tgg gtt gat cgg atg      240
   Pro Ser Phe Glu Phe Thr Asn Glu Ser Arg Leu Trp Val Asp Arg Met
   65                  70                  75                  80
   ctg gac tac aat gta cgt gga ggg aaa ctc aat cgg ggt ctc tct gtt      288
   Leu Asp Tyr Asn Val Arg Gly Gly Lys Leu Asn Arg Gly Leu Ser Val
                   85                  90                  95
   gtt gac agt ttc aaa ctt ttg aag caa ggc aat gat ttg act gag caa      336
   Val Asp Ser Phe Lys Leu Leu Lys Gln Gly Asn Asp Leu Thr Glu Gln
               100                 105                 110
   gag gtt ttc ctc tct tgt gct ctc ggt tgg tgc att gaa tgg ctc caa      384
   Glu Val Phe Leu Ser Cys Ala Leu Gly Trp Cys Ile Glu Trp Leu Gln
           115                 120                 125
   gct tat ttc ctt gtg ctt gat gat att atg gat aac tct gtc act cgc      432
   Ala Tyr Phe Leu Val Leu Asp Asp Ile Met Asp Asn Ser Val Thr Arg
       130                 135                 140
   cgt ggt caa cct tgc tgg ttc aga gtt cct cag gtt ggt atg gtt gcc      480
   Arg Gly Gln Pro Cys Trp Phe Arg Val Pro Gln Val Gly Met Val Ala
   145                 150                 155                 160
   atc aat gat ggg att cta ctt cgc aat cac atc cac agg att ctc aaa      528
   Ile Asn Asp Gly Ile Leu Leu Arg Asn His Ile His Arg Ile Leu Lys
                   165                 170                 175
   aag cat ttc cgt gat aag cct tac tat gtt gac ctt gtt gat ttg ttt      576
   Lys His Phe Arg Asp Lys Pro Tyr Tyr Val Asp Leu Val Asp Leu Phe
               180                 185                 190
   aat gag gtt gag ttg caa aca gct tgt ggc cag atg ata gat ttg atc      624
   Asn Glu Val Glu Leu Gln Thr Ala Cys Gly Gln Met Ile Asp Leu Ile
           195                 200                 205
   acc acc ttt gaa gga gaa aag gat ttg gcc aag tac tca ttg tca atc      672
   Thr Thr Phe Glu Gly Glu Lys Asp Leu Ala Lys Tyr Ser Leu Ser Ile
       210                 215                 220
   cac cgt cgt att gtc cag tac aaa acg gct tat tac tca ttt tat ctc      720
   His Arg Arg Ile Val Gln Tyr Lys Thr Ala Tyr Tyr Ser Phe Tyr Leu
   225                 230                 235                 240
   cct gtt gct tgt gcg ttg ctt atg gcg ggc gaa aat ttg gaa aac cat      768
   Pro Val Ala Cys Ala Leu Leu Met Ala Gly Glu Asn Leu Glu Asn His
                   245                 250                 255
   att gac gtg aaa aat gtt ctt gtt gac atg gga atc tac ttc caa gtg      816
   Ile Asp Val Lys Asn Val Leu Val Asp Met Gly Ile Tyr Phe Gln Val
               260                 265                 270
   cag gat gat tat ctg gat tgt ttt gct gat ccc gag acg ctt ggc aag      864
   Gln Asp Asp Tyr Leu Asp Cys Phe Ala Asp Pro Glu Thr Leu Gly Lys
           275                 280                 285
   ata gga aca gat ata gaa gat ttc aaa tgc tcg tgg ttg gtg gtt aag      912
   Ile Gly Thr Asp Ile Glu Asp Phe Lys Cys Ser Trp Leu Val Val Lys
       290                 295                 300
   gca tta gag cgc tgc agc gaa gaa caa act aag ata tta tat gag aac      960
   Ala Leu Glu Arg Cys Ser Glu Glu Gln Thr Lys Ile Leu Tyr Glu Asn
   305                 310                 315                 320
   tat ggt aaa ccc gac cca tcg aac gtt gct aaa gtg aag gat ctc tac     1008
   Tyr Gly Lys Pro Asp Pro Ser Asn Val Ala Lys Val Lys Asp Leu Tyr
                   325                 330                 335
   aaa gag ctg gat ctt gag gga gtt ttc atg gag tat gag agc aaa agc     1056
   Lys Glu Leu Asp Leu Glu Gly Val Phe Met Glu Tyr Glu Ser Lys Ser
               340                 345                 350
   tac gag aag ctg act gga gcg att gag gga cac caa agt aaa gca atc     1104
   Tyr Glu Lys Leu Thr Gly Ala Ile Glu Gly His Gln Ser Lys Ala Ile
           355                 360                 365
   caa gca gtg cta aaa tcc ttc ttg gct aag atc tac aag agg cag aag 1152
   Gln Ala Val Leu Lys Ser Phe Leu Ala Lys Ile Tyr Lys Arg Gln Lys
   370 375 380
   tag 1155
<210> 124
   <211> 384
   <212> PRT
   <213> Arabidopsis thaliana
<400> 124
   Met Ser Val Ser Cys Cys Cys Arg Asn Leu Gly Lys Thr Ile Lys Lys
   1 5 10 15
   Ala Ile Pro Ser His His Leu His Leu Arg Ser Leu Gly Gly Ser Leu
   20 25 30
   Tyr Arg Arg Arg Ile Gln Ser Ser Ser Met Glu Thr Asp Leu Lys Ser
   35 40 45
   Thr Phe Leu Asn Val Tyr Ser Val Leu Lys Ser Asp Leu Leu His Asp
   50 55 60
   Pro Ser Phe Glu Phe Thr Asn Glu Ser Arg Leu Trp Val Asp Arg Met
   65 70 75 80
   Leu Asp Tyr Asn Val Arg Gly Gly Lys Leu Asn Arg Gly Leu Ser Val
   85 90 95
   Val Asp Ser Phe Lys Leu Leu Lys Gln Gly Asn Asp Leu Thr Glu Gln
   100 105 110
   Glu Val Phe Leu Ser Cys Ala Leu Gly Trp Cys Ile Glu Trp Leu Gln
   115 120 125
   Ala Tyr Phe Leu Val Leu Asp Asp Ile Met Asp Asn Ser Val Thr Arg
   130 135 140
   Arg Gly Gln Pro Cys Trp Phe Arg Val Pro Gln Val Gly Met Val Ala
   145 150 155 160
   Ile Asn Asp Gly Ile Leu Leu Arg Asn His Ile His Arg Ile Leu Lys
   165 170 175
   Lys His Phe Arg Asp Lys Pro Tyr Tyr Val Asp Leu Val Asp Leu Phe
   180 185 190
   Asn Glu Val Glu Leu Gln Thr Ala Cys Gly Gln Met Ile Asp Leu Ile
   195 200 205
   Thr Thr Phe Glu Gly Glu Lys Asp Leu Ala Lys Tyr Ser Leu Ser Ile
   210 215 220
   His Arg Arg Ile Val Gln Tyr Lys Thr Ala Tyr Tyr Ser Phe Tyr Leu
   225 230 235 240
   Pro Val Ala Cys Ala Leu Leu Met Ala Gly Glu Asn Leu Glu Asn His
   245 250 255
   Ile Asp Val Lys Asn Val Leu Val Asp Met Gly Ile Tyr Phe Gln Val
   260 265 270
   Gln Asp Asp Tyr Leu Asp Cys Phe Ala Asp Pro Glu Thr Leu Gly Lys
   275 280 285
   Ile Gly Thr Asp Ile Glu Asp Phe Lys Cys Ser Trp Leu Val Val Lys
   290 295 300
   Ala Leu Glu Arg Cys Ser Glu Glu Gln Thr Lys Ile Leu Tyr Glu Asn
   305 310 315 320
   Tyr Gly Lys Pro Asp Pro Ser Asn Val Ala Lys Val Lys Asp Leu Tyr
   325 330 335
   Lys Glu Leu Asp Leu Glu Gly Val Phe Met Glu Tyr Glu Ser Lys Ser
   340 345 350
   Tyr Glu Lys Leu Thr Gly Ala Ile Glu Gly His Gln Ser Lys Ala Ile
           355                 360                 365
   Gln Ala Val Leu Lys Ser Phe Leu Ala Lys Ile Tyr Lys Arg Gln Lys
       370                 375                 380
<210> 125
   <211> 1101
   <212> DNA
   <213> Sinabs alba
<220>
   <221> CDS
   <222> (1)..(1101)
   <223>
<400> 125
   atg gct tct tca gtg act cct cta ggt tca tgg gtt ctt ctt cac cat       48
   Met Ala Ser Ser Val Thr Pro Leu Gly Ser Trp Val Leu Leu His His
   1               5                   10                  15
   cat cct tca act atc tta acc caa tcc aga tcc aga tct cct cct tct       96
   His Pro Ser Thr Ile Leu Thr Gln Ser Arg Ser Arg Ser Pro Pro Ser
               20                  25                  30
   ctc atc acc ctt aaa ccc atc tcc ctc act cca aaa cgc acc gtt tcg      144
   Leu Ile Thr Leu Lys Pro Ile Ser Leu Thr Pro Lys Arg Thr Val Ser
           35                  40                  45
   tct tct tcc tcc tct tcc ctc atc acc aaa gaa gac aac aac ctc aaa      192
   Ser Ser Ser Ser Ser Ser Leu Ile Thr Lys Glu Asp Asn Asn Leu Lys
       50                  55                  60
   tcc tct tcc tct tcc ttc gat ttc atg tct tac atc atc cgc aaa gcc      240
   Ser Ser Ser Ser Ser Phe Asp Phe Met Ser Tyr Ile Ile Arg Lys Ala
   65                  70                  75                  80
   gac tcc gtc aac aaa gcc tta gac tcc gcc gtc cct ctc cgg gag cca      288
   Asp Ser Val Asn Lys Ala Leu Asp Ser Ala Val Pro Leu Arg Glu Pro
                   85                  90                  95
   ctc aag atc cac gaa gcg atg cgt tac tct ctc ctc gcc gga gga aaa      336
   Leu Lys Ile His Glu Ala Met Arg Tyr Ser Leu Leu Ala Gly Gly Lys
               100                 105                 110
   cgc gtc aga cca gtt ctc tgc atc gcc gcg tgc gag cta gtc gga gga      384
   Arg Val Arg Pro Val Leu Cys Ile Ala Ala Cys Glu Leu Val Gly Gly
           115                 120                 125
   gaa gag tct tta gct atg ccg gcg cgt tgc gcc gtg gaa atg atc cac      432
   Glu Glu Ser Leu Ala Met Pro Ala Arg Cys Ala Val Glu Met Ile His
       130                 135                 140
   acc atg tcg ttg atc cac gac gac ttg cct tgt atg gat aac gac gat      480
   Thr Met Ser Leu Ile His Asp Asp Leu Pro Cys Met Asp Asn Asp Asp
   145                 150                 155                 160
   ctc cgc cgc gga aag ccc acg aat cac aaa gtt tac ggc gaa gac gtg      528
   Leu Arg Arg Gly Lys Pro Thr Asn His Lys Val Tyr Gly Glu Asp Val
                   165                 170                 175
   gcg gtt tta gcc gga gac gcg ctt ctt tcg ttc gcc ttc gag cat tta      576
   Ala Val Leu Ala Gly Asp Ala Leu Leu Ser Phe Ala Phe Glu His Leu
               180                 185                 190
   gcg tcg gct acg agc tcg gag gtt tct ccg gcg aga gtg gtt aga gct      624
   Ala Ser Ala Thr Ser Ser Glu Val Ser Pro Ala Arg Val Val Arg Ala
           195                 200                 205
   gtg gga gag ttg gct aaa gcc atc ggc acc gaa ggg ctc gtg gcg gga      672
   Val Gly Glu Leu Ala Lys Ala Ile Gly Thr Glu Gly Leu Val Ala Gly
       210                 215                 220
   caa gtg gtg gat ata agc agt gaa ggg ttg gac tta aac aac gtc gga      720
   Gln Val Val Asp Ile Ser Ser Glu Gly Leu Asp Leu Asn Asn Val Gly
   225                 230                 235                 240
   ttg gag cat ttg aag ttt ata cat ttg cat aaa acg gcg gcg ttg ctt      768
   Leu Glu His Leu Lys Phe Ile His Leu His Lys Thr Ala Ala Leu Leu
                   245                 250                 255
   gaa gct tca gcg gtt ttg ggt ggg atc atc ggt gga ggg agt gat gaa      816
   Glu Ala Ser Ala Val Leu Gly Gly Ile Ile Gly Gly Gly Ser Asp Glu
               260                 265                 270
   gag atc gag agg ctg agg aag ttc gcg agg tgt att ggg ttg ttg ttt      864
   Glu Ile Glu Arg Leu Arg Lys Phe Ala Arg Cys Ile Gly Leu Leu Phe
           275                 280                 285
   cag gtg gtt gat gat atc ttg gac gtg acg aaa tcg tct caa gaa ctg      912
   Gln Val Val Asp Asp Ile Leu Asp Val Thr Lys Ser Ser Gln Glu Leu
       290                 295                 300
   ggg aaa acc gct ggg aaa gat ttg att gct gat aag ttg act tat ccg      960
   Gly Lys Thr Ala Gly Lys Asp Leu Ile Ala Asp Lys Leu Thr Tyr Pro
   305                 310                 315                 320
   aag ctc atg ggt ttg gag aaa tcg aga gag ttc gct gag aag ttg aat      1008
   Lys Leu Met Gly Leu Glu Lys Ser Arg Glu Phe Ala Glu Lys Leu Asn
                   325                 330                 335
   aca gag gca cgt gat cag ctt tta ggg ttt gat tcc gac aag gtt gct      1056
   Thr Glu Ala Arg Asp Gln Leu Leu Gly Phe Asp Ser Asp Lys Val Ala
               340                 345                 350
   cct ttg ttg gct ttg gct aat tac att gcc aat aga cag aac tga          1101
   Pro Leu Leu Ala Leu Ala Asn Tyr Ile Ala Asn Arg Gln Asn
           355                 360                 365
<210> 126
   <211> 366
   <212> PRT
   <213> Sinabs alba
<400> 126
   Met Ala Ser Ser Val Thr Pro Leu Gly Ser Trp Val Leu Leu His His
   1               5                   10                  15
   His Pro Ser Thr Ile Leu Thr Gln Ser Arg Ser Arg Ser Pro Pro Ser
               20                  25                  30
   Leu Ile Thr Leu Lys Pro Ile Ser Leu Thr Pro Lys Arg Thr Val Ser
           35                  40                  45
   Ser Ser Ser Ser Ser Ser Leu Ile Thr Lys Glu Asp Asn Asn Leu Lys
       50                  55                  60
   Ser Ser Ser Ser Ser Phe Asp Phe Met Ser Tyr Ile Ile Arg Lys Ala
   65                  70                  75                  80
   Asp Ser Val Asn Lys Ala Leu Asp Ser Ala Val Pro Leu Arg Glu Pro
                   85                  90                  95
   Leu Lys Ile His Glu Ala Met Arg Tyr Ser Leu Leu Ala Gly Gly Lys
               100                 105                 110
   Arg Val Arg Pro Val Leu Cys Ile Ala Ala Cys Glu Leu Val Gly Gly
           115                 120                 125
   Glu Glu Ser Leu Ala Met Pro Ala Arg Cys Ala Val Glu Met Ile His
       130                 135                 140
   Thr Met Ser Leu Ile His Asp Asp Leu Pro Cys Met Asp Asn Asp Asp
   145                 150                 155                 160
   Leu Arg Arg Gly Lys Pro Thr Asn His Lys Val Tyr Gly Glu Asp Val
                   165                 170                 175
   Ala Val Leu Ala Gly Asp Ala Leu Leu Ser Phe Ala Phe Glu His Leu
               180                 185                 190
   Ala Ser Ala Thr Ser Ser Glu Val Ser Pro Ala Arg Val Val Arg Ala
           195                 200                 205
   Val Gly Glu Leu Ala Lys Ala Ile Gly Thr Glu Gly Leu Val Ala Gly
       210                 215                 220
   Gln Val Val Asp Ile Ser Ser Glu Gly Leu Asp Leu Asn Asn Val Gly
   225                 230                 235                 240
   Leu Glu His Leu Lys Phe Ile His Leu His Lys Thr Ala Ala Leu Leu
                   245                 250                 255
   Glu Ala Ser Ala Val Leu Gly Gly Ile Ile Gly Gly Gly Ser Asp Glu
               260                 265                 270
   Glu Ile Glu Arg Leu Arg Lys Phe Ala Arg Cys Ile Gly Leu Leu Phe
           275                 280                 285
   Gln Val Val Asp Asp Ile Leu Asp Val Thr Lys Ser Ser Gln Glu Leu
       290                 295                 300
   Gly Lys Thr Ala Gly Lys Asp Leu Ile Ala Asp Lys Leu Thr Tyr Pro
   305                 310                 315                 320
   Lys Leu Met Gly Leu Glu Lys Ser Arg Glu Phe Ala Glu Lys Leu Asn
                   325                 330                 335
   Thr Glu Ala Arg Asp Gln Leu Leu Gly Phe Asp Ser Asp Lys Val Ala
               340                 345                 350
   Pro Leu Leu Ala Leu Ala Asn Tyr Ile Ala Asn Arg Gln Asn
           355                 360                 365
<210> 127
   <211> 930
   <212> DNA
   <213> Erwinia uredovora
<220>
   <221> CDS
   <222> (1)..(930)
   <223>
<400> 127
   atg aat aat ccg tcg tta ctc aat cat gcg gtc gaa acg atg gca gtt       48
   Met Asn Asn Pro Ser Leu Leu Asn His Ala Val Glu Thr Met Ala Val
   1               5                   10                  15
   ggc tcg aaa agt ttt gcg aca gcc tca aag tta ttt gat gca aaa acc       96
   Gly Ser Lys Ser Phe Ala Thr Ala Ser Lys Leu Phe Asp Ala Lys Thr
               20                  25                  30
   cgg cgc agc gta ctg atg ctc tac gcc tgg tgc cgc cat tgt gac gat      144
   Arg Arg Ser Val Leu Met Leu Tyr Ala Trp Cys Arg His Cys Asp Asp
           35                  40                  45
   gtt att gac gat cag acg ctg ggc ttt cag gcc cgg cag cct gcc tta      192
   Val Ile Asp Asp Gln Thr Leu Gly Phe Gln Ala Arg Gln Pro Ala Leu
       50                  55                  60
   caa acg ccc gaa caa cgt ctg atg caa ctt gag atg aaa acg cgc cag      240
   Gln Thr Pro Glu Gln Arg Leu Met Gln Leu Glu Met Lys Thr Arg Gln
   65                  70                  75                  80
   gcc tat gca gga tcg cag atg cac gaa ccg gcg ttt gcg gct ttt cag      288
   Ala Tyr Ala Gly Ser Gln Met His Glu Pro Ala Phe Ala Ala Phe Gln
                   85                  90                  95
   gaa gtg gct atg gct cat gat atc gcc ccg gct tac gcg ttt gat cat      336
   Glu Val Ala Met Ala His Asp Ile Ala Pro Ala Tyr Ala Phe Asp His
               100                 105                 110
   ctg gaa ggc ttc gcc atg gat gta cgc gaa gcg caa tac agc caa ctg      384
   Leu Glu Gly Phe Ala Met Asp Val Arg Glu Ala Gln Tyr Ser Gln Leu
           115                 120                 125
   gat gat acg ctg cgc tat tgc tat cac gtt gca ggc gtt gtc ggc ttg      432
   Asp Asp Thr Leu Arg Tyr Cys Tyr His Val Ala Gly Val Val Gly Leu
       130                 135                 140
   atg atg gcg caa atc atg ggc gtg cgg gat aac gcc acg ctg gac cgc      480
   Met Met Ala Gln Ile Met Gly Val Arg Asp Asn Ala Thr Leu Asp Arg
   145                 150                 155                 160
   gcc tgt gac ctt ggg ctg gca ttt cag ttg acc aat att gct cgc gat      528
   Ala Cys Asp Leu Gly Leu Ala Phe Gln Leu Thr Asn Ile Ala Arg Asp
                   165                 170                 175
   att gtg gac gat gcg cat gcg ggc cgc tgt tat ctg ccg gca agc tgg      576
   Ile Val Asp Asp Ala His Ala Gly Arg Cys Tyr Leu Pro Ala Ser Trp
               180                 185                 190
   ctg gag cat gaa ggt ctg aac aaa gag aat tat gcg gca cct gaa aac      624
   Leu Glu His Glu Gly Leu Asn Lys Glu Asn Tyr Ala Ala Pro Glu Asn
           195                 200                 205
   cgt cag gcg ctg agc cgt atc gcc cgt cgt ttg gtg cag gaa gca gaa      672
   Arg Gln Ala Leu Ser Arg Ile Ala Arg Arg Leu Val Gln Glu Ala Glu
       210                 215                 220
   cct tac tat ttg tct gcc aca gcc ggc ctg gca ggg ttg ccc ctg cgt      720
   Pro Tyr Tyr Leu Ser Ala Thr Ala Gly Leu Ala Gly Leu Pro Leu Arg
   225                 230                 235                 240
   tcc gcc tgg gca atc gct acg gcg aag cag gtt tac cgg aaa ata ggt      768
   Ser Ala Trp Ala Ile Ala Thr Ala Lys Gln Val Tyr Arg Lys Ile Gly
                   245                 250                 255
   gtc aaa gtt gaa cag gcc ggt cag caa gcc tgg gat cag cgg cag tca      816
   Val Lys Val Glu Gln Ala Gly Gln Gln Ala Trp Asp Gln Arg Gln Ser
               260                 265                 270
   acg acc acg ccc gaa aaa tta acg ctg ctg ctg gcc gcc tct ggt cag      864
   Thr Thr Thr Pro Glu Lys Leu Thr Leu Leu Leu Ala Ala Ser Gly Gln
           275                 280                 285
   gcc ctt act tcc cgg atg cgg gct cat cct ccc cgc cct gcg cat ctc      912
   Ala Leu Thr Ser Arg Met Arg Ala His Pro Pro Arg Pro Ala His Leu
       290                 295                 300
   tgg cag cgc ccg ctc tag                                              930
   Trp Gln Arg Pro Leu
   305
<210> 128
   <211> 309
   <212> PRT
   <213> Erwinia uredovora
<400> 128
   Met Asn Asn Pro Ser Leu Leu Asn His Ala Val Glu Thr Met Ala Val
   1               5                   10                  15
   Gly Ser Lys Ser Phe Ala Thr Ala Ser Lys Leu Phe Asp Ala Lys Thr
               20                  25                  30
   Arg Arg Ser Val Leu Met Leu Tyr Ala Trp Cys Arg His Cys Asp Asp
           35                  40                  45
   Val Ile Asp Asp Gln Thr Leu Gly Phe Gln Ala Arg Gln Pro Ala Leu
       50                  55                  60
   Gln Thr Pro Glu Gln Arg Leu Met Gln Leu Glu Met Lys Thr Arg Gln
   65                  70                  75                  80
   Ala Tyr Ala Gly Ser Gln Met His Glu Pro Ala Phe Ala Ala Phe Gln
                   85                  90                  95
   Glu Val Ala Met Ala His Asp Ile Ala Pro Ala Tyr Ala Phe Asp His
               100                 105                 110
   Leu Glu Gly Phe Ala Met Asp Val Arg Glu Ala Gln Tyr Ser Gln Leu
           115                 120                 125
   Asp Asp Thr Leu Arg Tyr Cys Tyr His Val Ala Gly Val Val Gly Leu
       130                 135                 140
   Met Met Ala Gln Ile Met Gly Val Arg Asp Asn Ala Thr Leu Asp Arg
   145                 150                 155                 160
   Ala Cys Asp Leu Gly Leu Ala Phe Gln Leu Thr Asn Ile Ala Arg Asp
   165 170 175
   Ile Val Asp Asp Ala His Ala Gly Arg Cys Tyr Leu Pro Ala Ser Trp
   180 185 190
   Leu Glu His Glu Gly Leu Asn Lys Glu Asn Tyr Ala Ala Pro Glu Asn
   195 200 205
   Arg Gln Ala Leu Ser Arg Ile Ala Arg Arg Leu Val Gln Glu Ala Glu
   210 215 220
   Pro Tyr Tyr Leu Ser Ala Thr Ala Gly Leu Ala Gly Leu Pro Leu Arg
   225 230 235 240
   Ser Ala Trp Ala Ile Ala Thr Ala Lys Gln Val Tyr Arg Lys Ile Gly
   245 250 255
   Val Lys Val Glu Gln Ala Gly Gln Gln Ala Trp Asp Gln Arg Gln Ser
   260 265 270
   Thr Thr Thr Pro Glu Lys Leu Thr Leu Leu Leu Ala Ala Ser Gly Gln
   275 280 285
   Ala Leu Thr Ser Arg Met Arg Ala His Pro Pro Arg Pro Ala His Leu
   290 295 300
   Trp Gln Arg Pro Leu
   305
<210> 129
   <211> 1479
   <212> DNA
   <213> Erwinia uredovora
<220>
   <221> CDS
   <222> (1)..(1479)
   <223>
<400> 129
   atg aaa cca act acg gta att ggt gca ggc ttc ggt ggc ctg gca ctg        48
   Met Lys Pro Thr Thr Val Ile Gly Ala Gly Phe Gly Gly Leu Ala Leu
   1               5                   10                  15
   gca att cgt cta caa gct gcg ggg atc ccc gtc tta ctg ctt gaa caa        96
   Ala Ile Arg Leu Gln Ala Ala Gly Ile Pro Val Leu Leu Leu Glu Gln
               20                  25                  30
   cgt gat aaa ccc ggc ggt cgg gct tat gtc tac gag gat cag ggg ttt       144
   Arg Asp Lys Pro Gly Gly Arg Ala Tyr Val Tyr Glu Asp Gln Gly Phe
           35                  40                  45
   acc ttt gat gca ggc ccg acg gtt atc acc gat ccc agt gcc att gaa       192
   Thr Phe Asp Ala Gly Pro Thr Val Ile Thr Asp Pro Ser Ala Ile Glu
       50                  55                  60
   gaa ctg ttt gca ctg gca gga aaa cag tta aaa gag tat gtc gaa ctg       240
   Glu Leu Phe Ala Leu Ala Gly Lys Gln Leu Lys Glu Tyr Val Glu Leu
   65                  70                  75                  80
   ctg ccg gtt acg ccg ttt tac cgc ctg tgt tgg gag tca ggg aag gtc       288
   Leu Pro Val Thr Pro Phe Tyr Arg Leu Cys Trp Glu Ser Gly Lys Val
                   85                  90                  95
   ttt aat tac gat aac gat caa acc cgg ctc gaa gcg cag att cag cag       336
   Phe Asn Tyr Asp Asn Asp Gln Thr Arg Leu Glu Ala Gln Ile Gln Gln
               100                 105                 110
   ttt aat ccc cgc gat gtc gaa ggt tat cgt cag ttt ctg gac tat tca       384
   Phe Asn Pro Arg Asp Val Glu Gly Tyr Arg Gln Phe Leu Asp Tyr Ser
           115                 120                 125
   cgc gcg gtg ttt aaa gaa ggc tat cta aag ctc ggt act gtc cct ttt       432
   Arg Ala Val Phe Lys Glu Gly Tyr Leu Lys Leu Gly Thr Val Pro Phe
       130                 135                 140
   tta tcg ttc aga gac atg ctt cgc gcc gca cct caa ctg gcg aaa ctg       480
   Leu Ser Phe Arg Asp Met Leu Arg Ala Ala Pro Gln Leu Ala Lys Leu
   145                 150                 155                 160
   cag gca tgg aga agc gtt tac agt aag gtt gcc agt tac atc gaa gat       528
   Gln Ala Trp Arg Ser Val Tyr Ser Lys Val Ala Ser Tyr Ile Glu Asp
                   165                 170                 175
   gaa cat ctg cgc cag gcg ttt tct ttc cac tcg ctg ttg gtg ggc ggc       576
   Glu His Leu Arg Gln Ala Phe Ser Phe His Ser Leu Leu Val Gly Gly
               180                 185                 190
   aat ccc ttc gcc acc tca tcc att tat acg ttg ata cac gcg ctg gag      624
   Asn Pro Phe Ala Thr Ser Ser Ile Tyr Thr Leu Ile His Ala Leu Glu
           195                 200                 205
   cgt gag tgg ggc gtc tgg ttt ccg cgt ggc ggc acc ggc gca tta gtt      672
   Arg Glu Trp Gly Val Trp Phe Pro Arg Gly Gly Thr Gly Ala Leu Val
       210                 215                 220
   cag ggg atg ata aag ctg ttt cag gat ctg ggt ggc gaa gtc gtg tta      720
   Gln Gly Met Ile Lys Leu Phe Gln Asp Leu Gly Gly Glu Val Val Leu
   225                 230                 235                 240
   aac gcc aga gtc agc cat atg gaa acg aca gga aac aag att gaa gcc      768
   Asn Ala Arg Val Ser His Met Glu Thr Thr Gly Asn Lys Ile Glu Ala
                   245                 250                 255
   gtg cat tta gag gac ggt cgc agg ttc ctg acg caa gcc gtc gcg tca      816
   Val His Leu Glu Asp Gly Arg Arg Phe Leu Thr Gln Ala Val Ala Ser
               260                 265                 270
   aat gca gat gtg gtt cat acc tat cgc gac ctg tta agc cag cac cct      864
   Asn Ala Asp Val Val His Thr Tyr Arg Asp Leu Leu Ser Gln His Pro
           275                 280                 285
   gcc gcg gtt aag cag tcc aac aaa ctg cag act aag cgc atg agt aac      912
   Ala Ala Val Lys Gln Ser Asn Lys Leu Gln Thr Lys Arg Met Ser Asn
       290                 295                 300
   tct ctg ttt gtg ctc tat ttt ggt ttg aat cac cat cat gat cag ctc      960
   Ser Leu Phe Val Leu Tyr Phe Gly Leu Asn His His His Asp Gln Leu
   305                 310                 315                 320
   gcg cat cac acg gtt tgt ttc ggc ccg cgt tac cgc gag ctg att gac     1008
   Ala His His Thr Val Cys Phe Gly Pro Arg Tyr Arg Glu Leu Ile Asp
                   325                 330                 335
   gaa att ttt aat cat gat ggc ctc gca gag gac ttc tca ctt tat ctg     1056
   Glu Ile Phe Asn His Asp Gly Leu Ala Glu Asp Phe Ser Leu Tyr Leu
               340                 345                 350
   cac gcg ccc tgt gtc acg gat tcg tca ctg gcg cct gaa ggt tgc ggc     1104
   His Ala Pro Cys Val Thr Asp Ser Ser Leu Ala Pro Glu Gly Cys Gly
           355                 360                 365
   agt tac tat gtg ttg gcg ccg gtg ccg cat tta ggc acc gcg aac ctc     1152
   Ser Tyr Tyr Val Leu Ala Pro Val Pro His Leu Gly Thr Ala Asn Leu
       370                 375                 380
   gac tgg acg gtt gag ggg cca aaa cta cgc gac cgt att ttt gcg tac     1200
   Asp Trp Thr Val Glu Gly Pro Lys Leu Arg Asp Arg Ile Phe Ala Tyr
   385                 390                 395                 400
   ctt gag cag cat tac atg cct ggc tta cgg agt cag ctg gtc acg cac      1248
   Leu Glu Gln His Tyr Met Pro Gly Leu Arg Ser Gln Leu Val Thr His
                   405                 410                 415
   cgg atg ttt acg ccg ttt gat ttt cgc gac cag ctt aat gcc tat cat      1296
   Arg Met Phe Thr Pro Phe Asp Phe Arg Asp Gln Leu Asn Ala Tyr His
               420                 425                 430
   ggc tca gcc ttt tct gtg gag ccc gtt ctt acc cag agc gcc tgg ttt      1344
   Gly Ser Ala Phe Ser Val Glu Pro Val Leu Thr Gln Ser Ala Trp Phe
           435                 440                 445
   cgg ccg cat aac cgc gat aaa acc att act aat ctc tac ctg gtc ggc      1392
   Arg Pro His Asn Arg Asp Lys Thr Ile Thr Asn Leu Tyr Leu Val Gly
       450                 455                 460
   gca ggc acg cat ccc ggc gca ggc att cct ggc gtc atc ggc tcg gca      1440
   Ala Gly Thr His Pro Gly Ala Gly Ile Pro Gly Val Ile Gly Ser Ala
   465                 470                 475                 480
   aaa gcg aca gca ggt ttg atg ctg gag gat ctg ata tga                  1479
   Lys Ala Thr Ala Gly Leu Met Leu Glu Asp Leu Ile
                   485                 490
<210> 130
   <211> 492
   <212> PRT
   <213> Erwinia uredovora
<400> 130
   Met Lys Pro Thr Thr Val Ile Gly Ala Gly Phe Gly Gly Leu Ala Leu
   1               5                   10                  15
   Ala Ile Arg Leu Gln Ala Ala Gly Ile Pro Val Leu Leu Leu Glu Gln
               20                  25                  30
   Arg Asp Lys Pro Gly Gly Arg Ala Tyr Val Tyr Glu Asp Gln Gly Phe
           35                  40                  45
   Thr Phe Asp Ala Gly Pro Thr Val Ile Thr Asp Pro Ser Ala Ile Glu
       50                  55                  60
   Glu Leu Phe Ala Leu Ala Gly Lys Gln Leu Lys Glu Tyr Val Glu Leu
   65 70 75 80
   Leu Pro Val Thr Pro Phe Tyr Arg Leu Cys Trp Glu Ser Gly Lys Val
   85 90 95
   Phe Asn Tyr Asp Asn Asp Gln Thr Arg Leu Glu Ala Gln Ile Gln Gln
   100 105 110
   Phe Asn Pro Arg Asp Val Glu Gly Tyr Arg Gln Phe Leu Asp Tyr Ser
   115 120 125
   Arg Ala Val Phe Lys Glu Gly Tyr Leu Lys Leu Gly Thr Val Pro Phe
   130 135 140
   Leu Ser Phe Arg Asp Met Leu Arg Ala Ala Pro Gln Leu Ala Lys Leu
   145 150 155 160
   Gln Ala Trp Arg Ser Val Tyr Ser Lys Val Ala Ser Tyr Ile Glu Asp
   165 170 175
   Glu His Leu Arg Gln Ala Phe Ser Phe His Ser Leu Leu Val Gly Gly
   180 185 190
   Asn Pro Phe Ala Thr Ser Ser Ile Tyr Thr Leu Ile His Ala Leu Glu
   195 200 205
   Arg Glu Trp Gly Val Trp Phe Pro Arg Gly Gly Thr Gly Ala Leu Val
   210 215 220
   Gln Gly Met Ile Lys Leu Phe Gln Asp Leu Gly Gly Glu Val Val Leu
   225 230 235 240
   Asn Ala Arg Val Ser His Met Glu Thr Thr Gly Asn Lys Ile Glu Ala
   245 250 255
   Val His Leu Glu Asp Gly Arg Arg Phe Leu Thr Gln Ala Val Ala Ser
   260 265 270
   Asn Ala Asp Val Val His Thr Tyr Arg Asp Leu Leu Ser Gln His Pro
   275 280 285
   Ala Ala Val Lys Gln Ser Asn Lys Leu Gln Thr Lys Arg Met Ser Asn
   290 295 300
   Ser Leu Phe Val Leu Tyr Phe Gly Leu Asn His His His Asp Gln Leu
   305 310 315 320
   Ala His His Thr Val Cys Phe Gly Pro Arg Tyr Arg Glu Leu Ile Asp
   325 330 335
   Glu Ile Phe Asn His Asp Gly Leu Ala Glu Asp Phe Ser Leu Tyr Leu
   340 345 350
   His Ala Pro Cys Val Thr Asp Ser Ser Leu Ala Pro Glu Gly Cys Gly
   355 360 365
   Ser Tyr Tyr Val Leu Ala Pro Val Pro His Leu Gly Thr Ala Asn Leu
   370 375 380
   Asp Trp Thr Val Glu Gly Pro Lys Leu Arg Asp Arg Ile Phe Ala Tyr
   385 390 395 400
   Leu Glu Gln His Tyr Met Pro Gly Leu Arg Ser Gln Leu Val Thr His
   405 410 415
   Arg Met Phe Thr Pro Phe Asp Phe Arg Asp Gln Leu Asn Ala Tyr His
   420 425 430
   Gly Ser Ala Phe Ser Val Glu Pro Val Leu Thr Gln Ser Ala Trp Phe
   435 440 445
   Arg Pro His Asn Arg Asp Lys Thr Ile Thr Asn Leu Tyr Leu Val Gly
   450 455 460
   Ala Gly Thr His Pro Gly Ala Gly Ile Pro Gly Val Ile Gly Ser Ala
   465 470 475 480
   Lys Ala Thr Ala Gly Leu Met Leu Glu Asp Leu Ile
   485 490
<210> 131
   <211> 1725
   <212> DNA
   <213> Narcissus pseudonarcissus
<220>
   <221> CDS
   <222> (1)..(1725)
   <223>
<400> 131
   atg gct tct tcc act tgt tta att cat tct tcc tct ttt ggg gtt gga      48
   Met Ala Ser Ser Thr Cys Leu Ile His Ser Ser Ser Phe Gly Val Gly
   1               5                   10                  15
   gga aag aaa gtg aag atg aac acg atg att cga tcg aag ttg ttt tca      96
   Gly Lys Lys Val Lys Met Asn Thr Met Ile Arg Ser Lys Leu Phe Ser
               20                  25                  30
   att cgg tcg gct ttg gac act aag gtg tct gat atg agc gtc aat gct      144
   Ile Arg Ser Ala Leu Asp Thr Lys Val Ser Asp Met Ser Val Asn Ala
           35                  40                  45
   cca aaa gga ttg ttt cca cca gag cct gag cac tac agg ggg cca aag      192
   Pro Lys Gly Leu Phe Pro Pro Glu Pro Glu His Tyr Arg Gly Pro Lys
       50                  55                  60
   ctt aaa gtg gct atc att gga gct ggg ctc gct ggc atg tca act gca      240
   Leu Lys Val Ala Ile Ile Gly Ala Gly Leu Ala Gly Met Ser Thr Ala
   65                  70                  75                  80
   gtg gag ctt ttg gat caa ggg cat gag gtt gac ata tat gaa tcc aga      288
   Val Glu Leu Leu Asp Gln Gly His Glu Val Asp Ile Tyr Glu Ser Arg
                   85                  90                  95
   caa ttt att ggt ggt aaa gtc ggt tct ttt gta gat aag cgt gga aac      336
   Gln Phe Ile Gly Gly Lys Val Gly Ser Phe Val Asp Lys Arg Gly Asn
               100                 105                 110
   cat att gaa atg gga ctc cat gtg ttt ttt ggt tgc tat aac aat ctt      384
   His Ile Glu Met Gly Leu His Val Phe Phe Gly Cys Tyr Asn Asn Leu
           115                 120                 125
   ttc aga ctt atg aaa aag gta ggt gca gat gaa aat tta ctg gtg aag      432
   Phe Arg Leu Met Lys Lys Val Gly Ala Asp Glu Asn Leu Leu Val Lys
       130                 135                 140
   gat cat act cat acc ttt gta aac cga ggt gga gaa att ggt gaa ctt      480
   Asp His Thr His Thr Phe Val Asn Arg Gly Gly Glu Ile Gly Glu Leu
   145                 150                 155                 160
   gat ttc cga ctt ccg atg ggt gca cca tta cat ggt att cgt gca ttt      528
   Asp Phe Arg Leu Pro Met Gly Ala Pro Leu His Gly Ile Arg Ala Phe
                   165                 170                 175
   cta aca act aat caa ctg aag cct tat gat aaa gca agg aat gct gtg      576
   Leu Thr Thr Asn Gln Leu Lys Pro Tyr Asp Lys Ala Arg Asn Ala Val
               180                 185                 190
   gct ctt gcc ctt agc cca gtt gta cgt gct ctt att gat cca aat ggt      624
   Ala Leu Ala Leu Ser Pro Val Val Arg Ala Leu Ile Asp Pro Asn Gly
           195                 200                 205
   gca atg cag gat ata agg aac tta gat aat att agc ttt tct gat tgg      672
   Ala Met Gln Asp Ile Arg Asn Leu Asp Asn Ile Ser Phe Ser Asp Trp
       210                 215                 220
   ttc tta tcc aaa ggc ggt acc cgc atg agc atc caa agg atg tgg gat      720
   Phe Leu Ser Lys Gly Gly Thr Arg Met Ser Ile Gln Arg Met Trp Asp
   225                 230                 235                 240
   cca gtt gct tat gcc ctc gga ttt att gac tgt gat aat atc agt gcc      768
   Pro Val Ala Tyr Ala Leu Gly Phe Ile Asp Cys Asp Asn Ile Ser Ala
                   245                 250                 255
   cgt tgt atg ctt act ata ttt tct cta ttt gct act aag aca gaa gct      816
   Arg Cys Met Leu Thr Ile Phe Ser Leu Phe Ala Thr Lys Thr Glu Ala
               260                 265                 270
   tct ctg ttg cgt atg ttg aag ggt tcg cct gat gtt tac tta agc ggt      864
   Ser Leu Leu Arg Met Leu Lys Gly Ser Pro Asp Val Tyr Leu Ser Gly
           275                 280                 285
   cct ata aga aag tat att aca gat aaa ggt gga agg ttt cac cta agg      912
   Pro Ile Arg Lys Tyr Ile Thr Asp Lys Gly Gly Arg Phe His Leu Arg
       290                 295                 300
   tgg ggg tgt aga gag ata ctt tat gat gaa cta tca aat ggc gac aca      960
   Trp Gly Cys Arg Glu Ile Leu Tyr Asp Glu Leu Ser Asn Gly Asp Thr
   305                 310                 315                 320
   tat atc aca ggc att gca atg tcg aag gct acc aat aaa aaa ctt gtg     1008
   Tyr Ile Thr Gly Ile Ala Met Ser Lys Ala Thr Asn Lys Lys Leu Val
                   325                 330                 335
   aaa gct gac gtg tat gtt gca gca tgt gat gtt cct gga ata aaa agg     1056
   Lys Ala Asp Val Tyr Val Ala Ala Cys Asp Val Pro Gly Ile Lys Arg
               340                 345                 350
   ttg atc cca tcg gag tgg aga gaa tgg gat cta ttt gac aat atc tat      1104
   Leu Ile Pro Ser Glu Trp Arg Glu Trp Asp Leu Phe Asp Asn Ile Tyr
           355                 360                 365
   aaa cta gtt gga gtt cca gtt gtc act gtt cag ctt agg tac aat ggt      1152
   Lys Leu Val Gly Val Pro Val Val Thr Val Gln Leu Arg Tyr Asn Gly
       370                 375                 380
   tgg gtg aca gag atg caa gat ctg gaa aaa tca agg cag ttg aga gct      1200
   Trp Val Thr Glu Met Gln Asp Leu Glu Lys Ser Arg Gln Leu Arg Ala
   385                 390                 395                 400
   gca gta gga ttg gat aat ctt ctt tat act cca gat gca gac ttt tct      1248
   Ala Val Gly Leu Asp Asn Leu Leu Tyr Thr Pro Asp Ala Asp Phe Ser
                   405                 410                 415
   tgt ttt tct gat ctt gca ctc tcg tcg cct gaa gat tat tat att gaa      1296
   Cys Phe Ser Asp Leu Ala Leu Ser Ser Pro Glu Asp Tyr Tyr Ile Glu
               420                 425                 430
   gga caa ggg tcc cta ata cag gct gtt ctc acg cca ggg gat cca tac      1344
   Gly Gln Gly Ser Leu Ile Gln Ala Val Leu Thr Pro Gly Asp Pro Tyr
           435                 440                 445
   atg ccc cta cct aat gat gca att ata gaa aga gtt cgg aaa cag gtt      1392
   Met Pro Leu Pro Asn Asp Ala Ile Ile Glu Arg Val Arg Lys Gln Val
       450                 455                 460
   ttg gat tta ttc cca tcc tct caa ggc ctg gaa gtt cta tgg tct tcg      1440
   Leu Asp Leu Phe Pro Ser Ser Gln Gly Leu Glu Val Leu Trp Ser Ser
   465                 470                 475                 480
   gtg gtt aaa atc gga caa tcc cta tat cgg gag ggg cct gga aag gac      1488
   Val Val Lys Ile Gly Gln Ser Leu Tyr Arg Glu Gly Pro Gly Lys Asp
                   485                 490                 495
   cca ttc aga cct gat cag aag aca cca gta aaa aat ttc ttc ctt gca      1536
   Pro Phe Arg Pro Asp Gln Lys Thr Pro Val Lys Asn Phe Phe Leu Ala
               500                 505                 510
   ggt tca tac acc aaa cag gat tac att gac agt atg gaa gga gcg acc      1584
   Gly Ser Tyr Thr Lys Gln Asp Tyr Ile Asp Ser Met Glu Gly Ala Thr
           515                 520                 525
   cta tcg ggg aga caa gca gct gca tat atc tgc agc gcc ggt gaa gat      1632
   Leu Ser Gly Arg Gln Ala Ala Ala Tyr Ile Cys Ser Ala Gly Glu Asp
       530                 535                 540
   ctg gca gca ctt cgc aag aag atc gct gct gat cat cca gag caa ctg      1680
   Leu Ala Ala Leu Arg Lys Lys Ile Ala Ala Asp His Pro Glu Gln Leu
   545                 550                 555                 560
   atc aac aaa gat tct aac gtg tcg gat gaa ctg agt ctc gta taa          1725
   Ile Asn Lys Asp Ser Asn Val Ser Asp Glu Leu Ser Leu Val
   565 570
<210> 132
   <211> 574
   <212> PRT
   <213> Narcissus pseudonarcissus
<400> 132
   Met Ala Ser Ser Thr Cys Leu Ile His Ser Ser Ser Phe Gly Val Gly
   1 5 10 15
   Gly Lys Lys Val Lys Met Asn Thr Met Ile Arg Ser Lys Leu Phe Ser
   20 25 30
   Ile Arg Ser Ala Leu Asp Thr Lys Val Ser Asp Met Ser Val Asn Ala
   35 40 45
   Pro Lys Gly Leu Phe Pro Pro Glu Pro Glu His Tyr Arg Gly Pro Lys
   50 55 60
   Leu Lys Val Ala Ile Ile Gly Ala Gly Leu Ala Gly Met Ser Thr Ala
   65 70 75 80
   Val Glu Leu Leu Asp Gln Gly His Glu Val Asp Ile Tyr Glu Ser Arg
   85 90 95
   Gln Phe Ile Gly Gly Lys Val Gly Ser Phe Val Asp Lys Arg Gly Asn
   100 105 110
   His Ile Glu Met Gly Leu His Val Phe Phe Gly Cys Tyr Asn Asn Leu
   115 120 125
   Phe Arg Leu Met Lys Lys Val Gly Ala Asp Glu Asn Leu Leu Val Lys
   130 135 140
   Asp His Thr His Thr Phe Val Asn Arg Gly Gly Glu Ile Gly Glu Leu
   145 150 155 160
   Asp Phe Arg Leu Pro Met Gly Ala Pro Leu His Gly Ile Arg Ala Phe
                   165                 170                 175
   Leu Thr Thr Asn Gln Leu Lys Pro Tyr Asp Lys Ala Arg Asn Ala Val
               180                 185                 190
   Ala Leu Ala Leu Ser Pro Val Val Arg Ala Leu Ile Asp Pro Asn Gly
           195                 200                 205
   Ala Met Gln Asp Ile Arg Asn Leu Asp Asn Ile Ser Phe Ser Asp Trp
       210                 215                 220
   Phe Leu Ser Lys Gly Gly Thr Arg Met Ser Ile Gln Arg Met Trp Asp
   225                 230                 235                 240
   Pro Val Ala Tyr Ala Leu Gly Phe Ile Asp Cys Asp Asn Ile Ser Ala
                   245                 250                 255
   Arg Cys Met Leu Thr Ile Phe Ser Leu Phe Ala Thr Lys Thr Glu Ala
               260                 265                 270
   Ser Leu Leu Arg Met Leu Lys Gly Ser Pro Asp Val Tyr Leu Ser Gly
           275                 280                 285
   Pro Ile Arg Lys Tyr Ile Thr Asp Lys Gly Gly Arg Phe His Leu Arg
       290                 295                 300
   Trp Gly Cys Arg Glu Ile Leu Tyr Asp Glu Leu Ser Asn Gly Asp Thr
   305                 310                 315                 320
   Tyr Ile Thr Gly Ile Ala Met Ser Lys Ala Thr Asn Lys Lys Leu Val
                   325                 330                 335
   Lys Ala Asp Val Tyr Val Ala Ala Cys Asp Val Pro Gly Ile Lys Arg
               340                 345                 350
   Leu Ile Pro Ser Glu Trp Arg Glu Trp Asp Leu Phe Asp Asn Ile Tyr
           355                 360                 365
   Lys Leu Val Gly Val Pro Val Val Thr Val Gln Leu Arg Tyr Asn Gly
       370                 375                 380
   Trp Val Thr Glu Met Gln Asp Leu Glu Lys Ser Arg Gln Leu Arg Ala
   385                 390                 395                 400
   Ala Val Gly Leu Asp Asn Leu Leu Tyr Thr Pro Asp Ala Asp Phe Ser
                   405                 410                 415
   Cys Phe Ser Asp Leu Ala Leu Ser Ser Pro Glu Asp Tyr Tyr Ile Glu
               420                 425                 430
   Gly Gln Gly Ser Leu Ile Gln Ala Val Leu Thr Pro Gly Asp Pro Tyr
           435                 440                 445
   Met Pro Leu Pro Asn Asp Ala Ile Ile Glu Arg Val Arg Lys Gln Val
       450                 455                 460
   Leu Asp Leu Phe Pro Ser Ser Gln Gly Leu Glu Val Leu Trp Ser Ser
   465                 470                 475                 480
   Val Val Lys Ile Gly Gln Ser Leu Tyr Arg Glu Gly Pro Gly Lys Asp
                   485                 490                 495
   Pro Phe Arg Pro Asp Gln Lys Thr Pro Val Lys Asn Phe Phe Leu Ala
               500                 505                 510
   Gly Ser Tyr Thr Lys Gln Asp Tyr Ile Asp Ser Met Glu Gly Ala Thr
           515                 520                 525
   Leu Ser Gly Arg Gln Ala Ala Ala Tyr Ile Cys Ser Ala Gly Glu Asp
       530                 535                 540
   Leu Ala Ala Leu Arg Lys Lys Ile Ala Ala Asp His Pro Glu Gln Leu
   545                 550                 555                 560
   Ile Asn Lys Asp Ser Asn Val Ser Asp Glu Leu Ser Leu Val
                   565                 570
<210> 133
   <211> 1848
   <212> DNA
   <213> Lycopersicon esculentum
<220>
   <221> CDS
   <222> (1)..(1848)
   <223>
<400> 133
   atg tgt acc ttg agt ttt atg tat cct aat tca ctt ctt gat ggt acc        48
   Met Cys Thr Leu Ser Phe Met Tyr Pro Asn Ser Leu Leu Asp Gly Thr
   1               5                   10                  15
   tgc aag act gta gct ttg ggt gat agc aaa cca aga tac aat aaa cag        96
   Cys Lys Thr Val Ala Leu Gly Asp Ser Lys Pro Arg Tyr Asn Lys Gln
               20                  25                  30
   aga agt tct tgt ttt gac cct ttg ata att gga aat tgt act gat cag       144
   Arg Ser Ser Cys Phe Asp Pro Leu Ile Ile Gly Asn Cys Thr Asp Gln
           35                  40                  45
   cag cag ctt tgt ggc ttg agt tgg ggg gtg gac aag gct aag gga aga       192
   Gln Gln Leu Cys Gly Leu Ser Trp Gly Val Asp Lys Ala Lys Gly Arg
       50                  55                  60
   aga ggg ggt act gtt tcc aat ttg aaa gca gtt gta gat gta gac aaa       240
   Arg Gly Gly Thr Val Ser Asn Leu Lys Ala Val Val Asp Val Asp Lys
   65                  70                  75                  80
   aga gtg gag agc tat ggc agt agt gat gta gaa gga aat gag agt ggc       288
   Arg Val Glu Ser Tyr Gly Ser Ser Asp Val Glu Gly Asn Glu Ser Gly
                   85                  90                  95
   agc tat gat gcc att gtt ata ggt tca gga ata ggt gga ttg gtg gca       336
   Ser Tyr Asp Ala Ile Val Ile Gly Ser Gly Ile Gly Gly Leu Val Ala
               100                 105                 110
   gcg acg cag ctg gcg gtt aag gga gct aag gtt tta gtt ctg gag aag       384
   Ala Thr Gln Leu Ala Val Lys Gly Ala Lys Val Leu Val Leu Glu Lys
           115                 120                 125
   tat gtt att cct ggt gga agc tct ggc ttt tac gag agg gat ggt tat       432
   Tyr Val Ile Pro Gly Gly Ser Ser Gly Phe Tyr Glu Arg Asp Gly Tyr
       130                 135                 140
   aag ttt gat gtt ggt tca tca gtg atg ttt gga ttc agt gat aag gga      480
   Lys Phe Asp Val Gly Ser Ser Val Met Phe Gly Phe Ser Asp Lys Gly
   145                 150                 155                 160
   aac ctc aat tta att act caa gca ttg gca gca gta gga cgt aaa tta      528
   Asn Leu Asn Leu Ile Thr Gln Ala Leu Ala Ala Val Gly Arg Lys Leu
                   165                 170                 175
   gaa gtt ata cct gac cca aca act gta cat ttc cac ctg cca aat gac      576
   Glu Val Ile Pro Asp Pro Thr Thr Val His Phe His Leu Pro Asn Asp
               180                 185                 190
   ctt tct gtt cgt ata cac cga gag tat gat gac ttc att gaa gag ctt      624
   Leu Ser Val Arg Ile His Arg Glu Tyr Asp Asp Phe Ile Glu Glu Leu
           195                 200                 205
   gtg agt aaa ttt cca cat gaa aag gaa ggg att atc aaa ttt tac agt      672
   Val Ser Lys Phe Pro His Glu Lys Glu Gly Ile Ile Lys Phe Tyr Ser
       210                 215                 220
   gaa tgc tgg aag atc ttt aat tct ctg aat tca ttg gaa ctg aag tct      720
   Glu Cys Trp Lys Ile Phe Asn Ser Leu Asn Ser Leu Glu Leu Lys Ser
   225                 230                 235                 240
   ttg gag gaa ccc atc tac ctt ttt ggc cag ttc ttt aag aag ccc ctt      768
   Leu Glu Glu Pro Ile Tyr Leu Phe Gly Gln Phe Phe Lys Lys Pro Leu
                   245                 250                 255
   gaa tgc ttg act ctt gcc tac tat ttg ccc cag aat gct ggt agc atc      816
   Glu Cys Leu Thr Leu Ala Tyr Tyr Leu Pro Gln Asn Ala Gly Ser Ile
               260                 265                 270
   gct cgg aag tat ata aga gat cct ggg ttg ctg tct ttt ata gat gca      864
   Ala Arg Lys Tyr Ile Arg Asp Pro Gly Leu Leu Ser Phe Ile Asp Ala
           275                 280                 285
   gag tgc ttt atc gtg agt aca gtt aat gca tta caa aca cca atg atc      912
   Glu Cys Phe Ile Val Ser Thr Val Asn Ala Leu Gln Thr Pro Met Ile
       290                 295                 300
   aat gca agc atg gtt cta tgt gac aga cat ttt ggc gga atc aac tac      960
   Asn Ala Ser Met Val Leu Cys Asp Arg His Phe Gly Gly Ile Asn Tyr
   305                 310                 315                 320
   ccc gtt ggt gga gtt ggc gag atc gcc aaa tcc tta gca aaa ggc ttg     1008
   Pro Val Gly Gly Val Gly Glu Ile Ala Lys Ser Leu Ala Lys Gly Leu
                   325                 330                 335
   gat gat cac gga agt cag ata ctt tat agg gca aat gtt aca agt atc     1056
   Asp Asp His Gly Ser Gln Ile Leu Tyr Arg Ala Asn Val Thr Ser Ile
               340                 345                 350
   att ttg gac aat ggc aaa gct gtg gga gtg aag ctt tct gac ggg agg     1104
   Ile Leu Asp Asn Gly Lys Ala Val Gly Val Lys Leu Ser Asp Gly Arg
           355                 360                 365
   aag ttt tat gct aaa acc ata gta tcg aat gct acc aga tgg gat act      1152
   Lys Phe Tyr Ala Lys Thr Ile Val Ser Asn Ala Thr Arg Trp Asp Thr
       370                 375                 380
   ttt gga aag ctt tta aaa gct gag aat ctg cca aaa gaa gaa gaa aat      1200
   Phe Gly Lys Leu Leu Lys Ala Glu Asn Leu Pro Lys Glu Glu Glu Asn
   385                 390                 395                 400
   ttc cag aaa gct tat gta aaa gca cct tct ttt ctt tct att cat atg      1248
   Phe Gln Lys Ala Tyr Val Lys Ala Pro Ser Phe Leu Ser Ile His Met
                   405                 410                 415
   gga gtt aaa gca gat gta ctc cca cca gac aca gat tgt cac cat ttt      1296
   Gly Val Lys Ala Asp Val Leu Pro Pro Asp Thr Asp Cys His His Phe
               420                 425                 430
   gtc ctc gag gat gat tgg aca aat ttg gag aaa cca tat gga agt ata      1344
   Val Leu Glu Asp Asp Trp Thr Asn Leu Glu Lys Pro Tyr Gly Ser Ile
           435                 440                 445
   ttc ttg agt att cca aca gtt ctt gat tcc tca ttg gcc cca gaa gga      1392
   Phe Leu Ser Ile Pro Thr Val Leu Asp Ser Ser Leu Ala Pro Glu Gly
       450                 455                 460
   cac cat att ctt cac att ttt aca aca tcg agc att gaa gat tgg gag      1440
   His His Ile Leu His Ile Phe Thr Thr Ser Ser Ile Glu Asp Trp Glu
   465                 470                 475                 480
   gga ctc tct ccg aaa gac tat gaa gcg aag aaa gag gtt gtt gct gaa      1488
   Gly Leu Ser Pro Lys Asp Tyr Glu Ala Lys Lys Glu Val Val Ala Glu
                   485                 490                 495
   agg att ata agc aga ctt gaa aaa aca ctc ttc cca ggg ctt aag tca      1536
   Arg Ile Ile Ser Arg Leu Glu Lys Thr Leu Phe Pro Gly Leu Lys Ser
               500                 505                 510
   tct att ctc ttt aag gag gtg gga act cca aag acc cac aga cga tac      1584
   Ser Ile Leu Phe Lys Glu Val Gly Thr Pro Lys Thr His Arg Arg Tyr
           515                 520                 525
   ctt gct cgt gat agt ggt acc tat gga cca atg cca cgc gga aca cct      1632
   Leu Ala Arg Asp Ser Gly Thr Tyr Gly Pro Met Pro Arg Gly Thr Pro
       530                 535                 540
   aag gga ctc ctg gga atg cct ttc aat acc act gct ata gat ggt cta      1680
   Lys Gly Leu Leu Gly Met Pro Phe Asn Thr Thr Ala Ile Asp Gly Leu
   545                 550                 555                 560
   tat tgt gtt ggc gat agt tgc ttc cca gga caa ggt gtt ata gct gta      1728
   Tyr Cys Val Gly Asp Ser Cys Phe Pro Gly Gln Gly Val Ile Ala Val
                   565                 570                 575
   gcc ttt tca gga gta atg tgc gct cat cgt gtt gca gct gac tta ggg      1776
   Ala Phe Ser Gly Val Met Cys Ala His Arg Val Ala Ala Asp Leu Gly
               580                 585                 590
   ttt gaa aaa aaa tca gat gtg ctg gac agt gct ctt ctt aga cta ctt      1824
   Phe Glu Lys Lys Ser Asp Val Leu Asp Ser Ala Leu Leu Arg Leu Leu
           595                 600                 605
   ggt tgg tta agg aca cta gca tga                                      1848
   Gly Trp Leu Arg Thr Leu Ala
       610                 615
<210> 134
   <211> 615
   <212> PRT
   <213> Lycopersicon esculentum
<400> 134
   Met Cys Thr Leu Ser Phe Met Tyr Pro Asn Ser Leu Leu Asp Gly Thr
   1               5                   10                  15
   Cys Lys Thr Val Ala Leu Gly Asp Ser Lys Pro Arg Tyr Asn Lys Gln
               20                  25                  30
   Arg Ser Ser Cys Phe Asp Pro Leu Ile Ile Gly Asn Cys Thr Asp Gln
           35                  40                  45
   Gln Gln Leu Cys Gly Leu Ser Trp Gly Val Asp Lys Ala Lys Gly Arg
       50                  55                  60
   Arg Gly Gly Thr Val Ser Asn Leu Lys Ala Val Val Asp Val Asp Lys
   65                  70                  75                  80
   Arg Val Glu Ser Tyr Gly Ser Ser Asp Val Glu Gly Asn Glu Ser Gly
                   85                  90                  95
   Ser Tyr Asp Ala Ile Val Ile Gly Ser Gly Ile Gly Gly Leu Val Ala
               100                 105                 110
   Ala Thr Gln Leu Ala Val Lys Gly Ala Lys Val Leu Val Leu Glu Lys
   115 120 125
   Tyr Val Ile Pro Gly Gly Ser Ser Gly Phe Tyr Glu Arg Asp Gly Tyr
   130 135 140
   Lys Phe Asp Val Gly Ser Ser Val Met Phe Gly Phe Ser Asp Lys Gly
   145 150 155 160
   Asn Leu Asn Leu Ile Thr Gln Ala Leu Ala Ala Val Gly Arg Lys Leu
   165 170 175
   Glu Val Ile Pro Asp Pro Thr Thr Val His Phe His Leu Pro Asn Asp
   180 185 190
   Leu Ser Val Arg Ile His Arg Glu Tyr Asp Asp Phe Ile Glu Glu Leu
   195 200 205
   Val Ser Lys Phe Pro His Glu Lys Glu Gly Ile Ile Lys Phe Tyr Ser
   210 215 220
   Glu Cys Trp Lys Ile Phe Asn Ser Leu Asn Ser Leu Glu Leu Lys Ser
   225 230 235 240
   Leu Glu Glu Pro Ile Tyr Leu Phe Gly Gln Phe Phe Lys Lys Pro Leu
   245 250 255
   Glu Cys Leu Thr Leu Ala Tyr Tyr Leu Pro Gln Asn Ala Gly Ser Ile
   260 265 270
   Ala Arg Lys Tyr Ile Arg Asp Pro Gly Leu Leu Ser Phe Ile Asp Ala
   275 280 285
   Glu Cys Phe Ile Val Ser Thr Val Asn Ala Leu Gln Thr Pro Met Ile
   290 295 300
   Asn Ala Ser Met Val Leu Cys Asp Arg His Phe Gly Gly Ile Asn Tyr
   305 310 315 320
   Pro Val Gly Gly Val Gly Glu Ile Ala Lys Ser Leu Ala Lys Gly Leu
   325 330 335
   Asp Asp His Gly Ser Gln Ile Leu Tyr Arg Ala Asn Val Thr Ser Ile
   340 345 350
   Ile Leu Asp Asn Gly Lys Ala Val Gly Val Lys Leu Ser Asp Gly Arg
   355 360 365
   Lys Phe Tyr Ala Lys Thr Ile Val Ser Asn Ala Thr Arg Trp Asp Thr
   370 375 380
   Phe Gly Lys Leu Leu Lys Ala Glu Asn Leu Pro Lys Glu Glu Glu Asn
   385 390 395 400
   Phe Gln Lys Ala Tyr Val Lys Ala Pro Ser Phe Leu Ser Ile His Met
   405 410 415
   Gly Val Lys Ala Asp Val Leu Pro Pro Asp Thr Asp Cys His His Phe
   420 425 430
   Val Leu Glu Asp Asp Trp Thr Asn Leu Glu Lys Pro Tyr Gly Ser Ile
   435 440 445
   Phe Leu Ser Ile Pro Thr Val Leu Asp Ser Ser Leu Ala Pro Glu Gly
   450 455 460
   His His Ile Leu His Ile Phe Thr Thr Ser Ser Ile Glu Asp Trp Glu
   465 470 475 480
   Gly Leu Ser Pro Lys Asp Tyr Glu Ala Lys Lys Glu Val Val Ala Glu
   485 490 495
   Arg Ile Ile Ser Arg Leu Glu Lys Thr Leu Phe Pro Gly Leu Lys Ser
   500 505 510
   Ser Ile Leu Phe Lys Glu Val Gly Thr Pro Lys Thr His Arg Arg Tyr
   515 520 525
   Leu Ala Arg Asp Ser Gly Thr Tyr Gly Pro Met Pro Arg Gly Thr Pro
   530 535 540
   Lys Gly Leu Leu Gly Met Pro Phe Asn Thr Thr Ala Ile Asp Gly Leu
   545                 550                 555                 560
   Tyr Cys Val Gly Asp Ser Cys Phe Pro Gly Gln Gly Val Ile Ala Val
                   565                 570                 575
   Ala Phe Ser Gly Val Met Cys Ala His Arg Val Ala Ala Asp Leu Gly
               580                 585                 590
   Phe Glu Lys Lys Ser Asp Val Leu Asp Ser Ala Leu Leu Arg Leu Leu
           595                 600                 605
   Gly Trp Leu Arg Thr Leu Ala
       610                 615
<210> 135
   <211> 1233
   <212> DNA
   <213> Tagetes erecta
<220>
   <221> CDS
   <222> (1)..(1233)
   <223>
<400> 135
   atg gcc aca cac aaa ctc ctt caa ttc acc acc aat ctc cca cca tct      48
   Met Ala Thr His Lys Leu Leu Gln Phe Thr Thr Asn Leu Pro Pro Ser
   1               5                   10                  15
   tct tct tca atc tct act ggc tgt tca ctc tcc ccc ttc ttc ctc aaa      96
   Ser Ser Ser Ile Ser Thr Gly Cys Ser Leu Ser Pro Phe Phe Leu Lys
               20                  25                  30
   tca tct tct cat tcc cct aac cct cgc cga cac cgc cgc tcc gcc gta     144
   Ser Ser Ser His Ser Pro Asn Pro Arg Arg His Arg Arg Ser Ala Val
           35                  40                  45
   tgc tgc tct ttc gcc tca ctc gac tct gca aaa atc aaa gtc gtt ggc     192
   Cys Cys Ser Phe Ala Ser Leu Asp Ser Ala Lys Ile Lys Val Val Gly
       50                  55                  60
   gtc ggt ggt ggt ggc aac aat gcc gtt aac cgc atg att ggt agc ggc      240
   Val Gly Gly Gly Gly Asn Asn Ala Val Asn Arg Met Ile Gly Ser Gly
   65                  70                  75                  80
   tta cag ggt gtt gat ttt tac gcc att aac acg gac tca caa gcg ctt      288
   Leu Gln Gly Val Asp Phe Tyr Ala Ile Asn Thr Asp Ser Gln Ala Leu
                   85                  90                  95
   ctg caa tct gtt gca cat aac cct att caa att ggg gag ctt ttg act      336
   Leu Gln Ser Val Ala His Asn Pro Ile Gln Ile Gly Glu Leu Leu Thr
               100                 105                 110
   cgt gga tta ggt act ggt ggg aac ccg ctt ttg gga gaa cag gct gcg      384
   Arg Gly Leu Gly Thr Gly Gly Asn Pro Leu Leu Gly Glu Gln Ala Ala
           115                 120                 125
   gag gag tcg aag gaa gcg att ggg aat gcg ctt aaa ggg tcg gat ctt      432
   Glu Glu Ser Lys Glu Ala Ile Gly Asn Ala Leu Lys Gly Ser Asp Leu
       130                 135                 140
   gtg ttt ata aca gca ggt atg ggt ggt ggg acg ggt tcg ggt gct gct      480
   Val Phe Ile Thr Ala Gly Met Gly Gly Gly Thr Gly Ser Gly Ala Ala
   145                 150                 155                 160
   cca gtt gta gcg cag ata gcg aaa gaa gca ggg tat tta act gtt ggt      528
   Pro Val Val Ala Gln Ile Ala Lys Glu Ala Gly Tyr Leu Thr Val Gly
                   165                 170                 175
   gtt gta acg tac cca ttc agc ttt gaa ggc cgt aaa aga tca gta cag      576
   Val Val Thr Tyr Pro Phe Ser Phe Glu Gly Arg Lys Arg Ser Val Gln
               180                 185                 190
   gcg tta gag gct att gag aag ctg caa aag aac gtt gac aca ctt ata      624
   Ala Leu Glu Ala Ile Glu Lys Leu Gln Lys Asn Val Asp Thr Leu Ile
           195                 200                 205
   gtg att cca aat gac cgt ttg ctg gat att gct gat gaa aac acg cct      672
   Val Ile Pro Asn Asp Arg Leu Leu Asp Ile Ala Asp Glu Asn Thr Pro
       210                 215                 220
   ctt cag gat gct ttt ctt ctt gct gat gat gta ctc cgc caa gga gtt      720
   Leu Gln Asp Ala Phe Leu Leu Ala Asp Asp Val Leu Arg Gln Gly Val
   225                 230                 235                 240
   caa gga atc tca gat ata att aca ata cct ggg ctg gta aat gtg gac      768
   Gln Gly Ile Ser Asp Ile Ile Thr Ile Pro Gly Leu Val Asn Val Asp
                   245                 250                 255
   ttt gca gac gtt aaa gca gtc atg aaa gat tct gga act gca atg ctt      816
   Phe Ala Asp Val Lys Ala Val Met Lys Asp Ser Gly Thr Ala Met Leu
               260                 265                 270
   ggt gtc ggt gtt tcc tca agt aaa aac cga gct gaa gaa gca gct gaa        864
   Gly Val Gly Val Ser Ser Ser Lys Asn Arg Ala Glu Glu Ala Ala Glu
           275                 280                 285
   caa gca act ctt gct cct ttg att gga tca tca att caa tct gct aca        912
   Gln Ala Thr Leu Ala Pro Leu Ile Gly Ser Ser Ile Gln Ser Ala Thr
       290                 295                 300
   ggt gtt gtt tat aat att acc gga ggg aag gac ata act cta caa gaa        960
   Gly Val Val Tyr Asn Ile Thr Gly Gly Lys Asp Ile Thr Leu Gln Glu
   305                 310                 315                 320
   gtc aac agg gtt tct cag gtg gta aca agt ttg gca gat cca tca gca       1008
   Val Asn Arg Val Ser Gln Val Val Thr Ser Leu Ala Asp Pro Ser Ala
                   325                 330                 335
   aac att ata ttc ggg gca gtg gta gat gag aga tac aac ggg gag att       1056
   Asn Ile Ile Phe Gly Ala Val Val Asp Glu Arg Tyr Asn Gly Glu Ile
               340                 345                 350
   cat gtg acc att gtt gct act ggc ttt gcc cag tcg ttt cag aaa tct       1104
   His Val Thr Ile Val Ala Thr Gly Phe Ala Gln Ser Phe Gln Lys Ser
           355                 360                 365
   ctt ctt gct gac ccg aaa gga gca aaa ctt gtt gat aga aat caa gaa       1152
   Leu Leu Ala Asp Pro Lys Gly Ala Lys Leu Val Asp Arg Asn Gln Glu
       370                 375                 380
   cct aca caa cct ttg act tcc gcg aga tct ttg aca aca cct tct cct       1200
   Pro Thr Gln Pro Leu Thr Ser Ala Arg Ser Leu Thr Thr Pro Ser Pro
   385                 390                 395                 400
   gct ccg tct cgg tct agg aaa ctc ttc ttt taa                           1233
   Ala Pro Ser Arg Ser Arg Lys Leu Phe Phe
                   405                 410
<210> 136
   <211> 410
   <212> PRT
   <213> Tagetes erecta
<400> 136
   Met Ala Thr His Lys Leu Leu Gln Phe Thr Thr Asn Leu Pro Pro Ser
   1               5                   10                  15
   Ser Ser Ser Ile Ser Thr Gly Cys Ser Leu Ser Pro Phe Phe Leu Lys
               20                  25                  30
   Ser Ser Ser His Ser Pro Asn Pro Arg Arg His Arg Arg Ser Ala Val
           35                  40                  45
   Cys Cys Ser Phe Ala Ser Leu Asp Ser Ala Lys Ile Lys Val Val Gly
       50                  55                  60
   Val Gly Gly Gly Gly Asn Asn Ala Val Asn Arg Met Ile Gly Ser Gly
   65                  70                  75                  80
   Leu Gln Gly Val Asp Phe Tyr Ala Ile Asn Thr Asp Ser Gln Ala Leu
                   85                  90                  95
   Leu Gln Ser Val Ala His Asn Pro Ile Gln Ile Gly Glu Leu Leu Thr
               100                 105                 110
   Arg Gly Leu Gly Thr Gly Gly Asn Pro Leu Leu Gly Glu Gln Ala Ala
           115                 120                 125
   Glu Glu Ser Lys Glu Ala Ile Gly Asn Ala Leu Lys Gly Ser Asp Leu
       130                 135                 140
   Val Phe Ile Thr Ala Gly Met Gly Gly Gly Thr Gly Ser Gly Ala Ala
   145                 150                 155                 160
   Pro Val Val Ala Gln Ile Ala Lys Glu Ala Gly Tyr Leu Thr Val Gly
                   165                 170                 175
   Val Val Thr Tyr Pro Phe Ser Phe Glu Gly Arg Lys Arg Ser Val Gln
               180                 185                 190
   Ala Leu Glu Ala Ile Glu Lys Leu Gln Lys Asn Val Asp Thr Leu Ile
           195                 200                 205
   Val Ile Pro Asn Asp Arg Leu Leu Asp Ile Ala Asp Glu Asn Thr Pro
       210                 215                 220
   Leu Gln Asp Ala Phe Leu Leu Ala Asp Asp Val Leu Arg Gln Gly Val
   225                 230                 235                 240
   Gln Gly Ile Ser Asp Ile Ile Thr Ile Pro Gly Leu Val Asn Val Asp
   245 250 255
   Phe Ala Asp Val Lys Ala Val Met Lys Asp Ser Gly Thr Ala Met Leu
   260 265 270
   Gly Val Gly Val Ser Ser Ser Lys Asn Arg Ala Glu Glu Ala Ala Glu
   275 280 285
   Gln Ala Thr Leu Ala Pro Leu Ile Gly Ser Ser Ile Gln Ser Ala Thr
   290 295 300
   Gly Val Val Tyr Asn Ile Thr Gly Gly Lys Asp Ile Thr Leu Gln Glu
   305 310 315 320
   Val Asn Arg Val Ser Gln Val Val Thr Ser Leu Ala Asp Pro Ser Ala
   325 330 335
   Asn Ile Ile Phe Gly Ala Val Val Asp Glu Arg Tyr Asn Gly Glu Ile
   340 345 350
   His Val Thr Ile Val Ala Thr Gly Phe Ala Gln Ser Phe Gln Lys Ser
   355 360 365
   Leu Leu Ala Asp Pro Lys Gly Ala Lys Leu Val Asp Arg Asn Gln Glu
   370 375 380
   Pro Thr Gln Pro Leu Thr Ser Ala Arg Ser Leu Thr Thr Pro Ser Pro
   385 390 395 400
   Ala Pro Ser Arg Ser Arg Lys Leu Phe Phe
   405 410
<210> 137
   <211> 891
   <212> DNA
   <213> Tagetes erecta <220>
   <221> CDS
   <222> (1)..(891)
   <223>
<400> 137
   atg aca tcc ctg agg ttt cta aca gaa ccc tca ctt gta tgc tca tcc       48
   Met Thr Ser Leu Arg Phe Leu Thr Glu Pro Ser Leu Val Cys Ser Ser
   1               5                   10                  15
   act ttc ccc aca ttc aat ccc cta cac aaa acc cta act aaa cca aca       96
   Thr Phe Pro Thr Phe Asn Pro Leu His Lys Thr Leu Thr Lys Pro Thr
               20                  25                  30
   cca aaa ccc tac cca aag cca cca cca att cgc tcc gtc ctt caa tac      144
   Pro Lys Pro Tyr Pro Lys Pro Pro Pro Ile Arg Ser Val Leu Gln Tyr
           35                  40                  45
   aat cgc aaa cca gag ctc gcc gga gac act cca cga gtc gtc gca atc      192
   Asn Arg Lys Pro Glu Leu Ala Gly Asp Thr Pro Arg Val Val Ala Ile
       50                  55                  60
   gac gcc gac gtt ggt cta cgt aac ctc gat ctt ctt ctc ggt ctc gaa      240
   Asp Ala Asp Val Gly Leu Arg Asn Leu Asp Leu Leu Leu Gly Leu Glu
   65                  70                  75                  80
   aac cgc gtc aat tac acc gtc gtt gaa gtt ctc aac ggc gat tgc aga      288
   Asn Arg Val Asn Tyr Thr Val Val Glu Val Leu Asn Gly Asp Cys Arg
                   85                  90                  95
   ctc gac caa gcc cta gtt cgt gat aaa cgc tgg tca aat ttc gaa ttg      336
   Leu Asp Gln Ala Leu Val Arg Asp Lys Arg Trp Ser Asn Phe Glu Leu
               100                 105                 110
   ctt tgt att tca aaa cct agg tca aaa ttg cct tta gga ttt ggg gga      384
   Leu Cys Ile Ser Lys Pro Arg Ser Lys Leu Pro Leu Gly Phe Gly Gly
           115                 120                 125
   aaa gct tta gtt tgg ctt gat gca tta aaa gat agg caa gaa ggt tgc      432
   Lys Ala Leu Val Trp Leu Asp Ala Leu Lys Asp Arg Gln Glu Gly Cys
       130                 135                 140
   ccg gat ttt ata ctt ata gat tgt cct gca ggt att gat gcc ggg ttc      480
   Pro Asp Phe Ile Leu Ile Asp Cys Pro Ala Gly Ile Asp Ala Gly Phe
   145                 150                 155                 160
   ata acc gcc att aca ccg gct aac gaa gcc gta tta gtt aca aca cct      528
   Ile Thr Ala Ile Thr Pro Ala Asn Glu Ala Val Leu Val Thr Thr Pro
                   165                 170                 175
   gat att act gca ttg aga gat gca gat aga gtt aca ggc ttg ctt gaa        576
   Asp Ile Thr Ala Leu Arg Asp Ala Asp Arg Val Thr Gly Leu Leu Glu
               180                 185                 190
   tgt gat gga att agg gat att aaa atg att gtg aac aga gtt aga act        624
   Cys Asp Gly Ile Arg Asp Ile Lys Met Ile Val Asn Arg Val Arg Thr
           195                 200                 205
   gat ttg ata agg ggt gaa gat atg atg tca gtt ctt gat gtt caa gag        672
   Asp Leu Ile Arg Gly Glu Asp Met Met Ser Val Leu Asp Val Gln Glu
       210                 215                 220
   atg ttg gga ttg tca ttg ttg agt gat acc cga gga ttc gaa gtg att        720
   Met Leu Gly Leu Ser Leu Leu Ser Asp Thr Arg Gly Phe Glu Val Ile
   225                 230                 235                 240
   cgg agt acg aat aga ggg ttt ccg ctt gtg ttg aac aag cct ccg act        768
   Arg Ser Thr Asn Arg Gly Phe Pro Leu Val Leu Asn Lys Pro Pro Thr
                   245                 250                 255
   tta gca gga ttg gca ttt gag cag gct gct tgg aga ttg gtt gag caa        816
   Leu Ala Gly Leu Ala Phe Glu Gln Ala Ala Trp Arg Leu Val Glu Gln
               260                 265                 270
   gat agc atg aag gct gtg atg gtg gag gaa gaa cct aaa aag agg gga        864
   Asp Ser Met Lys Ala Val Met Val Glu Glu Glu Pro Lys Lys Arg Gly
           275                 280                 285
   ttt ttc tcg ttt ttt gga ggt tag tga                                    891
   Phe Phe Ser Phe Phe Gly Gly
       290                 295
<210> 138
   <211> 295
   <212> PRT
   <213> Tagetes erecta
<400> 138
   Met Thr Ser Leu Arg Phe Leu Thr Glu Pro Ser Leu Val Cys Ser Ser
   1               5                   10                  15
   Thr Phe Pro Thr Phe Asn Pro Leu His Lys Thr Leu Thr Lys Pro Thr
               20                  25                  30
   Pro Lys Pro Tyr Pro Lys Pro Pro Pro Ile Arg Ser Val Leu Gln Tyr
   35 40 45
   Asn Arg Lys Pro Glu Leu Ala Gly Asp Thr Pro Arg Val Val Ala Ile
   50 55 60
   Asp Ala Asp Val Gly Leu Arg Asn Leu Asp Leu Leu Leu Gly Leu Glu
   65 70 75 80
   Asn Arg Val Asn Tyr Thr Val Val Glu Val Leu Asn Gly Asp Cys Arg
   85 90 95
   Leu Asp Gln Ala Leu Val Arg Asp Lys Arg Trp Ser Asn Phe Glu Leu
   100 105 110
   Leu Cys Ile Ser Lys Pro Arg Ser Lys Leu Pro Leu Gly Phe Gly Gly
   115 120 125
   Lys Ala Leu Val Trp Leu Asp Ala Leu Lys Asp Arg Gln Glu Gly Cys
   130 135 140
   Pro Asp Phe Ile Leu Ile Asp Cys Pro Ala Gly Ile Asp Ala Gly Phe
   145 150 155 160
   Ile Thr Ala Ile Thr Pro Ala Asn Glu Ala Val Leu Val Thr Thr Pro
   165 170 175
   Asp Ile Thr Ala Leu Arg Asp Ala Asp Arg Val Thr Gly Leu Leu Glu
   180 185 190
   Cys Asp Gly Ile Arg Asp Ile Lys Met Ile Val Asn Arg Val Arg Thr
   195 200 205
   Asp Leu Ile Arg Gly Glu Asp Met Met Ser Val Leu Asp Val Gln Glu
   210 215 220
   Met Leu Gly Leu Ser Leu Leu Ser Asp Thr Arg Gly Phe Glu Val Ile
   225 230 235 240
   Arg Ser Thr Asn Arg Gly Phe Pro Leu Val Leu Asn Lys Pro Pro Thr
                   245                 250                 255
   Leu Ala Gly Leu Ala Phe Glu Gln Ala Ala Trp Arg Leu Val Glu Gln
               260                 265                 270
   Asp Ser Met Lys Ala Val Met Val Glu Glu Glu Pro Lys Lys Arg Gly
           275                 280                 285
   Phe Phe Ser Phe Phe Gly Gly
       290                 295
<210> 139
   <211> 332
   <212> DNA
   <213> Tagetes erecta
<220>
   <221> CDS
   <222> (1)..(330)
   <223>
<400> 139
   aag ctt gca cga gcc tct ctc tat ttt tac act tca atg gcg gca gca        48
   Lys Leu Ala Arg Ala Ser Leu Tyr Phe Tyr Thr Ser Met Ala Ala Ala
   1               5                   10                  15
   att gct gtc cct tgt agc tca aga cca ttt ggc tta ggt cga atg cgg        96
   Ile Ala Val Pro Cys Ser Ser Arg Pro Phe Gly Leu Gly Arg Met Arg
               20                  25                  30
   tta ctt ggt cat aaa ccc aca acc ata act tgt cac ttc ccc ttt tct       144
   Leu Leu Gly His Lys Pro Thr Thr Ile Thr Cys His Phe Pro Phe Ser
           35                  40                  45
   ttt tct atc aaa tca ttt acc cca att gtt agg ggc aga aga tgt act       192
   Phe Ser Ile Lys Ser Phe Thr Pro Ile Val Arg Gly Arg Arg Cys Thr
       50                  55                  60
   gtt tgt ttt gtt gcc ggt ggc gac agt aat agt aac agt aat aat aat       240
   Val Cys Phe Val Ala Gly Gly Asp Ser Asn Ser Asn Ser Asn Asn Asn
   65                  70                  75                  80
   agt gac agt aat agt aat aat ccg ggt ctg gat tta aac ccg gcg gtt        288
   Ser Asp Ser Asn Ser Asn Asn Pro Gly Leu Asp Leu Asn Pro Ala Val
                   85                  90                  95
   atg aac cgt aac cgt ttg gtt gaa gaa aaa atg gag agg tcg ac             332
   Met Asn Arg Asn Arg Leu Val Glu Glu Lys Met Glu Arg Ser
               100                 105                 110
<210> 140
   <211> 110
   <212> PRT
   <213> Tagetes erecta
<400> 140
   Lys Leu Ala Arg Ala Ser Leu Tyr Phe Tyr Thr Ser Met Ala Ala Ala
   1               5                   10                  15
   Ile Ala Val Pro Cys Ser Ser Arg Pro Phe Gly Leu Gly Arg Met Arg
               20                  25                  30
   Leu Leu Gly His Lys Pro Thr Thr Ile Thr Cys His Phe Pro Phe Ser
           35                  40                  45
   Phe Ser Ile Lys Ser Phe Thr Pro Ile Val Arg Gly Arg Arg Cys Thr
       50                  55                  60
   Val Cys Phe Val Ala Gly Gly Asp Ser Asn Ser Asn Ser Asn Asn Asn
   65                  70                  75                  80
   Ser Asp Ser Asn Ser Asn Asn Pro Gly Leu Asp Leu Asn Pro Ala Val
                   85                  90                  95
   Met Asn Arg Asn Arg Leu Val Glu Glu Lys Met Glu Arg Ser
               100                 105                 110
<210> 141
   <211> 332
   <212> DNA
   <213> Tagetes erceta
<220>
   <221> misc_feature
   <222> (1)..(332)
   <223> b-Hydroxylase Sense-Fragment
<400> 141
   aagcttgcac gagcctctct ctatttttac acttcaatgg cggcagcaat tgctgtccct      60
   tgtagctcaa gaccatttgg cttaggtcga atgcggttac ttggtcataa acccacaacc     120
   ataacttgtc acttcccctt ttctttttct atcaaatcat ttaccccaat tgttaggggc     180
   agaagatgta ctgtttgttt tgttgccggt ggcgacagta atagtaacag taataataat     240
   agtgacagta atagtaataa tccgggtctg gatttaaacc cggcggttat gaaccgtaac     300
   cgtttggttg aagaaaaaat ggagaggtcg ac                                   332
<210> 142
   <211> 332
   <212> DNA
   <213> Tagetes erecta
<220>
   <221> misc_feature
   <222> (1)..(332)
   <223> b-Hydroxylase Antisense-Fragment
<400> 142
   gaattcggca cgagcctctc tctattttta cacttcaatg gcggcagcaa ttgctgtccc      60
   ttgtagctca agaccatttg gcttaggtcg aatgcggtta cttggtcata aacccacaac     120
   cataacttgt cacttcccct tttctttttc tatcaaatca tttaccccaa ttgttagggg     180
   cagaagatgt actgtttgtt ttgttgccgg tggcgacagt aatagtaaca gtaataataa     240
   tagtgacagt aatagtaata atccgggtct ggatttaaac ccggcggtta tgaaccgtaa     300
   ccgtttggtt gaagaaaaaa tggagaggat cc                                   332

## Beschreibung

Die vorliegende Erfindung betrifft die Verwendung von astaxanthinhaltigen Pflanzen oder Pflanzenteilen der Gattung Tagetes zur oralen Verabreichung an Tiere, Verfahren zur Herstellung von Tierfutterzubereitungen, ein Verfahren zum Pigmentieren von Tieren oder Tierprodukten sowie ein Verfahren zur Herstellung pigmentierter Tiere und Tierprodukte.

Aufgrund seiner farbgebenden Eigenschaften wird Astaxanthin als Pigmentierstoff in der Tierernährung, insbesondere in der Forelien-, Lachs- und Shrimpzucht verwendet.

Die Herstellung von Astaxanthin erfolgt heutzulage größtenteils durch chemische Syntheseverfahren. Natürliches Astaxanthin, wird heutzulage in biotechnologischen Ver∼ fahren in kleinen Mengen durch Kultivierung von Algen, beispielsweise *Haematococcus pluvialis* oder durch Fermentation von gentechnologisch optimierten Mikroorganismen und anschließender isolierung gewonnen.

Synthetisches oder durch Isolierung gewonnenes natürliches Astaxanthin wird durch spezielle. Formulierungstechniken zur Erhöhung der Lagerfähigkeit chemisch und/oder physikalich stabilisiert und für den jeweiligen Verwendungszweck entsprechend der gewünschten Appilkationsbereiche und Biovertügbarkeiten aufbereitet.

Im US "Code of Federal Regulations, title 21", Band 1, §73,295 der USA werden Extakte und Mehl von Tagetes beschrieben, die als Additive in Hühnerfutler eingesetzt werden und gegebenenfalls, mit Xanthophyll versetzt werden können. Solche Extrakte von Tagetes enthalten, da sie von gentechnisch unveränderten Pflanzen stammen kein Astaxanthin.

In der WO 99/07867 wird eine Methode beschrieben zur Veränderung des Xanthophyllgehalts in Samen von Ölpflanzen. Es wird weiter auf die Möglichkeit hingewiesen, entsprechende Caotinoide aus dem Öl entsprechend veränderter Pflanzen zu extrahieren.

WO00/32783 beschreibt Methoden zur Regulation des Carotinoidgehalts in Tagetes; Dafür werden Gene aus Tagetes zur Verfügung gestellt, die zur Überexpression oder Suppression endogener Gene in Tagetes verwendet werden sollen. Derartig hergestellte Tagetes enthalten kein Astaxanthin.

WO003/077950 beschreibt die Verwendung von Extrakten aus Tagetes als Grundstoff für die Synthese von Astaxanthin aus in diesen Extrakten enthaltenen Vorstufen von Astaxanthin. Die beschriebenen Tagetespflanzen oder Extrakte selber enthalten kein Astaxanthin.

WO 9201754 beschreibt eine astaxanthinhaltige Wildtyppflanze der Spezies Adonis aestivalis. Ferner offenbart das Dokument die Verwendung der astaxanthinhaltigen Petalen von Adonis aestivalis sowie deren Extrakte als Fischfutter oder als Zusatz in Fischfutter zur Pigmentierung von Fischen.

Die Verwendung von Adonis aestivalis als pflanzliche Queile für Astaxanithin zur Pigmentierung von Fischen im Stand der Technik weist jedoch den Nachteil auf, dass der Ertrag an astaxanthinhaltiger Biomasse und damit an astsxanthinhaltigem Pflanzenma∼ terial pro Anbaufläche sehr gering ist, und somit nur doch kostenintensiven Anbau großer Flächen eine befriedigende Menge an astaxanthinhaltigem Pflanzenmaterial erhalten werden kann. Dies führt zu hohen Kosten bei der Herstellung entsprechender Pigmentiermittel. In der WO03/080849 werden transgene Pflanzen, zum Beispiel Tagetes beschrieben, die Astaxanthin produzieren sowie die Verwendung dieser Pflanzen zur Herstellung von astaxanthinhastigen Extrakten. Diese Extrakte sollen unter anderem als Zusatz für Futtermittel verwendet werden,

Aus dem Stand der Technik sind daher nur Tagetespflanzen bekannt, die kein Astaxanthin enthalten oder die, wenn sie Astaxanthin enthalten, zur Extraktion dieses Carotinoids zur Herstellung von Futterusatzmitteln eingesetzt werden.

Der Erfindung lag daher die Aufgabe zugrunde, astaxanthinhaltige Pflanzen oder Pflanzenteile zur oralen Verabreichung an Tiere zur Verfügung zu stellen,

Demgemäss wurde gefunden, dass astaxanthinhaltige Pflanzen oder Pflanzenteile der Gattung Tagetes zur oralen Verabreichung an Tiere verwendet werden können.

In einer bevorzugten Ausführungsform werden die astaxanthinhaitigen Pflanzen oder Pflanzenteile der Gattung Tagetes zur Pigmentierung von Tieren und der entsprechenden Tierprodukte verwendet.

Unter astaxanthinhaltigen Pflanzen der Gattung Tagetes werden bevorzugt Pflanzen der Gattung Tagetes verstanden, die in mindestens einem Teil der Pflanze einen Gehalt an Astaxanthin aufweisen. Das Astaxanthin kann in freier Form in Form von Fettsäure-Di- oder Monoester vorliegen. Bevorzugte Pflanzen der Gattung Tagetes sind Pflanzen ausgewählt aus den Spezies Tagetes erecta, Tagetes patula, die auch als Marigoid bezeichnet werden, Tagetes lucida, Tagetes pringlei, Tagetes palmeri, Tagetes minuta, Tagetes lemmonii, Tagetes tenuifolia oder Tagetes campanulata, besonders bevorzugt *Tagetes erecta* oder *Tagetes patula.*

Unter astaxanthinhaltigen Pflanzenteilen von Pflanzen der Gattung Tagetes werden vorzugsweise Teile von Pflanzen verstanden, die in mindestens einem Teil des Pflanzenteils einen Gehalt an Astaxanthin aufweisen. Bevorzugte Pflanzenteile sind beispielsweise Blüten, Blütenköpfe oder besonders bevorzugt Blütenblätter, die auch als Petalen bezeichnet werden.

Wildtyppflanzen der Gattung Tagetes weisen kein Astaxanthin jedoch Carotinoide wie Lutein und Zeaxanthin in Blüten auf. Es wurde jedoch erfindungsgemäß gefunden, dass die Pflanzen der Gattung Tagetes beispielsweise durch genetische Veränderung in die Lage versetzt werden können, Astaxanthin herzustellen.

In einer bevorzugten Ausführungsform werden die Pflanzen der Gattung Tagetes beispielsweise dadurch in die Lage versetzt Astaxanthin herzustellen, indem in den genetisch veränderten Pflanzen der Gattung Tagetes im Vergleich zum Wildtyp eine Ketolase-Aktivität verursacht wird.

Unter Ketolase-Aktivität wird die Enzymaktivität einer Ketolase verstanden.

Unter einer Ketolase wird ein Protein verstanden, das die enzymatische Aktivität aufweist, am, gegebenenfalls substituierten, β-lonon-Ring von Carotinoiden eine Keto-Gruppe einzuführen.

Insbesondere wird unter einer Ketolase ein Protein verstanden, das die enzymatische Aktivität aufweist, β-Carotin in Canthaxanthin umzuwandeln.

Dementsprechend wird unter Ketolase-Aktivität die in einer bestimmten Zeit durch das Protein Ketolase umgesetzte Menge β-Carotin bzw. gebildete Menge Canthaxanthin verstanden.

Unter dem Begriff "Wildtyp" wird erfindungsgemäß die entsprechende nicht genetisch veränderte Ausgangspflanze der Gattung Tagetes verstanden.

Je nach Zusammenhang kann unter dem Begriff "Pflanze" die Ausgangspflanze (Wildtyp) der Gattung Tagetes oder eine erfindungsgemäße, genetisch veränderte Pflanze der Gattung Tagetes oder beides verstanden werden.

Vorzugsweise wird unter "Wildtyp" für die Verursachung der Ketolase-Aktivität, für die nachstehend beschriebene Erhöhung der Hydroxylase-Aktivität, für die nachstehend beschriebene Erhöhung der β-Cyclase-Aktivität, und für die nachstehend beschriebene Reduzierung der ε-Cyclase-Aktivität und die Erhöhung des Gehalts an Astaxanthin jeweils eine Referenzpflanze verstanden.

Diese Referenzpflanze der Gattung Tagetes ist Tagetes erecta, Tagetes patula, Tagetes lucida, Tagetes pringlei, Tagetes palmeri, Tagetes minuta oder Tagetes campanulata, besonders bevorzugt *Tagetes erecta,* ganz besonders bevorzugt Tagetes erecta L., Accession number: TAG 72, Sorte Orangenprinz, erhältlich aus der Genbank des IPK, Corrensstr. 3, D-06466 Gatersleben.

Die Bestimmung der Ketolase-Aktivität in erfindungsgemäßen genetisch veränderten Pflanzen der Gattung Tagetes und in Wildtyp- bzw. Referenzpflanzen erfolgt vorzugsweise unter folgenden Bedingungen:

Die Bestimmung der Ketolase-Aktivität in Pflanzenmaterial erfolgt in Anlehnung an die Methode von Frazer et al., (J. Biol. Chem. 272(10): 6128-6135, 1997). Die Ketolase-Aktivität in pflanzlichen Extrakten wird mit den Substraten beta-Carotin und Canthaxanthin in Gegenwart von Lipid (Sojalecithin) und Detergens (Natriumcholat) bestimmt. Substrat/Produkt-Verhältnisse aus den Ketolase-Assays werden mittels HPLC ermittelt.

Die erfindungsgemäße genetisch veränderte Pflanze der Gattung Tagetes weist in dieser, bevorzugten Ausführungsform im Vergleich zum genetisch nicht veränderten Wildtyp eine Ketolase-Aktivität, vorzugsweise in Blütenblättern, auf und ist somit vorzugsweise in der Lage, transgen eine Ketolase zu exprimieren.

In einer weiter bevorzugten Ausführungsform erfolgt die Verursachung der Ketolase-Aktivität in den Pflanzen der Gattung Tagetes durch Verursachung der Genexpression einer Nukleinsäure kodierend eine Ketolase.

In dieser bevorzugten Ausführungsform erfolgt die Verursachung der Genexpression einer Nukleinsäure kodierend eine Ketolase vorzugsweise durch Einbringen von Nukleinsäuren, die Ketolasen kodieren in die Ausgangspflanze der Gattung Tagetes.

Dazu kann prinzipiell jedes Ketolase-Gen, also jede Nukleinsäuren die eine Ketolase codiert verwendet werden.

Alle in der Beschreibung erwähnten Nukleinsäuren können beispielsweise eine RNA-, DNA- oder cDNA-Sequenz sein.

Bei genomischen Ketolase-Sequenzen aus eukaryontischen Quellen, die Introns enthalten, sind für den Fall das die Wirtspflanze der Gattung Tagetes nicht in der Lage ist oder nicht in die Lage versetzt werden kann, die entsprechenden Ketolase zu exprimieren, bevorzugt bereits prozessierte Nukleinsäuresequenzen, wie die entsprechenden cDNAs zu verwenden.

Beispiele für Nukleinsäuren, kodierend eine Ketolase und die entsprechenden Ketolasen, die im erfindungsgemäßen Verfahren verwendet werden können sind beispielsweise Sequenzen aus
Haematoccus pluvialis, insbesondere aus Haematoccus pluvialis Flotow em. Wille (Accession NO: X86782; Nukleinsäure: SEQ ID NO: 1, Protein SEQ ID NO: 2),
Haematoccus pluvialis, NIES-144 (Accession NO: D45881; Nukleinsäure: SEQ ID NO: 3, Protein SEQ ID NO: 4),
Agrobacterium aurantiacum (Accession NO: D58420; Nukleinsäure: SEQ ID NO: 5, Protein SEQ ID NO: 6),
Alicaligenes spec. (Accession NO: D58422; Nukleinsäure: SEQ ID NO: 7, Protein SEQ ID NO: 8),
Paracoccus marcusii (Accession NO: Y15112; Nukleinsäure: SEQ ID NO: 9, Protein SEQ ID NO: 10).
Synechocystis sp. Strain PC6803 (Accession NO: NP442491; Nukleinsäure: SEQ ID NO: 11, Protein SEQ ID NO: 12).
Bradyrhizobium sp. (Accession NO: AF218415; Nukleinsäure: SEQ ID NO: 13, Protein SEQ ID NO: 14).
Nostoc sp. Strain PCC7120 (Accession NO: AP003592, BAB74888; Nukleinsäure: SEQ ID NO: 15, Protein SEQ ID NO: 16),
*Nostoc punctiforme ATTC 29133,* Nukleinsäure: Acc.-No. NZ_AABC01000195, Basenpaar 55,604 bis 55,392 (SEQ ID NO: 81); Protein: Acc.-No. ZP_00111258 (SEQ ID NO: 82) (als putatives Protein annotiert),
*Nostoc punctiforme ATTC 29133,* Nukleinsäure: Acc.-No. NZ_AABC01000196, Basenpaar 140,571 bis 139,810 (SEQ ID NO: 83), Protein: (SEQ ID NO: 84) (nicht annotiert), *Synechococcus sp. WH 8102,* Nukleinsäure: Acc.-No. NZ_AABD01000001, Basenpaar 1,354,725-1,355,528 (SEO ID NO: 85), Protein: Acc.-No. ZP_00115639 (SEO ID NO: 86) (als putatives Protein annotiert),
Haematococcus pluvialis (Accession NO: AF534876, AAN03484; Nukleinsäure: SEQ ID NO: 97, Protein : SEQ ID NO: 98),
Paracoccus sp. MBIC1143, (Accession NO: D58420, P54972; Nukleinsäure: SEQ ID NO: 99, Protein : SEQ ID NO: 100),
Brevundimonas aurantiaca (Accession NO: AY166610, AAN86030; Nukleinsäure: SEO ID NO: 101, Protein : SEQ ID NO: 102,)
Nodularia spumigena NSOR10 (Accession NO: AY210783, AAO64399; Nukleinsäure: SEQ ID NO: 103, Protein : SEQ ID NO: 104) und
Deinococcus radiodurans R1 (Accession NO: E75561, AE001872; Nukleinsäure: SEQ ID NO: 105, Protein : SEQ ID NO: 106).

Weitere natürliche Beispiele für Ketolasen und Ketolase-Gene, die im erfindungsgemäßen Verfahren verwendet werden können, lassen sich beispielsweise aus verschiedenen Organismen, deren genomische Sequenz bekannt ist, durch Identitätsvergleiche der Aminosäuresequenzen oder der entsprechenden rückübersetzten Nukleinsäuresequenzen aus Datenbanken mit den vorstehend beschriebenen Sequenzen und insbesondere mit den Sequenzen SEQ ID NO: 2 und/oder 16 leicht auffinden.

Weitere natürliche Beispiele für Ketolasen und Ketolase-Gene lassen sich weiterhin ausgehend von den vorstehend beschriebenen Nukleinsäuresequenzen, insbesondere ausgehend von den Sequenzen SEQ ID NO: 2 und/oder 16 aus verschiedenen Organismen, deren genomische Sequenz nicht bekannt ist, durch Hybridisierungstechniken in an sich bekannter Weise leicht auffinden.

Die Hybridisierung kann unter moderaten (geringe Stringenz) oder vorzugsweise unter stringenten (hohe Stringenz) Bedingungen erfolgen.

Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Beispielhaft können die Bedingungen während des Waschschrittes ausgewählt sein aus dem Bereich von Bedingungen begrenzt von solchen mit geringer Stringenz (mit 2X SSC bei 50_C) und solchen mit hoher Stringenz (mit 0.2X SSC bei 50_C, bevorzugt bei 65_C) (20X SSC: 0,3 M Natriumcitrat, 3 M Natriumchlorid, pH 7.0).

Darüberhinaus kann die Temperatur während des Waschschrittes von moderaten Bedingungen bei Raumtemperatur, 22_C, bis zu stringenten Bedingungen bei 65_C angehoben werden.

Beide Parameter, Salzkonzentration und Temperatur, können gleichzeitig variiert werden, auch kann einer der beiden Parameter konstant gehalten und nur der andere variiert werden. Während der Hybridisierung können auch denaturierende Agenzien wie zum Beispiel Formamid oder SDS eingesetzt werden. In Gegenwart von 50 % Formamid wird die Hybridisierung bevorzugt bei 42_C ausgeführt.

Einige beispielhafte Bedingungen für Hybridisierung und Waschschritt sind infolge gegeben:
(1) Hybridiserungsbedingungen mit zum Beispiel
   (i) 4X SSC bei 65_C, oder
   (ii) 6X SSC bei 45_C, oder
   (iii) 6X SSC bei 68_C, 100 mg/ml denaturierter Fischsperma-DNA, oder
   (iv) 6X SSC, 0.5 % SDS, 100 mg/ml denaturierte, fragmentierte Lachssperma-DNA bei 68_C, oder
   (v) 6XSSC, 0.5 % SDS, 100 mg/ml denaturierte, fragmentierte Lachssperma-DNA, 50 % Formamid bei 42_C, oder
   (vi) 50 % Formamid, 4X SSC bei 42_C, oder
   (vii) 50 % (vol/vol) Formamid, 0.1 % Rinderserumalbumin, 0.1 % Ficoll, 0.1 % Polyvinylpyrrolidon, 50 mM Natriumphosphatpuffer pH 6.5, 750 mM NaCl, 75 mM Natriumcitrat bei 42_C, oder
   (viii) 2X oder 4X SSC bei 50_C (moderate Bedingungen), oder
   (ix) 30 bis 40 % Formamid, 2X oder 4X SSC bei 42_ (moderate Bedingungen).
(2) Waschschritte für jeweils 10 Minuten mit zum Beispiel
   (i) 0.015 M NaCl/0.0015 M Natriumcitrat/0.1 % SDS bei 50_C, oder
   (ii) 0.1 X SSC bei 65_C, oder
   (iii) 0.1 X SSC, 0.5 % SDS bei 68_C, oder
   (iv) 0.1 X SSC, 0.5 % SDS, 50 % Formamid bei 42_C, oder
   (v) 0.2X SSC, 0.1 % SDS bei 42_C, oder
   (vi) 2X SSC bei 65_C (moderate Bedingungen).

In einer bevorzugten Ausführungsform der erfindungsgemäßen genetisch veränderten Planzen der Gattung Tagetes bringt man Nukleinsäuren ein, die ein Protein kodieren, enthaltend die Aminosäuresequenz SEQ ID NO: 2 oder eine von dieser Sequenz durch Substitution, Insertion oder Deletion von Aminosäuren abgeleitete Sequenz, die eine Identität von mindestens 20 %, vorzugsweise mindestens 30 %, bevorzugter mindestens 40 %, bevorzugter mindestens 50 %, bevorzugter mindestens 60 %, bevorzugter mindestens 70 %, bevorzugter mindestens 80 %, besonders bevorzugt mindestens 90 % auf Aminosäureebene mit der Sequenz SEQ ID NO: 2 und die enzymatische Eigenschaft einer Ketolase aufweist.

Dabei kann es sich um eine natürliche Ketolase-Sequenz handeln, die wie vorstehend beschrieben durch Identitätsvergleich der Sequenzen aus anderen Organismen gefunden werden kann oder um eine künstliche Ketolase-Sequenz die ausgehend von der Sequenz SEQ ID NO: 2 durch künstliche Variation, beispielsweise durch Substitution, Insertion oder Deletion von Aminosäuren abgewandelt wurde.

In einer weiteren, bevorzugten Ausführungsform der erfindungsgemäßen Verfahren bringt man Nukleinsäuren ein die ein Protein kodieren, enthaltend die Aminosäuresequenz SEQ ID NO: 16 oder eine von dieser Sequenz durch Substitution, Insertion oder Deletion von Aminosäuren abgeleitete Sequenz, die eine Identität von mindestens 20 %, vorzugsweise mindestens 30 %, bevorzugter mindestens 40 %, bevorzugter mindestens 50 %, bevorzugter mindestens 60 %, bevorzugter mindestens 70 %, bevorzugter mindestens 80 %, besonders bevorzugt mindestens 90 % auf Aminosäureebene mit der Sequenz SEQ ID NO: 16 und die enzymatische Eigenschaft einer Ketolase aufweist.

Dabei kann es sich um eine natürliche Ketolase-Sequenz handeln, die, wie vorstehend beschrieben, durch ldentitätsvergleich der Sequenzen aus anderen Organismen gefunden werden kann oder um eine künstliche Ketolase-Sequenz die ausgehend von der Sequenz SEQ. ID NO: 16 durch künstliche Variation, beispielsweise durch Substitution, Insertion oder Deletion von Aminosäuren abgewandelt wurde.

Unter dem Begriff "Substitution" ist in der Beschreibung der Austausch einer oder mehrerer Aminosäuren durch eine oder mehrere Aminosäuren zu verstehen. Bevorzugt werden sog. konservative Austausche durchgeführt, bei denen die ersetzte Aminosäure eine ähnliche Eigenschaft hat wie die ursprüngliche Aminosäure, beispielsweise Austausch von Glu durch Asp, Gln durch Asn, Val durch Ile, Leu durch Ile, Ser durch Thr.

Deletion ist das Ersetzen einer Aminosäure durch eine direkte Bindung. Bevorzugte Positionen für Deletionen sind die Termini des Polypeptides und die Verknüpfungen zwischen den einzelnen Proteindomänen.

Insertionen sind Einfügungen von Aminosäuren in die Polypeptidkette, wobei formal eine direkte Bindung durch ein oder mehrere Aminosäuren ersetzt wird.

Unter Identität zwischen zwei Proteinen wird die Identität der Aminosäuren über die jeweils gesamte Proteinlänge verstanden, insbesondere die Identität die durch Vergleich mit Hilfe der Lasergene Software der Firma DNASTAR, inc. Madison, Wisconsin (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

### Multiple alignment parameter:

| | |
|---|---|
| Gap penalty | 10 |
| Gap length penalty | 10 |
| Pairwise alignment parameter: | |
| K-tuple | 1 |
| Gap penalty | 3 |
| Window | 5 |
| Diagonals saved | 5 |

Unter einem Protein, das eine Identität von mindestens 20 % auf Aminosäureebene mit der Sequenz SEQ ID NO: 2 oder 16 aufweist, wird dementsprechend ein Protein verstanden, das bei einem Vergleich seiner Sequenz mit der Sequenz SEQ ID NO: 2 oder 16, insbesondere nach obigen Programmlogarithmus mit obigem Parametersatz eine Identität von mindestens 20 % aufweist.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der tagetesspezifischen codon usage häufig verwendet werden. Die codon usage lässt sich anhand von Computerauswertungen anderer, bekannter Gene von Pflanzen der Gattung Tagetes leicht ermitteln.

In einer besonders bevorzugten Ausführungsform bringt man eine Nukleinsäure, enthaltend die Sequenz SEQ ID NO: 1 in die Pflanze der Gattung ein.

In einer weiteren, besonders bevorzugten Ausführungsform bringt man eine Nukleinsäure, enthaltend die Sequenz SEQ ID NO: 15 in die Pflanze der Gattung ein.

Alle vorstehend erwähnten Ketolase-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, S. 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer besonderes bevorzugten Ausführungsform der erfindungsgemäßen Verfahrens verwendet man genetisch veränderte Pflanzen der Gattung Tagetes, die in Blüten die höchste Expressionsrate einer Ketolase aufweisen.

Vorzugsweise wird dies dadurch erreicht, daß die Genexpression der Ketolase unter Kontrolle eines blütenspezifischen Promotors erfolgt. Beispielsweise werden dazu die vorstehend beschriebenen Nukleinsäuren, wie nachstehend ausführlich beschrieben, in einem Nukleinsäurekonstrukt, funktionell verknüpft mit einem blütenspezifischen Promotor in die Pflanze der Gattung Tagetes eingebracht.

Besonders bevorzugte Pflanzen der Gattung Tagetes als Ausgangspflanzen oder erfindungsgemäße genetisch veränderte Pflanzen sind Pflanzen ausgewählt aus den Spezies Tagetes erecta, Tagetes patula, die auch als Marigold bezeichnet werden, Tagetes lucida, Tagetes pringlei, Tagetes palmeri, Tagetes minuta, Tagetes lemmonii, Tagetes tenuifolia, oder Tagetes campanulata, besonders bevorzugt *Tagetes erecta* oder *Tagetes patula.*

In einer bevorzugten Ausführungsform werden genetisch veränderte Pflanzen der Gattung Tagetes verwendet, die gegenüber dem Wildtyp zusätzlich eine erhöhte Hydroxylase-Aktivität und/oder β-Cyclase-Aktivität aufweisen.

Unter Hydroxylase-Aktivität die Enzymaktivität einer Hydroxylase verstanden.

Unter einer Hydroxylase wird ein Protein verstanden, das die enzymatische Aktivität aufweist, am, gegebenenfalls substituierten, β-lonon-Ring von Carotinoiden eine Hydroxy-Gruppe einzuführen.

Insbesondere wird unter einer Hydroxylase ein Protein verstanden, das die enzymatische Aktivität aufweist, β-Carotin in Zeaxanthin oder Cantaxanthin in Astaxanthin umzuwandeln.

Dementsprechend wird unter Hydroxyase-Aktivität die in einer bestimmten Zeit durch das Protein Hydroxylase umgesetzte Menge β-Carotin oder Cantaxanthin bzw. gebildete Menge Zeaxanthin oder Astaxanthin verstanden.

Bei einer erhöhten Hydroxylase-Aktivität gegenüber dem Wildtyp wird somit im Vergleich zum Wildtyp in einer bestimmten Zeit durch das Protein Hydroxylase die umgesetzte Menge β-Carotin oder Cantaxantin bzw. die gebildete Menge Zeaxanthin oder Astaxanthin erhöht.

Vorzugsweise beträgt diese Erhöhung der Hydroxylase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Hydroxylase-Aktivität des Wildtyps.

Unter β-Cyclase-Aktivität wird die Enzymaktivität einer β-Cyclase verstanden.

Unter einer β-Cyclase wird ein Protein verstanden, das die enzymatische Aktivität aufweist, einen endständigen, linearen Rest von Lycopin in einen β-lonon-Ring zu überführen.

Insbesondere wird unter einer β-Cyclase ein Protein verstanden, das die enzymatische Aktivität aufweist, γ-Carotin in β-Carotin umzuwandeln.

Dementsprechend wird unter β-Cyclase-Aktivität die in einer bestimmten Zeit durch das Protein β-Cyclase umgesetzte Menge γ-Carotin bzw. gebildete Menge β-Carotin verstanden.

Bei einer erhöhten β-Cyclase -Aktivität gegenüber dem Wildtyp wird somit im Vergleich zum Wildtyp in einer bestimmten Zeit durch das Protein β-Cyclase die umgesetzte Menge γ-Carotin bzw. die gebildete Menge β-Carotin erhöht.

Vorzugsweise beträgt diese Erhöhung der β-Cyclase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der β-Cyclase-Aktivität des Wildtyps.

Die Bestimmung der Hydroxylase-Aktivität in erfindungsgemäßen genetisch veränderten Pflanzen und in Wildtyp- bzw. Referenzpflanzen erfolgt vorzugsweise unter folgenden Bedingungen:

Die Aktivität der Hydroxylase wird nach Bouvier et al. (Biochim. Biophys. Acta 1391 (1998), 320-328) *in vitro* bestimmt. Es wird zu einer bestimmten Menge an Pflanzenextrakt Ferredoxin, Ferredoxin-NADP Oxidoreductase, Katalase, NADPH sowie beta-Carotin mit Mono- und Digalaktosylglyzeriden zugegeben.

Besonders bevorzugt erfolgt die Bestimmung der Hydroxylase-Aktivität unter folgenden Bedingungen nach Bouvier, Keller, d'Harlingue und Camara (Xanthophyll biosynthesis: molecular and functional characterization of carotenoid hydroxylases from pepper fruits (Capsicum annuum L.; Biochim. Biophys. Acta 1391 (1998), 320-328):

Der in-vitro Assay wird in einem Volumen von 0.250 ml Volumen durchgeführt. Der Ansatz enthält 50 mM Kaliumphosphat (pH 7.6), 0.025 mg Ferredoxin von Spinat, 0.5 Einheiten Ferredoxin-NADP+ Oxidoreduktase von Spinat, 0.25 mM NADPH, 0.010 mg beta-Carotin (in 0.1 mg Tween 80 emulgiert), 0.05 mM einer Mischung von Mono- und Digalaktosylglyzeriden (1:1), 1 Einheit Katalyse, 200 Mono- und Digalaktosylglyzeriden, (1:1), 0.2 mg Rinderserumalbumin und Pflanzenextrakt in unterschiedlichem Volumen. Die Reaktionsmischung wird 2 Stunden bei 30C inkubiert. Die Reaktionsprodukte werden mit organischem Lösungsmittel wie Aceton oder Chloroform/Methanol (2:1) extrahiert und mittels HPLC bestimmt.

Die Bestimmung der β-Cyclase-Aktivität in erfindungsgemäßen genetisch veränderten Pflanzen und in Wildtyp- bzw. Referenzpflanzen erfolgt vorzugsweise unter folgenden Bedingungen:

Die Aktivität der β-Cyclase wird nach Fraser und Sandmann (Biochem. Biophys. Res. Comm. 185(1) (1992) 9-15)*in vitro* bestimmt. Es werden zu einer bestimmten Menge an Pflanzenextrakt Kaliumphosphat als Puffer (ph 7.6), Lycopin als Substrat, Stromaprotein von Paprika, NADP+, NADPH und ATP zugegeben.

Besonders bevorzugt erfolgt die Bestimmung der Hydroxylase-Aktivität unter folgenden Bedingungen nach Bouvier, d'Harlingue und Camara (Molecular Analysis of carotenoid cyclae inhibition; Arch. Biochem. Biophys. 346(1) (1997) 53-64): Der in-vitro Assay wird in einem Volumen von 250 ∝l Volumen durchgeführt. Der Ansatz enthält 50 mM Kaliumphosphat (pH 7.6),unterschiedliche Mengen an Pflanzenextrakt, 20 nM Lycopin, 250 ∝g an chromoplastidärem Stromaprotein aus Paprika, 0.2 mM NADP+, 0.2 mM NADPH und 1 mM ATP. NADP/NADPH und ATP werden in 10 ml Ethanol mit 1 mg Tween 80 unmittelbar vor der Zugabe zum Inkubationsmedium gelöst. Nach einer Reaktionszeit von 60 Minuten bei 30C wird die Reaktion durch Zugabe von Chloroform/Methanol (2:1) beendet. Die in Chloroform extrahierten Reaktionsprodukte werden mittels HPLC analysiert.

Ein alternativer Assay mit radioaktivem Substrat ist beschrieben in Fraser und Sandmann (Biochem. Biophys. Res. Comm. 185(1) (1992) 9-15).

Die Erhöhung der Hydroxylase-Aktivität und/oder β-Cyclase-Aktivität kann durch verschiedene Wege erfolgen, beispielsweise durch Ausschalten von hemmenden Regulationsmechanismen auf Expressions- und Proteinebene oder durch Erhöhung der Genexpression von Nukleinsäuren kodierend eine Hydroxylase und/oder von Nukleinsäuren kodierend eine β-Cyclase gegenüber dem Wildtyp.

Die Erhöhung der Genexpression der Nukleinsäuren kodierend eine Hydroxylase und/oder die Erhöhung der Genexpression der Nukleinsäure kodierend eine β-Cyclase gegenüber dem Wildtyp kann ebenfalls durch verschiedene Wege erfolgen, beispielsweise durch Induzierung des Hydroxylase-Gens und/oder β-Cyclase-Gens durch Aktivatoren oder durch Einbringen von einer oder mehrerer Hydroxylase-Genkopien und/oder β-Cyclase-Genkopien, also durch Einbringen mindestens einer Nukleinsäure kodierend eine Hydroxylase und/oder mindestens einer Nukleinsäure kodierend eine ε-Cyclase in die Pflanze der Gattung Tagetes.

Unter Erhöhung der Genexpression einer Nukleinsäure codierend eine Hydroxylase und/oder β-Cyclase wird erfindungsgemäß auch die Manipulation der Expression der Pflanzen der Gattung Tagetes eigenen, endogenen Hydroxylase und/oder β-Cyclase verstanden.

Dies kann beispielsweise durch Veränderung der Promotor DNA-Sequenz für Hydroxylasen und/oder β-Cyclasen kodierende Gene erreicht werden. Eine solche Veränderung, die eine erhöhte Expressionsrate des Gens zur Folge hat, kann beispielsweise durch Deletion oder Insertion von DNA Sequenzen erfolgen.

Es ist, wie vorstehend beschrieben, möglich, die Expression der endogenen Hydroxylase und/oder β-Cyclase durch die Applikation exogener Stimuli zu verändern. Dies kann durch besondere physiologische Bedingungen, also durch die Applikation von Fremdsubstanzen erfolgen.

Des weiteren kann eine veränderte bzw. erhöhte Expression eines endogenen Hydroxylase- und/oder β-Cyclase-Gens dadurch erzielt werden, dass ein in der nicht transformierten Pflanze nicht vorkommendes Regulator-Protein mit dem Promotor dieses Gens in Wechselwirkung tritt.

Solch ein Regulator kann ein chimäres Protein darstellen, welches aus einer DNA-Bindedomäne und einer Transkriptionsaktivator-Domäne besteht, wie beispielsweise in WO 96/06166 beschrieben.

In einer bevorzugten Ausführungsform erfolgt die Erhöhung der Genexpression einer Nukleinsäure kodierend eine Hydroxylase und/oder die Erhöhung der Genexpression einer Nukleinsäure kodierend eine β-Cyclase durch Einbringen von mindestens einer Nukleinsäure kodierend eine Hydroxylase und/oder durch Einbringen von mindestens einer Nukleinsäure kodierend eine β-Cyclase in die Pflanze der Gattung Tagetes. Dazu kann prinzipiell jedes Hydroxylase-Gen bzw. jedes β-Cyclase-Gen, also jede Nukleinsäure, die eine Hydroxylase und jede Nukleinsäure, die eine β-Cyclase codiert, verwendet werden.

Bei genomischen Hydroxylase-bzw. β-Cyclase-Nukleinsäure-Sequenzen aus eukaryontischen Quellen, die Introns enthalten, sind für den Fall das die Wirtspflanze nicht in der Lage ist oder nicht in die Lage versetzt werden kann, die entsprechende Hydroxylase bzw. β-Cyclase zu exprimieren, bevorzugt bereits prozessierte Nukleinsäuresequenzen, wie die entsprechenden cDNAs zu verwenden.

Ein Beispiel für ein Hydroxylase-Gen ist eine Nukleinsäure, kodierend eine Hydroxylase aus Haematococcus pluvialis, Accession AX038729, WO 0061764); (Nukleinsäure: SEQ ID NO: 17, Protein: SEQ ID NO: 18).

sowie Hydroxylasen der folgenden Accession Nummern:
IemblCAB55626.1, CAA70427.1, CAA70888.1, CAB55625.1, AF499108_1, AF315289_1, AF296158_1, AAC49443.1, NP_194300.1, NP_200070.1, AAG10430.1, CAC06712.1, AAM88619.1, CAC95130.1, AAL80006.1, AF162276_1, AA053295.1, AAN85601.1, CRTZ_ERWHE, CRTZ_PANAN, BAB79605.1, CRTZ_ALCSP, CRTZ_AGRAU, CAB56060.1, ZP_00094836.1, AAC44852.1, BAC77670.1, NP_745389.1, NP_344225.1. NP_849490.1, ZP_00087019.1, NP_503072,1, NP_852012.1, NP_115929.1, ZP_00013255.1

Eine besonders bevorzugte Hydroxylase ist weiterhin die Hydroxylase aus Tomate (Accession Y14809) (Nukleinsäure: SEQ ID NO: 107; Protein: SEQ ID NO. 108).

Beispiele für β-Cyclase-Gene sind:
eine Nukleinsäure, codierend eine β-Cyclase aus Tomate (Accession X86452).(Nukleinsäure: SEQ ID NO: 19, Protein: SEQ ID NO: 20),.

Sowie β-Cyc!asen der folgenden Accesion Nummern:
S66350 lycopene beta-cyclase (EC 5.5.1.-) - tomato
CAA60119 lycopene synthase [Capsicum annuum]
S66349 lycopene beta-cyclase (EC 5.5.1.-) - common tobacco
CAA57386 lycopene cyclase [Nicotiana tabacum]
AAM21152 lycopene beta-cyclase [Citrus sinensis]
AAD38049 lycopene cyclase [Citrus x paradisi]
AAN86060 lycopene cyclase [Citrus unshiu]
AAF44700 lycopene beta-cyclase [Citrus sinensis]
AAK07430 lycopene beta-cyclase [Adonis palaestina]
AAG 10429 beta cyclase [Tagetes erecta]
AAA81880 lycopene cyclase
AAB53337 Lycopene beta cyclase
AAL92175 beta-lycopene cyclase [Sandersonia aurantiaca]
CAA67331 lycopene cyclase [Narcissus pseudonarcissus]
AAM45381 beta cyclase [Tagetes erecta]
AAO18661 lycopene beta-cyclase [Zea mays]
AAG21133 chromoplast-specific lycopene beta-cyclase [Lycopersicon esculentum]
AAF18989 lycopene beta-cyclase [Daucus carota]
ZP_001140 hypothetical protein [Prochlorococcus marinus str. MIT9313]
ZP_001050 hypothetical protein [Prochlorococcus marinus subsp. pastoris str. CCMP1378]
ZP_001046 hypothetical protein [Prochlorococcus marinus subsp. pastoris str. CCMP1378]
ZP_001134 hypothetical protein [Prochlorococcus marinus str. MIT9313]
ZP_001150 hypothetical protein [Synechococcus sp. WH 8102]
AAF10377 lycopene cyclase [Deinococcus radiodurans]
BAA29250 393aa long hypothetical protein [Pyrococcus horikoshii]
BAC77673 lycopene beta-monocyclase [marine bacterium P99-3]
AAL01999 lycopene cyclase [Xanthobacter sp. Py2]
ZP_000190 hypothetical protein [Chloroflexus aurantiacus]
ZP_000941 hypothetical protein [Novosphingobium aromaticivorans]
AAF78200 lycopene cyclase [Bradyrhizobium sp. ORS278]
BAB79602 crtY [Pantoea agglomerans pv. milletiae]
CAA64855 lycopene cyclase [Streptomyces griseus]
AAA21262 dycopene cyclase [Pantoea agglomerans]
C37802 crtY protein - Erwinia uredovora
BAB79602 crtY [Pantoea agglomerans pv. milletiae]
AAA64980 lycopene cyclase [Pantoea agglomerans]
AAC44851 lycopene cyclase
BAA09593 Lycopene cyclase [Paracoccus sp. MBIC1143]
ZP_000941 hypothetical protein [Novosphingobium aromaticivorans]
CAB56061 lycopene beta-cyclase [Paracoccus marcusii]
BAA20275 lycopene cyclase [Erythrobacter longus]
ZP_000570 hypothetical protein [Thermobifida fusca]
ZP_000190 hypothetical protein [Chloroflexus aurantiacus]
AAK07430 lycopene beta-cyclase [Adonis palaestina]
CAA67331 lycopene cyclase [Narcissus pseudonarcissus]
AAB53337 Lycopene beta cyclase
BAC77673 lycopene beta-monocyclase [marine bacterium P99-3]

Eine besonders bevorzugte β-Cyclase ist weiterhin die chromoplastenspezifische β-Cyclase aus Tomate (AAG21133) (Nukleinsäure: SEQ ID No. 109; Protein: SEQ ID No. 110)

In den erfindungsgemäßen bevorzugten transgenen Pflanzen der gattung Tagetes liegt also in dieser bevorzugten Ausführungsform gegenüber dem Wildtyp mindestens ein weiteres Hydroxylase-Gen und/oder β-Cyclase-Gen vor.

In dieser bevorzugten Ausführungsform weist die genetisch veränderte Pflanze beispielsweise mindestens eine exogene Nukleinsäure, kodierend eine Hydroxylase oder mindestens zwei endogene Nukleinsäuren, kodierend eine Hydroxylase und/oder mindestens eine exogene Nukleinsäure, kodierend eine β-Cyclase oder mindestens zwei endogene Nukleinsäuren, kodierend eine β-Cyclase auf.

Bevorzugt verwendet man in vorstehend beschriebener bevorzugter Ausführungsform als Hydroxylase-Gene Nukleinsäuren, die Proteine kodieren, enthaltend die Aminosäuresequenz SEQ ID NO: 18 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70%, noch bevorzugter mindestens 90 %, am bevorzugtesten mindestens 95 % auf Aminosäureebene mit der Sequenz SEQ. ID. NO: 18, und die die enzymatische Eigenschaft einer Hydroxylase aufweisen.

Weitere Beispiele für Hydroxylasen und Hydroxylase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID. NO: 18 leicht auffinden.

Weitere Beispiele für Hydroxylasen und Hydroxylase-Gene lassen sich weiterhin beispielsweise ausgehend von der Sequenz SEQ ID NO: 17 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der Hydroxylase-Aktivität Nukleinsäuren in Organismen eingebracht, die Proteine kodieren, enthaltend die Aminosäuresequenz der Hydroxylase der Sequenz SEQ ID NO: 18.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ. ID. NO: 17 in den Organismus ein.

Bevorzugt verwendet man in vorstehend beschriebener bevorzugter Ausführungsform als β-Cyclase-Gene Nukleinsäuren, die Proteine kodieren, enthaltend die Aminosäuresequenz SEQ ID NO: 20 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 20, und die die enzymatische Eigenschaft einer β-Cyclase aufweisen.

Weitere Beispiele für β-Cyclasen und β-Cyclase-Gene lassen sich beispielsweise aus verschiedenen Organismen, deren genomische Sequenz bekannt ist, wie vorstehend beschrieben durch Homologievergleiche der Aminosäuresequenzen oder der entsprechenden rückübersetzten Nukleinsäuresequenzen aus Datenbanken mit der SEQ ID NO: 20 leicht auffinden.

Weitere Beispiele für β-Cyclasen und β-Cyclase-Gene lassen sich weiterhin beispielsweise ausgehend von der Sequenz SEQ ID NO: 19 aus verschiedenen Organismen deren genomische Sequenz nicht bekannt ist, durch Hybridisierungs- und PCR-Techniken in an sich bekannter Weise leicht auffinden.

In einer weiter besonders bevorzugten Ausführungsform werden zur Erhöhung der β-Cyclase-Aktivität Nukleinsäuren in Organismen eingebracht, die Proteine kodieren, enthaltend die Aminosäuresequenz der β-Cyclase der Sequenz SEQ. ID. NO: 20.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ. ID. NO: 19 in den Organismus ein.

Alle vorstehend erwähnten Hydroxylase-Gene oder β-Cyclase-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer weiter bevorzugten Ausführungsform des Verfahrens weisen die Pflanzen der Gattung Tagetes gegenüber dem Wildtyp zusätzlich eine reduzierte ε-Cyclase-Aktivität auf.

Unter ε-Cyclase-Aktivität wird die Enzymaktivität einer ε-Cyclase verstanden.

Unter einer ε-Cyclase wird ein Protein verstanden, das die enzymatische Aktivität aufweist, einen endständigen, linearen Rest von Lycopin in einen ε-lonon-Ring zu überführen.

Unter einer ε-Cyclase wird daher insbesondere ein Protein verstanden, das die enzymatische Aktivität aufweist, Lycopin in δ-Carotin umzuwandeln.

Dementsprechend wird unter ε-Cyclase-Aktivität die in einer bestimmten Zeit durch das Protein ε-Cyclase umgesetzte Menge Lycopin bzw. gebildete Menge δ-Carotin verstanden.

Bei einer reduzierten ε-Cyclase-Aktivität gegenüber dem Wildtyp wird somit im Vergleich zum Wildtyp in einer bestimmten Zeit durch das Protein ε-Cyclase die umgesetzte Menge Lycopin bzw. die gebildete Menge δ-Carotin reduziert.

Unter einer reduzierten ε-Cyclase-Aktivität wird vorzugsweise die teilweise oder im wesentlichen vollständige, auf unterschiedliche zellbiologische Mechanismen beruhende Unterbindung oder Blockierung der Funktionalität einer ε-Cyclase in einer pflanzlichen Zelle, Pflanze oder einem davon abgeleiteten Teil, Gewebe, Organ, Zellen oder Samen verstanden.

Die Reduzierung der ε-Cyclase-Aktivität in Pflanzen gegenüber dem Wildtyp kann beispielsweise durch Reduzierung der ε-Cyclase-Proteinmenge, oder der ε-Cyclase-mRNA-Menge in der Pflanze erfolgen. Dementsprechend kann eine gegenüber dem Wildtyp reduzierte ε-Cyclase-Aktivität direkt bestimmt werden oder über die Bestimmung der ε-Cyclase-Proteinmenge oder der ε-Cyclase-mRNA-Menge der erfindungsgemäßen Pflanze im Vergleich zum Wildtyp erfolgen.

Eine Reduzierung der ε-Cyclase-Aktivität umfasst eine mengenmäßige Verringerung einer ε-Cyclase bis hin zu einem im wesentlichen vollständigen Fehlen der ε-Cyclase (d.h. fehlende Nachweisbarkeit von ε-Cyclase-Aktivität oder fehlende immunologische Nachweisbarkeit der ε-Cyclase). Vorzugsweise wird die ε-Cyclase-Aktivität (bzw. die ε-Cyclase-Proteinmenge oder die ε-Cyclase-mRNA-Menge) in der Pflanze, besonders bevorzugt in Blüten im Vergleich zum Wildtyp um mindestens 5 %, weiter bevorzugt um mindestens 20 %, weiter bevorzugt um mindestens 50 %, weiter bevorzugt um 100 % reduziert. Insbesondere meint "Reduzierung" auch das vollständigen Fehlen der ε-Cyclase-Aktivität (bzw. des ε-Cyclase-Proteins oder der ε-Cyclase-mRNA).

Die Bestimmung der ε-Cyclase-Aktivität in erfindungsgemäßen genetisch veränderten Pflanzen und in Wildtyp- bzw. Referenzpflanzen erfolgt vorzugsweise unter folgenden Bedingungen:

Die ε-Cyclase-Aktivität kann nach Fraser und Sandmann (Biochem. Biophys. Res. Comm. 185(1) (1992) 9-15)*in vitro* bestimmt werden, wenn zu einer bestimmten Menge an Pflanzenextrakt Kaliumphosphat als Puffer (ph 7.6), Lycopin als Substrat, Stromaprotein von Paprika, NADP+, NADPH und ATP zugegeben werden.

Die Bestimmung der ε-Cyclase-Aktivität in erfindungsgemäßen genetisch veränderten Pflanzen und in Wildtyp- bzw. Referenzpflanzen erfolgt besonders bevorzugt nach Bouvier, d'Harlingue und Camara (Molecular Analysis of carotenoid cyclase inhibition; Arch. Biochem. Biophys. 346(1) (1997) 53-64):

Der in-vitro Assay wird in einem Volumen von 0.25 ml durchgeführt. Der Ansatz enthält 50 mM Kaliumphosphat (pH 7.6),unterschiedliche Mengen an Pflanzenextrakt, 20 nM Lycopin, 0.25 mg an chromoplastidärem Stromaprotein aus Paprika, 0.2 mM NADP+, 0.2 mM NADPH und 1 mM ATP. NADP/NADPH und ATP werden in 0.01 ml Ethanol mit 1 mg Tween 80 unmittelbar vor der Zugabe zum Inkubationsmedium gelöst. Nach einer Reaktionszeit von 60 Minuten bei 30C wird die Reaktion durch Zugabe von Chloroform/Methanol (2:1) beendet. Die in Chloroform extrahierten Reaktionsprodukte werden mittels HPLC analysiert.

Ein alternativer Assay mit radioaktivem Substrat ist beschrieben in Fraser und Sandmann (Biochem. Biophys. Res. Comm. 185(1) (1992) 9-15). Eine weitere analytische Methode ist beschrieben in Beyer, Kröncke und Nievelstein (On the mechanism of the lycopene isomerase/cyclase reaction in Narcissus pseudonarcissus L. chromopast,; J. Biol. Chem. 266(26) (1991) 17072-17078).

Vorzugsweise erfolgt die Reduzierung der ε-Cyclase-Aktivität in Pflanzen durch mindestens eines der nachfolgenden Verfahren:
a) Einbringen mindestens einer doppelsträngigen ε-Cyclase Ribonukleinsäuresequenz, nachstehend auch ε-Cyclase-dsRNA genannt, oder einer deren Expression gewährleistenden Expressionskassette oder Expressionskassetten. Umfasst sind solche Verfahren, bei denen die ε-Cyclase-dsRNA gegen ein ε-Cyclase-Gen (also genomische DNA-Sequenzen wie die Promotorsequenz) oder ein ε-Cyclase-Transkript (also mRNA-Sequenzen) gerichtet ist,
b) Einbringen mindestens einer ε-Cyctase antisense-Ribonukleinsäuresequenz, nachstehend auch ε-Cyclase-antisenseRNA genannt, oder einer deren Expression gewährleistenden Expressionskassette. Umfasst sind solche Verfahren, bei denen die ε-Cyclase-antisenseRNA gegen ein ε-Cyclase-Gen (also genomische DNA-Sequenzen) oder ein ε-Cyclase-Gentranskript (also RNA-Sequenzen) gerichtet ist. Umfasst sind auch α-anomere Nukleinsäuresequenzen,
c) Einbringen mindestens einer ε-Cyclase-antisenseRNA kombiniert mit einem Ribozym oder einer deren Expression gewährleistenden Expressionskassette
d) Einbringen mindestens einer ε-Cyclase sense-Ribonukleinsäuresequenz , nachstehend auch ε-Cyclase-senseRNA genannt, zur Induktion einer Kosuppression oder einer deren Expression gewährleistenden Expressionskassette
e) Einbringen mindestens eines DNA- oder Protein-bindenden Faktors gegen ein ε-Cyclase-Gen, -RNA oder -Protein oder einer dessen Expression gewährleistenden Expressionskassette
f) Einbringen mindestens einer den ε-Cyclase RNA-Abbau bewirkenden viralen Nukleinsäuresequenz oder einer deren Expression gewährleistenden Expressionskassette
g) Einbringen mindestens eines Konstruktes zur Erzeugung eines Funktionsverlustes, wie beispielsweise die Generierung von Stopp-Kodons oder eine Verschiebungen im Leseraster, an einem ε-Cyclase-Gen beispielsweise durch Erzeugung einer Insertion, Deletion, Inversion oder Mutation in einem ε-Cyclase-Gen. Bevorzugt können Knockout-Mutanten mittels gezielter Insertion in besagtes ε-Cyclase-Gen durch homologe Rekombination oder Einbringen von sequenzspezifischen Nukleasen gegen ε-Cyclase-Gensequenzen generiert werden.

Dem Fachmann ist bekannt, dass auch weitere Verfahren im Rahmen der vorliegenden Erfindung zur Verminderung einer ε-Cyclase bzw. seiner Aktivität oder Funktion eingesetzt werden können. Beispielsweise kann auch das Einbringen einer dominant-negativen Variante einer ε-Cyclase oder einer deren Expression gewährleistenden Expressionskassette vorteilhaft sein. Dabei kann jedes einzelne dieser Verfahren eine Verminderung der Proteinmenge, mRNA-Menge und/oder Aktivität einer ε-Cyclase bewirken. Auch eine kombinierte Anwendung ist denkbar. Weitere Methoden sind dem Fachmann bekannt und können die Behinderung oder Unterbindung der Prozessierung der ε-Cyclase, des Transports der ε-Cyclase oder dessen mRNA, Hemmung der Ribosomenanlagerung, Hemmung des RNA-Spleißens, Induktion eines ε-Cyclase-RNA abbauenden Enzyms und/oder Hemmung der Translationselongation oder -termination umfassen.

Die einzelnen bevorzugten Verfahren seien infolge durch beispielhafte Ausführungsformen beschrieben:
a) Einbringen einer doppelsträngigen ε-Cyclase-Ribonukleinsäuresequenz (ε-Cyclase-dsRNA)

Das Verfahren der Genregulation mittels doppelsträngiger RNA ("double-stranded RNA interference"; dsRNAi) ist bekannt und beispielsweise in Matzke MA et al. (2000) Plant Mol Biol 43:401-415; Fire A. et al (1998) Nature 391:806-811; WO 99/32619; WO 99/53050; WO 00/68374; WO 00/44914; WO 00/44895; WO 00/49035 oder WO 00/63364 beschrieben. Auf die in den angegebenen Zitaten beschriebenen Verfahren und Methoden wird hiermit ausdrücklich Bezug genommen.

Unter "Doppelsträngiger Ribonukleinsäuresequenz" wird erfindungsgemäß eine oder mehr Ribonukleinsäuresequenzen, die aufgrund komplementärer Sequenzen theoretisch, beispielsweise gemäß den Basenpaarregeln von Waston und Crick und/oder faktisch, beispielsweise aufgrund von Hybridisierungsexperimenten, in vitro und/oder in vivo in der Lage sind, doppelsträngige RNA-Strukturen auszubilden.

Dem Fachmann ist bewusst, dass die Ausbildung von doppelsträngigen RNA-Strukturen, einen Gleichgewichtszustand darstellt. Bevorzugt ist das Verhältnis von doppelsträngigen Molekülen zu entsprechenden dissozierten Formen mindestens 1 zu 10, bevorzugt 1:1, besonders bevorzugt 5:1, am meisten bevorzugt 10:1.

Unter einer doppelsträngigen ε-Cyclase-Ribonukleinsäuresequenz oder auch ε-Cyclase-dsRNA wird vorzugsweise ein RNA-Molekül verstanden, das einen Bereich mit Doppel-Strang-Struktur aufweist und in diesem Bereich eine Nukleinsäuresequenz enthält, die
a) mit mindestens einem Teil des Pflanze eigenen ε-Cyclase-Transkripts identisch ist und/oder
b) mit mindestens einem Teil der Pflanze eigenen ε-Cyclase-Promotor-Sequenz identisch ist.
Im erfindungsgemäßen Verfahren bringt man daher zur Reduzierung der ε-Cyclase-Aktivität bevorzugt in die Pflanze eine RNA ein, die einen Bereich mit Doppel-Strang-Struktur aufweist und in diesem Bereich eine Nukleinsäuresequenz enthält, die
a) mit mindestens einem Teil des Pflanze eigenen ε-Cyclase-Transkripts identisch ist und/oder
b) mit mindestens einem Teil der Pflanze eigenen ε-Cyclase-Promotor-Sequenz identisch ist.

Unter dem Begriff "ε-Cyclase-Transkript" wird der transkripierte Teil eines ε-Cyclase-Gens verstanden, der neben der ε-Cyclase kodierenden Sequenz beispielsweise auch nichtkodierende Sequenzen, wie beispielsweise auch UTRs enthält.

Unter einer RNA, die "mit mindestens einem Teil der Pflanze eigenen ε-Cyclase-Promotor-Sequenz identisch ist", ist vorzugsweise gemeint, dass die RNA-Sequenz mit mindestens einem Teil des theoretischen Transkriptes der ε-Cyclase-Promotor-Sequenz, also der entsprechenden RNA-Sequenz, identisch ist.

Unter "einem Teil" des Pflanze eigenen ε-Cyclase-Transkripts bzw. der Pflanze eigenen ε-Cyclase-Promotor-Sequenz werden Teilsequenzen verstanden, die von wenigen Basenpaaren bis hin zu vollständigen Sequenzen des Transkripts bzw. der Promotorssequenz reichen können. Die optimale Länger der Teilsequenzen kann der Fachmann durch Routineversuche leicht ermitteln.

In der Regel beträgt die Länge der Teilsequenzen mindestens 10 Basen und höchstens 2 kb, bevorzugt mindestens 25 Basen und höchstens 1,5 kb, besonders bevorzugt mindestens 50 Basen und höchstens 600 Basen, ganz besonders bevorzugt mindestens 100 Basen und höchstens 500, am meisten bevorzugt mindestens 200 Basen oder mindestens 300 Basen und höchstens 400 Basen.

Vorzugsweise werden die Teilsequenzen so ausgesucht, dass eine möglichst hohe Spezifität erreicht wird und nicht Aktivitäten anderer Enzyme reduziert werden, deren Verminderung nicht erwünscht ist. Es ist daher vorteilhaft für die Teilsequenzen der ε-Cyclase-dsRNA Teile des ε-Cyclase Transkripts und/oder Teilsequenzen der ε-Cyclase-Promotor-Sequenzen zu wählen, die nicht in anderen Aktivitäten auftreten.

In einer besonders bevorzugten Ausführungsform enthält daher die ε-Cyclase-dsRNA eine Sequenz, die mit einem Teil der Pflanze eigenen ε-Cyclase-Transkripts identisch ist und das 5'-Ende oder das 3'-Ende der Pflanze eigenen Nukleinsäure, codierend eine ε-Cyclase enthält. Insbesondere sind nichttranslatierte Bereiche im 5' oder 3' des Transkriptes geeignet, selektive Doppel-Strang-Strukturen herzustellen.

Ein weiterer Gegenstand der Erfindung bezieht sich auf doppelsträngige RNA-Moleküle (dsRNA-Moleküle), die bei Einbringen in einen pflanzlichen Organismus (oder eine davon abgeleitete Zelle, Gewebe, Organ oder Vermehrungsmaterial) die Verminderung einer ε-Cyclase bewirken.

Ein doppelsträngige RNA-Molekül zur Reduzierung der Expression einer ε-Cyclase (ε-Cyclase-dsRNA) umfasst dabei bevorzugt
a) einen "sense"-RNA-Strang umfassend mindestens eine Ribonukleotidsequenz, die im wesentlichen identisch ist zu mindestens einem Teil eines "sense"-RNA-ε-Cyclase Transkriptes, und
b) einen "antisense"-RNA-Strang, der zu dem RNA-"sense"-Strang unter a) im wesentlichen, bevorzugt vollständig, komplementären ist.

Zur Transformation der Pflanze mit einer ε-Cyclase-dsRNA wird bevorzugt ein Nukleinsäurekonstrukt verwendet, das in die Pflanze eingebracht wird und das in der Pflanze in die ε-Cyclase-dsRNA transkripiert wird.

Daher betrifft die vorliegende Erfindung auch ein
Nukleinsäurekonstrukt, transkripierbar in
a) einen "sense"-RNA-Strang umfassend mindestens eine Ribonukleotidsequenz, die im wesentlichen identisch ist zu mindestens einem Teil des "sense"-RNA-ε-Cyclase Transkriptes, und
b) einen "antisense"-RNA-Strang, der zu dem RNA-sense-Strang unter a) im wesentlichen - bevorzugt vollständig - komplementär ist.

Diese Nukleinsäurekonstrukte werden im folgenden auch Expressionskassetten oder Expressionsvektoren genannt.

In Bezug auf die dsRNA-Moleküle wird unter ε-Cyclase-Nukleinsäuresequenz, bzw. das entsprechende Transkript bevorzugt die Sequenz gemäß SEQ ID NO: 38 oder ein Tel derselben verstanden.

"Im wesentlichen identisch" meint, dass die dsRNA Sequenz auch Insertionen, Deletionen sowie einzelne Punktmutationen im Vergleich zu der ε-Cyclase Zielsequenz aufweisen kann und dennoch eine effizient Verminderung der Expression bewirkt. Bevorzugt beträgt die Homologie mindestens 75 %, bevorzugt mindestens 80 %, ganz besonders bevorzugt mindestens 90 % am meisten bevorzugt 100 % zwischen dem "sense"-Strang einer inhibitorischen dsRNA und mindestens einem Teil des "sense"-RNA-Transkriptes eines ε-Cyclase-Gens, bzw. zwischen dem "antisense"-Strang dem komplementären Strang eines ε-Cyclase-Gens.

Eine 100%ige Sequenzidentität zwischen dsRNA und einem ε-Cyclase Gentranskript ist nicht zwingend erforderlich, um eine effiziente Verminderung der ε-Cyclase Expression zu bewirken. Demzufolge besteht der Vorteil, dass das Verfahren tolerant ist gegenüber Sequenzabweichungen, wie sie infolge genetischer Mutationen, Polymorphismen oder evolutionärer Divergenzen vorliegen können. So ist es beispielsweise möglich mit der dsRNA, die ausgehend von der ε-Cyclase Sequenz des einen Organismus generiert wurde, die ε-Cyclase Expression in einem anderen Organismus zu unterdrücken. Zu diesem Zweck umfasst die dsRNA bevorzugt Sequenzbereiche von ε-Cyclase-Gentranskripten, die konservierten Bereichen entsprechen. Besagte konservierte Bereiche können aus Sequenzvergleichen leicht abgeleitet werden.

Alternativ, kann eine "im wesentlichen identische" dsRNA auch als Nukleinsäuresequenz definiert werden, die befähigt ist, mit einem Teil eines ε-Cyclase Gentranskriptes zu hybridisieren (z.B. in 400 mM NaCl, 40 mM PIPES pH 6,4, 1 mM EDTA bei 50°C oder 70°C für 12 bis 16 h).

"Im wesentlichen komplementär" meint, dass der "antisense"-RNA-Strang auch Insertionen, Deletionen sowie einzelne Punktmutationen im Vergleich zu dem Komplement des "sense"-RNA-Stranges aufweisen kann. Bevorzugt beträgt die Homologie mindestens 80 %, bevorzugt mindestens 90 %, ganz besonders bevorzugt mindestens 95 %, am meisten bevorzugt 100 % zwischen dem "antisense"-RNA-Strang und dem Komplement des "sense"-RNA-Stranges.

In einer weiteren Ausführungsform umfasst die ε-Cyclase-dsRNA
a) einen "sense"-RNA-Strang umfassend mindestens eine Ribonukleotidsequenz, die im wesentlichen identisch ist zu mindestens einem Teil des "sense"-RNA-Transkriptes des Promotorbereichs eines ε-Cyclase-Gens, und
b) einen "antisense"-RNA-Strang, der zu dem RNA-"sense"-Strang unter a) im wesentlichen - bevorzugt vollständig - komplementären ist.

Das entsprechende, bevorzugt zur Transformation der Pflanzen zu verwendende, Nukleinsäurekonstrukt, umfasst
a) einen "sense"-DNA-Strang der im wesentlichen identisch ist zu mindestens einem Teil des Promotorbereichs eines ε-Cyclase-Gens, und
b) einen "antisense"-DNA-Strang, der zu dem DNA-"sense"-Strang unter a) im wesentlichen - bevorzugt vollständig - komplementär ist.

Vorzugsweise wird unter dem Promotorbereich einer ε-Cyclase eine Sequenz gemäß SEQ ID NO: 47 oder ein Teil der selben verstanden.

Zur Herstellung der ε-Cyclase-dsRNA-Sequenzen zur Reduzierung der ε-Cyclase-Aktivität werden, insbesondere für *Tagetes erecta,* besonders bevorzugt die folgenden Teil-Sequenzen verwendet:
SEQ ID NO: 40: Sense-Fragment der 5'terminalen Region der ε-Cyclase
SEQ ID NO: 41: Antisense-Fragment der 5'terminalen Region der ε-Cyclase
SEQ ID NO: 42: Sense-Fragment der 3'terminalen Region der ε-Cyclase
SEQ ID NO: 43: Antisense-Fragment der 3'terminalen Region der ε-Cyclase
SEQ ID NO: 47: Sense-Fragment des ε-Cyclase-Promotors
SEQ ID NO: 48: Antisense-Fragment des ε-Cyclase-Promotors

Die dsRNA kann aus einem oder mehr Strängen von Polyribonukleotiden bestehen. Natürlich können, um den gleichen Zweck zu erreichen, auch mehrere individuelle dsRNA Moleküle, die jeweils einen der oben definierten Ribonukleotidsequenzabschnitte umfassen, in die Zelle oder den Organismus eingebracht werden.

Die doppelsträngige dsRNA-Struktur kann ausgehend von zwei komplementären, separaten RNA-Strängen oder - bevorzugt - ausgehend von einem einzelnen, selbstkomplementären RNA-Strang gebildet werden. In diesem Fall sind "sense"-RNA-Strang und "antisense"-RNA-Strang bevorzugt kovalent in Form eines invertierten "Repeats" miteinander verbunden.

Wie z.B. in WO 99/53050 beschrieben, kann die dsRNA auch eine Haarnadelstruktur umfassen, indem "sense"- und "antisense"-Strang durch eine verbindende Sequenz ("Linker"; beispielsweise ein Intron) verbunden werden. Die selbstkomplementären dsRNA-Strukturen sind bevorzugt, da sie lediglich die Expression einer RNA-Sequenz erfordern und die komplementären RNA-Stränge stets in einem äquimolaren Verhältnis umfassen. Bevorzugt ist die verbindende Sequenz ein Intron (z.B. ein Intron des ST-LS1 Gens aus Kartoffel; Vancanneyt GF et al. (1990) Mol Gen Genet 220(2):245-250).

Die Nukleinsäuresequenz kodierend für eine dsRNA kann weitere Elemente beinhalten, wie beispielsweise Transkriptionsterminationssignale oder Polyadenylierungssignale.

Ist die dsRNA jedoch gegen die Promotorsequenz einer ε-Cyclase gerichtet, so umfasst sie bevorzugt keine Transkriptionsterminationssignale oder Polyadenylierungssignale. Dies ermöglicht eine Retention der dsRNA im Nukleus der Zelle und verhindert eine Verteilung der dsRNA in der gesamten Pflanze "Spreadinng").

Sollen die zwei Stränge der dsRNA in einer Zelle oder Pflanze zusammengebracht werden, so kann dies beispielhaft auf folgende Art geschehen:
a) Transformation der Zelle oder Pflanze mit einem Vektor, der beide Expressionskassetten umfasst,
b) Kotransformation der Zelle oder Pflanze mit zwei Vektoren, wobei der eine die Expressionskassetten mit dem "sense"-Strang, der andere die Expressionskassetten mit dem "antisense°-Strang umfasst.
c) Kreuzung von zwei individuellen Pflanzenlinien, wobei die eine die Expressionskassetten mit dem "sense"-Strang, die andere die Expressionskassetten mit dem "antisense"-Strang umfasst.

Die Bildung der RNA Duplex kann entweder außerhalb der Zelle oder innerhalb derselben initiiert werden.

Die dsRNA kann entweder in vivo oder in vitro synthetisiert werden. Dazu kann eine DNA-Sequenz kodierend für eine dsRNA in eine Expressionskassette unter Kontrolle mindestens eines genetischen Kontrollelementes (wie beispielsweise einem Promotor) gebracht werden. Eine Polyadenylierung ist nicht erforderlich, ebenso müssen keine Elemente zur Initiierung einer Translation vorhanden sein. Bevorzugt ist die Expressionskassette für die MP-dsRNA auf dem Transformationskonstrukt oder dem Transformationsvektor enthalten.

In einer besonders bevorzugten Auführungsform erfolgt die Expression der dsRNA ausgehend von einem Expressionskonstrukt unter funktioneller Kontrolle eines blütenspezifischen Promotors, besonders bevorzugt unter der Kontrolle des Promotors beschrieben durch SEQ ID NO: 28 oder eines funktionell äquivalenten Teils desselben.

Die Expressionskassetten kodierend für den "antisense"- und/oder den "sense"-Strang einer ε-Cyclase -dsRNA oder für den selbstkomplementären-Strang der dsRNA, werden dazu bevorzugt in einen Transformationsvektor insertiert und mit den unten beschriebenen Verfahren in die pflanzliche Zelle eingebracht. Für das erfindungsgemäße Verfahren ist eine stabile Insertion in das Genom vorteilhaft.

Die dsRNA kann in einer Menge eingeführt werden, die zumindest eine Kopie pro Zelle ermöglicht. Höhere Mengen (z.B. mindestens 5, 10, 100, 500 oder 1000 Kopien pro Zelle) können ggf. eine effizienter Verminderung bewirken.
b) Einbringen einer antisense-Ribonukleinsäuresequenz einer ε-Cyclase (ε-Cyclase-antisenseRNA)

Verfahren zur Verminderung eines bestimmten Proteins durch die "antisense"-Technologie sind vielfach - auch in Pflanzen - beschrieben (Sheehy et al. (1988) Proc Natl Acad Sci USA 85: 8805-8809; US 4,801,340; Mol JN et al. (1990) FEBS Lett 268(2):427-430). Das antisense Nukleinsäuremolekül hybridisiert bzw. bindet mit der zellulären mRNA und/oder genomischen DNA kodierend für das zu vermindernde ε-Cyclase. Dadurch wird die Transkription und/oder Translation der ε-Cyclase unterdrückt. Die Hybridisierung kann auf konventionelle Art über die Bildung einer stabilen Duplex oder - im Fall von genomischer DNA - durch Bindung des antisense Nukleinsäuremoleküls mit der Duplex der genomischen DNA durch spezifische Wechselwirkung in der großen Furche der DNA-Helix entstehen.

Eine ε-Cyclase-antisenseRNA kann unter Verwendung der für diese ε-Cyclase kodierenden Nukleinsäuresequenz, beispielsweise der Nukleinsäuresequenz gemäß SEQ ID NO: 38 nach den Basenpaarregeln von Watson und Crick abgeleitet werden. Die ε-Cyclase-antisenseRNA kann zu der gesamten transkribierten mRNA der ε-Cyclase komplementär sein, sich auf die kodierende Region beschränken oder nur aus einem Oligonukleotid bestehen, das zu einem Teil der kodierenden oder nicht-kodierenden Sequenz der mRNA komplementär ist. So kann das Oligonukleotid beispielsweise komplementär zu der Region sein, die den Translationsstart für die ε-Cyclase umfasst. Die ε-Cyclase-antisenseRNA kann eine Länge von zum Beispiel 5, 10, 15, 20, 25, 30, 35, 40, 45 oder 50 Nukleotide haben, kann aber auch länger sein und mindestens 100, 200, 500, 1000, 2000 oder 5000 Nukleotide umfassen. ε-Cyclase-antisenseRNAs werden im Rahmen des erfindungsgemäßen Verfahrens bevorzugt rekombinant in der Zielzelle exprimiert..

In einer besonders bevorzugten Ausführungsform erfolgt die Expression der antisenseRNA ausgehend von einem Expressionskonstrukt unter funktioneller Kontrolle eines blütenspezifischen Promotors, besonders bevorzugt unter der Kontrolle des Promotors beschrieben durch SEQ ID NO: 28 oder eines funktionell äquivalenten Teils desselben.

Besagte Expressionskassetten können Teil eines Transformationskonstruktes oder Transformationsvektors sein, oder aber auch im Rahmen einer Kotransformation eingeführt werden.

In einer weiteren bevorzugten Ausführungsform kann die Expression einer ε-Cyclase durch Nukleotidsequenzen inhibiert werden, die komplementär zu der regulatorischen Region eines ε-Cyclase-Gens (z.B. einem ε-Cyclase Promoter und/oder Enhancer) sind und triple-helikale Strukturen mit der dortigen DNA-Doppelhelix ausbilden, so dass die Transkription des ε-Cyclase-Gens reduziert wird. Entsprechende Verfahren sind beschrieben (Helene C (1991) Anticancer Drug Res 6(6):569-84; Helene C et al. (1992) Ann NY Acad Sci 660:27-36; Maher LJ (1992) Bioassays 14(12):807- 815).

In einer weiteren Ausführungsform kann die ε-Cyclase-antisenseRNA eine α-anomere Nukleinsäure sein. Derartige α-anomere Nukleinsäuremoleküle bilden spezifische doppelsträngige Hybride mit komplementärer RNA in denen, - im Unterschied zu den konventionellen β-Nukleinsäuren - die beiden Stränge parallel zueinander verlaufen (Gautier C et al. (1987) Nucleic Acids Res 15:6625-6641).

### c) Einbringen einer ε-Cyclase-antisenseRNA kombiniert mit einem Ribozym

Vorteilhaft kann die oben beschriebene antisense-Strategie mit einem Ribozym-Verfahren gekoppelt werden. Katalytische RNA-Moleküle oder Ribozyme können an jede beliebige Ziel-RNA angepasst werden und spalten das Phosphodiester-Gerüst an spezifischen Positionen, wodurch die Ziel-RNA funktionell deaktiviert wird (Tanner NK (1999) FEMS Microbiol Rev 23(3):257-275). Das Ribozym wird dadurch nicht selber modifiziert, sondern ist in der Lage, weitere Ziel-RNA-Moleküle analog zu spalten, wodurch es die Eigenschaften eines Enzyms erhält. Der Einbau von Ribozymsequenzen in "antisense"-RNAs verleiht eben diesen "antisense"-RNAs diese enzymähnliche, RNA-spaltende Eigenschaft und steigert so deren Effizienz bei der Inaktivierung der Ziel-RNA. Die Herstellung und Verwendung entsprechender Ribozym-"antisense"-RNA-Moleküle ist beschrieben (u.a. bei Haseloff et al. (1988) Nature 334: 585-591); Haselhoff und Gerlach (1988) Nature 334:585-591; Steinecke P et al. (1992) EMBO J 11(4):1525-1530; de Feyter R et al. (1996) Mol Gen Genet. 250(3):329-338).

Auf diese Art können Ribozyme (z.B. "Hammerhead"-Ribozyme; Haselhoff und Gerlach (1988) Nature 334:585-591) verwendet werden, um die mRNA eines zu vermindernden ε-Cyclases katalytisch zu spalten und so die Translation zu verhindern. Die Ribozym-Technologie kann die Effizienz einer antisense-Strategie erhöhen. Verfahren zur Expression von Ribozymen zur Verminderung bestimmter Proteine sind beschrieben in (EP 0 291 533, EP 0 321 201, EP 0 360 257). In pflanzlichen Zellen ist eine Ribozym-Expression ebenfalls beschrieben (Steinecke P et al. (1992) EMBO J 11(4):1525-1530; de Feyter R et al. (1996) Mol Gen Genet. 250(3):329-338). Geeignete Zielsequenzen und Ribozyme können zum Beispiel wie bei "Steinecke P, Ribozymes, Methods in Cell Biology 50, Galbraith et al. eds, Academic Press, Inc. (1995), S. 449-460" beschrieben, durch Sekundärstrukturberechnungen von Ribozym- und Ziel-RNA sowie durch deren Interaktion bestimmt werden (Bayley CC et al. (1992) Plant Mol Biol. 18(2):353-361; Lloyd AM and Davis RW et al. (1994) Mol Gen Genet. 242(6):653-657). Beispielsweise können Derivate der Tetrahymena L-19 IVS RNA konstruiert werden, die komplementäre Bereiche zu der mRNA des zu supprimierenden ε-Cylases aufweisen (siehe auch US 4,987,071 und US 5,116,742). Alternativ können solche Ribozyme auch über einen Selektionsprozess aus einer Bibliothek diverser Ribozyme identifiziert werden (Bartel D und Szostak JW (1993) Science 261:1411-1418).

### d) Einbringen einer sense-Ribonukleinsäuresequenz einer ε-Cyclase (ε-Cyclase-senseRNA) zur Induktion einer Kosuppression

Die Expression einer ε-Cyclase Ribonukleinsäuresequenz (oder eines Teils derselben) in sense-Orientierung kann zu einer Kosuppression des entsprechenden ε-Cyclase-Gens führen. Die Expression von sense-RNA mit Homologie zu einem endogenen ε-Cyctasegen kann die Expression desselben vermindern oder ausschalten, ähnlich wie es für antisense Ansätze beschrieben wurde (Jorgensen et al. (1996) Plant Mol Biol 31(5):957-973; Gering et al. (1991) Proc Natl Acad Sci USA 88:1770-1774; Smith et al. (1990) Mol Gen Genet 224:447-481; Napoli et al. (1990) Plant Cell 2:279-289; Van der Krol et al. (1990) Plant Cell 2:291-99). Dabei kann das eingeführte Konstrukt das zu vermindernde, homologe Gen ganz oder nur teilweise repräsentieren. Die Möglichkeit zur Translation ist nicht erforderlich. Die Anwendung dieser Technologie auf Pflanzen ist beschrieben (z.B. Napoli et al. (1990) Plant Cell 2:279-289; in US 5,034,323.

Bevorzugt wird die Kosuppression unter Verwendung einer Sequenz realisiert, die im wesentlichen identisch ist zu zumindest einem Teil der Nukleinsäuresequenz kodierend für eine ε-Cyclase, beispielsweise der Nukleinsäuresequenz gemäß SEQ ID NO: 38. Bevorzugt ist die ε-Cyclase-senseRNA so gewählt, dass es nicht zu einer Translation der ε-Cyclase oder eines Teils desselben kommen kann. Dazu kann beispielsweise der 5'-untranslatierte oder 3'-untranslatierte Bereich gewählt oder aber das ATG-Startkodon deletiert oder mutiert werden.

### e) Einbringen von DNA-oder Protein-bindende Faktoren gegen ε-Cyclase Gene, - RNAs oder Proteine

Eine Verminderung einer ε-Cyclase Expression ist auch mit spezifischen DNAbindenden Faktoren z.B. mit Faktoren vom Typ der Zinkfingertranskriptionsfaktoren möglich. Diese Faktoren lagern sich an die genomische Sequenz des endogenen Zielgens, bevorzugt in den regulatorischen Bereichen, an und bewirken eine Verminderung der Expression. Entsprechende Verfahren zur Herstellung entsprechender Faktoren sind beschrieben (Dreier B et al. (2001) J Biol Chem 276(31):29466-78; Dreier B et al. (2000) J Mol Biol 303(4):489-502; Beerli RR et al. (2000) Proc Natl Acad Sci USA 97 (4):1495-1500; Beerli RR et al. (2000) J Biol Chem 275(42):32617-32627; Segal DJ and Barbas CF 3rd. (2000) Curr Opin Chem Biol 4(1):34-39; Kang JS and Kim JS (2000) J Biol Chem 275(12):8742-8748; Beerli RR et al. (1998) Proc Natl Acad Sci USA 95(25):14628- 14633; Kim JS et al. (1997) Proc Natl Acad Sci USA 94(8):3616 -3620; Klug A (1999) J Mol Biol 293(2):215-218; Tsai SY et al. (1998) Adv Drug Deliv Rev 30(1-3):23-31; Mapp AK et al. (2000) Proc Natl Acad Sci USA 97(8):3930-3935; Sharrocks AD et al. (1997) Int J Biochem Cell Biol 29(12):1371-1387; Zhang L et al. (2000) J Biol Chem 275(43):33850-33860).

Die Selektion dieser Faktoren kann unter Verwendung eines beliebigen Stückes eines ε-Cyclase-Gens erfolgen. Bevorzugt liegt dieser Abschnitt im Bereich der Promotorregion. Für eine Genunterdrückung kann er aber auch im Bereich der kodierenden Exons oder Introns liegen.

Ferner können Faktoren in eine Zelle eingebracht werden, die die ε-Cyclase selber inhibieren. Diese proteinbindenden Faktoren können z.B. Aptamere (Famulok M und Mayer G (1999) Curr Top Microbiol Immunol 243:123-36) oder Antikörper bzw. Antikörperfragmente oder einzelkettige Antikörper sein. Die Gewinnung dieser Faktoren ist beschrieben (Owen M et al. (1992) Biotechnology (N Y) 10(7):790-794; Franken E et al. (1997) Curr Opin Biotechnol 8(4):411-416; Whitelam (1996) Trend Plant Sci 1:286-272).

### f) Einbringen von den ε-Cyclase RNA-Abbau bewirkenden viralen Nukleinsäuresequenzen und Expressionskonstrukten

Die ε-Cyclase Expression kann effektiv auch durch Induktion des spezifischen ε-Cyclase RNA-Abbaus durch die Pflanze mit Hilfe eines viralen Expressionssystems (Amplikon; Angell SM et al. (1999) Plant J 20(3):357-362) realisiert werden. Diese Systeme - auch als "VIGS" (viral induced gene silencing) bezeichnet - bringen Nukleinsäuresequenzen mit Homologie zu dem Transkript einer zu vermindernden ε-Cyclase mittels viraler Vektoren in die Pflanze ein. Die Transkription wird sodann - vermutlich mediiert durch pflanzliche Abwehrmechanismen gegen Viren - abgeschaltet. Entsprechende Techniken und Verfahren sind beschrieben (Ratcliff F et al. (2001) Plant J 25(2):237-45; Fagard M und Vaucheret H (2000) Plant Mol Biol 43(2-3):285-93; Anandalakshmi R et al. (1998) Proc Natl Acad Sci USA 95(22):13079-84; Ruiz MT (1998) Plant Cell 10(6):937-46).

Bevorzugt wird die VIGS-vermittelte Verminderung unter Verwendung einer Sequenz realisiert, die im wesentlichen identisch ist zu zumindest einem Teil der Nukleinsäuresequenz kodierend für ein ε-Cyclase, beispielsweise der Nukleinsäuresequenz gemäß SEQ ID NO: 1 .

### g) Einbringen von Konstrukten zur Erzeugung eines Funktionsverlustes oder einer Funktionsminderung an ε-Cyclase-Genen

Dem Fachmann sind zahlreiche Verfahren bekannt, wie genomische Sequenzen gezielt modifiziert werden können. Dazu zählen insbesondere Verfahren wie die Erzeugung von Knockout-Mutanten mittels gezielter homologen Rekombination z.B. durch Generierung von Stopp-Kodons, Verschiebungen im Leseraster etc. (Hohn B und Puchta H (1999) Proc Natl Acad Sci USA 96:8321-8323) oder die gezielte Deletion oder Inversion von Sequenzen mittels z.B. sequenzspezifischer Rekombinasen oder Nukleasen (s.u.)

Die Verminderung der ε-Cyclase-Menge, -Funktion und/oder -Aktivität kann auch durch eine gezielte Insertion von Nukleinsäuresequenzen (z.B. der im Rahmen der erfindungsgemäßen Verfahrens zu insertierenden Nukleinsäuresequenz) in die Sequenz kodierend für eine ε-Cyclase (z.B. mittels intermolekularer homologer Rekombination) realisiert werden. Im Rahmen dieser Ausführungsform verwendet man bevorzugt ein DNA-Konstrukt, das zumindest einen Teil der Sequenz eines ε-Cyclasegens oder benachbarter Sequenzen umfasst, und so mit diesen in der Zielzelle gezielt rekombinieren kann, so dass durch eine Deletion, Addition oder Substitution mindestens eines Nukleotids das ε-Cyclase-Gen so verändert wird, dass die Funktionalität des ε-Cyclase-Gens reduziert oder gänzlich aufgehoben wird. Die Veränderung kann auch die regulativen Elemente (z.B. den Promotor) des ε-Cyclase-Gens betreffen, so dass die kodierende Sequenz unverändert bleibt, eine Expression (Transkription und/oder Translation) jedoch unterbleibt und reduziert wird. Bei der konventionellen homologen Rekombination ist die zu insertierende Sequenz an ihrem 5'- und/oder 3'-Ende von weiteren Nukleinsäuresequenzen (A' bzw. B') flankiert, die eine ausreichende Länge und Homologie zu entsprechenden Sequenzen des ε-Cyclase-Gens (A bzw. B) für die Ermöglichung der homologen Rekombination aufweisen. Die Länge liegt in der Regel in einem Bereich von mehreren hundert Basen bis zu mehreren Kilobasen (Thomas KR und Capecchi MR (1987) Cell 51:503; Strepp et al. (1998) Proc Natl Acad Sci USA 95(8):4368-4373). Für die homologe Rekombination wird die pflanzliche Zelle mit dem Rekombinationskonstrukt unter Verwendung der unten beschriebenen Verfahren transformiert und erfolgreich rekombinierte Klone basierend auf der infolge inaktivierten ε-Cyclase selektioniert.

In einer weiteren bevorzugten Ausführungsform wird die Effizienz der Rekombination gesteigert durch Kombination mit Verfahren, die die homologe Rekombination fördern. Solche Verfahren sind beschrieben und umfassen beispielhaft die Expression von Proteinen wie RecA oder die Behandlung mit PARP-Inhibitoren. Es konnte gezeigt werden, dass die intrachromosomale homologe Rekombination in Tabakpflanzen durch die Verwendung von PARP-Inhibitoren erhöht werden kann (Puchta H et al. (1995) Plant J 7:203-210). Durch den Einsatz dieser Inhibitoren kann die Rate der homologen Rekombination in den Rekombinationskonstrukten nach Induktion des sequenzspezifischen DNA-Doppelstrangbruches und damit die Effizienz der Deletion der Transgensequenzen weiter erhöht werden. Verschiedene PARP Inhibitoren können dabei zum Einsatz kommen. Bevorzugt umfasst sind Inhibitoren wie 3-Aminobenzamid, 8-Hydroxy-2-methylquinazolin-4-on (NU1025), 1,11b-Dihydro-[2H]benzopyrano-[4,3,2-de]isoquinolin-3-on (GPI 6150), 5-Aminoisoquinolinon, 3,4-Dihydro-5-[4-(1-piperidinyl)butoxy]-1(2H)-isoquinolinon oder die in WO 00/26192, WO 00/29384,

WO 00/32579, WO 00/64878, WO 00/68206, WO 00/67734, WO 01/23386 und WO 01/23390 beschriebenen Substanzen.

Weitere geeignete Methoden sind die Einführung von Nonsense-Mutationen in endogene Markerprotein Gene zum Beispiel mittels Einführung von RNA/DNA-Oligonukleotiden in die Pflanze (Zhu et al. (2000) Nat Biotechnol 18(5):555-558) oder die Generierung von Knockout-Mutanten mit Hilfe von z.B. T-DNA-Mutagenese (Koncz et al., Plant Mol. Biol. 1992, 20(5):963-976). Punktmutationen können auch mittels DNA-RNA Hybriden erzeugt werden, die auch als "chimeraplasty" bekannt sind (Cole-Strauss et al. (1999) Nucl Acids Res 27(5):1323-1330; Kmiec (1999) Gene therapy American Scientist 87(3):240-247).

Die Methoden der dsRNAi, der Kosuppression mittels sense-RNA und der "VIGS" ("virus induced gene silencing") werden auch als "post-transcriptional gene silencing" (PTGS) oder transcriptional gene silencing" (TGS) bezeichnet. PTGSfTGS-Verfahren sind besonders vorteilhaft, weil die Anforderungen an die Homologie zwischen dem zu vermindernden Markerprotein-Gen und der transgen exprimierten sense- oder dsRNA-Nukleinsäuresequenz geringer sind als beispielsweise bei einem klassischen antisense-Ansatz. So kann man unter Verwendung der Markerprotein-Nukleinsäuresequenzen aus einer Art auch die Expression von homologen Markerprotein-Proteinen in anderen Arten effektiv vermindern, ohne, dass die Isolierung und Strukturaufklärung der dort vorkommenden Markerprotein-Homologen zwingend erforderlich wäre. Dies erleichtert erheblich den Arbeitsaufwand.

In einer besonders bevorzugten Ausführungsform des erfindungsgemäßen Verfahrens erfolgt die Reduzierung der ε-Cyclase-Aktivität gegenüber dem Wildtyp durch:
a) Einbringen mindestens einer doppelsträngigen ε-Cyclase Ribonukleinsäuresequenz oder einer deren Expression gewährleistenden Expressionskassette oder Expressionskassetten in Pflanzen und/oder
b) Einbringen mindestens einer ε-Cyclase antisense-Ribonukleinsäuresequenzen oder einer deren Expression gewährleistenden Expressionskassette in Pflanzen.

In einer ganz besonders bevorzugten Ausführungsform erfolgt die Reduzierung der ε-Cyclase-Aktivität gegenüber dem Wildtyp durch Einbringen mindestens einer doppelsträngigen ε-Cyclase Ribonukleinsäuresequenz oder einer deren Expression gewährleistenden Expressionskassette oder Expressionskassetten in Pflanzen.

In einer bevorzugten Ausführungsform werden genetisch veränderte Pflanzen verwendet, die in Blüten die geringste Expressionsrate einer ε-Cyclase aufweisen.

Dies wird bevorzugt dadurch erreicht, dass die Reduzierung der ε-Cyclase-Aktivität blütenspezifisch, besonders bevorzugt blütenblattspezifisch erfolgt.

In der vorstehend beschriebenen, besonders bevorzugten Ausführungsform wird dies dadurch erreicht, dass die Transkription der ε-Cyclase-dsRNA-Sequenzen unter Kontrolle eines blütenspezifischen Promotors oder noch bevorzugter unter Kontrolle eines blütenblattspezifischen Promotors erfolgt.

In einer weiter bevorzugten Ausführungsform werden Pflanzen kultiviert, die zusätzlich gegenüber dem Wildtyp eine erhöhte Aktivität mindestens einer der Aktivitäten, ausgewählt aus der Gruppe HMG-CoA-Reduktase-Aktivität, (E)-4-Hydroxy-3-Methylbut-2-enyl-Diphosphat-Reduktase-Aktivität, 1-Deoxy-D-Xylose-5-Phosphat-Synthase-Aktivität, 1-Deoxy-D-Xylose-5-Phosphat-Reduktoisomerase-Aktivität, Isopentenyl-Diphosphat-Δ-Isomerase-Aktivität, Geranyl-Diphosphat-Synthase-Aktivität, Farnesyl-Diphosphat-Synthase-Aktivität, Geranyl-Geranyl-Diphosphat-Synthase-Aktivität, Phytoen-Synthase-Aktivität, Phytoen-Desaturase-Aktivität, Zeta-Carotin-Desaturase-Aktivität, crtISO-Aktivität, FtsZ-Aktivität und MinD-Aktivität aufweisen.

Unter HMG-CoA-Reduktase-Aktivität wird die Enzymaktivität einer HMG-CoA-Reduktase (3-Hydroxy-3-Methyl-Glutaryl-Coenzym-A-Reduktase) verstanden.

Unter einer HMG-CoA-Reduktase wird ein Protein verstanden, das die enzymatische Aktivität aufweist, 3-Hydroxy-3-Methyl-Glutaryl-Coenzym-A in Mevalonat umzuwandeln.

Dementsprechend wird unter HMG-CoA-Reduktase-Aktivität die in einer bestimmten Zeit durch das Protein HMG-CoA-Reduktase umgesetzte Menge 3-Hydroxy-3-Methyl-Glutaryl-Coenzym-A bzw. gebildete Menge Mevalonat verstanden.

Bei einer erhöhten HMG-CoA-Reduktase-Aktivität gegenüber dem Wildtyp wird somit im Vergleich zum Wildtyp in einer bestimmten Zeit durch das Protein HMG-CoA-Reduktase die umgesetzte Menge 3-Hydroxy-3-Methyl-Glutaryl-Coenzym-A bzw. die gebildete Menge Mevalonat erhöht.

Vorzusgweise beträgt diese Erhöhung der HMG-CoA-Reduktase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der HMG-CoA-Reduktase-Aktivität des Wildtyps.Unter HMG-CoA-Reduktase-Aktivität wird die Enzymaktivität einer HMG-CoA-Reduktase verstanden.

Die Bestimmung der HMG-CoA-Reduktase-Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7.4), 10 mM MgCl2, 10 mM KCI, 1 mM EDTA, 1 mM EGTA, 0.1% (v/v) Triton X-100, 2 mM ε-Aminocapronsäure, 10% Glyzerin, 5 mM KHC03. Kurz vor der Extraktion wird 2 mM DTT und 0.5 mM PMSF zugegeben.

Die Aktivität der HMG-CoA-Reduktase kann nach veröffentlichen Beschreibungen gemessen werden (z.B. Schaller, Grausem, Benveniste, Chye, Tan, Song und Chua, Plant Physiol. 109 (1995), 761-770; Chappell, Wolf, Proulx, Cuellar und Saunders, Plant Physiol. 109 (1995) 1337-1343). Pflanzengewebe kann in kaltem Puffer (100 mM Kaliumphosphat (pH 7.0), 4 mM MgCl₂, 5 mM DTT) homogenisiert und extrahiert werden. Das Homogenisat wird 15 Minuten lang bei 10.000g bei 4C zentrifugiert. Der Überstand wird danach bei 100.000g für 45-60 Minuten nochmals zentrifugiert. Die Aktivität der HMG-CoA-Reduktase wird im Überstand und im Pellet der mikrosomalen Fraktion (nach dem Resuspendieren in 100 mM Kaliumphosphat (pH 7.0) und 50 mM DTT) bestimmt. Aliquots der Lösung und der Suspension (der Proteingehalt der Suspension entspricht etwa 1-10 ∝g) werden in 100 mM Kaliumphosphat-Puffer (pH 7,0 mit 3 mM NADPH und 20 ∝M (¹⁴C)HMG-CoA (58 ∝Ci/∝M) idealerweise in einem Volumen von 26 ∝I für 15-60 Minuten bei 30C inkubiert. Die Reaktion wird terminiert durch die Zugabe von 5 ∝I Mevalonatlacton (1 mg/ml) und 6 N HCl. Nach Zugabe wird die Mischung bei Raumtemperatur 15 Minuten inkubiert. Das in der Reaktion gebildete (¹⁴C)-Mevalonat wird quantifiziert, indem 125 ∝I einer gesättigten Kaliumphosphat-Lösung (pH 6.0) und 300 ∝I Ethylacetat zugegeben werden. Die Mischung wird gut vermischt und zentrifugiert. Mittels Szintillationsmessung kann die Radioaktivität bestimmt werden.

Unter (E)-4-Hydroxy-3-Methylbut-2-enyl-Diphosphat-Reduktase-Aktivität, auch lytB oder IspH bezeichnet, wird die Enzymaktivität einer (E)-4-Hydroxy-3-Methylbut-2-enyl-Diphosphat-Reduktase verstanden.

Unter einer (E)-4-Hydroxy-3-Methylbut-2-enyl-Diphosphat-Reduktase wird ein Protein verstanden, das die enzymatische Aktivität aufweist, (E)-4-Hydroxy-3-Methylbut-2-enyl-Diphosphat in Isopentenyldiphosphat und Dimethylallyldiphosphate umzuwandeln.

Dementsprechend wird unter (E)-4-Hydroxy-3-Methylbut-2-enyl-Diphosphat-Reduktase-Aktivität die in einer bestimmten Zeit durch das Protein (E)-4-Hydroxy-3-Methylbut-2-enyl-Diphosphat-Reduktase umgesetzte Menge (E)-4-Hydroxy-3-Methylbut-2-enyl-Diphosphat bzw. gebildete Menge Isopentenyldiphosphat und/oder Dimethylallyldiphosphat verstanden.

Bei einer erhöhten (E)-4-Hydroxy-3-Methylbut-2-enyl-Diphosphat-Reduktase -Aktivität gegenüber dem Wildtyp wird somit im Vergleich zum Wildtyp in einer bestimmten Zeit durch das Protein (E)-4-Hydroxy-3-Methylbut-2-enyl-Diphosphat-Reduktase die umgesetzte Menge (E)-4-Hydroxy-3-Methylbut-2-enyl-Diphosphat bzw. die gebildete Menge lsopentenyldiphosphat und/oder Dimethylallyldiphosphat erhöht.

Vorzugsweise beträgt diese Erhöhung der (E)-4-Hydroxy-3-Methylbut-2-enyl-Diphosphat-Reduktase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der (E)-4-Hydroxy-3-Methylbut-2-enyl-Diphosphat-Reduktase -Aktivität des Wildtyps.

Die Bestimmung der (E)-4-Hydroxy-3-Methylbut-2-enyl-Diphosphat-Reduktase-Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7.4), 10 mM MgCl2, 10 mM KCI, 1 mM EDTA, 1 mM EGTA, 0.1 % (v/v) Triton X-100, 2 mM _{ε}-Aminocapronsäure, 10 % Glyzerin, 5 mM KHCO3. Kurz vor der Extraktion wird 2 mM DTT und 0,5 mM PMSF zugegeben.

Die Bestimmung der (E)-4-Hydroxy-3-Methylbut-2-enyl-Diphosphat-Reduktase-Aktivität kann über einen immunologischen Nachweis erbracht werden. Die Herstellung spezifischer Antikörper ist durch Rohdich und Kollegen (Rohdich, Hecht, Gärtner, A-dam, Krieger, Amslinger, Arigoni, Bacher und Eisenreich: Studies on the nonmevalonate terpene biosynthetic pathway: metabolic role of IspH (LytB) protein, Natl. Acad. Natl. Sci. USA 99 (2002), 1158-1163) beschrieben worden. Zur Bestimmung der katalytischen Aktivität bschreiben Altincicek und Kollegen (Altincicek, Duin, Reichenberg, Hedderich, Kollas, Hintz, Wagner, Wiesner, Beck und Jomaa: LytB protein catalyzes the terminal step of the 2-C-methyl-D-erythritol-4-phosphate pathway of isoprenoid biosynthesis; FEBS Letters 532 (2002,) 437-440) ein in vitro-System, welches die Reduktion von (E)-4-hydroxy-3-methyl-but-2-enyl diphosphat in die Isopentenyldiphosphat und Dimethylallyldiphosphat verfolgt.

Unter 1-Deoxy-D-Xylose-5-Phosphat-Synthase-Aktivität wird die Enzymaktivität einer 1 Deoxy-D-Xylose-5-Phosphat-Synthase verstanden.

Unter einer 1-Deoxy-D-Xylose-5-Phosphat-Synthase wird ein Protein verstanden, das die enzymatische Aktivität aufweist, Hydroxyethyl-ThPP und Glycerinaldehyd-3-Phosphat in 1-Deoxy-D-Xylose-5-Phosphat umzuwandeln.

Dementsprechend wird unter 1-Deoxy-D-Xylose-5-Phosphat-Synthase -Aktivität die in einer bestimmten Zeit durch das Protein 1-Deoxy-D-Xylose-5-Phosphat-Synthase umgesetzte Menge Hydroxyethyl-ThPP und/oder Glycerinaldehyd-3-Phosphat bzw. gebildete Menge -Deoxy-D-Xylose-5-Phosphat verstanden.

Bei einer erhöhten 1-Deoxy-D-Xylose-5-Phosphat-Synthase -Aktivität gegenüber dem Wildtyp wird somit im Vergleich zum Wildtyp in einer bestimmten Zeit durch das Protein 1-Deoxy-D-Xylose-5-Phosphat-Synthase die umgesetzte Menge Hydroxyethyl-ThPP und/oder Glycerinaldehyd-3-Phosphat bzw. die gebildete Menge -Deoxy-D-Xylose-5-Phosphat erhöht.

Vorzugsweise beträgt diese Erhöhung der 1-Deoxy-D-Xylose-5-Phosphat-Synthase - Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der 1-Deoxy-D-Xylose-5-Phosphat-Synthase-Aktivität des Wildtyps.

Die Bestimmung der 1-Deoxy-D-Xylose-5-Phosphat-Synthase-Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7.4), 10 mM MgCl2, 10 mM KCI, 1 mM EDTA, 1 mM EGTA, 0,1 % (v/v) Triton X-100, 2 mM ε-Aminocapronsäure, 10 % Glyzerin, 5 mM KHC03. Kurz vor der Extraktion wird 2 mM DTT und 0,5 mM PMSF zugegeben.

Die Reaktionslösung (50-200 ul) für die Bestimmung der D-1-Deoxyxylulose-5-Phosphat-Synthase-Aktivität (DXS) besteht aus 100 mM Tris-HCl (pH 8.0), 3 mM MgCl₂, 3 mM MnCl₂, 3 mM ATP, 1 mM Thiamindiphosphat, 0.1% Tween-60, 1 mM Kaliumfluorid, 30 ∝M (2-¹⁴C)-Pyruvat (0.5 ∝Ci), 0.6 mM DL-Glyerinaldehyd-3-phosphat. Der Pflanzenextrakt wird 1 bis 2 Stunden in der Reaktionslösung bei 37C inkubiert. Danach wird die Reaktion durch Erhitzen auf 80C für 3 Minuten gestoppt. Nach Zentrifugation bei 13.000 Umdrehungen/Minute für 5 Minuten wird der Überstand evaporiert, der Rest in 50 ∝I Methanol resuspendiert, auf eine TLC-Platte für Dünnschichtchromatographie (Silica-Gel 60, Merck, Darmstadt) aufgetragen und in N-Propylalkohol-/Ethylacetat/Wasser (6:1:3; v/v/v) aufgetrennt. Dabei trennt sich radioaktiv markiertes D-1-deoxyxylulose-5-phosphat (oder D-1-deoxyxylulose) von (2-¹⁴C)-Pyruvat. Die Quantifizierung erfolgt mittels Scintillationszähler. Die Methode wurde beschrieben in Harker und Bramley (FEBS Letters 448 (1999) 115-119). Alternativ wurde ein fluorometrischer Assay zur Bestimmung der DXS-Synthaseaktivität von Querol und Kollegen beschrieben (Analytical Biochemistry 296 (2001) 101-105).

Unter 1-Deoxy-D-Xylose-5-Phosphat-Reduktoisomerase -Aktivität wird die Enzymaktivität einer 1-Deoxy-D-Xylose-5-Phosphat-Reduktoisomerase verstanden.

Unter einer 1-Deoxy-D-Xylose-5-Phosphat-Reduktoisomerase wird ein Protein verstanden, das die enzymatische Aktivität aufweist, 1-Deoxy-D-Xylose-5-Phosphat in β-Carotin umzuwandeln.

Dementsprechend wird unter 1-Deoxy-D-Xylose-5-Phosphat-Reduktoisomerase - Aktivität die in einer bestimmten Zeit durch das Protein 1-Deoxy-D-Xylose-5-Phosphat-Reduktoisomerase umgesetzte Menge 1-Deoxy-D-Xylose-5-Phosphat bzw. gebildete Menge Isopenetenyl-Diphosphat verstanden.

Bei einer erhöhten 1-Deoxy-D-Xylose-5-Phosphat-Reduktoisomerase -Aktivität gegenüber dem Wildtyp wird somit im Vergleich zum Wildtyp in einer bestimmten Zeit durch das Protein 1-Deoxy-D-Xylose-5-Phosphat-Reduktoisomerase die umgesetzte Menge 1-Deoxy-D-Xylose-5-Phosphat bzw. die gebildete Menge Isopenetenyl-Diphosphat erhöht.

Vorzugsweise beträgt diese Erhöhung der 1-Deoxy-D-Xylose-5-Phosphat-Reduktoisomerase -Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % 1-Deoxy-D-Xylose-5-Phosphat-Reduktoisomerase -Aktivität des Wildtyps.

Die Bestimmung der 1-Deoxy-D-Xylose-5-Phosphat-Reduktoisomerase -Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7,4), 10 mM MgCl2, 10 mM KCI, 1 mM EDTA, 1 mM EGTA, 0,1 % (v/v) Triton X-100, 2 mM ε-Aminocapronsäure, 10 % Glyzerin, 5 mM KHCO3. Kurz vor der Extraktion wird 2 mM DTT und 0,5 mM PMSF zugegeben.

Die Aktivität der D-1-Deoxyxylulose-5-Phosphat-Reduktoisomerase (DXR) wird gemessen in einem Puffer aus 100 mM Tris-HCl (pH 7,5), 1 mM MnCl₂, 0,3 mM NADPH und 0,3 mM 1-Deoxy-D-Xylulose-4-Phosphat, welches z.B. enzymatisch synthetisiert werden kann (Kuzuyama, Takahashi, Watanabe und Seto: Tetrahedon letters 39 (1998) 4509-4512). Die Reaktion wird durch Zugabe des Pflanzenextraktes gestartet. Das Reaktionsvolumen kann typischerweis 0,2 bis 0,5 mL betragen; die Inkubation erfolgt bei 37C über 30-60 Minuten. Während dieser Zeit wird die Oxidation von NADPH photometrisch bei 340 nm verfolgt.

Unter lsopentenyl-Diphosphat-Δ-Isomerase -Aktivität wird die Enzymaktivität einer Isopentenyl-Diphosphat-Δ-Isomerase verstanden.

Unter einer lsopentenyl-Diphosphat-D-Isomerase wird ein Protein verstanden, das die enzymatische Aktivität aufweist, lsopenetenyl-Diphosphat in Dimethylallylphosphat umzuwandeln.

Dementsprechend wird unter lsopentenyl-Diphosphat-D-Isomerase-Aktivität die in einer bestimmten Zeit durch das Protein Isopentenyl-Diphosphat-D-Isomerase umgesetzte Menge Isopenetenyl-Diphosphat bzw. gebildete Menge Dimethylallylphosphat verstanden.

Bei einer erhöhten Isopentenyl-Diphosphat-D-Isomerase-Aktivität gegenüber dem Wildtyp wird somit im Vergleich zum Wildtyp in einer bestimmten Zeit durch das Protein Isopentenyl-Diphosphat-D-Isomerase die umgesetzte Menge Isopenetenyl-Diphosphat bzw. die gebildete Menge Dimethylallylphosphat erhöht.

Vorzugsweise beträgt diese Erhöhung der lsopentenyl-Diphosphat-Δ-Isomerase - Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der lsopentenyl-Diphosphat-Δ-Isomerase Aktivität des Wildtyps.

Die Bestimmung der Isopentenyl-Diphosphat-Δ-Isomerase-Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7,4), 10 mM MgCl2, 10 mM KCI, 1 mM EDTA, 1 mM EGTA, 0,1 % (v/v) Triton X-100, 2 mM ε-Aminocapronsäure, 10 % Glyzerin, 5 mM KHCO3. Kurz vor der Extraktion wird 2 mM DTT und 0,5 mM PMSF zugegeben.

Aktivitätsbestimmungen der Isopentenyl-Diphosphat-Isomerase (IPP-Isomerase) können nach der von Fraser und Kollegen vorgestellten Methode (Fraser, Römer, Shipton, Mills, Kiano, Misawa, Drake, Schuch und Bramley: Evaluation of transgenic tomato plants expressing an additional phytoene synthase in a fruit-specific manner; Proc. Natl. Acad. Sci. USA 99 (2002), 1092-1097, basierend auf Fraser, Pinto, Holloway und Bramley, Plant Journal 24 (2000), 551-558) durchgeführt werden. Für Enzymmessungen werden Inkubationen mit 0,5 ∝ Ci (1-¹⁴C)IPP (Isopentenylpyrophosphat) (56 mCi/mmol, Amersham plc) als Substrat in 0,4 M Tris-HCl (pH 8,0) mit 1 mM DTT, 4 mM MgCl₂, 6 mM Mn Cl₂, 3 mM ATP, 0,1 % Tween 60, 1 mM Kaliunfluorid in einem Volumen von etwa 150-500 ∝I durchgeführt. Extrakte werden mit Puffer gemischt (z.B. im Verhältnis 1:1) und für wenigstens 5 Stunden bei 28°C inkubiert. Danach wird etwa 200 ∝I Methanol zugegeben und durch Zugabe von konzentrierter Salzsäure (Endkonzentration 25 %) eine Säurehydrolyse für etwa 1 Stunde bei 37C durchgeführt. Anschließend erfolgt eine zweimalige Extraktion (jeweils 500 ∝I) mit Petrolether (versetzt mit 10% Diethylether). Die Radioaktivität in einem Aliquot der Hyperphase wird mittels Szintillationszähler bestimmt. Die spezifische Enzymaktivität kann bei kurzer Inkubation von 5 Minuten bestimmt werden, da kurze Reaktionszeiten die Bildung von Reaktionsnebenprodukten unterdrückt (siehe Lützow und Beyer: The isopentenyl-diphosphate Δisomerase and its relation to the phytoene synthase complex in daffodil chromoplasts; Biochim. Biophys. Acta 959 (1988),118-126)

Unter Geranyl-Diphosphat-Synthase -Aktivität wird die Enzymaktivität einer Geranyl-Diphosphat-Synthase verstanden.

Unter einer Geranyl-Diphosphat-Synthase wird ein Protein verstanden, das die enzymatische Aktivität aufweist, Isopentenyl-Diphosphat und Dimethylallylphosphat in Geranyl-Diphosphat umzuwandeln.

Dementsprechend wird unter Geranyl-Diphosphat-Synthase-Aktivität die in einer bestimmten Zeit durch das Protein Geranyl-Diphosphat-Synthase umgesetzte Menge Isopentenyl-Diphosphat und/oder Dimethylallylphosphat bzw. gebildete Menge Geranyl-Diphosphat verstanden.

Bei einer erhöhten Geranyl-Diphosphat-Synthase-Aktivität gegenüber dem Wildtyp wird somit im Vergleich zum Wildtyp in einer bestimmten Zeit durch das Protein Geranyl-Diphosphat-Synthase die umgesetzte Menge Isopentenyl-Diphosphat und/oder Dimethylallylphosphat bzw. die gebildete Menge Geranyl-Diphosphat erhöht.

Vorzugsweise beträgt diese Erhöhung der Geranyl-Diphosphat-Synthase -Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Geranyl-Diphosphat-Synthase-Aktivität des Wildtyps.

Die Bestimmung der Geranyl-Diphosphat-Synthase-Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7.4), 10 mM MgC12, 10 mM KCI, 1 mM EDTA, 1 mM EGTA, 0,1 % (v/v) Triton X-100, 2 mM ε-Aminocapronsäure, 10 % Glyzerin, 5 mM KHC03. Kurz vor der Extraktion wird 2 mM DTT und 0,5 mM PMSF zugegeben.

Die Aktivität der Geranyl-Diphosphat-Synthase (GPP-Synthase) kann in 50 mM Tris-HCl (pH 7.6), 10 mM MgCl₂, 5 mM MnCl₂, 2 mM DTT, 1 mM ATP, 0.2 % Tween-20, 5 ∝M (¹⁴C)IPP und 50 ∝M DMAPP (Dimethylallylpyrophosphat) nach Zugabe von Pflanzenextrakt bestimmt werden (nach Bouvier, Suire, d'Harlingue, Backhaus und Camara: Meolcular cloning of geranyl diphosphate synthase and compartmentation of monoterpene synthesis in plant cells, Plant Journal 24 (2000,) 241-252). Nach der Inkubation von z.B. 2 Stunden bei 37C werden die Reaktionsprodukte dephosphyryliert (nach Koyama, Fuji und Ogura: Enzymatic hydrolysis of polyprenyl pyrophosphats, Methods Enzymol. 110 (1985), 153-155) und mittels Dünnschichtchromatographie und Messung der inkorporierten Radioaktivität analysiert (Dogbo, Bardat, Quennemet und Camara: Metabolism of plastid terpenoids: In vitrp inhibition of phytoene synthesis by phenethyl pyrophosphate derivates, FEBS Letters 219 (1987) 211-215).

Unter Farnesyl-Diphosphat-Synthase -Aktivität wird die Enzymaktivität einer Farnesyl-Diphosphat-Synthase verstanden.

Unter einer Farnesyl-Diphosphat-Synthase wird ein Protein verstanden, das die enzymatische Aktivität aufweist, Geranyl-Diphosphate und Isopentenyl-Diphosphat in Farnesyl-Diphosphat umzuwandeln.

Dementsprechend wird unter Farnesyl-Diphosphat-Synthase-Aktivität die in einer bestimmten Zeit durch das Protein Farnesyl-Diphosphat-Synthase umgesetzte Menge Geranyl-Diphosphate und/oder lsopentenyl-Diphosphat bzw. gebildete Menge Farnesyl-Diphosphat verstanden.

Bei einer erhöhten Farnesyl-Diphosphat-Synthase -Aktivität gegenüber dem Wildtyp wird somit im Vergleich zum Wildtyp in einer bestimmten Zeit durch das Protein Farnesyl-Diphosphat-Synthase die umgesetzte Menge Geranyl-Diphosphate und/oder Isopentenyl-Diphosphat bzw. die gebildete Menge Farnesyl-Diphosphat erhöht.

Vorzugsweise beträgt diese Erhöhung der Farnesyl-Diphosphat-Synthase -Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Farnesyl-Diphosphat-Synthase-Aktivität des Wildtyps.

Die Bestimmung der Farnesyl-Diphosphat-Synthase-Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7.4), 10 mM MgCl2, 10 mM KCI, 1 mM EDTA, 1 mM EGTA, 0,1 % (v/v) Triton X-100, 2 mM ε-Aminocapronsäure, 10 % Glyzerin, 5 mM KHC03. Kurz vor der Extraktion wird 2 mM DTT und 0,5 mM PMSF zugegeben.

Die Aktivität der Franesylpyrophosphat-Snthase (FPP-Synthase) kann nach einer Vorschrift von Joly und Edwards (Journal of Biological Chemistry 268 (1993), 26983-26989) bestimmt werden. Danach wird die Enzymaktivität in einem Puffer aus 10 mM HEPES (pH 7,2), 1 mM MgCl₂, 1 mM Dithiothreitol, 20 ∝M Geranylpyrophosphat und 40 ∝M (1-¹⁴C) Isopentenylpyrophosphat (4 Ci/mmol) gemessen. Die Reaktionsmischung wird bei 37°C inkubiert; die Reaktion wird durch Zugabe von 2,5 N HCl (in 70 % Ethanol mit 19 ∝g/ml Farnesol) gestoppt. Die Reaktionsproduckte werden somit durch Säurehydrolyse bei 37C innerhalb von 30 Minuten hydrolysiert. Durch Zugabe von 10% NaOH wird die Mischung neutralisiert und mit Hexan ausgeschüttelt. Ein Aliquot der Hexanphase kann zur Bestimmung der eingebauten Radioaktivität mittels Szintillationszähler gemessen werden.

Alternativ können nach Inkubation von Pflanzenextrakt und radioaktiv markierten IPP die Reaktionsprodukte mittels Dünnschichtchromatographie (Silica-Gel SE60, Merck) in Benzol/Methanol (9:1) getrennt werden. Radioaktiv markierte Produkte werden eluiert und die Radioaktivität bestimmt (nach Gaffe, Bru, Causse, Vidal, Stamitti-Bert, Carde und Gallusci: LEFPS1, a tomato farnesyl pyrophosphate gene highly expressed during early fruit development; Plant Physiology 123 (2000) 1351-1362).

Unter Geranyl-Geranyl-Diphosphat-Synthase -Aktivität wird die Enzymaktivität einer Geranyl-Geranyl-Diphosphat-Synthase verstanden.

Unter einer Geranyl-Geranyl-Diphosphat-Synthase wird ein Protein verstanden, das die enzymatische Aktivität aufweist, Farnesyl-Diphosphat und Isopentenyl-Diphosphat in Geranyl-Geranyl-Diphosphat umzuwandeln.

Dementsprechend wird unter Geranyl-Geranyl-Diphosphat-Synthase-Aktivität die in einer bestimmten Zeit durch das Protein Geranyl-Geranyl-Diphosphat-Synthase umgesetzte Menge Farnesyl-Diphosphat und/oder Isopentenyl-Diphosphat bzw. gebildete Menge Geranyl-Geranyl-Diphosphat verstanden.

Bei einer erhöhten Geranyl-Geranyl-Diphosphat-Synthase -Aktivität gegenüber dem Wildtyp wird somit im Vergleich zum Wildtyp in einer bestimmten Zeit durch das Protein Geranyl-Geranyl-Diphosphat-Synthase die umgesetzte Menge Farnesyl-Diphosphat und/oder lsopentenyl-Diphosphat bzw. die gebildete Menge Geranyl-Geranyl-Diphosphat erhöht.

Vorzugsweise beträgt diese Erhöhung der Geranyl-Geranyl-Diphosphat-Synthase - Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der β-Cyclase-Aktivität des Wildtyps.

Die Bestimmung der Geranyl-Geranyl-Diphosphat-Synthase -Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7,4), 10 mM MgCl2, 10 mM KCI, 1 mM EDTA, 1 mM EGTA, 0,1 % (v/v) Triton X-100, 2 mM ε-Aminocapronsäure, 10 % Glyzerin, 5 mM KHCO3. Kurz vor der Extraktion wird 2 mM DTT und 0,5 mM PMSF zugegeben.

Aktivitätsmessungen der Geranylgeranypyrophosphat-Synthase (GGPP-Synthase) können nach der von Dogbo und Camara beschriebenen Methode (in Biochim. Biophys. Acta 920 (1987), 140-148: Purification of isopentenyl pyrophosphate isomerase and geranylgeranyl pyrophosphate synthase from Capsicum chromoplasts by affinity chromatography) bestimmt werden. Dazu wird einem Puffer (50 mM Tris-HCl (pH 7,6), 2 mM MgCl₂, 1 mM MnCl₂, 2 mM Dithiothreitol, (1-¹⁴C)IPP (0,1 ∝Ci, 10 ∝M), 15 ∝M DMAPP, GPP oder FPP) mit einem Gesamtvolumen von etwa 200 ∝I Pflanzenextrakt zugesetzt. Die Inkubation kann für 1-2 Stunden (oder länger) bei 30C erfolgen. Die Reaktion wird durch Zugabe von 0,5 ml Ethanol und 0,1 ml 6N HCl. Nach 10minütiger Inkubation bei 37°C wird die Reaktionsmischung mit 6N NaOH neutralisiert, mit 1 ml Wasser vermischt und mit 4 ml Diethylether ausgeschüttelt. In einem Aliquot (z.B. 0,2 mL) der Etherphase wird mittels Szintillationszählung die Menge an Radioaktivität bestimmt. Alternativ können nach Säurehydrolyse die radioaktiv markierten Prenylalkohole in Ether ausgeschüttelt werden und mit HPLC (25 cm-Säule Spherisorb ODS-1, 5∝m; Elution mit Methanol/Wasser (90:10; v/v) bei einer Flussrate von 1 ml/min) getrennt und mittels Radioaktivitätsmonitor quantifiziert werden (nach Wiedemann, Misawa und Sandmann: Purification and enzymatic characterization of the geranylgeranyl pyrophosphate synthase from Erwinia uredovora after expression in Escherichia coli;

Unter Phytoen-Synthase -Aktivität wird die Enzymaktivität einer Phytoen-Synthase verstanden.

Unter einer Phytoen-Synthase wird ein Protein verstanden, das die enzymatische Aktivität aufweist, einen endständigen, linearen Rest von Lycopin in einen β-Ionon-Ring zu überführen.

Insbesondere wird unter einer Phytoen-Synthase ein Protein verstanden, das die enzymatische Aktivität aufweist, Geranyl-Geranyl-Diphosphat in Phytoen umzuwandeln.

Dementsprechend wird unter Phytoen-Synthase -Aktivität die in einer bestimmten Zeit durch das Protein Phytoen-Synthase umgesetzte Menge Geranyl-Geranyl-Diphosphat bzw. gebildete Menge Phytoen verstanden.

Bei einer erhöhten Phytoen-Synthase -Aktivität gegenüber dem Wildtyp wird somit im Vergleich zum Wildtyp in einer bestimmten Zeit durch das Protein Phytoen-Synthase die umgesetzte Menge Geranyl-Geranyl-Diphosphat bzw. die gebildete Menge Phytoen erhöht.

Vorzugsweise beträgt diese Erhöhung der Phytoen-Synthase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Phytoen-Synthase-Aktivität des Wildtyps.

Die Bestimmung der Phytoen-Synthase-Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7,4), 10 mM MgCl2, 10 mM KCI, 1 mM EDTA, 1 mM EGTA, 0,1 % (v/v) Triton X-100, 2 mM ε-Aminocapronsäure, 10 % Glyzerin, 5 mM KHCO3. Kurz vor der Extraktion wird 2 mM DTT und 0,5 mM PMSF zugegeben.

Aktivitästbestimmungen der Phytoenesynthase (PSY) können nach der von Fraser und Kollegen vorgestellten Methode (Fraser, Romer, Shipton, Mills, Kiano, Misawa, Drake, Schuch und Bramley: Evaluation of transgenic tomato plants expressing an additional phytoene synthase in a fruit-specific manner; Proc. Natl. Acad. Sci. USA 99 (2002), 1092-1097, basierend auf Fraser, Pinto, Holloway und Bramley, Plant Journal 24 (2000) 551-558) durchgeführt werden. Für Enzymmessungen werden Inkubationen mit (³H)Geranylgeranyl-pyrophosphat (15 mCi/mM, American Radiolabeled Chemicals, St. Louis) als Substrat in 0.4 M Tris-HCl (pH 8,0) mit 1 mM DTT, 4 mM MgCl₂, 6 mM Mn Cl₂, 3 mM ATP, 0,1 % Tween 60, 1mM Kaliunfluorid durchgeführt. Pflanzenextrakte werden mit Puffer gemischt, z B. 295 ∝I Puffer mit Extrakt in einem Gesamtvolumen von 500 ∝I. Inkubiert wird für wenigstens 5 Stunden bei 28C. Anschließend wird Phytoene durch zweimaliges Ausschütteln (jeweils 500 ∝I) mit Chloroform extrahiert. Das während der Reaktion gebildete radioaktiv markierte Phytoene wird mittels Dünnschichtchromatographie auf Silicaplatten in Methanol/Wasser (95:5; v/v) getrennt. Phytoene kann in einer Jod-angereicherten Atmosphäre (durch Erhitzen weniger lodkristalle) auf den Silicaplatten identifiziert werden. Ein Phytoene-Standard dient als Referenz. Die Menge an radioaktiv markiertem Produckt wird mittels Messung im Szintillationszähler bestimmt. Alternativ kann Phytoene auch mittels HPLC, die mit einem Radioaktivitätsdetektor versehen ist, quantifiziert werden (Fraser, Albrecht und Sandmann: Development of high performance liquid chromatographic systems for the separation of radiolabeled carotenes and precursors formed in specific enzymatic reactions; J. Chromatogr. 645 (1993) 265-272).

Unter Phytoen-Desaturase-Aktivität wird die Enzymaktivität einer Phytoen-Desaturase verstanden.

Unter einer Phytoen-Desaturase wird ein Protein verstanden, das die enzymatische Aktivität aufweist, Phytoen in Phytofluen und/oder Phytofluen in ζ-Carotin (Zetacarotin) umzuwandeln.

Dementsprechend wird unter Phytoen-Desaturase-Aktivität die in einer bestimmten Zeit durch das Protein Phytoen-Desaturase umgesetzte Menge Phytoen bzw. Phytofluen bzw. gebildete Menge Phytofluen bzw. ζ-Carotin verstanden.

Bei einer erhöhten Phytoen-Desaturase-Aktivität gegenüber dem Wildtyp wird somit im Vergleich zum Wildtyp in einer bestimmten Zeit durch das Protein Phytoen-Desaturase die umgesetzte Menge Phytoen bzw. Phytofluen bzw. die gebildete Menge Phytofluen bzw. ζ-Carotin erhöht.

Vorzugsweise beträgt diese Erhöhung der Phytoen-Desaturase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Phytoen-Desaturase-Aktivität des Wildtyps.

Die Bestimmung der Phytoen-Desaturase-Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7,4), 10 mM MgCl2, 10 mM KCI, 1 mM EDTA, 1 mM EGTA, 0,1 % (v/v) Triton X-100, 2 mM ε-Aminocapronsäure, 10 % Glyzerin, 5 mM KHC03. Kurz vor der Extraktion wird 2 mM DTT und 0,5 mM PMSF zugegeben.

Die Aktivität der Phytoendesaturase (PDS) kann durch die Inkorporation von radioaktiv markiertem (¹⁴C)-Phytoen in ungesättigte Carotine gemessen werden (nach Römer, Fraser, Kiano, Shipton, Misawa, Schuch und Bramley: Elevation of the provitamin A content of transgenic tomato plants; Nature Biotechnology 18 (2000) 666-669). Radioaktiv markiertes Phytoene kann synthetisiert werden nach Fraser (Fraser, De la Rivas, Mackenzie, Bramley: Phycomyces blakesieanus CarB mutants: their use in assays of phytoene desaturase; Phytochemistry 30 (1991), 3971-3976). Membranen von Plastiden des Zielgewebes können mit 100 mM MES-Puffer (pH 6,0) mit 10 mM MgCl₂ und 1 mM Dithiothreitol in einem Gesamtvolumen von 1 mL inkubiert werden. In Aceton gelöstes (¹⁴C)-Phytoen (etwa 100.000 Zerfälle/Minute für jeweils eine Inkubation) wird zugegeben, wobei die Acetonkonzentration 5 % (v/v) nicht übersteigen sollte. Diese Mischung wird bei 28C für etwa 6 bis 7 Stunden im Dunklen unter Schütteln inkubiert. Danach werden Pigmente dreimal mit etwa 5 mL Petrolether (mit 10 % Diethylether versetzt) extrahiert und mittels HPLC getrennt und quantifiziert.

Alternativ kann die Aktivität der Phytoenedesaturase nach Fraser et al. (Fraser, Misawa, Linden, Yamano, Kobayashi und Sandmann: Expression in Escherichia coli, purification, and reactivation of the recombinant Erwinia uredovora phytoene desaturase, Journal of Biological Chemistry 267 (1992), 19891-9895) gemessen werden.

Unter Zeta-Carotin-Desaturase-Aktivität wird die Enzymaktivität einer Zeta-Carotin-Desaturase verstanden.

Unter einer Zeta-Carotin-Desaturase wird ein Protein verstanden, das die enzymatische Aktivität aufweist, ζ-Carotin in Neurosporin und/oder Neurosporin in Lycopin umzuwandeln.

Dementsprechend wird unter Zeta-Carotin-Desaturase-Aktivität die in einer bestimmten Zeit durch das Protein Zeta-Carotin-Desaturase umgesetzte Menge ζ-Carotin oder Neurosporin bzw. gebildete Menge Neurosporin oder Lycopin verstanden.

Bei einer erhöhten Zeta-Carotin-Desaturase-Aktivität gegenüber dem Wildtyp wird somit im Vergleich zum Wildtyp in einer bestimmten Zeit durch das Protein Zeta-Carotin-Desaturase die umgesetzte Menge ζ-Carotin oder Neurosporin bzw. die gebildete Menge Neurosporin oder Lycopin erhöht.

Vorzugsweise beträgt diese Erhöhung der Zeta-Carotin-Desaturase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Zeta-Carotin-Desaturase - Aktivität des Wildtyps.

Die Bestimmung der Zeta-Carotin-Desaturase-Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7,4), 10 mM MgCl2, 10 mM KCl, 1 mM EDTA, 1 mM EGTA, 0,1 % (v/v) Triton X-100, 2 mM ε-Aminocapronsäure, 10 % Glyzerin, 5 mM KHCO3. Kurz vor der Extraktion wird 2 mM DTT und 0,5 mM PMSF zugegeben.

Analysen zur Bestimmung der ζ-Carotin-Desaturase (ZDS-Desaturase) können in 0.2 M Kaliumphosphat (pH 7.8, Puffervolumen von etwa 1 ml) durchgeführt werden. Die Anlysemethode dazu wurde von Breitenbach und Kollegen (Breitenbach, Kuntz, Takaichi und Sandmann: Catalytic properties of an expressed and purified higher plant type ξ-carotene desaturase from Capsicum annuum; European Journal of Biochemistry. 265(1):376-383, 1999 Oct ) publiziert. Jeder Analyseansatz enthält 3 mg Phosphytidylcholin, das in 0,4 M Kaliumphosphatpuffer (pH 7,8) suspendiert ist, 5 ∝g ξ-Carotin oder Neurosporene, 0,02 % Butylhydroxytoluol, 10 ∝I Decyl-Plastochinon (1 mM methanolische Stammlösung) und Pflanzenextrakt. Das Volumen des Pflanzenextraktes muß der Menge an vorhandener ZDS-Desaturase-Aktivität angepasst werden, um Quantifizierungen in einem linearen Messbereich zu ermöglichen. Inkubationen erfolgen typischerweise für etwa 17 Stunden bei kräftigem Schütteln (200 Umdrehungen/Minute) bei etwa 28°C im Dunklen. Carotinoide werden durch Zugabe von 4 ml Aceton bei 50°C für 10 Minuten unter Schütteln extrahiert. Aus dieser Mischung werden die Carotinoide in eine Petroletherpahse (mit 10 % Diethylether) überführt. Die Dethylether/Petroletherphase wird unter Stickstoff evaporiert, die Carotinoide wieder in 20 ∝I gelöst und mittels HPLC getrennt und quantifiziert.

Unter crtISO -Aktivität wird die Enzymaktivität eines crtISO-Proteins verstanden.

Unter einem crtISO-Proteins wird ein Protein verstanden, das die enzymatische Aktivität aufweist, 7,9,7',9'-tetra-cis-Lycopin in all-trans-Lycopin umzuwandeln.

Dementsprechend wird unter crtISO-Aktivität die in einer bestimmten Zeit durch das Protein b-Cyclase umgesetzte Menge 7,9,7',9'-tetra-cis-Lycopin bzw. gebildete Menge all-trans-Lycopin verstanden.

Bei einer erhöhten crtISO-Aktivität gegenüber dem Wildtyp wird somit im Vergleich zum Wildtyp in einer bestimmten Zeit durch das crtISO-Proteins die umgesetzte Menge 7,9,7',9'-tetra-cis-Lycopin bzw. die gebildete Menge all-trans-Lycopin erhöht.

Vorzugsweise beträgt diese Erhöhung der crtISO-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der crtISO-Aktivität des Wildtyps.

Die Bestimmung der crtISO-Aktivität in erfindungsgemäßen genetisch veränderten Pflanzen und in Wildtyp- bzw. Referenzpflanzen erfolgt vorzugsweise unter folgenden Bedingungen:

Eingefrorenes Pflanzenmaterial wird durch intensives Mösern in flüssigem Stickstoff homogenisiert und mit Extraktionspuffer in einem Verhältnis von 1:1 bis 1:20 extrahiert. Das jeweilige Verhältnis richtet sich nach den Enzymaktivitäten in dem verfügbaren Pflanzenmaterial, sodaß eine Bestimmung und Quantifizierung der Enzymaktivitäten innerhalb des linearen Messbereiches möglicht ist. Typischerweise kann der Extraktionspuffer bestehen aus 50 mM HEPES-KOH (pH 7,4), 10 mM MgCl2, 10 mM KCI, 1 mM EDTA, 1 mM EGTA, 0,1 % (v/v) Triton X-100, 2 mM ε-Aminocapronsäure, 10 % Glyzerin, 5 mM KHC03. Kurz vor der Extraktion wird 2 mM DTT und 0,5 mM PMSF zugegeben.

Unter FtsZ-Aktivität wird die physiologische Aktivität eines FtsZ-Proteins verstanden. Unter einem FtsZ-Protein wird ein Protein verstanden, das an der Zellteilungs und Plastidenteilungs fördernde Wirkung hat und Homologien zu Tubulinproteinen aufweist.

Unter MinD -Aktivität wird die physiologische Aktivität eines MinD -Proteins verstanden.

Unter einem MinD -Protein wird ein Protein verstanden, das eine multifunktionele Rolle bei der Zellteilung aufweist. Es ist eine Membran-assoziierte ATPase und kann innerhalb der Zelle eine oszillierende Bewegung von Pol zu Pol zeigen.

Weiterhin kann die Erhöhung der Aktivität von Enzymen des Nicht-Mevalonatweges zu einer weiteren Erhöhung des gewünschten Ketocarotenoid-Endproduktes führen. Beipiele hierfür sind die 4-Diphosphocytidyl-2-C-Methyl-D-Erythritol-Synthase, die 4-Diphosphocytidyl-2-C-Methyl-D-Erythritol-Kinase und die 2-C-Methyl-D-Erythritol-2,4-cyclodiphoshat-Synthase. Durch Änderungen der Genexpression der entsprechenden Gene kann die Aktivität der genannten Enzyme erhöht werden. Die veränderten Konzentrationen der relavanten Proteine können standardgemäß mittels Antikörpern und entsprechenden Blotting-techniken nachgewiesen werden. Die Erhöhung der HMG-CoA-Reduktase-Aktivität und/oder (E)-4-Hydroxy-3-Methylbut-2-enyl-Diphosphat-Reduktase-Aktivität und/oder 1-Deoxy-D-Xylose-5-Phosphat-Synthase-Aktivität und/oder 1-Deoxy-D-Xylose-5-Phosphat-Reduktoisomerase-Aktivität und/oder Isopentenyl-Diphosphat-Δ-Isomerase-Aktivität und/oder Geranyl-Diphosphat-Synthase-Aktivität und/oder Farnesyl-Diphosphat-Synthase-Aktivität und/oder Geranyl-geranyl-Diphosphat-Synthase-Aktivität und/oder Phytoen-Synthase-Aktivität und/oder Phytoen-Desaturase-Aktivität und/oder Zeta-Carotin-Desaturase-Aktivität und/oder crtISO-Aktivität und/oder FtsZ-Aktivität und/oder MinD-Aktivität kann durch verschiedene Wege erfolgen, beispielsweise durch Ausschalten von hemmenden Regulationsmechanismen auf Expressions- und Proteinebene oder durch Erhöhung der Genexpression von Nukleinsäuren kodierend eine HMG-CoA-Reduktase und/oder Nukleinsäuren kodierend eine (E)-4-Hydroxy-3-Methylbut-2-enyl-Diphosphat-Reduktase und/oder Nukleinsäuren kodierend eine 1-Deoxy-D-Xylose-5-Phosphat-Synthase und/oder Nukleinsäuren kodierend eine 1-Deoxy-D-Xylose-5-Phosphat-Reduktoisomerase und/oder Nukleinsäuren kodierend eine Isopentenyl-Diphosphat-o-Isomerase und/oder Nukleinsäuren kodierend eine Geranyl-Diphosphat-Synthase und/oder Nukleinsäuren kodierend eine Farnesyl-Diphosphat-Synthase und/oder Nukleinsäuren kodierend eine Geranyl-geranyl-Diphosphat-Synthase und/oder Nukleinsäuren kodierend eine Phytoen-Synthase und/oder Nukleinsäuren kodierend eine Phytoen-Desaturase und/oder Nukleinsäuren kodierend eine Zeta-Carotin-Desaturase und/oder Nukleinsäuren kodierend ein crtISO-Protein und/oder Nukleinsäuren kodierend ein FtsZ-Protein und/oder Nukleinsäuren kodierend ein MinD-Protein gegenüber dem Wildtyp.

Die Erhöhung der Genexpression der Nukleinsäuren kodierend eine HMG-CoA-Reduktase und/oder Nukleinsäuren kodierend eine (E)-4-Hydroxy-3-Methylbut-2-enyl-Diphosphat-Reduktase und/oder Nukleinsäuren kodierend eine 1-Deoxy-D-Xylose-5-Phosphat-Synthase und/oder Nukleinsäuren kodierend eine 1-Deoxy-D-Xylose-5-Phosphat-Reduktoisomerase und/oder Nukleinsäuren kodierend eine Isopentenyl-Diphosphat-Δ-Isomerase und/oder Nukleinsäuren kodierend eine Geranyl-Diphosphat-Synthase und/oder Nukleinsäuren kodierend eine Farnesyl-Diphosphat-Synthase und/oder Nukleinsäuren kodierend eine Geranyl-geranyl-Diphosphat-Synthase und/oder Nukleinsäuren kodierend eine Phytoen-Synthase und/oder Nukleinsäuren kodierend eine Phytoen-Desaturase und/oder Nukleinsäuren kodierend eine Zeta-Carotin-Desaturase und/oder Nukleinsäuren kodierend ein crtiSO-Protein und/oder Nukleinsäuren kodierend ein FtsZ-Protein und/oder Nukleinsäuren kodierend ein MinD-Protein gegenüber dem Wildtyp kann ebenfalls durch verschiedene Wege erfolgen, beispielsweise durch Induzierung des HMG-CoA-Reduktase-Gens und/oder (E)-4-Hydroxy-3-Methylbut-2-enyl-Diphosphat-Reduktase-Gens und/oder 1-Deoxy-D-Xylose-5-Phosphat-Synthase-Gens und/oder 1-Deoxy-D-Xylose-5-Phosphat-Reduktoisomerase-Gens und/oder Isopentenyl-Diphosphat-Δ-Isomerase-Gens und/oder Geranyl-Diphosphat-Synthase-Gens und/oder Farnesyl-Diphosphat-Synthase-Gens und/oder Geranyl-geranyl-Diphosphat-Synthase-Gens und/oder Phytoen-Synthase-Gens und/oder Phytoen-Desaturase-Gens und/oder Zeta-Carotin-Desaturase-Gens und/oder crtISO-Gens und/oder FtsZ-Gens und/oder MinD-Gens durch Aktivatoren oder durch Einbringen von einer oder mehrerer Kopien des HMG-CoA-Reduktase-Gens und/oder (E)-4-Hydroxy-3-Methylbut-2-enyl-Diphosphat-Reduktase-Gens und/oder 1-Deoxy-D-Xylose-5-Phosphat-Synthase-Gens und/oder 1-Deoxy-D-Xylose-5-Phosphat-Reduktoisomerase-Gens und/oder Isopentenyl-Diphosphat-Δ-Isomerase-Gens und/oder Geranyl-Diphosphat-Synthase-Gens und/oder Farnesyl-Diphosphat-Synthase-Gens und/oder Geranyl-geranyl-Diphosphat-Synthase-Gens und/oder Phytoen-Synthase-Gens und/oder Phytoen-Desaturase-Gens und/oder Zeta-Carotin-Desaturase-Gens und/oder crtISO-Gens und/oder FtsZ-Gens und/oder MinD-Gens, also durch Einbringen mindestens einer Nukleinsäure kodierend eine HMG-CoA-Reduktase und/oder mindestens einer Nukleinsäure kodierend eine (E)-4-Hydroxy-3-Methylbut-2-enyl-Diphosphat-Reduktase und/oder mindestens einer Nukleinsäure kodierend eine 1-Deoxy-D-Xylose-5-Phosphat-Synthase und/oder mindestens einer Nukleinsäure kodierend eine 1-Deoxy-D-Xylose-5-Phosphat-Reduktoisomerase und/oder mindestens einer Nukleinsäure kodierend eine Isopentenyl-Diphosphat-Δ-Isomerase und/oder mindestens einer Nukleinsäure kodierend eine Geranyl-Diphosphat-Synthase und/oder mindestens einer Nukleinsäure kodierend eine Farnesyl-Diphosphat-Synthase und/oder mindestens einer Nukleinsäure kodierend eine Geranyl-geranyl-Diphosphat-Synthase und/oder mindestens einer Nukleinsäure kodierend eine Phytoen-Synthase und/oder mindestens einer Nukleinsäure kodierend eine Phytoen-Desaturase und/oder mindestens einer Nukleinsäure kodierend eine Zeta-Carotin-Desaturase und/oder mindestens einer Nukleinsäure kodierend ein crtISO-Protein und/oder mindestens einer Nukleinsäure kodierend ein FtsZ-Protein und/oder mindestens einer Nukleinsäure kodierend ein MinD-Protein in die Pflanze.

Unter Erhöhung der Genexpression einer Nukleinsäure codierend eine HMG-CoA-Reduktase und/oder (E)-4-Hydroxy-3-Methylbut-2-enyl-Diphosphat-Reduktase und/oder 1-Deoxy-D-Xylose-5-Phosphat-Synthase und/oder 1-Deoxy-D-Xylose-5-Phosphat-Reduktoisomerase und/oder Isopentenyl-Diphosphat-A-Isomerase und/oder Geranyl-Diphosphat-Synthase und/oder Farnesyl-Diphosphat-Synthase und/oder Geranyl-geranyl-Diphosphat-Synthase und/oder Phytoen-Synthase und/oder Phytoen-Desaturase und/oder Zeta-Carotin-Desaturase und/oder ein crtISO-Protein und/oder FtsZ-Protein und/oder MinD-Protein wird erfindungsgemäß auch die Manipulation der Expression der Pflanzen eigenen, endogenen HMG-CoA-Reduktase und/oder (E)-4-Hydroxy-3-Methylbut-2-enyl-Diphosphat-Reduktase und/oder 1-Deoxy-D-Xylose-5-Phosphat-Synthase und/oder 1-Deoxy-D-Xylose-5-Phosphat-Reduktoisomerase und/oder Isopentenyl-Diphosphat-Δ-Isomerase und/oder Geranyl-Diphosphat-Synthase und/oder Farnesyl-Diphosphat-Synthase und/oder Geranyl-geranyl-Diphosphat-Synthase und/oder Phytoen-Synthase und/oder Phytoen-Desaturase und/oder Zeta-Carotin-Desaturase und/oder des Pflanzen eigenen crtISO-Proteins und/oder FtsZ-Proteins und/oder MinD-Proteins verstanden.

Dies kann beispielsweise durch Veränderung der entsprechenden Promotor DNA-Sequenz erreicht werden. Eine solche Veränderung, die eine erhöhte Expressionsrate des Gens zur Folge hat, kann beispielsweise durch Deletion oder Insertion von DNA Sequenzen erfolgen.

In einer bevorzugten Ausführungsform erfolgt die Erhöhung der Genexpression einer Nukleinsäure kodierend eine HMG-CoA-Reduktase und/oder die Erhöhung der Genexpression einer Nukleinsäure kodierend eine (E)-4-Hydroxy-3-Methylbut-2-enyl-Diphosphat-Reduktase und/oder die Erhöhung der Genexpression einer Nukleinsäure kodierend eine 1-Deoxy-D-Xylose-5-Phosphat-Synthase und/oder die Erhöhung der Genexpression einer Nukleinsäure kodierend eine 1-Deoxy-D-Xylose-5-Phosphat-Reduktoisomerase und/oder die Erhöhung der Genexpression einer Nukleinsäure kodierend eine Isopentenyl-Diphosphat-Δ-Isomerase und/oder die Erhöhung der Genexpression einer Nukleinsäure kodierend eine Geranyl-Diphosphat-Synthase und/oder die Erhöhung der Genexpression einer Nukleinsäure kodierend eine Farnesyl-Diphosphat-Synthase und/oder die Erhöhung der Genexpression einer Nukleinsäure kodierend eine Geranyl-geranyl-Diphosphat-Synthase und/oder die Erhöhung der Genexpression einer Nukleinsäure kodierend eine Phytoen-Synthase und/oder die Erhöhung der Genexpression einer Nukleinsäure kodierend eine Phytoen-Desaturase und/oder die Erhöhung der Genexpression einer Nukleinsäure kodierend eine Zeta-Carotin-Desaturase und/oder die Erhöhung der Genexpression einer Nukleinsäure kodierend ein crtISO-Protein und/oder die Erhöhung der Genexpression einer Nukleinsäure kodierend ein FtsZ-Protein und/oder die Erhöhung der Genexpression einer Nukleinsäure kodierend ein MinD-Protein durch Einbringen von mindestens einer Nukleinsäure kodierend eine HMG-CoA-Reduktase und/oder durch Einbringen von mindestens einer Nukleinsäure kodierend eine (E)-4-Hydroxy-3-Methylbut-2-enyl-Diphosphat-Reduktase und/oder durch Einbringen von mindestens einer Nukleinsäure kodierend eine 1-Deoxy-D-Xylose-5-Phosphat-Synthase und/oder durch Einbringen von mindestens einer Nukleinsäure kodierend eine 1-Deoxy-D-Xylose-5-Phosphat-Reduktoisomerase und/oder durch Einbringen von mindestens einer Nukleinsäure kodierend eine Isopentenyl-Diphosphat-Δ-Isomerase und/oder durch Einbringen von mindestens einer Nukleinsäure kodierend eine Geranyl-Diphosphat-Synthase und/oder durch Einbringen von mindestens einer Nukleinsäure kodierend eine Farnesyl-Diphosphat-Synthase und/oder durch Einbringen von mindestens einer Nukleinsäure kodierend eine Geranyl-geranyl-Diphosphat-Synthase und/oder durch Einbringen von mindestens einer Nukleinsäure kodierend eine Phytoen-Synthase und/oder durch Einbringen von mindestens einer Nukleinsäure kodierend eine Phytoen-Desaturase und/oder durch Einbringen von mindestens einer Nukleinsäure kodierend eine Zeta-Carotin-Desaturase und/oder durch Einbringen von mindestens einer Nukleinsäure kodierend ein crtiSO-Protein und/oder durch Einbringen von mindestens einer Nukleinsäure kodierend ein FtsZ-Protein und/oder durch Einbringen von mindestens einer Nukleinsäure kodierend ein MinD-Protein in die Pflanze.

Dazu kann prinzipiell jedes HMG-CoA-Reduktase-Gen bzw. (E)-4-Hydroxy-3-Methylbut-2-enyl-Diphosphat-Reduktase-Gen bzw. 1-Deoxy-D-Xylose-5-Phosphat-Synthase-Gen bzw. 1-Deoxy-D-Xylose-5-Phosphat-Reduktoisomerase-Gen bzw. Isopentenyl-Diphosphat-Δ-Isomerase-Gen bzw. Geranyl-Diphosphat-Synthase-Gen bzw. Farnesyl-Diphosphat-Synthase-Gen bzw. Geranyl-geranyl-Diphosphat-Synthase-Gen bzw. Phytoen-Synthase-Gen bzw. Phytoen-Desaturase-Gen bzw. Zeta-Carotin-Desaturase-Gen bzw. crtiSO-Gen bzw. FtsZ-Gen bzw. MinD-Gen verwendet werden.

Bei genomischen HMG-CoA-Reduktase-Sequenzen bzw. (E)-4-Hydroxy-3-Methylbut-2-enyl-Diphosphat-Reduktase-Sequenzen bzw. 1-Deoxy-D-Xylose-5-Phosphat-Synthase-Sequenzen bzw. 1-Deoxy-D-Xylose-5-Phosphat-Reduktoisomerase-Sequenzen bzw. Isopentenyl-Diphosphat-Δ-Isomerase-Sequenzen bzw. Geranyl-Diphosphat-Synthase-Sequenzen bzw. Farnesyl-Diphosphat-Synthase-Sequenzen bzw. Geranyl-geranyl-Diphosphat-Synthase-Sequenzen bzw. Phytoen-Synthase-Sequenzen bzw. Phytoen-Desaturase-Sequenzen bzw. Zeta-Carotin-Desaturase-Sequenzen bzw. crtISO-Sequenzen bzw. FtsZ-Sequenzen bzw. MinD-Sequenzen aus eukaryontischen Quellen, die Introns enthalten, sind für den Fall das die Wirtspflanze nicht in der Lage ist oder nicht in die Lage versetzt werden kann, die entsprechenden Proteine zu exprimieren, bevorzugt bereits prozessierte Nukleinsäuresequenzen, wie die entsprechenden cDNAs zu verwenden.

In den erfindungsgemäßen bevorzugten transgenen Pflanzen liegt also in dieser bevorzugten Ausführungsform gegenüber dem Wildtyp mindestens ein weiteres HMG-CoA-Reduktase-Gen und/oder (E)-4-Hydroxy-3-Methylbut-2-enyl-Diphosphat-Reduktase-Gen und/oder 1-Deoxy-D-Xylose-5-Phosphat-Synthase-Gen und/oder 1-Deoxy-D-Xylose-5-Phosphat-Reduktoisomerase-Gen und/oder Isopentenyl-Diphosphat-Δ-Isomerase-Gen und/oder Geranyl-Diphosphat-Synthase-Gen und/oder Farnesyl-Diphosphat-Synthase-Gen und/oder Geranyl-geranyl-Diphosphat-Synthase-Gen und/oder Phytoen-Synthase-Gen und/oder Phytoen-Desaturase-Gen und/oder Zeta-Carotin-Desaturase-Gen und/oder crtISO-Gen und/oder FtsZ-Gen und/oder MinD-Gen vor.

In dieser bevorzugten Ausführungsform weist die genetisch veränderte Pflanze beispielsweise mindestens eine exogene Nukleinsäure, kodierend eine HMG-CoA-Reduktase oder mindestens zwei endogene Nukleinsäuren, kodierend eine HMG-CoA-Reduktase und/oder mindestens eine exogene Nukleinsäure, kodierend eine (E)-4-Hydroxy-3-Methylbut-2-enyl-Diphosphat-Reduktase oder mindestens zwei endogene Nukleinsäuren, kodierend eine (E)-4-Hydroxy-3-Methylbut-2-enyl-Diphosphat-Reduktase und/oder mindestens eine exogene Nukleinsäure, kodierend eine 1-Deoxy-D-Xylose-5-Phosphat-Synthase oder mindestens zwei endogene Nukleinsäuren, kodierend eine 1-Deoxy-D-Xylose-5-Phosphat-Synthase und/oder mindestens eine exogene Nukleinsäure, kodierend eine 1-Deoxy-D-Xylose-5-Phosphat-Reduktoisomerase oder mindestens zwei endogene Nukleinsäuren, kodierend eine 1-Deoxy-D-Xylose-5-Phosphat-Reduktoisomerase und/oder mindestens eine exogene Nukleinsäure, kodierend eine Isopentenyl-Diphosphat-Δ-Isomerase oder mindestens zwei endogene Nukleinsäuren, kodierend eine Isopentenyl-Diphosphat-Δ-Isomerase und/oder mindestens eine exogene Nukleinsäure, kodierend eine Geranyl-Diphosphat-Synthase oder mindestens zwei endogene Nukleinsäuren, kodierend eine Geranyl-Diphosphat-Synthase und/oder mindestens eine exogene Nukleinsäure, kodierend eine Farnesyl-Diphosphat-Synthase oder mindestens zwei endogene Nukleinsäuren, kodierend eine Farnesyl-Diphosphat-Synthase und/oder mindestens eine exogene Nukleinsäure, kodierend eine Geranyl-geranyl-Diphosphat-Synthase oder mindestens zwei endogene Nukleinsäuren, kodierend eine Geranyl-geranyl-Diphosphat-Synthase und/oder mindestens eine exogene Nukleinsäure, kodierend eine Phytoen-Synthase oder mindestens zwei endogene Nukleinsäuren, kodierend eine Phytoen-Synthase und/oder mindestens eine exogene Nukleinsäure, kodierend eine Phytoen-Desaturase oder mindestens zwei endogene Nukleinsäuren, kodierend eine Phytoen-Desaturase und/oder mindestens eine exogene Nukleinsäure, kodierend eine Zeta-Carotin-Desaturase oder mindestens zwei endogene Nukleinsäuren, kodierend eine Zeta-Carotin-Desaturase und/oder mindestens eine exogene Nukleinsäure, kodierend ein crtISO-Protein oder mindestens zwei endogene Nukleinsäuren, kodierend ein crtISO-Protein und/oder mindestens eine exogene Nukleinsäure, kodierend ein FtsZ-Protein oder mindestens zwei endogene Nukleinsäuren, kodierend eine FtsZ-Protein und/oder mindestens eine exogene Nukleinsäure, kodierend ein MinD-Protein oder mindestens zwei endogene Nukleinsäuren, kodierend ein MinD-Protein auf.

Beispiele für HMG-CoA-Reduktase-Gene sind:

Eine Nukleinsäure, kodierend eine HMG-CoA-Reduktase aus Arabidopsis thaliana, Accession NM_106299; (Nukleinsäure: SEQ ID NO: 111, Protein: SEQ ID NO: 112),

sowie weitere HMG-CoA-Reduktase -Gene aus anderen Organismen mit den folgenden Accession Nummern:
P54961, P54870, P54868, P54869, O02734, P22791, P54873, P54871, P23228, P13704, P54872, Q01581, P17425, P54874, P54839, P14891, P34135, O64966, P29057, P48019, P48020, P12683, P43256, Q9XEL8, P34136, 064967, P29058, P48022, Q41437, P12684, Q00583, Q9XHL5, Q41438, Q9YAS4, O76819, O28538, Q9Y7D2, P54960, O51628, P48021, Q03163, P00347, P14773, Q12577, Q59468, P04035, O24594, P09610, Q58116, 026662, Q01237, Q01559, Q12649, O74164, 059469, P51639, Q10283, O08424, P20715, P13703, P13702, Q96UG4, Q8SQZ9, O15888, Q9TUM4, P93514, Q39628, P93081, P93080, Q944T9, Q40148, Q84MM0, Q84LS3, Q9Z9N4, Q9KLM0

Beispiele für (E)-4-Hydroxy-3-Methylbut-2-enyl-Diphosphat-Reduktase-Gene sind:

Eine Nukleinsäure, kodierend eine (E)-4-Hydroxy-3-Methylbut-2-enyl-Diphosphat-Reduktase aus Arabidopsis thaliana (lytB/ISPH), ACCESSION AY168881, (Nukleinsäure: SEQ ID NO: 113, Protein: SEQ ID NO: 114),

sowie weitere (E)-4-Hydroxy-3-Methylbut-2-enyl-Diphosphat-Reduktase -Gene aus anderen Organismen mit den folgenden Accession Nummern:
T04781, AF270978_1, NP_485028.1. NP_442089.1, NP_681832.1, ZP_00110421.1,
ZP_00071594.1. ZP_00114706.1. ISPH_SYNY3, ZP_00114087.1. ZP_00104269.1,
AF398145_1, AF398146_1, AAD55762.1, AF514843_1, NP_622970.1, NP_348471.1,
NP_562001.1, NP_223698.1, NP_781941.1, ZP_00080042.1, NP_859669.1.
NP_214191.1, ZP_00086191.1, ISPH_VIBCH, NP_230334.1. NP_742768.1,
NP_302306.1, ISPH_MYCLE, NP_602581.1, ZP_00026966.1, NP_520563.1,
NP_253247.1, NP_282047.1, ZP_30038210.1. ZP_00064913.1, CAA61555.1,
ZP_00125365.1, ISPH_ACICA, EAA24703.1, ZP_00013067.1, ZP_00029164.1,
NP_790656.1, NP_217899.1, NP_641592.1, NP_636532.1, NP_719076.1,
NP_660497.1, NP_422155.1, NP_715446.1, ZP_00090692.1, NP_759496.1.
ISPH_BURPS, ZP_00129657.1, NP_215626.1, NP_335584.1, ZP_00135016.1,
NP_789585.1. NP_787770.1, NP_769647.1. ZP_00043336.1. NP_242248.1,
ZP_00008555.1, NP_246603.1, ZP_00030951.1, NP_670994.1. NP_404120.1.
NP_540376.1. NP_733653.1. NP_697503.1. NP_840730.1. NP_274828.1.
NP_796916.1. ZP_00123390.1, NP_824386.1, NP_737689.1, ZP_00021222.1,
NP_757521.1, NP_390395.1, ZP_00133322.1, CAD76178.1, NP_600249.1.
NP_454660.1. NP_712601.1, NP_385018.1. NP_751989.1

Beispiele für 1-Deoxy-D-Xylose-5-Phosphat-Synthase -Gene sind:

Eine Nukleinsäure, kodierend eine 1-Deoxy-D-Xylose-5-Phosphat-Synthase aus Lycopersicon esculentum, ACCESSION #AF143812 (Nukleinsäure: SEQ ID NO:115, Protein: SEQ ID NO: 116),

sowie weitere 1-Deoxy-D-Xylose-5-Phosphat-Synthase -Gene aus anderen Organismen mit den folgenden Accession Nummern:
AF143812_1, DXS_CAPAN, CAD22530.1, AF182286_1, NP_193291.1, T52289,
AAC49368.1, AAP14353.1, D71420, DXS_ORYSA, Auf443590_1. BAB02345.1,
CAA09804.2, NP_850620.1. CAD22155.2, AAM65798.1, NP_566686.1, CAD22531.1,
AAC33513.1, CAC08458.1, AAG10432.1, T08140, AAP14354.1, AF428463_1,
ZP_00010537.1, NP_769291.1, AAK59424.1, NP_107784.1, NP_697464.1,
NP_540415.1. NP_196699.1, NP_384986.1, ZP_00096461.1, ZP_00013656.1,
NP_353769.1, BAA83576.1, ZP_00005919.1, ZP_00006273.1. NP_420871.1,
AAM48660.1, DXS_RHOCA, ZP_00045608.1, ZP_00031686.1. NP_841218.1.
ZP_00022174.1, ZP_00086851.1, NP_742690.1. NP_520342.1, ZP_00082120.1,
NP_790545.1, ZP_00125266.1. CAC17468.1, NP_252733.1, ZP_00092466.1,
NP_439591.1, NP_414954.1, NP_752465.1, NP_622918.1, NP_286162.1,
NP_836085.1, NP_706308.1, ZP_00081148.1. NP_797065.1. NP_213598.1,
NP_245469.1, ZP_00075029.1, NP_455016.1, NP_230536.1, NP_459417.1,
NP_274863.1, NP_283402.1, NP_759318.1. NP_406652.1, DXS_SYNLE,
DXS_SYNP7, NP_440409.1. ZP_00067331.1, ZP_00122853.1, NP_717142.1,
ZP_00104889.1, NP_243645.1. NP_681412.1, DXS_SYNEL, NP_637787.1,
DXS_CHLTE, ZP_00129863.1, NP_661241.1, DXS_XANCP, NP_470738.1,
NP_484643.1. ZP_00108360.1, NP_833890.1, NP_846629.1, NP_658213.1.
NP_642879.1, ZP_00039479.1, ZP_00060584.1, ZP_00041364.1, ZP_00117779.1,
NP_299528.1

Beispiele für 1-Deoxy-D-Xylose-5-Phosphat-Reduktoisomerase-Gene sind:

Eine Nukleinsäure, kodierend eine 1-Deoxy-D-Xylose-5-Phosphat-Reduktoisomerase aus Arabidopsis thaliana, ACCESSION #AF148852, (Nukleinsäure: SEQ ID NO: 137 , Protein: SEQ ID NO: 138),

sowie weitere 1-Deoxy-D-Xylose-5-Phosphat-Reduktoisomerase-Gene aus anderen Organismen mit den folgenden Accession Nummern:
AF148852, AY084775, AY054682, AY050802, AY045634, AY081453, AY091405,
AY098952, AJ242588, AB009053, AY202991, NP_201085.1, T52570, AF331705_1,
BAB16915.1, AF367205_1, AF250235_1, CAC03581.1, CAD22156.1, AF182287_1,
DXR_MENPI, ZP_00071219.1. NP_488391.1, ZP_00111307.1, DXR_SYNLE,
AAP56260.1, NP_681831.1, NP_442113.1, ZP_00115071.1, ZP_00105106.1,
ZP_00113484.1, NP_833540.1, NP_657789.1, NP_661031.1, DXR_BACHD,
NP_833080.1, NP_845693.1, NP_562610.1. NP_623020.1, NP_810915.1.
NP_243287.1, ZP_00118743.1, NP_464842.1, NP_470690.1, ZP_00082201.1,
NP_781898.1, ZP_00123667.1, NP_348420.1. NP_604221.1, ZP_00053349.1,
ZP_00064941.1, NP_246927.1, NP_389537.1, ZP_00102576.1, NP_519531.1,
AF124757_19, DXR_ZYMMO, NP_713472.1, EP_459225.1, NP_454827.1,
ZP_00045738.1. NP_743754.1, DXR_PSEPK, ZP_00130352.1, NP_702530.1,
NP_841744.1, NP_438967.1, AF514841 _1, NP_706118.1, ZP_00125845.1,
NP_404661.1, NP_285867.1, NP_240064.1, NP_414715.1, ZP_00094058.1,
NP_791365.1, ZP_00012448.1, ZP_00015132.1, ZP_00091545.1. NP_629822.1,
NP_771495.1, NP_798691.1, NP_231885.1, NP_252340.1. ZP_00022353.1,
NP_355549.1, NP_420724.1. ZP_00085169.1, EAA17616.1, NP_273242.1,
Nu_219574.1, NP_387094.1. NP_296721.1, ZP_00004209.1, NP_823739.1.
NP_282934.1, BAA77848.1, NP_660577.1. NP_760741.1, NP_641750.1.
NP_636741.1, NP_829309.1. NP_298338.1, NP_444964.1, NP_717246.1.
NP_224545.1. ZP_00038451.1, OXR_KITGR, NP_778563.1.

Beispiele für Isopentenyl-Diphosphat-Δ-Isomerase-Gene sind:

Eine Nukleinsäure, kodierend eine Isopentenyl-Diphosphat-Δ-Isomerase aus Adonis palaestina clone ApIP128, (ipiAa1), ACCESSION #AF188060, veröffentlicht durch Cunningham,F.X. Jr. and Gantt,E.: Identification of multi-gene families encoding isopentenyl diphosphate isomerase in plants by heterologous complementation in Escherichia coli, Plant Cell Physiol. 41 (1), 119-123 (2000) (Nukleinsäure: SEQ ID NO: 117, Protein: SEQ ID NO: 118),

sowie weitere lsopentenyl-Diphosphat-Δ-lsomerase-Gene aus anderen Organismen mit den folgenden Accession Nummern:
Q38929, 048964, Q39472, Q13907, 035586, P58044, 042641, 035760, Q10132, P15496, Q9YB30, Q8YNH4, Q42553, 027997, P50740, 051627,048965, Q8KFR5, Q39471, Q39664, Q9RVE2, Q01335, Q9HHE4, Q9BXS1, Q9KWF6, Q9CIF5, Q88WB6, Q92BX2, Q8Y7A5, Q8TT35 Q9KK75, Q8NN99, Q8XD58, Q8FE75, Q46822, Q9HP40, P72002, P26173, Q9Z5D3, Q8Z3X9, Q8ZM82, Q9X7Q6, 013504, Q9HFW8, QBNJL9, Q9UUQI , Q9NH02, Q9M6K9, Q9M6K5, Q9FXR6, 081691, Q9S7C4, Q8S3L8, Q9M592, Q9M6K3, Q9M6K7, Q9FV48, Q9LLB6, Q9AVJ1, Q9AVG8, Q9M6K6. Q9AVJ5. Q9M6K2, Q9AYS5, Q9M6K8, Q9AVG7, Q8S3L7, Q8W250, Q941E1, Q9AV18, Q9AYS6, Q9SAY0, Q9M6K4, Q8GVZ0, Q84RZ8, Q8KZ12, Q8KZ66, QBFND7, Q88QC9, 08BFZ6, BAC26382, CAD94476.

Beispiele für Geranyl-Diphosphat-Synthase -Gene sind:

Eine Nukleinsäure, kodierend eine Geranyl-Diphosphat-Synthase aus Arabidopsis thaliana, ACCESSION #Y17376, Bouvier,F., Suire,C., d'Harlingue,A., Backhaus,R.A. and Camara,B.; Molecular cloning of geranyl diphosphate synthase and compartmentation of monoterpene synthesis in plant cells, Plant J. 24 (2), 241-252 (2000) (Nukleinsäure: SEQ ID NO: 119, Protein: SEQ ID NO: 120),

sowie weitere Geranyl-Diphosphat-Synthase-Gene aus anderen Organismen mit den folgenden Accession Nummern:
Q9FT89, Q8LKJ2, 09FSW8, Q8LKJ3, Q9SBR3, 49SBR4, Q9FET8, Q8LKJ1, Q84LG 1, Q9JK86

Beispiele für Farnesyl-Diphosphat-Synthase-Gene sind:

Eine Nukleinsäure, kodierend eine Farnesyl-Diphosphat-Synthase aus Arabidopsis thaliana (FPS1), ACCESSION #U80605, veröffentlicht durch Cunillera,N., Arro,M., Delourme,D., Karst,F., Boronat,A. und Ferrer,A.: Arabidopsis thaliana contains two differentially expressed farnesyl-diphosphate synthase genes, J. Biol. Chem. 271 (13), 7774-7780 (1996), (Nukleinsäure: SEQ ID NO: 121, Protein: SEQ ID NO:122),

sowie weitere Farnesyl-Diphosphat-Synthase-Gene aus anderen Organismen mit den folgenden Accession Nummern:
P53799, P37268, Q02769, Q09152, P49351, 024241, Q43315, P49352, 024242, P49350, P08836, P14324, P49349, P08524, 066952, Q08291, P54383, Q45220, P57537, Q8K9A0, P22939, P45204, 066126, P55539, Q9SWH9, Q9AV17, Q9FRX2, Q9AYS7, Q941E8, Q9FXR9, Q9ZWF6, Q9FXR8, Q9AR37, O50009,Q941E9,Q8RVK7, Q8RVQ7, 004882, Q93RA8, Q93RB0, Q93RB4, Q93RB5,Q93RB3, Q93RB1, Q93RB2, Q920E5.

Beispiele für Geranyl-geranyl-Diphosphat-Synthase -Gene sind:

Eine Nukleinsäure, kodierend eine Geranyl-geranyl-Diphosphat-Synthase aus Sinaps alba, ACCESSION #X98795, veröffentlicht durch Bonk,M., Hoffmann,B., Von Lintig,J., Schledz,M., Al-Babili,S., Hobeika,E., Kleinig,H. and Beyer,P.: Chloroplast import of four carotenoid biosynthetic enzymes in vitro reveals differential fates prior to membrane binding and oligomeric assembly, Eur. J. Biochem. 247 (3), 942-950 (1997), (Nukleinsäure: SEQ ID NO: 123, Protein: SEQ ID NO:124),

sowie weitere Geranyl-geranyl-Diphosphat-Synthase-Gene aus anderen Organismen mit den folgenden Accession Nummern:
P22873, P34802 ,P56966, P80042, Q42698, Q92236, O95749, Q9WTN0, Q50727, P24322, P39464, Q9FXR3, Q9AYN2, Q9FXR2, Q9AVG6. Q9FRW4, Q9SXZ5, Q9AVJ7, Q9AYN1, Q9AVJ4, Q9FXR7, Q8LSC5, Q9AVJ6, Q8LSC4, Q9AVJ3, Q9SSU0, Q9SXZ6, Q9SST9, Q9AVJ0, Q9AVI9, Q9FRW3, Q9FXR5, Q94IF0, Q9FRX1. Q9K567, Q93RA9, Q93QX8, CAD95619, EAA31459

Beispiele für Phytoen-Synthase -Gene sind:

Eine Nukleinsäure, kodierend eine Phytoen-Synthase aus Erwinia uredovora, ACCESSION # D90087; veröffentlicht durch Misawa,N., Nakagawa,M., Kobayashi,K., Yamano,S., Izawa,Y.,Nakamura,K. und Harashima,K.: Elucidation of the Erwinia uredovora carotenoid biosynthetic pathway by functional analysis of gene products expressed in Escherichia coli; J. Bacteriol. 172 (12), 6704-6712 (1990), (Nukleinsäure: SEQ ID NO: 125, Protein: SEQ ID NO: 126),

sowie weitere Phytoen-Synthase -Gene aus anderen Organismen mit den folgenden Accession Nummern:
CAB39693, BAC69364, AAF10440, CAA45350, BAA20384, AAM72615, BAC09112, CAA48922, P_001091, CAB84588, AAF41518, CAA48155, AAD38051, AAF33237, AAG10427, AAA34187, BAB73532, CAC19567, AAM62787, CAA55391, AAB65697, AAM45379, CAC27383, AAA32836, AAK07735, BAA84763, P_000205, AAB60314, P_001163, P_000718, AAB71428, AAA34153, AAK07734, CAA42969, CAD76176, CAA68575, P_000130, P_001142, CAA47625, CAA85775, BAC14416, CAA79957, BAC76563, P_000242, P_000551, AAL02001, AAK15621, CAB94795, AAA91951, P_000448

Beispiele für Phytoen-Desaturase-Gene sind:

Eine Nukleinsäure, kodierend eine Phytoen-Desaturase aus Erwinia uredovora, ACCESSION # D90087; veröffentlicht durch Misawa,N., Nakagawa,M., Kobayashi,K., Yamano,S., Izawa,Y.,Nakamura,K. und Harashima,K.: Elucidation of the Erwinia uredovora carotenoid biosynthetic pathway by functional analysis of gene products expressed in Escherichia coli; J. Bacteriol. 172 (12), 6704-6712 (1990), (Nukleinsäure: SEQ ID NO: 127, Protein: SEQ ID NO: 128),

sowie weitere Phytoen-Desaturase -Gene aus anderen Organismen mit den folgenden Accession Nummern:
AAL15300, A39597, CAA42573, AAK51545, BAB08179, CAA48195, BAB82461, AAK92625, CAA55392, AAG10426, AAD02489, AAO24235, AAC12846, AAA99519, AAL38046, CAA60479, CAA75094, ZP_001041, ZP_001163, CAA39004, CAA44452, ZP_001142, ZP_000718, BAB82462, AAM45380, CAB56040, ZP_001091, BAC09113, AAP79175, AAL80005, AAM72642, AAM72043, ZP_000745, ZP_001141, BAC07889, CAD55814, ZP_001041, CAD27442, CAE00192, ZP_001163, ZP_000197, BAA18400, AAG10425, ZP_001119, AAF13698, 2121278A, AAB35386, AAD02462, BAB68552, CAC85667, AAK51557, CAA12062, AAG51402, AAM63349, AAF85796, BAB74081, AAA91161, CAB56041, AAC48983, AAG 14399, CAB65434, BAB73487, ZP_001117, ZP_000448, CAB39695, CAD76175, BAC69363, BAA17934, ZP_000171, AAF65586, ZP_000748, BAC07074, ZP_001133, CAA64853, BAB74484, ZP_001156, AAF23289, AAG28703, AAP09348, AAM71569, BAB69140, ZP_000130, AAF41516, AAG 18866, CAD95940, NP_656310, AAG10645, ZP_000276, ZP_000192, ZP_000186, AAM94364, EAA31371, ZP_000612, BAC75676, AAF65582

Beispiele für Zeta-Carotin-Desaturase-Gene sind:

Eine Nukleinsäure, kodierend eine Zeta-Carotin-Desaturase aus Narcissus pseudonarcissus, ACCESSION #AJ224683, veröffentlicht durch AI-Babili,S., Oelschlegel,J. and Beyer,P.: A cDNA encoding for beta carotene desaturase (Accession No.AJ224683) from Narcissus pseudonarcissus L.. (PGR98-103), Plant Physiol. 117, 719-719 (1998), (Nukleinsäure: SEQ ID NO: 129, Protein: SEQ ID NO: 130),

sowie weitere Zeta-Carotin-Desaturase-Gene aus anderen Organismen mit den folgenden Accession Nummern:
Q9R6X4, Q38893, Q9SMJ3, Q9SE20, Q9ZTP4, 049901, P74306, Q9FV46, Q9RCT2, ZDS_NARPS, BAB68552.1, CAC85667.1, AF372617_1, ZDS_TARER, CAD55814.1, CAD27442.1, 2121278A, ZDS_CAPAN, ZDS_LYCES, NP_187138.1, AAM63349.1, ZDS_ARATH, AAA91161.1, ZDS_MAIZE, AAG14399.1, NP_441720.1, NP_486422.1, ZP_00111920.1, CAB56041.1, ZP_00074512.1, ZP_00116357.1, NP_681127.1, ZP_00114185.1, ZP_00104126.1, CAB65434.1, NP_662300.1

Beispiele für crtISO-Gene sind:

Eine Nukleinsäure, kodierend eine crtISO aus Lycopersicon esculentum; ACCESSION #AF416727, veröffentlicht durch Isaacson,T., Ronen,G., Zamir,D. and Hirschberg,J.: Cloning of tangerine from tomato reveals a carotenoid isomerase essential for the production of beta-carotene and xanthophylls in plants; Plant Cell 14 (2), 333-342 (2002), (Nukleinsäure: SEQ ID NO: 131, Protein: SEQ ID NO:132),

sowie weitere crtISO -Gene aus anderen Organismen mit den folgenden Accession Nummern:
AAM53952

Beispiele für FtsZ-Gene sind:

Eine Nukleinsäure, kodierend eine FtsZ aus Tagetes erecta, ACCESSION #AF251346, veröffentlicht durch Moehs,C.P., Tian,L., Osteryoung,K.W. and Dellapenna,D.: Analysis of carotenoid biosynthetic gene expression during marigold petal development Plant Mol. Biol. 45 (3), 281-293 (2001), (Nukleinsäure: SEQ ID NO: 133, Protein: SEQ ID NO: 134),

sowie weitere FtsZ -Gene aus anderen Organismen mit den folgenden Accession Nummern:
CAB89286.1, AF205858_1, NP_200339.1, CAB89287.1, CAB41987.1, AAA82068.1, T06774,AF383876_1, BAC57986.1, CAD22047.1, BAB91150.1, ZP_00072546.1, NP_440816.1, T51092, N_683172.1, BAA85116.1, N_487898.1, JC4289, BAA82871.1, NP_781763.1, BAC57987.1, ZP_00111461.1, T51088, NP_190843.1, ZP_00060035.1, NP_846285.1, AAL07180.1, NP_243424.1, NP_833626.1, AAN04561.1, AAN04557.1, CAD22048.1, T51089, NP_692394.1, NP_623237.1, NP_565839.1, T51090, CAA07676.1, NP_113397.1, T51087, CAC44257.1, E84778, ZP_00105267.1, BAA82091.1, ZP_00112790.1, BAA96782.1, NP_348319.1, NP_471472.1, ZP_00115870.1, NP_465556.1, NP_389412.1, BAA82090.1, NP_562681.1, AAM22891.1, NP_371710.1, NP_764416.1, CAB95028.1, FTSZ_STRGR, AF120117_1, NP_827300.1, JE0282, NP_626341.1, AAC45639.1, NP_785689.1, NP_336679.1, NP_738660.1, ZP_00057764.1, AAC32265.1, NP_814733.1, FTSZ_MYCKA, NP_216666.1, CAA75616.1, N_301700.1, NP_601357.1, ZP_00046269.1, CAA70158.1, ZP_00037834.1, NP_268026.1, FTSZ_ENTH_R, NP_787643.1, NP_346105.1, AAC32264.1, JC5548, AAC95440.1, NP_710793.1, NP_687509.1, NP_269594.1, AAC32266.1, NP_720988.1, NP_657875.1, ZP_00094865.1, ZP_00080499.1, ZP_00043589.1, JC7087, NP_660559.1, AAC46069.1, AF179611_14, AAC44223.1, NP_404201.1.

Beispiele für MinD -Gene sind:

Eine Nukleinsäure, kodierend eine MinD aus Tagetes erecta, ACCESSION #AF251019, veröffentlicht durch Moehs,C.P., Tian,L., Osteryoung,K.W. und Dellapenna,D.: Analysis of carotenoid biosynthetic gene expression during marigold petal development; Plant Mol. Biol. 45 (3), 281-293 (2001), (Nukleinsäure: SEQ ID NO: 135, Protein: SEQ ID NO: 136),

sowie weitere MinD -Gene mit den folgenden Accession Nummern:
NP_197790.1, BAA90628.1, NP_038435.1, NP_045875.1, AAN33031.1, N_050910.1, CAB53105.1, NP_050687.1, N_682807.1, NP_487496.1, ZP_00111708.1, ZP_00071109.1, NP_442592.1, NP_603083.1, NP_782631.1, ZP_00097367.1, ZP_00104319.1, NP_294476.1, NP_622555.1, NP_563054.1, NP_347881.1, ZP_00113908.1, NP_834154.1, NP_658480.1, ZP_00059858.1, NP_470915.1, NP_243893.1, NP_465069.1, ZP_00116155.1, NP_390677.1, NP_692970.1, N_298610.1, NP_207129.1, ZP_00038874.1, N_778791.1, NP_223033.1, NP_641561.1, NP_636499.1, ZP_00088714.1, NP_213595.1, NP_743889.1, NP_231594.1, ZP_00085067.1, NP_797252.1, ZP_00136593.1, NP_251934.1, NP_405629.1, NP_759144.1, ZP_00102939.1, NP_793645.1, N_699517.1, NP_460771.1, N_860754.1, N_456322.1, NP_718163.1, NP_229666.1, NP_357356.1, NP_541904.1, NP_287414.1, NP_660660.1, ZP_00128273.1, NP_103411.1, NP_785789.1, NP_715361.1, AF149810_1, NP_841854.1, NP_437893.1, ZP_00022726.1, EAA24844.1, ZP_00029547.1, NP_521484.1, NP_240148.1, NP_770852.1, AF345908_2, NP_777923.1, ZP_00048879.1, NP_579340.1, NP_143455.1, NP_126254.1, NP_142573.1, NP_613505.1, NP_127112.1, NP_712786.1, NP_578214.1, NP_069530.1, NP_247526.1, AAA85593.1, NP_212403.1, NP_782258.1, ZP_00058694.1, NP_247137.1, NP_219149.1, NP_276946.1, NP_614522.1, ZP_00019288.1, CAD78330.1

Bevorzugt verwendet man in vorstehend beschriebener bevorzugter Ausführungsform als HMG-CoA-Reduktase-Gene Nukleinsäuren, die Proteine kodieren, enthaltend die Aminosäuresequenz SEQ ID NO: 112 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 112, und die die enzymatische Eigenschaft einer HMG-CoA-Reduktase aufweisen.

Weitere Beispiele für HMG-CoA-Reduktasen und HMG-CoA-Reduktase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 112 leicht auffinden.

Weitere Beispiele für HMG-CoA-Reduktasen und HMG-CoA-Reduktase-Gene lassen sich weiterhin beispielsweise ausgehend von der Sequenz SEQ ID NO: 111 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der HMG-CoA-Reduktase-Aktivität Nukleinsäuren in Organismen eingebracht, die Proteine kodieren, enthaltend die Aminosäuresequenz der HMG-CoA-Reduktase der Sequenz SEQ ID NO: 112.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 111 in den Organismus ein.

Bevorzugt verwendet man in vorstehend beschriebener bevorzugter Ausführungsform als (E)-4-Hydroxy-3-Methylbut-2-enyl-Diphosphat-Reduktase-Gene Nukleinsäuren, die Proteine kodieren, enthaltend die Aminosäuresequenz SEQ ID NO: 114 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70%, noch bevorzugter mindestens 90 %, am bevorzugtesten mindestens 95 % auf Aminosäureebene mit der Sequenz SEQ ID NO: 114, und die die enzymatische Eigenschaft einer (E)-4-Hydroxy-3-Methylbut-2-enyl-Diphosphat-Reduktase aufweisen.

Weitere Beispiele für (E)-4-Hydroxy-3-Methylbut-2-enyl-Diphosphat-Reduktasen und (E)-4-Hydroxy-3-Methylbut-2-enyl-Diphosphat-Reduktase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 114 leicht auffinden.

Weitere Beispiele für (E)-4-Hydroxy-3-Methylbut-2-enyl-Diphosphat-Reduktasen und (E)-4-Hydroxy-3-Methylbut-2-enyl-Diphosphat-Reduktase-Gene lassen sich weiterhin beispielsweise ausgehend von der Sequenz SEQ ID NO: 113 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der (E)-4-Hydroxy-3-Methylbut-2-enyl-Diphosphat-Reduktase-Aktivität Nukleinsäuren in Organismen eingebracht, die Proteine kodieren, enthaltend die Aminosäuresequenz der (E)-4-Hydroxy-3-Methylbut-2-enyl-Diphosphat-Reduktase der Sequenz SEQ ID NO: 114.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 113 in den Organismus ein.

Bevorzugt verwendet man in vorstehend beschriebener bevorzugter Ausführungsform als (1-Deoxy-D-Xylose-5-Phosphat-Synthase-Gene Nukleinsäuren, die Proteine kodieren, enthaltend die Aminosäuresequenz SEQ ID NO: 116 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70%, noch bevorzugter mindestens 90 %, am bevorzugtesten mindestens 95 % auf Aminosäureebene mit der Sequenz SEQ ID NO: 116, und die die enzymatische Eigenschaft einer (1-Deoxy-D-Xylose-5-Phosphat-Synthase aufweisen.

Weitere Beispiele für (1-Deoxy-D-Xylose-5-Phosphat-Synthasen und (1-Deoxy-D-Xylose-5-Phosphat-Synthase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 116 leicht auffinden.

Weitere Beispiele für (1-Deoxy-D-Xylose-5-Phosphat-Synthasen und (1-Deoxy-D-Xylose-5-Phosphat-Synthase-Gene lassen sich weiterhin beispielsweise ausgehend von der Sequenz SEQ ID NO: 115 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der (1-Deoxy-D-Xylose-5-Phosphat-Synthase-Aktivität Nukleinsäuren in Organismen eingebracht, die Proteine kodieren, enthaltend die Aminosäuresequenz der (1-Deoxy-D-Xylose-5-Phosphat-Synthase der Sequenz SEQ ID NO: 116.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 115 in den Organismus ein.

Bevorzugt verwendet man in vorstehend beschriebener bevorzugter Ausführungsform als 1-Deoxy-D-Xylose-5-Phosphat-Reduktoisomerase-Gene Nukleinsäuren, die Proteine kodieren, enthaltend die Aminosäuresequenz SEQ ID NO: 138 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 138, und die die enzymatische Eigenschaft einer 1-Deoxy-D-Xylose-5-Phosphat-Reduktoisomerase aufweisen.

Weitere Beispiele für 1-Deoxy-D-Xylose-5-Phosphat-Reduktoisomerasen und 1-Deoxy-D-Xylose-5-Phosphat-Reduktoisomerase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 138 leicht auffinden.

Weitere Beispiele für 1-Deoxy-D-Xylose-5-Phosphat-Reduktoisomerasen und 1-Deoxy-D-Xylose-5-Phosphat-Reduktoisomerase-Gene lassen sich weiterhin beispielsweise ausgehend von der Sequenz SEQ ID NO: 137 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der 1-Deoxy-D-Xylose-5-Phosphat-Reduktoisomerase-Aktivität Nukleinsäuren in Organismen eingebracht, die Proteine kodieren, enthaltend die Aminosäuresequenz der 1-Deoxy-D-Xylose-5-Phosphat-Reduktoisomerase der Sequenz SEQ ID NO: 138.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 137 in den Organismus ein.

Bevorzugt verwendet man in vorstehend beschriebener bevorzugter Ausführungsform als Isopentenyl-D-Isomerase-Gene Nukleinsäuren, die Proteine kodieren, enthaltend die Aminosäuresequenz SEQ ID NO: 118 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 118, und die die enzymatische Eigenschaft einer Isopentenyl-D-Isomerase aufweisen.

Weitere Beispiele für Isopentenyl-D-Isomerasen und Isopentenyl-D-Isomerase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 118 leicht auffinden.

Weitere Beispiele für Isopentenyl-D-Isomerasen und Isopentenyl-D-Isomerase-Gene lassen sich weiterhin beispielsweise ausgehend von der Sequenz SEQ ID NO: 117 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der Isopentenyl-D-Isomerase-Aktivität Nukleinsäuren in Organismen eingebracht, die Proteine kodieren, enthaltend die Aminosäuresequenz der lsopentenyl-D-Isomerase der Sequenz SEQ ID NO: 118.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 117 in den Organismus ein.

Bevorzugt verwendet man in vorstehend beschriebener bevorzugter Ausführungsform als Geranyl-Diphosphat-Synthase-Gene Nukleinsäuren, die Proteine kodieren, enthaltend die Aminosäuresequenz SEQ ID NO: 120 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 120, und die die enzymatische Eigenschaft einer Geranyl-Diphosphat-Synthase aufweisen.

Weitere Beispiele für Geranyl-Diphosphat-Synthasen und Geranyl-Diphosphat-Synthase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 120 leicht auffinden.

Weitere Beispiele für Geranyl-Diphosphat-Synthasen und Geranyl-Diphosphat-Synthase-Gene lassen sich weiterhin beispielsweise ausgehend von der Sequenz SEQ ID NO: 119 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der Geranyl-Diphosphat-Synthase-Aktivität Nukleinsäuren in Organismen eingebracht, die Proteine kodieren, enthaltend die Aminosäuresequenz der Geranyl-Diphosphat-Synthase der Sequenz SEQ ID NO: 120.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 119 in den Organismus ein.

Bevorzugt verwendet man in vorstehend beschriebener bevorzugter Ausführungsform als Farnesyl-Diphosphat-Synthase-Gene Nukleinsäuren, die Proteine kodieren, enthaltend die Aminosäuresequenz SEQ ID NO: 122 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 122, und die die enzymatische Eigenschaft einer Farnesyl-Diphosphat-Synthase aufweisen.

Weitere Beispiele für Farnesyl-Diphosphat-Synthasen und Farnesyl-Diphosphat-Synthase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 122 leicht auffinden.

Weitere Beispiele für Farnesyl-Diphosphat-Synthasen und Farnesyl-Diphosphat-Synthase-Gene lassen sich weiterhin beispielsweise ausgehend von der Sequenz SEQ ID NO: 121 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der Farnesyl-Diphosphat-Synthase-Aktivität Nukleinsäuren in Organismen eingebracht, die Proteine kodieren, enthaltend die Aminosäuresequenz der Farnesyl-Diphosphat-Synthase der Sequenz SEQ ID NO: 122.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 121 in den Organismus ein.

Bevorzugt verwendet man in vorstehend beschriebener bevorzugter Ausführungsform als Geranyl-geranyl-Diphosphat-Synthase-Gene Nukleinsäuren, die Proteine kodieren, enthaltend die Aminosäuresequenz SEQ ID NO: 124 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 124, und die die enzymatische Eigenschaft einer Geranyl-geranyl-Diphosphat-Synthase aufweisen.

Weitere Beispiele für Geranyl-geranyl-Diphosphat-Synthasen und Geranyl-geranyl-Diphosphat-Synthase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 124 leicht auffinden.

Weitere Beispiele für Geranyl-geranyl-Diphosphat-Synthasen und Geranyl-geranyl-Diphosphat-Synthase-Gene lassen sich weiterhin beispielsweise ausgehend von der Sequenz SEQ ID NO: 123 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der Geranyl-geranyl-Diphosphat-Synthase-Aktivität Nukleinsäuren in Organismen eingebracht, die Proteine kodieren, enthaltend die Aminosäuresequenz der Geranyl-geranyl-Diphosphat-Synthase der Sequenz SEQ ID NO: 124.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 123 in den Organismus ein.

Bevorzugt verwendet man in vorstehend beschriebener bevorzugter Ausführungsform als Phytoen-Synthase-Gene Nukleinsäuren, die Proteine kodieren, enthaltend die Aminosäuresequenz SEQ ID NO: 126 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 126, und die die enzymatische Eigenschaft einer Phytoen-Synthase aufweisen.

Weitere Beispiele für Phytoen-Synthasen und Phytoen-Synthase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 126 leicht auffinden.

Weitere Beispiele für Phytoen-Synthasen und Phytoen-Synthase-Gene lassen sich weiterhin beispielsweise ausgehend von der Sequenz SEQ ID NO: 125 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der Phytoen-Synthase-Aktivität Nukleinsäuren in Organismen eingebracht, die Proteine kodieren, enthaltend die Aminosäuresequenz der Phytoen-Synthase der Sequenz SEO ID NO: 126.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 125 in den Organismus ein.

Bevorzugt verwendet man in vorstehend beschriebener bevorzugter Ausführungsform als Phytoen-Desaturase-Gene Nukleinsäuren, die Proteine kodieren, enthaltend die Aminosäuresequenz SEQ ID NO: 128 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 128, und die die enzymatische Eigenschaft einer Phytoen-Desaturase aufweisen.

Weitere Beispiele für Phytoen-Desaturasen und Phytoen-Desaturase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 128 leicht auffinden.

Weitere Beispiele für Phytoen-Desaturasen und Phytoen-Desaturase-Gene lassen sich weiterhin beispielsweise ausgehend von der Sequenz SEQ ID NO: 127 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der Phytoen-Desaturase-Aktivität Nukleinsäuren in Organismen eingebracht, die Proteine kodieren, enthaltend die Aminosäuresequenz der Phytoen-Desaturase der Sequenz SEQ ID NO: 128.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 127 in den Organismus ein.

Bevorzugt verwendet man in vorstehend beschriebener bevorzugter Ausführungsform als Zeta-Carotin-Desaturase-Gene Nukleinsäuren, die Proteine kodieren, enthaltend die Aminosäuresequenz SEQ ID NO: 130 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 130, und die die enzymatische Eigenschaft einer Zeta-Carotin-Desaturase aufweisen.

Weitere Beispiele für Zeta-Carotin-Desaturasen und Zeta-Carotin-Desaturase-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SEQ ID NO: 130 leicht auffinden.

Weitere Beispiele für Zeta-Carotin-Desaturasen und Zeta-Carotin-Desaturase-Gene lassen sich weiterhin beispielsweise ausgehend von der Sequenz SEQ ID NO: 129 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der Zeta-Carotin-Desaturase-Aktivität Nukleinsäuren in Organismen eingebracht, die Proteine kodieren, enthaltend die Aminosäuresequenz der Zeta-Carotin-Desaturase der Sequenz SEQ ID NO: 130.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 129 in den Organismus ein.

Bevorzugt verwendet man in vorstehend beschriebener bevorzugter Ausführungsform als Crtlso-Gene Nukleinsäuren, die Proteine kodieren, enthaltend die Aminosäuresequenz SEQ ID NO: 132 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 132, und die die enzymatische Eigenschaft einer Crtlso aufweisen.

Weitere Beispiele für Crtlson und Crtlso-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 132 leicht auffinden.

Weitere Beispiele für Crtlson und Crtlso-Gene lassen sich weiterhin beispielsweise ausgehend von der Sequenz SEQ ID NO: 131 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der Crtlso-Aktivität Nukleinsäuren in Organismen eingebracht, die Proteine kodieren, enthaltend die Aminosäuresequenz der Crtlso der Sequenz SEQ ID NO: 132.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 131 in den Organismus ein.

Bevorzugt verwendet man in vorstehend beschriebener bevorzugter Ausführungsform als FtsZ-Gene Nukleinsäuren, die Proteine kodieren, enthaltend die Aminosäuresequenz SEQ ID NO: 134 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 134, und die die enzymatische Eigenschaft einer FtsZ aufweisen.

Weitere Beispiele für FtsZn und FtsZ-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 134 leicht auffinden.

Weitere Beispiele für FtsZn und FtsZ-Gene lassen sich weiterhin beispielsweise ausgehend von der Sequenz SEQ ID NO: 133 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der FtsZ-Aktivität Nukleinsäuren in Organismen eingebracht, die Proteine kodieren, enthaltend die Aminosäuresequenz der FtsZ der Sequenz SEQ ID NO: 134

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 133 in den Organismus ein.

Bevorzugt verwendet man in vorstehend beschriebener bevorzugter Ausführungsform als MinD-Gene Nukleinsäuren, die Proteine kodieren, enthaltend die Aminosäuresequenz SEQ ID NO: 136 oder eine von dieser Sequenz durch Substitution, Insertion oder Deletion von Aminosäuren abgeleitete Sequenz, die eine Identität von mindestens 30 %, vorzugsweise mindestens 50 %, bevorzugter mindestens 70 %, noch bevorzugter mindestens 90 %, am bevorzugtesten mindestens 95 % auf Aminosäureebene mit der Sequenz SEQ ID NO: 136, und die die enzymatische Eigenschaft einer MinD aufweisen.

Weitere Beispiele für MinDn und MinD-Gene lassen sich beispielsweise aus verschiedenen Organismen, deren genomische Sequenz bekannt ist, wie vorstehend beschrieben, durch Homologievergleiche der Aminosäuresequenzen oder der entsprechenden rückübersetzten Nukleinsäuresequenzen aus Datenbanken mit der SeQ ID NO: 136 leicht auffinden.

Weitere Beispiele für MinDn und MinD-Gene lassen sich weiterhin beispielsweise ausgehend von der Sequenz SEQ ID NO: 135 aus verschiedenen Organismen deren genomische Sequenz nicht bekannt ist, wie vorstehend beschrieben, durch Hybridisierungs- und PCR-Techniken in an sich bekannter Weise leicht auffinden.

In einer weiter besonders bevorzugten Ausführungsform werden zur Erhöhung der MinD-Aktivität Nukleinsäuren in Organismen eingebracht, die Proteine kodieren, enthaltend die Aminosäuresequenz der MinD der Sequenz SEQ ID NO: 136.

Geeignete Nukleinsäuresequenzen sind beispielsweise durch Rückübersetzung der Polypeptidsequenz gemäß dem genetischen Code erhältlich.

Bevorzugt werden dafür solche Codons verwendet, die entsprechend der pflanzenspezifischen codon usage häufig verwendet werden. Die codon usage lässt sich anhand von Computerauswertungen anderer, bekannter Gene der betreffenden Organismen leicht ermitteln.

In einer besonders bevorzugten Ausführungsform bringt man eine Nukleinsäure, enthaltend die Sequenz SEQ ID NO: 135 in den Organismus ein.

Alle vorstehend erwähnten HMG-CoA-Reduktase-Gene, (E)-4-Hydroxy-3-Methylbut-2-enyl-Diphosphat-Reduktase-Gene, 1-Deoxy-D-Xylose-5-Phosphat-Synthase-Gene, 1-Deoxy-D-Xylose-5-Phosphat-Reduktoisomerase-Gene, Isopentenyl-Diphosphat-Δ-Isomerase-Gene, Geranyl-Diphosphat-Synthase-Gene, Farnesyl-Diphosphat-Synthase-Gene, Geranyl-geranyl-Diphosphat-Synthase-Gene, Phytoen-Synthase-Gene, Phytoen-Desaturase-Gene, Zeta-Carotin-Desaturase-Gene, crtISO-Gene, FtsZ-Gene oder MinD-Gene sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

In einer weiter bevorzugten Ausführungsform des Verfahrens weisen die Pflanzen gegenüber dem Wildtyp zusätzlich eine reduzierte endogene β-Hydroxylase Aktivität auf.

Unter einer reduzierten Aktivität wird, wie vorstehend erwähnt, vorzugsweise die teilweise oder im wesentlichen vollständige, auf unterschiedliche zellbiologische Mechanismen beruhende Unterbindung oder Blockierung der Funktionalität eines Enzyms in einer pflanzlichen Zelle, Pflanze oder einem davon abgeleiteten Teil, Gewebe, Organ, Zellen oder Samen verstanden.

Die Reduzierung einer Aktivität in Pflanzen gegenüber dem Wildtyp kann beispielsweise durch Reduzierung der Proteinmenge, oder der mRNA-Menge in der Pflanze erfolgen. Dementsprechend kann eine gegenüber dem Wildtyp reduzierte Aktivität direkt bestimmt werden oder über die Bestimmung der Proteinmenge oder der mRNA-Menge der erfindungsgemäßen Pflanze im Vergleich zum Wildtyp erfolgen.

Eine Reduzierung einer Aktivität umfasst eine mengenmäßige Verringerung eines Proteins bis hin zu einem im wesentlichen vollständigen Fehlen des Proteins (d.h. fehlende Nachweisbarkeit der entsprechenden Aktivität oder fehlende immunologische Nachweisbarkeit des entsprechenden Proteins).

Unter endogener β-Hydroxylase -Aktivität wird die Enzymaktivität der endogenen, pflanzeneigenen β-Hydroxylase verstanden.

Unter einer endogenen β-Hydroxylase wird eine endogene, pflanzeneigene Hxdroxylase wie vorstehend beschrieben, verstanden. Ist beispielsweise Tagetes errecta die genetisch zu verändernde Zielpflanze, so wird unter der endogenen β-Hydroxylase die β-Hydoxylase von Tagetes errecta verstanden.

Unter einer endogenen β-Hydroxylase wird demnach insbesondere ein pflanzeneigenes Protein verstanden, das die enzymatische Aktivität aufweist, β-Carotin in Zeaxanthin umzuwandeln.

Dementsprechend wird unter endogener β-Hydroxylase -Aktivität die in einer bestimmten Zeit durch das Protein endogene β-Hydroxylase umgesetzte Menge β-Carotin bzw. gebildete Menge Zeaxanthin verstanden.

Bei einer reduzierten endogenen β-Hydroxylase-Aktivität gegenüber dem Wildtyp wird somit im Vergleich zum Wildtyp in einer bestimmten Zeit die durch das Protein endogene β-Hydroxylase umgesetzte Menge β-Carotin bzw. die gebildete Menge Zeaxanthin reduziert.

Vorzugsweise beträgt diese Reduzierung der endogenen β-Hydroxylase-Aktivität mindestens 5 %, weiter bevorzugt mindestens 20 %, weiter bevorzugt mindestens 50 %, weiter bevorzugt 100 %. Besonders bevorzugt ist die endogenen β-Hydroxylase-Aktivität komplett ausgeschaltet.

Es wurde überraschenderweise gefunden, dass es bei Pflanzen die mehrheitlich Carotinoide des α-Carotin-Weges, wie beispielsweise Lutein, herstellen, wie beispielsweise Pflanzen der Gattung Tagetes, vorteilhaft ist, die Aktivität der endogenen β-Hydroxylase zu reduzieren und gegebenenfalls die Aktivität einer heterologen Hydroxylase zu erhöhen. Besonders bevorzugt werden dabei Hydroxylasen oder funktionelle Äquivalente davon verwendet, die aus Pflanzen stammen, die mehrheitlich Carotinoide des β-Carotin-Weges herstellen, wie beispielsweiese die vorstehend beschriebene β-Hydroxylase aus Tomate (Nukleinsäure: SEQ ID No. 107, Protein: SEQ ID No. 108).

Die Bestimmung der endogenen β-Hydroxylase Aktivtät erfolgt wie vorstehend beschrieben analog zur Bestimmung der Hydroxylase-Aktivität.

Vorzugsweise erfolgt die Reduzierung der endogenen β-Hydroxylase-Aktivität in Pflanzen durch mindestens eines der nachfolgenden Verfahren:
a) Einbringen mindestens einer doppelsträngigen endogenen β-Hydroxylase Ribonukleinsäuresequenz, nachstehend auch endogene β-Hydroxylase-dsRNA genannt, oder einer deren Expression gewährleistenden Expressionskassette oder Expressionskassetten.

Umfasst sind solche Verfahren, bei denen die endogene β-Hydroxylase-dsRNA gegen ein endogenes β-Hydroxylase-Gen (also genomische DNA-Sequenzen wie die Promotorsequenz) oder ein endogenes β-Hydroxylase-Transkript (also mRNA-Sequenzen) gerichtet ist,
b) Einbringen mindestens einer endogenen β-Hydroxylase antisense-Ribonukleinsäuresequenz, nachstehend auch endogene β-Hydroxylase-antisenseRNA genannt, oder einer deren Expression gewährleistenden Expressionskassette. Umfasst sind solche Verfahren, bei denen die endogene β-Hydroxylase-antisenseRNA gegen ein endogenes β-Hydroxylase-Gen (also genomische DNA-Sequenzen) oder ein endogenes β-Hydroxylase-Gentranskript (also RNA-Sequenzen) gerichtet ist. Umfasst sind auch α-anomere Nukleinsäuresequenzen,
c) Einbringen mindestens einer endogenen β-Hydroxylase-antisenseRNA kombiniert mit einem Ribozym oder einer deren Expression gewährleistenden Expressionskassette
d) Einbringen mindestens einer endogenen β-Hydroxylase sense-Ribonukleinsäuresequenz, nachstehend auch endogene β-HydroxylasesenseRNA genannt, zur Induktion einer Kosuppression oder einer deren Expression gewährleistenden Expressionskassette
e) Einbringen mindestens eines DNA- oder Protein-bindenden Faktors gegen ein endogenes β-Hydroxylase-Gen, -RNA oder -Protein oder einer dessen Expression gewährleistenden Expressionskassette
f) Einbringen mindestens einer, den endogenen β-Hydroxylase RNA-Abbau bewirkenden viralen Nukleinsäuresequenz oder einer deren Expression gewährleistenden Expressionskassette
g) Einbringen mindestens eines Konstruktes zur Erzeugung eines Funktionsverlustes, wie beispielsweise die Generierung von Stopp-Kodons oder eine Verschiebungen im Leseraster, an einem endogenen β-Hydroxylase-Gen beispielsweise durch Erzeugung einer Insertion, Deletion, Inversion oder Mutation in einem endogenen β-Hydroxylase-Gen. Bevorzugt können Knockout-Mutanten mittels gezielter Insertion in besagtes endogenes β-Hydroxylase-Gen durch homologe Rekombination oder Einbringen von sequenzspezifischen Nukleasen gegen endogene β-Hydroxylase-Gensequenzen generiert werden.

Dem Fachmann ist bekannt, dass auch weitere Verfahren im Rahmen der vorliegenden Erfindung zur Verminderung einer endogenen β-Hydroxylase bzw. seiner Aktivität oder

Funktion eingesetzt werden können. Beispielsweise kann auch das Einbringen einer dominant-negativen Variante einer endogenen β-Hydroxylase oder einer deren Expression gewährleistenden Expressionskassette vorteilhaft sein. Dabei kann jedes einzelne dieser Verfahren eine Verminderung der Proteinmenge, mRNA-Menge und/oder Aktivität einer endogenen β-Hydroxylase bewirken. Auch eine kombinierte Anwendung ist denkbar. Weitere Methoden sind dem Fachmann bekannt und können die Behinderung oder Unterbindung der Prozessierung der endogenen β-Hydroxylase, des Transports der Zeaxanthin-Epoxidase und/oder endogenen β-Hydroxylase oder dessen mRNA, Hemmung der Ribosomenanlagerung, Hemmung des RNA-Spleißens, Induktion eines endogenen β-Hydroxylase-RNA abbauenden Enzyms und/oder Hemmung der Translationselongation oder -termination umfassen.

Die einzelnen bevorzugten Verfahren seien infolge durch beispielhafte Ausführungsformen beschrieben:
a) Einbringen einer doppelsträngigen, endogenen β-Hydroxylase-Ribonukleinsäuresequenz (endogene β-Hydroxylase-dsRNA)

Das Verfahren der Genregulation mittels doppelsträngiger RNA wurde vorstehend für die Reduzierung der ε-Cyclase-Aktivität ausführlich beschrieben. Analog lässt sich dieses Verfahren für die Reduzierung der endogenen β-Hydroxylase-Aktivität durchführen.

Unter einer doppelsträngigen endogenen β-Hydroxylase-Ribonukleinsäuresequenz oder auch endogenen β-Hydroxylase-dsRNA wird vorzugsweise ein RNA-Molekül verstanden, das einen Bereich mit Doppel-Strang-Struktur aufweist und in diesem Bereich eine Nukleinsäuresequenz enthält, die
a) mit mindestens einem Teil des Pflanze eigenen endogenen β-Hydroxylase-Transkripts identisch ist und/oder
b) mit mindestens einem Teil der Pflanze eigenen endogenen β-Hydroxylase-Promotor-Sequenz identisch ist.

Im erfindungsgemäßen Verfahren bringt man daher zur Reduzierung der endogenen β-Hydroxylase-Aktivität bevorzugt in die Pflanze eine RNA ein, die einen Bereich mit Doppel-Strang-Struktur aufweist und in diesem Bereich eine Nukleinsäuresequenz enthält, die
a) mit mindestens einem Teil des Pflanze eigenen endogenen β-Hydroxylase-Transkripts identisch ist und/oder
b) mit mindestens einem Teil der Pflanze eigenen endogenen β-Hydroxylase-Promotor-Sequenz identisch ist.

Unter dem Begriff "endogenes β-Hydroxylase-Transkript" wird der transkripierte Teil eines eines endogenen β-Hydroxylase-Gens verstanden, der neben der endogenen β-Hydroxylase kodierenden Sequenz beispielsweise auch nichtkodierende Sequenzen, wie beispielsweise auch UTRs enthält.

Unter einer RNA, die "mit mindestens einem Teil der Pflanze eigenen endogenen β-Hydroxylase-Promotor-Sequenz identisch ist", ist vorzugsweise gemeint, dass die RNA-Sequenz mit mindestens einem Teil des theoretischen Transkriptes der endogenen β-Hydroxylase-Promotor-Sequenz, also der entsprechenden RNA-Sequenz, identisch ist.

Unter "einem Teil" des Pflanze eigenen endogenen β-Hydroxylase-Transkripts bzw. der Pflanze eigenen endogenen β-Hydroxylase-Promotor-Sequenz werden Teilsequenzen verstanden, die von wenigen Basenpaaren bis hin zu vollständigen Sequenzen des Transkripts bzw. der Promotorssequenz reichen können. Die optimale Länge der Teilsequenzen kann der Fachmann durch Routineversuche leicht ermitteln.

In der Regel beträgt die Länge der Teilsequenzen mindestens 10 Basen und höchstens 2 kb, bevorzugt mindestens 25 Basen und höchstens 1,5 kb, besonders bevorzugt mindestens 50 Basen und höchstens 600 Basen, ganz besonders bevorzugt mindestens 100 Basen und höchstens 500, am meisten bevorzugt mindestens 200 Basen oder mindestens 300 Basen und höchstens 400 Basen.

Vorzugsweise werden die Teilsequenzen so ausgesucht, dass eine möglichst hohe Spezifität erreicht wird und nicht Aktivitäten anderer Enzyme reduziert werden, deren Verminderung nicht erwünscht ist. Es ist daher vorteilhaft für die Teilsequenzen der der endogenen β-Hydroxylase-dsRNA Teile des endogenen β-Hydroxylase Transkripts und/oder Teilsequenzen der endogenen β-Hydroxylase-Promotor-Sequenzen zu wählen, die nicht in anderen Aktivitäten auftreten.

In einer besonders bevorzugten Ausführungsform enthält daher die endogene β-Hydroxylase-dsRNA eine Sequenz, die mit einem Teil des Pflanze eigenen endogenen β-Hydroxylase-Transkripts identisch ist und das 5'-Ende oder das 3'-Ende der Pflanze eigenen Nukleinsäure, codierend eine endogene β-Hydroxylase enthält. Insbesondere sind nichttranslatierte Bereiche im 5' oder 3' des Transkriptes geeignet, selektive Doppel-Strang-Strukturen herzustellen.

Ein weiterer Gegenstand der Erfindung bezieht sich auf doppelsträngige RNA-Moleküle (dsRNA-Moleküle), die bei Einbringen in einen pflanzlichen Organismus (oder eine davon abgeleitete Zelle, Gewebe, Organ oder Vermehrungsmaterial) die Verminderung einer endogenen β-Hydroxylase bewirken.

Ferner betrifft die Erfindung ein doppelsträngiges RNA-Molekül zur Reduzierung der Expression einer endogenen β-Hydroxylase (endogene β-Hydroxylase-dsRNA) umfassend dabei bevorzugt
a) einen "sense"-RNA-Strang umfassend mindestens eine Ribonukleotidsequenz, die im wesentlichen identisch ist zu mindestens einem Teil eines "sense"-RNA-endogene β-Hydroxylase-Transkriptes, und
b) einen "antisense"-RNA-Strang, der zu dem RNA-"sense"-Strang unter a) im wesentlichen, bevorzugt vollständig, komplementären ist.

Zur Transformation der Pflanze mit einer endogenen β-Hydroxylase-dsRNA wird bevorzugt ein Nukleinsäurekonstrukt verwendet, das in die Pflanze eingebracht wird und das in der Pflanze in die endogene β-Hydroxylase-dsRNA transkripiert wird.

Ferner betrifft die vorliegende Erfindung auch ein Nukleinsäurekonstrukt, transkripierbar in
a) einen "sense"-RNA-Strang umfassend mindestens eine Ribonukleotidsequenz, die im wesentlichen identisch ist zu mindestens einem Teil des "sense"-RNA-endogene β-Hydroxylase Transkriptes, und
b) einen "antisense"-RNA-Strang, der zu dem RNA-sense-Strang unter a) im wesentlichen - bevorzugt vollständig - komplementär ist.

Diese Nukleinsäurekonstrukte werden im folgenden auch Expressionskassetten oder Expressionsvektoren genannt.

In Bezug auf die dsRNA-Moleküle wird unter der endogenen β-Hydroxylase Nukleinsäuresequenz, bzw. das entsprechende Transkript bevorzugt die Sequenz gemäß SEQ ID NO: 139 oder ein Teil derselben verstanden.

"Im wesentlichen identisch" meint, dass die dsRNA Sequenz auch Insertionen, Deletionen sowie einzelne Punktmutationen im Vergleich zu der endogenen β-Hydroxylase Zielsequenz aufweisen kann und dennoch eine effizient Verminderung der Expression bewirkt. Bevorzugt beträgt die Homologie mindestens 75 %, bevorzugt mindestens 80 %, ganz besonders bevorzugt mindestens 90 % am meisten bevorzugt 100 % zwischen dem "sense"-Strang einer inhibitorischen dsRNA und mindestens einem Teil des "sense"-RNA-Transkriptes eines endogenen β-Hydroxylase-Gens, bzw. zwischen dem "antisense"-Strang dem komplementären Strang eines endogenen β-Hydroxylase-Gens.

Eine 100%ige Sequenzidentität zwischen dsRNA und einem endogenen β-Hydroxylase Gentranskript ist nicht zwingend erforderlich, um eine effiziente Verminderung der endogenen β-Hydroxylase Expression zu bewirken. Demzufolge besteht der Vorteil, dass das Verfahren tolerant ist gegenüber Sequenzabweichungen, wie sie infolge genetischer Mutationen, Polymorphismen oder evolutionärer Divergenzen vorliegen können. So ist es beispielsweise möglich mit der dsRNA, die ausgehend von der endogenen β-Hydroxylase Sequenz des einen Organismus generiert wurde, die endogene β-Hydroxylase Expression in einem anderen Organismus zu unterdrücken. Zu diesem Zweck umfasst die dsRNA bevorzugt Sequenzbereiche von endogenen β-Hydroxylase-Gentranskripten, die konservierten Bereichen entsprechen. Besagte konservierte Bereiche können aus Sequenzvergleichen leicht abgeleitet werden.

Alternativ, kann eine "im wesentlichen identische" dsRNA auch als Nukleinsäuresequenz definiert werden, die befähigt ist, mit einem Teil eines endogenen β-Hydroxylase Gentranskriptes zu hybridisieren (z.B. in 400 mM NaCl, 40 mM PIPES pH 6,4, 1 mM EDTA bei 50°C oder 70°C für 12 bis 16 h).

"Im wesentlichen komplementär" meint, dass der "antisense"-RNA-Strang auch Insertionen, Deletionen sowie einzelne Punktmutationen im Vergleich zu dem Komplement des "sense"-RNA-Stranges aufweisen kann. Bevorzugt beträgt die Homologie mindestens 80 %, bevorzugt mindestens 90 %, ganz besonders bevorzugt mindestens 95 %, am meisten bevorzugt 100 % zwischen dem "antisense"-RNA-Strang und dem Komplement des "sense"-RNA-Stranges.

In einer weiteren Ausführungsform umfasst die endogene β-Hydroxylase-dsRNA
a) einen "sense"-RNA-Strang umfassend mindestens eine Ribonukleotidsequenz, die im wesentlichen identisch ist zu mindestens einem Teil des "sense"-RNA-Transkriptes des Promotorbereichs eines endogenen β-Hydroxylase-Gens, und
b) einen "antisense"-RNA-Strang, der zu dem RNA-"sense"-Strang unter a) im wesentlichen - bevorzugt vollständig - komplementären ist.

Das entsprechende, bevorzugt zur Transformation der Pflanzen zu verwendende, Nukleinsäurekonstrukt, umfasst
a) einen "sense"-DNA-Strang der im wesentlichen identisch ist zu mindestens einem Teil des Promotorbereichs eines endogenen β-Hydroxylase-Gens, und
b) einen "antisense"-DNA-Strang, der zu dem DNA-"sense"-Strang unter a) im wesentlichen - bevorzugt vollständig - komplementär ist.

Zur Herstellung der endogenen β-Hydroxylase-Sequenzen zur Reduzierung der endogenen β-Hydroxylase-Aktivität werden, insbesondere für *Tagetes erecta,* besonders bevorzugt die folgenden Teil-Sequenzen verwendet:
SEQ ID NO: 141:Sense-Fragment der 5'terminalen Region der endogenen β-Hydroxylase
SEQ ID NO: 142:Antisense-Fragment der 5'terminalen Region der endogenen β-Hydroxylase

Die dsRNA kann aus einem oder mehr Strängen von Polyribonukleotiden bestehen. Natürlich können, um den gleichen Zweck zu erreichen, auch mehrere individuelle dsRNA Moleküle, die jeweils einen der oben definierten Ribonukleotidsequenzabschnitte umfassen, in die Zelle oder den Organismus eingebracht werden.

Die doppelsträngige dsRNA-Struktur kann ausgehend von zwei komplementären, separaten RNA-Strängen oder - bevorzugt - ausgehend von einem einzelnen, selbstkomplementären RNA-Strang gebildet werden. In diesem Fall sind "sense"-RNA-Strang und "antisense"-RNA-Strang bevorzugt kovalent in Form eines invertierten "Repeats" miteinander verbunden.

Wie z.B. in WO 99/53050 beschrieben, kann die dsRNA auch eine Haarnadelstruktur umfassen, indem "sense"- und "antisense"-Strang durch eine verbindende Sequenz ("Linker"; beispielsweise ein Intron) verbunden werden. Die selbstkomplementären dsRNA-Strukturen sind bevorzugt, da sie lediglich die Expression einer RNA-Sequenz erfordern und die komplementären RNA-Stränge stets in einem äquimolaren Verhältnis umfassen. Bevorzugt ist die verbindende Sequenz ein Intron (z.B. ein Intron des ST-LS1 Gens aus Kartoffel; Vancanneyt GF et al. (1990) Mol Gen Genet 220(2):245-250).

Die Nukleinsäuresequenz kodierend für eine dsRNA kann weitere Elemente beinhalten, wie beispielsweise Transkriptionsterminationssignale oder Polyadenylierungssignale.

Weitere bevorzugte Ausführungsformen für die Reduzierung der endogenen β-Hydroxylase Aktivität ergeben sich analog der vorstehend beschriebenen, bevorzugten

Ausführungsformen der Reduzierung der ε-Cyclase-Aktivität unter Austausch der e-Cyclase durch endogene β-Hydroxylase.

Besonders bevorzugt werden im erfindungsgemäßen Verfahren genetisch veränderte Pflanzen mit folgende Kombinationen genetischer Veränderungen verwendet:
Genetisch veränderte Pflanzen, die im Vergleich zum Wildtyp eine erhöhte oder verursachte Ketolase-Aktivität in Blütenblättern und eine erhöhte Hydroxylase-Aktivität aufweisen,
genetisch veränderte Pflanzen, die im Vergleich zum Wildtyp eine erhöhte oder verursachte Ketolase-Aktivität in Blütenblättern und eine erhöhte β-Cyclase-Aktivität aufweisen,
genetisch veränderte Pflanzen, die im Vergleich zum Wildtyp eine erhöhte oder verursachte Ketolase-Aktivität in Blütenblättern und eine reduzierte ε-Cyclase-Aktivität aufweisen,
genetisch veränderte Pflanzen, die im Vergleich zum Wildtyp eine erhöhte oder verursachte Ketolase-Aktivität in Blütenblättern und eine erhöhte Hydroxylase-Aktivität und eine erhöhte β-Cyclase-Aktivität aufweisen,
genetisch veränderte Pflanzen, die im Vergleich zum Wildtyp eine erhöhte oder verursachte Ketolase-Aktivität in Blütenblättern und eine erhöhte Hydroxylase-Aktivität und eine reduzierte ε-Cyclase-Aktivität aufweisen,
genetisch veränderte Pflanzen, die im Vergleich zum Wildtyp eine erhöhte oder verursachte Ketolase-Aktivität in Blütenblättern und eine erhöhte β-Cyclase-Aktivität und eine reduzierte ε-Cyclase-Aktivität aufweisen,sowie
genetisch veränderte Pflanzen, die im Vergleich zum Wildtyp eine erhöhte oder verursachte Ketolase-Aktivität in Blütenblättern und eine erhöhte Hydroxylase-Aktivität und eine erhöhte β-Cyclase-Aktivität und eine reduzierte ε-Cyclase-Aktivität aufweisen.
genetisch veränderte Pflanzen, die im Vergleich zum Wildtyp eine erhöhte oder verursachte Ketolase-Aktivität in Blütenblättern, eine reduzierte ε-Cyclase-Aktivität und eine erhöhte β-Cyclase-Aktivität aufweisen,
genetisch veränderte Pflanzen, die im Vergleich zum Wildtyp eine erhöhte oder verursachte Ketolase-Aktivität in Blütenblättern, eine reduzierte ε-Cyclase-Aktivität und eine reduzierte endogene β-Hydroxylase-Aktivität aufweisen,
genetisch veränderte Pflanzen, die im Vergleich zum Wildtyp eine erhöhte oder verursachte Ketolase-Aktivität in Blütenblättern, eine reduzierte ε-Cyclase-Aktivität und eine erhöhte Hydroxylase-Aktivität aufweisen,
genetisch veränderte Pflanzen, die im Vergleich zum Wildtyp eine erhöhte oder verursachte Ketolase-Aktivität in Blütenblättern, eine erhöhte β-Cyclase-Aktivität und eine erhöhte Hydroxylase-Aktivität aufweisen,
genetisch veränderte Pflanzen, die im Vergleich zum Wildtyp eine erhöhte oder verursachte Ketolase-Aktivität in Blütenblättern, eine erhöhte β-Cyclase-Aktivität und eine reduzierte endogene β-Hydroxylase-Aktivität aufweisen,
genetisch veränderte Pflanzen, die im Vergleich zum Wildtyp eine erhöhte oder verursachte Ketolase-Aktivität in Blütenblättern und eine erhöhte β-Cyclase-Aktivität aufweisen,
genetisch veränderte Pflanzen, die im Vergleich zum Wildtyp eine erhöhte oder verursachte Ketolase-Aktivität in Blütenblättern, eine reduzierte e-Cyclase-Aktivität und mindestens eine weitere erhöhte Aktivität, ausgewählt aus der Gruppe HMG-CoA-Reduktase-Aktivität, (E)-4-Hydroxy-3-Methylbut-2-enyl-Diphosphat-Reduktase-Aktivität, 1-Deoxy-D-Xylose-5-Phosphat-Synthase-Aktivität, 1-Deoxy-D-Xylose-5-Phosphat-Reduktoisomerase-Aktivität, Isopentenyl-Diphosphat-D-Isomerase-Aktivität, Geranyl-Diphosphat-Synthase-Aktivität, Farnesyl-Diphosphat-Synthase-Aktivität, Geranyl-Geranyl-Diphosphat-Synthase-Aktivität, Phytoen-Synthase-Aktivität, Phytoen-Desaturase-Aktivität, Zeta-Carotin-Desaturase-Aktivität, crtISO-Aktivität, FtsZ-Aktivität und MinD-Aktivität aufweisen.
genetisch veränderte Pflanzen, die im Vergleich zum Wildtyp eine erhöhte oder verursachte Ketolase-Aktivität in Blütenblättern, eine reduzierte ε-Cyclase-Aktivität, eine erhöhte β-Cyclase-Aktivität und eine erhöhte Hydroxylase-Aktivität aufweisen,
genetisch veränderte Pflanzen, die im Vergleich zum Wildtyp eine erhöhte oder verursachte Ketolase-Aktivität in Blütenblättern, eine reduzierte ε-Cyclase-Aktivität, eine erhöhte β-Cyclase-Aktivität und eine reduzierte endogene β-Hydroxylase-Aktivität aufweisen,
genetisch veränderte Pflanzen, die im Vergleich zum Wildtyp eine erhöhte oder verursachte Ketolase-Aktivität in Blütenblättern, eine reduzierte ε-Cyclase-Aktivität und eine erhöhte β-Cyclase-Aktivität aufweisen,
genetisch veränderte Pflanzen, die im Vergleich zum Wildtyp eine erhöhte oder verursachte Ketolase-Aktivität in Blütenblättern, eine reduzierte ε-Cyclase-Aktivität und eine erhöhte Hydroxylase-Aktivität aufweisen,
genetisch veränderte Pflanzen, die im Vergleich zum Wildtyp eine erhöhte oder verursachte Ketolase-Aktivität in Blütenblättern, eine reduzierte ε-Cyclase-Aktivität und eine reduzierte endogene β-Hydroxylase-Aktivität aufweisen.
genetisch veränderte Pflanzen, die im Vergleich zum Wildtyp eine erhöhte oder verursachte Ketolase-Aktivität in Blütenblättern, eine reduzierte ε-Cyclase-Aktivität, eine erhöhte Hydroxylase-Aktivität und eine reduzierte endogene β-Hydroxylase-Aktivität aufweisen,
genetisch veränderte Pflanzen, die im Vergleich zum Wildtyp eine erhöhte oder verursachte Ketolase-Aktivität in Blütenblättern, eine erhöhte β-Cyclase-Aktivität, eine erhöhte Hydroxylase-Aktivität und eine reduzierte endogene β-Hydroxylase-Aktivität aufweisen,
genetisch veränderte Pflanzen, die im Vergleich zum Wildtyp eine erhöhte oder verursachte Ketolase-Aktivität in Blütenblättern, eine reduzierte e-Cyclase-Aktivität, eine erhöhte b-Cyclase-Aktivität und mindestens eine weitere erhöhte Aktivität, ausgewählt aus der Gruppe HMG-CoA-Reduktase-Aktivität, (E)-4-Hydroxy-3-Methylbut-2-enyl-Diphosphat-Reduktase-Aktivität, 1-Deoxy-D-Xylose-5-Phosphat-Synthase-Aktivität, 1-Deoxy-D-Xylose-5-Phosphat-Reduktoisomerase-Aktivität, Isopentenyl-Diphosphat-D-Isomerase-Aktivität, Geranyl-Diphosphat-Synthase-Aktivität, Farnesyl-Diphosphat-Synthase-Aktivität, Geranyl-Geranyl-Diphosphat-Synthase-Aktivität, Phytoen-Synthase-Aktivität, Phytoen-Desaturase-Aktivität, Zeta-Carotin-Desaturase-Aktivität, crtISO-Aktivität, FtsZ-Aktivität und MinD-Aktivität aufweisen.
genetisch veränderte Pflanzen, die im Vergleich zum Wildtyp eine erhöhte oder verursachte Ketolase-Aktivität in Blütenblättern, eine reduzierte ε-Cyclase-Aktivität, eine erhöhte β-Cyclase-Aktivität, eine erhöhte Hydroxylase-Aktivität und eine reduzierte endogene β-Hydroxylase-Aktivität aufweisen,
genetisch veränderte Pflanzen, die im Vergleich zum Wildtyp eine erhöhte oder verursachte Ketolase-Aktivität in Blütenblättern, eine reduzierte ε-Cyclase-Aktivität, eine erhöhte β-Cyclase-Aktivität und eine erhöhte Hydroxylase-Aktivität aufweisen,
genetisch veränderte Pflanzen, die im Vergleich zum Wildtyp eine erhöhte oder verursachte Ketolase-Aktivität in Blütenblättern, eine reduzierte ε-Cyclase-Aktivität, eine erhöhte β-Cyclase-Aktivität und eine reduzierte endogene β-Hydroxylase-Aktivität aufweisen,
genetisch veränderte Pflanzen, die im Vergleich zum Wildtyp eine erhöhte oder verursachte Ketolase-Aktivität in Blütenblättern, eine reduzierte e-Cyclase-Aktivität, eine erhöhte Hydroxylase-Aktivität und mindestens eine weitere erhöhte Aktivität, ausgewählt aus der Gruppe HMG-CoA-Reduktase-Aktivität, (E)-4-Hydroxy-3-Methylbut-2-enyl-Diphosphat-Reduktase-Aktivität, 1-Deoxy-D-Xylose-5-Phosphat-Synthase-Aktivität, 1-Deoxy-D-Xylose-5-Phosphat-Reduktoisomerase-Aktivität, Isopentenyl-Diphosphat-D-Isomerase-Aktivität, Geranyl-Diphosphat-Synthase-Aktivität, Farnesyl-Diphosphat-Synthase-Aktivität, Geranyl-Geranyl-Diphosphat-Synthase-Aktivität, Phytoen-Synthase-Aktivität, Phytoen-Desaturase-Aktivität, Zeta-Carotin-Desaturase-Aktivität, crtISO-Aktivität, FtsZ-Aktivität und MinD-Aktivität aufweisen,
genetisch veränderte Pflanzen, die im Vergleich zum Wildtyp eine erhöhte oder verursachte Ketolase-Aktivität in Blütenblättern, eine reduzierte e-Cyclase-Aktivität, eine reduzierte endogene b-Hydroxylase-Aktivität und mindestens eine weitere erhöhte Aktivität, ausgewählt aus der Gruppe HMG-CoA-Reduktase-Aktivität, (E)-4-Hydroxy-3-Methylbut-2-enyl-Diphosphat-Reduktase-Aktivität, 1-Deoxy-D-Xylose-5-Phosphat-Synthase-Aktivität, 1-Deoxy-D-Xylose-5-Phosphat-Reduktoisomerase-Aktivität, lsopentenyl-Diphosphat-D-Isomerase-Aktivität, Geranyl-Diphosphat-Synthase-Aktivität, Farnesyl-Diphosphat-Synthase-Aktivität, Geranyl-Geranyl-Diphosphat-Synthase-Aktivität, Phytoen-Synthase-Aktivität, Phytoen-Desaturase-Aktivität, Zeta-Carotin-Desaturase-Aktivität, crtISO-Aktivität, FtsZ-Aktivität und MinD-Aktivität aufweisen,
genetisch veränderte Pflanzen, die im Vergleich zum Wildtyp eine erhöhte oder verursachte Ketolase-Aktivität in Blütenblättern, eine erhöhte b-Cyclase-Aktivität, eine erhöhte Hydroxylase-Aktivität und mindestens eine weitere erhöhte Aktivität, ausgewählt aus der Gruppe HMG-CoA-Reduktase-Aktivität, (E)-4-Hydroxy-3-Methylbut-2-enyl-Diphosphat-Reduktase-Aktivität, 1-Deoxy-D-Xylose-5-Phosphat-Synthase-Aktivität, 1-Deoxy-D-Xylose-5-Phosphat-Reduktoisomerase-Aktivität, Isopentenyl-Diphosphat-Δ-Isomerase-Aktivität, Geranyl-Diphosphat-Synthase-Aktivität, Farnesyl-Diphosphat-Synthase-Aktivität, Geranyl-Geranyl-Diphosphat-Synthase-Aktivität, Phytoen-Synthase-Aktivität, Phytoen-Desaturase-Aktivität, Zeta-Carotin-Desaturase-Aktivität, crtISO-Aktivität, FtsZ-Aktivität und MinD-Aktivität aufweisen,
genetisch veränderte Pflanzen, die im Vergleich zum Wildtyp eine erhöhte oder verursachte Ketolase-Aktivität in Blütenblättern, eine erhöhte b-Cyclase-Aktivität, eine reduzierte endogene b-Hydroxylase-Aktivität und mindestens eine weitere erhöhte Aktivität, ausgewählt aus der Gruppe HMG-CoA-Reduktase-Aktivität, (E)-4-Hydroxy-3-Methylbut-2-enyl-Diphosphat-Reduktase-Aktivität, 1-Deoxy-D-Xylose-5-Phosphat-Synthase-Aktivität, 1-Deoxy-D-Xylose-5-Phosphat-Reduktoisomerase-Aktivität, Isopentenyl-Diphosphat-D-Isomerase-Aktivität, Geranyl-Diphosphat-Synthase-Aktivität, Farnesyl-Diphosphat-Synthase-Aktivität, Geranyl-Geranyl-Diphosphat-Synthase-Aktivität, Phytoen-Synthase-Aktivität, Phytoen-Desaturase-Aktivität, Zeta-Carotin-Desaturase-Aktivität, crtISO-Aktivität, FtsZ-Aktivität und MinD-Aktivität aufweisen,
genetisch veränderte Pflanzen, die im Vergleich zum Wildtyp eine erhöhte oder verursachte Ketolase-Aktivität in Blütenblättern, eine reduzierte ε-Cyclase-Aktivität, eine erhöhte β-Cyclase-Aktivität und eine erhöhte Hydroxylase-Aktivität und eine reduzierte b-Hydroxylase-Aktivität aufweisen,
genetisch veränderte Pflanzen, die im Vergleich zum Wildtyp eine erhöhte oder verursachte Ketolase-Aktivität in Blütenblättern, eine reduzierte e-Cyclase-Aktivität, eine erhöhte b-Cyclase-Aktivität,eine erhöhte Hydroxylase-Aktivität und mindestens eine weitere erhöhte Aktivität, ausgewählt aus der Gruppe HMG-CoA-Reduktase-Aktivität, (E)-4-Hydroxy-3-Methylbut-2-enyl-Diphosphat-Reduktase-Aktivität, 1-Deoxy-D-Xylose-5-Phosphat-Synthase-Aktivität, 1-Deoxy-D-Xylose-5-Phosphat-Reduktoisomerase-Aktivität, Isopentenyl-Diphosphat-D-Isomerase-Aktivität, Geranyl-Diphosphat-Synthase-Aktivität, Farnesyl-Diphosphat-Synthase-Aktivität, Geranyl-Geranyl-Diphosphat-Synthase-Aktivität, Phytoen-Synthase-Aktivität, Phytoen-Desaturase-Aktivität, Zeta-Carotin-Desaturase-Aktivität, crtISO-Aktivität, FtsZ-Aktivität und MinD-Aktivität aufweisen,
genetisch veränderte Pflanzen, die im Vergleich zum Wildtyp eine erhöhte oder verursachte Ketolase-Aktivität in Blütenblättern, eine reduzierte e-Cyclase-Aktivität, eine erhöhte b-Cyclase-Aktivität, eine reduzierte endogene b-Hydroxylase-Aktivität und mindestens eine weitere erhöhte Aktivität, ausgewählt aus der Gruppe HMG-CoA-Reduktase-Aktivität, (E)-4-Hydroxy-3-Methylbut-2-enyl-Diphosphat-Reduktase-Aktivität, 1-Deoxy-D-Xylose-5-Phosphat-Synthase-Aktivität, 1-Deoxy-D-Xylose-5-Phosphat-Reduktoisomerase-Aktivität, Isopentenyl-Diphosphat-D-Isomerase-Aktivität, Geranyl-Diphosphat-Synthase-Aktivität, Farnesyl-Diphosphat-Synthase-Aktivität, Geranyl-Geranyl-Diphosphat-Synthase-Aktivität, Phytoen-Synthase-Aktivität, Phytoen-Desaturase-Aktivität, Zeta-Carotin-Desaturase-Aktivität, crtISO-Aktivität, FtsZ-Aktivität und MinD-Aktivität aufweisen.

Besonders bevorzugte, genetisch veränderte Pflanzen, weisen im Vergleich zum Wildtyp eine erhöhte oder verursachte Ketolase-Aktivität in Blütenblättern, eine erhöhte β-Cyclase-Aktivität und eine erhöhte Hydroxylase-Aktivität auf, wobei
die erhöhte Ketolase Aktivität dadurch verursacht wird, dass man Nukleinsäuren einbringt, die ein Protein kodieren, enthaltend die Aminosäuresequenz SEQ ID NO: 2 oder eine von dieser Sequenz durch Substitution, Insertion oder Deletion von Aminosäuren abgeleitete Sequenz, die eine Identität von mindestens 20 % auf Aminosäureebene mit der Sequenz SEQ ID NO: 2 und die enzymatische Eigenschaft einer Ketolase aufweist,
die erhöhte β-Cyclase-Aktivität dadurch verursacht wird, dass man Nukleinsäure einbringt, kodierend eine β-Cyclase, enthaltend die Aminosäuresequenz SEQ ID NO: 110 oder eine von dieser Sequenz durch Substitution, Insertion oder Deletion von Aminosäuren abgeleitete Sequenz, die eine Identität von mindestens 20 % auf Aminosäureebene mit der Sequenz SEQ ID NO: 110 aufweist
und die erhöhte Hydroxylase-Aktivität dadurch verursacht wird, dass man Nukleinsäuren einbringt, kodierend eine Hydroxylase, enthaltend die Aminosäuresequenz SEQ ID NO: 108 oder eine von dieser Sequenz durch Substitution, Insertion oder Deletion von Aminosäuren abgeleitete Sequenz, die eine Identität von mindestens 20 % auf Aminosäureebene mit der Sequenz SEQ ID NO: 108 aufweist.

Besonders bevorzugte, genetisch veränderte Pflanzen, weisen im Vergleich zum Wildtyp eine erhöhte oder verursachte Ketolase-Aktivität in Blütenblättern, eine reduzierte ε-Cyclase-Aktivität, eine erhöhte β-Cyclase-Aktivität, eine erhöhte Hydroxylase-Aktivität und eine reduzierte endogene β-Hydroxylase-Aktivität auf, wobei
die erhöhte Ketolase Aktivität dadurch verursacht wird, dass man Nukleinsäuren einbringt, die ein Protein kodieren, enthaltend die Aminosäuresequenz SEQ ID NO: 2 oder eine von dieser Sequenz durch Substitution, Insertion oder Deletion von Aminosäuren abgeleitete Sequenz, die eine Identität von mindestens 20 % auf Aminosäureebene mit der Sequenz SEQ ID NO: 2 und die enzymatische Eigenschaft einer Ketolase aufweist,
die erhöhte β-Cyclase-Aktivität dadurch verursacht wird, dass man Nukleinsäure einbringt, kodierend eine β-Cyclase, enthaltend die Aminosäuresequenz SEQ ID NO: 110 oder eine von dieser Sequenz durch Substitution, Insertion oder Deletion von Aminosäuren abgeleitete Sequenz, die eine Identität von mindestens 20 % auf Aminosäureebene mit der Sequenz SEQ ID NO: 100 aufweist,
die erhöhte Hydroxylase-Aktivität dadurch verursacht wird, dass man Nukleinsäuren einbringt, kodierend eine Hydroxylase, enthaltend die Aminosäuresequenz SEQ ID NO: 108 oder eine von dieser Sequenz durch Substitution, Insertion oder Deletion von Aminosäuren abgeleitete Sequenz, die eine Identität von mindestens 20 % auf Aminosäureebene mit der Sequenz SEQ ID NO: 108 aufweist, und die reduzierte ε-Cyclase-Aktivität und eine reduzierte endogene β-Hydroxylase-Aktivität gemäß den vorstehend beschriebenen, bevorzugten Ausführungsformen verursacht wird.

Die Herstellung dieser genetisch veränderten Pflanzen der Gattung Tagetes kann, wie nachstehend beschrieben, beispielsweise durch Einbringen einzelner Nukleinsäurekonstrukte (Expressionskassetten) oder durch Einbringen von Mehrfachkonstrukten erfolgen, die bis zu zwei, drei oder vier der beschriebenen Aktivitäten enthalten.

Im folgenden wird exemplarisch die Herstellung genetisch veränderter Pflanzen mit erhöhter oder verursachter Ketolase-Aktivität in Blütenblättern beschrieben. Die Erhöhung weiterer Aktivitäten, wie beispielsweise der Hydroxylase-Aktivität und/oder der β-Cyclase-Aktivität und/oder der HMG-CoA-Reduktase-Aktivität und/oder der (E)-4-Hydroxy-3-Methylbut-2-enyl-Diphosphat-Reduktase-Aktivität und/oder der 1-Deoxy-D-Xylose-5-Phosphat-Synthase-Aktivität und/oder der 1-Deoxy-D-Xylose-5-Phosphat-Reduktoisomerase-Aktivität und/oder der Isopentenyl-Diphosphat-D-Isomerase-Aktivität und/oder der Granyl-Diphosphat-Synthase-Aktivität und/oder der Farnesyl-Diphospaht-Synthase-Aktivität und/oder der Geranyl-geranyl-Diphosphat-Synthase-Aktivität und/oder der Phytoen-Synthase-Aktivität und/oder der Phytoen-Desaturase-Aktivität und/oder der Zeta-Carotin-Desaturase-Aktivität und/oder der crtISO-Aktivität und/oder der FtsZ-Aktivität und/oder der MinD-Aktivität kann analog unter Verwendung von Nukleinsäuresequenzen kodierend eine Hydroxylase bzw. β-Cyclase bzw. Nukleinsäuren kodierend eine HMG-CoA-Reduktase und/oder Nukleinsäuren kodierend eine (E)-4-Hydroxy-3-Methylbut-2-enyl-Diphosphat-Reduktase und/oder Nukleinsäuren kodierend eine 1-Deoxy-D-Xylose-5-Phosphat-Synthase und/oder Nukleinsäuren kodierend eine 1-Deoxy-D-Xylose-5-Phosphat-Reduktoisomerase und/oder Nukleinsäuren kodierend eine Isopentenyl-Diphosphat-D-Isomerase und/oder Nukleinsäuren kodierend eine Geranyl-Diphosphat-Synthase und/oder Nukleinsäuren kodierend eine Farnesyl-Diphosphat-Synthase und/oder Nukleinsäuren kodierend eine Geranyl-geranyl-Diphosphat-Synthase und/oder Nukleinsäuren kodierend eine Phytoen-Synthase und/oder Nukleinsäuren kodierend eine Phytoen-Desaturase und/oder Nukleinsäuren kodierend eine Zeta-Carotin-Desaturase und/oder Nukleinsäuren kodierend ein crtlso-Protein und/oder Nukleinsäuren kodierend ein FtsZ-Protein und/oder Nukleinsäuren kodierend ein MinD-Protein anstelle von Nukleinsäuresequenzen kodierend eine Ketolase erfolgen. Die Reduzierung weiterer Aktivitäten, wie beispielsweise die Reduzierung der ε-Cyclase-Aktivität bzw. der endogenen b-Hydroxylase-Aktivität kann analog unter Verwendung von anti-ε-Cyclase-Nukleinsäuresequenzen oder ε-Cyclase-Inverted-Repaet-Nukleinsäuresequenz bzw. unter Verwendung von anti-endogenen b-Hydroxylase-Nukleinsäuresequenzen oder endogenen b-Hydroxylase-Inverted-Repeat-Nukleinsäuresequenzen anstelle von Nukleinsäuresequenzen kodierend eine Ketolase erfolgen. Die Transformation kann bei den Kombinationen von genetischen Veränderungen einzeln oder durch Mehrfachkonstrukte erfolgen.

Die Herstellung der transgenen Pflanzen der Gattung Tagetes erfolgt vorzugsweise durch Transformation der Ausgangspflanzen, mit einem Nukleinsäurekonstrukt, das die vorstehend beschriebenen Nukleinsäuren codierend eine Ketolase enthält, die mit einem oder mehreren Regulationssignalen funktionell verknüpft sind, die die Transkription und Translation in Pflanzen gewährleisten.

Diese Nukleinsäurekonstrukte, in denen die kodierende Nukleinsäuresequenz mit einem oder mehreren Regulationssignalen funktionell verknüpft sind, die die Transkription und Translation in Pflanzen gewährleisten, werden im folgenden auch Expressionskassetten genannt.

Vorzugsweise enthalten die Regulationssignale einen oder mehrere Promotoren, die die Transkription und Translation in Pflanzen gewährleisten.

Die Expressionskassetten beinhalten Regulationssignale, also regulative Nukleinsäuresequenzen, welche die Expression der kodierenden Sequenz in der Wirtszelle steuern. Gemäß einer bevorzugten Ausführungsform umfasst eine Expressionskassette stromaufwärts, d.h. am 5'-Ende der kodierenden Sequenz, einen Promotor und stromabwärts, d.h. am 3'-Ende, ein Polyadenylierungssignal und gegebenenfalls weitere regulatorische Elemente, welche mit der dazwischenliegenden kodierenden Sequenz für mindestens eines der vorstehend beschriebenen Gene operativ verknüpft sind. Unter einer operativen Verknüpfung versteht man die sequenzielle Anordnung von Promotor, kodierender Sequenz, Terminator und ggf. weiterer regulativer Elemente derart, das jedes der regulativen Elemente seine Funktion bei der Expression der kodierenden Sequenz bestimmungsgemäß erfüllen kann.

Im folgenden werden beispielhaft die bevorzugten Nukleinsäurekonstrukte, Expressionskassetten und Vektoren für Pflanzen der Gattung Tagetes und Verfahren zur Herstellung von transgenen Pflanzen der Gattung Tagetes, sowie die transgenen Pflanzen der Gattung Tagetes selbst beschrieben.

Die zur operativen Verknüpfung bevorzugten aber nicht darauf beschränkten Sequenzen sind Targeting-Sequenzen zur Gewährleistung der subzellulären Lokalisation im Apoplasten, in der Vakuole, in Plastiden, im Mitochondrium, im Endoplasmatischen Retikulum (ER), im Zellkern, in Ölkörperchen oder anderen Kompartimenten und Translationsverstärker wie die 5'-Führungssequenz aus dem Tabak-Mosaik-Virus (Gallie et al., Nucl. Acids Res. 15 (1987), 8693 -8711).

Als Promotoren der Expressionskassette ist grundsätzlich jeder Promotor geeignet, der die Expression von Fremdgenen in Pflanzen steuern kann.

"Konstitutiver" Promotor meint solche Promotoren, die eine Expression in zahlreichen, bevorzugt allen, Geweben über einen größeren Zeitraum der Pflanzenentwicklung, bevorzugt zu allen Zeitpunkten der Pflanzenentwicklung, gewährleisten.

Vorzugsweise verwendet man insbesondere einen pflanzlichen Promotor oder einen Promotor, der einem Pflanzenvirus entstammt. Insbesondere bevorzugt ist der Promotor des 35S-Transkriptes des CaMV Blumenkohlmosaikvirus (Franck et al. (1980) Cell 21:285-294; Odell et al. (1985) Nature 313:810-812; Shewmaker et al. (1985) Virology 140:281-288; Gardner et al. (1986) Plant Mol Biol 6:221-228) oder der 19S CaMV Promotor (US 5,352,605; WO 84/02913; Benfey et al. (1989) EMBO J 8:2195-2202).

Ein weiterer geeigneter konstitutiver Promotor ist der pds Promoter (Pecker et al. (1992) Proc. Natl. Acad. Sci USA 89: 4962-4966) oder der "Rubisco small subunit (SSU)"-Promotor (US 4,962,028), der LeguminB-Promotor (GenBank Acc.-Nr. X03677), der Promotor der Nopalinsynthase aus Agrobacterium, der TR-Doppelpromotor, der OCS (Octopin Synthase) Promotor aus Agrobacterium, der Ubiquitin Promotor (Holtorf S et al. (1995) Plant Mol Biol 29:637-649), den Ubiquitin 1 Promotor (Christensen et al. (1992) Plant Mol Biol 18:675-689; Bruce et al. (1989) Proc Natl Acad Sci USA 86:9692-9696), den Smas Promotor, den Cinnamylalkoholdehydrogenase-Promotor (US 5,683,439), die Promotoren der vakuolärer ATPase Untereinheiten oder der Promotor eines prolinreichen Proteins aus Weizen (WO 91/13991), der Pnit-Promoter (Y07648.L, Hillebrand et al. (1998), Plant. Mol. Biol. 36, 89-99, Hillebrand et al. (1996), Gene, 170, 197-200) sowie weitere Promotoren von Genen, deren konstitutive Expression in Pflanzen dem Fachmann bekannt ist.

Die Expressionskassetten können auch einen chemisch induzierbaren Promotor enthalten (Übersichtsartikel: Gatz et al. (1997) Annu Rev Plant Physiol Plant Mol Biol 48:89-108), durch den die Expression des Ketolase-Gens in der Pflanze zu einem bestimmten Zeitpunkt gesteuert werden kann. Derartige Promotoren, wie z.B. der PRP1 Promotor (Ward et al. (1993) Plant Mol Biol 22:361-366), durch Salicylsäure induzierbarer Promotor (WO 95/19443), ein durch Benzolsulfonamid-induzierbarer Promotor (EP 0 388 186), ein durch Tetrazyklin-induzierbarer Promotor (Gatz et al. (1992) Plant J 2:397-404), ein durch Abscisinsäure induzierbarer Promotor (EP 0 335 528) bzw. ein durch Ethanol- oder Cyclohexanon-induzierbarer Promotor (WO 93/21334) können ebenfalls verwendet werden.

Ferner sind Promotoren bevorzugt, die durch biotischen oder abiotischen Stress induziert werden wie beispielsweise der pathogen-induzierbare Promotor des PRP1-Gens (Ward et al. (1993) Plant Mol Biol 22:361-366), der hitzeinduzierbare hsp70- oder hsp80-Promoter aus Tomate (US 5,187,267), der kälteinduzierbare alpha-Amylase Promoter aus der Kartoffel (WO 96/12814), der licht-induzierbare PPDK Promotor oder der verwundungsinduzierte pinll-Promoter (EP375091).

Pathogen-induzierbare Promotoren umfassen die von Genen, die infolge eines Pathogenbefalls induziert werden wie beispielsweise Gene von PR-Proteinen, SAR-Proteinen, b-1,3-Glucanase, Chitinase usw. (beispielsweise Redolfi et al. (1983) Neth J Plant Pathol 89:245-254; Uknes, et al. (1992) The Plant Cell 4:645-656; Van Loon (1985) Plant Mol Viral 4:111-116; Marineau et al. (1987) Plant Mol Biol 9:335-342; Matton et al. (1987) Molecular Plant-Microbe Interactions 2:325-342; Somssich et al. (1986) Proc Natl Acad Sci USA 83:2427-2430; Somssich et al. (1988) Mol Gen Genetics 2:93-98; Chen et al. (1996) Plant J 10:955-966; Zhang and Sing (1994) Proc Natl Acad Sci USA 91:2507-2511; Warner, et al. (1993) Plant J 3:191-201; Siebertz et al. (1989) Plant Cell 1:961-968(1989).

Umfasst sind auch verwundungs-induzierbare Promotoren wie der des pinll Gens (Ryan (1990) Ann Rev Phytopath 28:425-449; Duan et al. (1996) Nat Biotech 14:494-498), des wun1 und wun2-Gens (US 5,428,148), des win1- und win2-Gens (Stanford et al. (1989) Mol Gen Genet 215:200-208), des Systemin (McGurl et al. (1992) Science 225:1570-1573), des WIP1-Gens (Rohmeier et al. (1993) Plant Mol Biol 22:783-792; Ekelkamp et al. (1993) FEBS Letters 323:73-76), des MPI-Gens (Corderok et al. (1994) The Plant J 6(2):141-150) und dergleichen.

Weitere geeignete Promotoren sind beispielsweise fruchtreifung-spezifische Promotoren, wie beispielsweise der fruchtreifung-spezifische Promotor aus Tomate (WO 94/21794, EP 409 625). Entwicklungsabhängige Promotoren schließt zum Teil die gewebespezifischen Promotoren ein, da die Ausbildung einzelner Gewebe naturgemäß entwicklungsabhängig erfolgt.

Weiterhin sind insbesondere solche Promotoren bevorzugt, die die Expression in Geweben oder Pflanzenteilen sicherstellen, in denen beispielsweise die Biosynthese von Ketocarotinoiden bzw. dessen Vorstufen stattfindet. Bevorzugt sind beispielsweise Promotoren mit Spezifitäten für die Antheren, Ovarien, Petalen, Sepalen, Blüten, Blätter, Stengel und Wurzeln und Kombinationen hieraus.

Knollen-, Speicherwurzel- oder Wurzel-spezifische Promotoren sind beispielsweise der Patatin Promotor Klasse I (B33) oder der Promotor des Cathepsin D Inhibitors aus Kartoffel.

Blattspezifische Promotoren sind beispielsweise der Promotor der cytosolischen FBPase aus Kartoffel (WO 97/05900), der SSU Promotor (small subunit) der Rubisco (Ribulose-1,5-bisphosphatcarboxylase) oder der ST-LSI Promotor aus Kartoffel (Stockhaus et al. (1989) EMBO J 8:2445-2451).

Blütenspezifische Promotoren sind beispielsweise der Phytoen Synthase Promotor (WO 92/16635) oder der Promotor des P-rr Gens (WO 98/22593) oder der AP3 Promoter aus Arabidopsis thaliana (siehe Beispiel 1).

Antheren-spezifische Promotoren sind beispielsweise der 5126-Promotor (US 5,689,049, US 5,689,051), den glob-I Promotor oder der g-Zein Promotor.

Weitere zur Expression in Pflanzen geeignete Promotoren sind beschrieben in Rogers et al. (1987) Meth in Enzymol 153:253-277; Schardl et al. (1987) Gene 61:1-11 und Berger et al. (1989) Proc Natl Acad Sci USA 86:8402-8406).

Alle in der vorliegenden Anmeldung beschriebenen Promotoren ermöglichen in der Regel die Expression der Ketolase in Blütenblättern der erfindungsgemäßen Pflanzen.

Besonders bevorzugt im erfindungsgemäßen Verfahren sind konstitutive, blütenspezifische und insbesondere blütenblattspezifische Promotoren.

Die Herstellung einer Expressionskassette erfolgt vorzugsweise durch Fusion eines geeigneten Promotors mit einer vorstehend beschriebenen Nukleinsäure kodierend eine Ketolase und vorzugsweise einer zwischen Promotor und Nukleinsäure-Sequenz inserierten Nukleinsäure, die für ein plastidenspezifisches Transitpeptid kodiert, sowie einem Polyadenylierungssignal nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley-Interscience (1987) beschrieben sind.

Die vorzugsweise insertierte Nukleinsäuren kodierend ein plastidäres Transitpeptid, gewährleisten die Lokalisation in Plastiden und insbesondere in Chromoplasten.

Es können auch Expressionskassetten verwendet werden, deren Nukleinsäure-Sequenz für ein Ketolase-Fusionsprotein kodiert, wobei ein Teil des Fusionsproteins ein Transitpeptid ist, das die Translokation des Polypeptides steuert. Bevorzugt sind für die Chromoplasten spezifische Transitpeptide, welche nach Translokation der Ketolase in die Chromoplasten vom Ketolase-Teil enzymatisch abgespalten werden.

Insbesondere bevorzugt ist das Transitpeptid, das von der plastidären *Nicotiana tabacum* Transketolase oder einem anderen Transitpeptid (z.B. dem Transitpeptid der kleinen Untereinheit der Rubisco (rbcS) oder der Ferredoxin NADP Oxidoreduktase als auch der lsopentenylpyrophosphat Isomerase-2) oder dessen funktionellem Äquivalent abgeleitet ist.

Besonders bevorzugt sind Nukleinsäure-Sequenzen von drei Kassetten des Plastiden-Transitpeptids der plastidären Transketolase aus Tabak in drei Leserastern als Kpnl/BamHI Fragmente mit einem ATG-Codon in der Ncol Schnittstelle:
pTP09
pTP10
pTP11

Weitere Beispiele für ein plastidäres Transitpeptid sind das Transitpeptid der plastidären Isopentenyl-pyrophosphat Isomerase-2 (IPP-2) aus Arabisopsis thaliana und das Transitpeptid der kleinen Untereinheit der Ribulosebisphosphat Carboxylase (rbcS) aus Erbse (Guerineau, F, Woolston, S, Brooks, L, Mullineaux, P (1988) An expression cassette for targeting foreign proteins into the chloroplasts. Nucl. Acids Res. 16: 11380).

Die erfindungsgemäßen Nukleinsäuren können synthetisch hergestellt oder natürlich gewonnen sein oder eine Mischung aus synthetischen und natürlichen Nukleinsäure-Bestandteilen enthalten, sowie aus verschiedenen heterologen Genabschnitten verschiedener Organismen bestehen.

Bevorzugt sind, wie vorstehend beschrieben, synthetische Nukleotid-Sequenzen mit Kodons, die von Pflanzen der Gattung Tagetes bevorzugt werden. Diese von Pflanzen bevorzugten Kodons können aus Kodons mit der höchsten Proteinhäufigkeit bestimmt werden, die in den meisten interessanten Pflanzenspezies exprimiert werden.

Bei der Präparation einer Expressionskassette können verschiedene DNA-Fragmente manipuliert werden, um eine Nukleotid-Sequenz zu erhalten, die zweckmäßigerweise in der korrekten Richtung liest und die mit einem korrekten Leseraster ausgestattet ist. Für die Verbindung der DNA-Fragmente miteinander können an die Fragmente Adaptoren oder Linker angesetzt werden.

Zweckmäßigerweise können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, meistens 1 bis 8, vorzugsweise 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zur Wirtspflanze sein. Die Expressionskassette beinhaltet vorzugsweise in der 5'-3'-Transkriptionsrichtung den Promotor, eine kodierende Nukleinsäuresequenz oder ein Nukleinsäurekonstrukt und eine Region für die transkriptionale Termination. Verschiedene Terminationsbereiche sind gegeneinander beliebig austauschbar.

Beispiele für einen Terminator sind der 35S-Terminator (Guerineau et al. (1988) Nucl Acids Res. 16: 11380), der nos Terminator (Depicker A, Stachel S, Dhaese P, Zambryski P, Goodman HM. Nopaline synthase: transcript mapping and DNA sequence. J Mol Appl Genet. 1982;1(6):561-73) oder der ocs Terminator (Gielen, J, de Beuckeleer, M, Seurinck, J, Debroek, H, de Greve, H, Lemmers, M, van Montagu, M, Schell, J (1984) The complete sequence of the TL-DNA of the Agrobacterium tumefaciens plasmid pTiAch5. EMBO J. 3: 835-846).

Ferner können Manipulationen, die passende Restriktionsschnittstellen bereitstellen oder die überflüssige DNA oder Restriktionsschnittstellen entfernen, eingesetzt werden. Wo Insertionen, Deletionen oder Substitutionen wie z.B. Transitionen und Transversionen in Frage kommen, können *in vitro*-Mutagenese, "primer-repair", Restriktion oder Ligation verwendet werden.

Bei geeigneten Manipulationen, wie z.B. Restriktion, "chewing-back" oder Auffüllen von Überhängen für "bluntends", können komplementäre Enden der Fragmente für die Ligation zur Verfügung gestellt werden.

Bevorzugte Polyadenylierungssignale sind pflanzliche Polyadenylierungssignale, vorzugsweise solche, die im wesentlichen T-DNA-Polyadenylierungssignale aus Agrobacterium tumefaciens, insbesondere des Gens 3 der T-DNA (Octopin Synthase) des Ti-Plasmids pTiACH5 entsprechen (Gielen et al., EMBO J. 3 (1984), 835 ff) oder funktionelle Äquivalente.

Die Übertragung von Fremdgenen in das Genom einer Pflanze wird als Transformation bezeichnet.

Dazu können an sich bekannte Methoden zur Transformation und Regeneration von Pflanzen aus Pflanzengeweben oder Pflanzenzellen zur transienten oder stabilen Transformation genutzt werden.

Geeignete Methoden zur Transformation von Pflanzen sind die Protoplastentransformation durch Polyethylenglykol-induzierte DNA-Aufnahme, das biolistische Verfahren mit der Genkanone - die sogenannte particle bombardment Methode, die Elektroporation, die Inkubation trockener Embryonen in DNA-haltiger Lösung, die Mikroinjektion und der, vorstehend beschriebene, durch Agrobacterium vermittelte Gentransfer. Die genannten Verfahren sind beispielsweise in B. Jenes et al., Techniques for Gene Transfer, in: Transgenic Plants, Vol. 1, Engineering and Utilization, herausgegeben von S.D. Kung und R. Wu, Academic Press (1993), 128-143 sowie in Potrykus, Annu. Rev. Plant Physiol. Plant Molec. Biol. 42 (1991), 205-225) beschrieben.

Vorzugsweise wird das zu exprimierende Konstrukt in einen Vektor kloniert, der geeignet ist, Agrobacterium tumefaciens zu transformieren, beispielsweise pBin19 (Bevan et al., Nucl. Acids Res. 12 (1984), 8711) oder besonders bevorzugt pSUN2, pSUN3, pSUN4 oder pSUN5 (WO 02/00900).

Mit einem Expressionsplasmid transformierte Agrobakterien können in bekannter Weise zur Transformation von Pflanzen verwendet werden, z.B. indem verwundete Blätter oder Blattstücke in einer Agrobakterienlösung gebadet und anschließend in geeigneten Medien kultiviert werden.

Zur bevorzugten Herstellung von genetisch veränderten Pflanzen, im folgenden auch transgene Pflanzen bezeichnet, wird die fusionierte Expressionskassette, die eine Ketolase exprimiert, in einen Vektor, beispielsweise pBin19 oder insbesondere pSUN2 kloniert, der geeignet ist, in *Agrobacterium tumefaciens transformiert zu werden* Mit einem solchen Vektor transformierte Agrobakterien können dann in bekannter Weise zur Transformation von Pflanzen, insbesondere von Kulturpflanzen verwendet werden, indem beispielsweise verwundete Blätter oder Blattstücke in einer Agrobakterienlösung gebadet und anschließend in geeigneten Medien kultiviert werden.

Die Transformation von Pflanzen durch Agrobakterien ist unter anderem bekannt aus F.F. White, Vectors for Gene Transfer in Higher Plants; in Transgenic Plants, Vol. 1, Engineering and Utilization, herausgegeben von S.D. Kung und R. Wu, Academic Press, 1993, S. 15-38. Aus den transformierten Zellen der verwundeten Blätter bzw. Blattstücke können in bekannter Weise transgene Pflanzen regeneriert werden, die ein in die Expressionskassette integriertes Gen für die Expression einer Nukleinsäure codierend eine Ketolase enthalten.

Zur Transformation einer Wirtspflanze der Gattung Tagetes mit einer für eine Ketolase kodierenden Nukleinsäure wird eine Expressionskassette als Insertion in einen rekombinanten Vektor eingebaut, dessen Vektor-DNA zusätzliche funktionelle Regulationssignale, beispielsweise Sequenzen für Replikation oder Integration enthält. Geeignete Vektoren sind unter anderem in "Methods in Plant Molecular Biology and Biotechnology" (CRC Press), Kap. 6/7, S. 71-119 (1993) beschrieben.

Unter Verwendung der oben zitierten Rekombinations- und Klonierungstechniken können die Expressionskassetten in geeignete Vektoren kloniert werden, die ihre Vermehrung, beispielsweise in *E. coli,* ermöglichen. Geeignete Klonierungsvektoren sind u.a. pJIT117 (Guerineau et al. (1988) Nucl. Acids Res.16 :11380), pBR332, pUC-Serien, M13mp-Serien und pACYC184. Besonders geeignet sind binäre Vektoren, die sowohl in *E*. *coli* als auch in Agrobakterien replizieren können.

Dabei kann je nach Wahl des Promotors die Expression konstitutiv oder vorzugsweise spezifisch in den Blütenblättern erfolgen.

Die erfindungsgemäßen genetisch veränderten Pflanzen der Gattung Tagetes weisen im Vergleich zum Wildtyp einen Gehalt an Astaxanthin, insbesondere in Petalen auf.

Wie vorstehend erwähnt, betrifft die Erfindung die Verwendung astaxanthinhaltiger Pflanzen oder Pflanzenteile der Gattung Tagetes zur oralen Verabreichung an Tiere

In einer bevorzugten Ausführungsform werden die astaxanthinhaitigen Pflanzen oder Pflanzenteile der Gattung Tagetes zur Pigmentierung von Tieren und der entsprechenden Tierprodukte verwendet.

Unter "Pigmentierung" wird erfindungsgemäß vorzugsweise die Intensivierung oder Verursachung einer Farbe zumindest eines Teils eines Tieres oder Tierproduktes des pigmentierten Tieres im Vergleich zum nicht pigmentierten Tier verstanden. Astaxanthinhaltige Pigmentierstoffe pigmentieren und verursachen oder intensivieren in der Regel einen rosa bis rosa-roten Farbton.

Bevorzugte Tiere die durch die erfindungsgemäße orale Verabreichung pigmentiert werden können sind Tiere, ausgewählt aus der Gruppe Fische, Crustaceae oder Vögel, insbesondere Galliformes und Anatridae.

Bevorzugte Fische sind Salmoniden, insbesondere Lachs oder Forelle.

Bevorzugte Crustaceae sind Shrimps oder Krebse.

Bevorzugte Galliformes sind Hühner, Enten oder Gänse.

Bevorzugter Anatridae ist Flamingo.

Je nach pigmentiertem Tier werden vorzugsweise unter pigmentierten Tierprodukten insbesondere Fleisch für Lachs oder Forelle, Haut für Hühner, Enten oder Gänse, Feder für Hühner, Enten, Gänse oder Flamingo und Et bzw. Eidotter für Hühner, Enten oder Gänse verstanden.

Die orale Verabreichung der astaxanthinhaltigen Pflanzen oder Pflanzenteile der Gattung Tagetes an Tiere kann direkt erfolgen oder über orale Verabreichung von Tierfutterzubereitungen, denen zuvor die astaxanthinhaltigen Pflanzen oder Pflanzenteile der Gattung Tagetes beigemischt wurden.

In einer bevorzugten Ausführungsform werden die astaxanthinhaltigen Pflanzen oder Pflanzenteile der Gattung Tagetes

Tierfutterzubereitungen beigemischt und die Tierfutterzubereitung an Tiere oral verabreicht.

Dabei ist eis vorteilhaft, die astaxanthinhaltigen Pflanzen oder Pflanzenteile der Gattung Tagetes vor der Beimischung zu Tierfutterzubereitungen in eine Form zu prozessieren, die eine Beimischung zu entsprechenden Tierfutterzubereitung ermöglicht und vorzugsweise zu einer hohen Stabilität und Bioverfügbarkeit von Astaxanthin im jeweiligen Anwendungsbereich führt.

Je nach Tier, an das die orale Verabreichung erfolgen soll und damit je nach Tierfutterzubereitung können dazu verschiedene Prozessierungsschritte vorteilhaft sein

Für astaxanthinhaltigen Pflanzen oder Pflanzenteile der Gattung Tagetes, ist es in dieser Ausführungsform vorteilhaft, die astaxanthinhaltigen Pflanzen oder Pflanzenteile, insbesondere Blütenköpfe und Petalen zu trocknen und/oder zu zerkleinern. Besonders bevorzugt liegen die astaxanthinhaltigen Pflanzen oder Pflanzenteile der Gattung Tagetes in Pulverform vor.

Jede wie auch immer gestaltete Ausführungsform der astaxanthinhaltigen Pflanzen oder Pflanzenteile der Gattung Tagetes, ob prozessiert oder nicht prozessiert, kann in an sich bekannter Weise Tierfutterzubereitungen beigemischt werden.

Die Erfindung betrifft ferner ein Verfahren zur Herstellung von Tierfutterzubereitungen durch Zusammenfügen von astaxanthinhaitigen Pflanzen oder Pflanzenteilen der Gattung Tagetes und üblichen Tierfuttermitteln.

Eine bevorzugte Ausführungsform des Verfahrens ist **dadurch gekennzeichnet dass** die astaxanthinhaltigen Pflanzen oder Pflanzenteile der Gattung Tagetes vor dem Zusammenfügen mit Tierfuttermittein in eine Form prozessiert werden, die ein Zusammenfügen mit Tierfuttermitteln ermöglicht.

Beispielsweise für Fische können die Fischfutterzubereitungen weitere Übliche Fischfutterkomponenten enthalten, wie beispielsweise Fischmehl und/oder andere Proteine, Öle, wie beispielsweise Fischöle, Getreide, Vitamine, Mineralien, Konservierungsstoffe und gegebenenfalls Medikamente in Üblichen Mengen.

Eine typische Fischfutterrezeptur für Forellen setzt sich beispielsweise aus folgenden Komponenten zusammen:

| ***Komponenten*** | ***Gew.-%*** | **Einwaage f. 500 kg** ***kg*** |
|---|---|---|
| Fischmehl | 30,00 | 150,00 |
| Sojavoilfettbohnen | 20,00 | 100,00 |
| Weizenquelistärke | 18,00 | 90,00 |
| Vitamin-Prämix | 0,80 | 4,00 |
| Cholinchlorid (50%) | 0,20 | 1,00 |
| Weizenkleber | 20,00 | 100,00 |
| Sipernat 50S | 3,00 | 15,00 |
| Fischöl | 8,00 | 40,00 |

Eine typische Fischfutterrezeptur für Lachse setzt sich beispielsweise aus folgenden Komponenten zusammen:

| ***Komponenten*** | ***Gew.-%*** |
|---|---|
| Fischmehl | 75,00 |
| Pflanzliches Protein | 5,00 |
| Getreide | 7,80 |
| Vitaminw/Mineralien | 1,00 |
| Antioxidan- | 0,20 |
| tien/Konservierungsstoffe | |
| Fischöl | 11,00 |

In einer Ausführungsform werden die astaxanthinhaltigen Pflanzen oder Pflanzenteile der Gattung Tagetes den Tierfutterzubereitungen in getrockneter und zerkleinerter Pulverform beigemischt.

Die so erhaltenen Tierfutterzubereitungen, enthaltend astaxanthinhaltige Pflanzen oder Pflanzenteile der Gattung Tagetes, können bei Fischfutter bei spielsweise in an sich bekannter Weise pelletiert oder besonders vorteilhaft extrudiert werden.

In einer weiteren, bevorzugten Ausführungsform werden die astaxanthinhaltigen Pflanzen oder Pflanzenteile der Gattung Tagetes o direkt an Tiere oral verabreicht.

Dabei ist es vorteilhaft, die astaxanthinhaltigen Pflanzen oder der Gattung Tagetes vor der Verabreichung in eine Form zu prozessieren, die eine direkte orale Verabreichung an Tiere ermöglicht und vorzugsweise zu einer hohen Stabilität und Bioverfügbarkeit von Astaxanthin im jeweiligen Answendungsbereich führt.

Je nach Tier, an das die orale Verabreichung erfolgen soll und damit je nach Tierfutierzubereitung können dazu verschiedene Prozessierungsschritte vorteilhaft sein.

Für astaxanthinhaltigen Pflanzen oder Pflanzenteile der Gattung Tagetes, ist es in dieser Ausführungsform vorteilhaft, die astaxanthinhaltigen Pflanzen oder Pflanzenteile, insbesondere Blütenköpfe und Petalen zu trocknen und/oder zu zerkleinern. Besonders bevorzugt Hegen die astaxanthinhaltigen Pflanzen oder Pflanzenteile der Gattung Tagetes in Pulverform vor.

Jede wie auch immer gestaltete Ausführungsform der astaxanthinhaltigen Pflanzen oder Pflanzenteile der Gattung Tagetes, ob prozessiert oder nicht prozessiert, kann in an sich bekannter Weise oral an Tiere verabreicht werden.

Die Erfindung betrifft ferner ein Verfahren zur Pigmentierung von Tieren oder Tierprodukten durch orale Verabreichung von astaxanthinhaltigen Pflanzen oder Pflanzenteilen der Gattung Tagetes an Tiere.

Ferner betrifft die Erfindung ein Verfahren zur Herstellung von pigmentierien Tieren oder Tierprodukten durch orale Verabreichung von astaxanthinhaltigen Pflanzen oder Pflanzenteiten der Gattung Tagetes an Tiere.

Die Erfindung betrifft ferner die Verwendung von astaxanthinhaltigen Pflanzen oder Pflanzenteilen der Gattung Tagetes als Tierfutter oder Tierfutterzusatz.

Die Pigmentiermittel, enthaltend astaxanthinhaltige Pflanzen oder Pflanzenteile der Gattung Tagetes bzw. Tierfuttermittel enthaltend diese Pigmentiermittel weisen weiterhin den Vorteil einer hohen Lagerstabilität und Bioverfügbarkeit des Pigments Astaxanthin auf.

Die Erfindung wird durch die nun folgenden Beispiele erläutert, ist aber nicht auf diese beschränkt:

### Beispiel I

### Herstellung astaxanthinhaltiger, genetisch veränderter Pflanzen der Gattung Tagetes

Allgemeine Experimentelle Bedingungen:
Sequenzanalyse rekombinanter DNA

Die Sequenzierung rekombinanter DNA-Moleküle erfolgte mit einem Laserfluoreszenz-DNA-Sequenzierer der Firma Licor (Vertrieb durch MWG Biotech, Ebersbach) nach der Methode von Sanger (Sanger et al., Proc. Natl. Acad. Sci. USA 74 (1977), 5463-5467).

### Beispiel I.1:

### Amplifikation einer cDNA, die die gesamte Primärsequenz der Ketolase aus Haematococcus pluvialis Flotow em. Wille codiert

Die cDNA, die für die Ketolase aus *Haematococcus pluvialis* codiert, wurde mittels PCR aus *Haematococcus pluvialis* (Stamm 192.80 der "Sammlung von Algenkulturen der Universität Göttingen")Suspensionskultur amplifiziert.

Für die Präparation von Total-RNA aus einer Suspensionskultur von *Haematococcus pluvialis* (Stamm 192.80), die 2 Wochen mit indirektem Tageslicht bei Raumtemperatur in *Haematococcus*-_Medium (1.2 g/I Natriumacetat, 2 g/I Hefeextrakt, 0.2 g/I MgCl2x6H2O, 0.02 CaCl2x2H2O; pH 6.8; nach Autoklavieren Zugabe von 400 mg/l L-Asparagin, 10 mg/l FeSO4xH2O) gewachsen war, wurden die Zellen geerntet, in flüssigem Stickstoff eingefroren und im Mörser pulverisiert. Anschließend wurden 100 mg der gefrorenen, pulverisierten Algenzellen in ein Reaktionsgefäß überführt und in 0.8 ml Trizol-Puffer (LifeTechnologies) aufgenommen. Die Suspension wurde mit 0.2 ml Chloroform extrahiert. Nach 15 minütiger Zentrifugation bei 12 000 g wurde der wässrige Überstand abgenommen und in ein neues Reaktionsgefäß überführt und mit einem Volumen Ethanol extrahiert. Die RNA wurde mit einem Volumen Isopropanol gefällt, mit 75% Ethanol gewaschen und das Pellet in DEPC Wasser (über Nacht Inkubation von Wasser mit 1/1000 Volumen Diethylpyrocarbonat bei Raumtemperatur, anschließend autoklaviert) gelöst. Die RNA-Konzentration wurde photometrisch bestimmt.

Für die cDNA-Synthese wurden 2.5 ug Gesamt-RNA für 10 min bei 60_C denaturiert, für 2 min auf Eis abgekühlt und mittels eines cDNA-Kits (Ready-to-go-you-prime-beads, Pharmacia Biotech) nach Herstellerangaben unter Verwendung eines antisense spezifischen Primers (PR1 SEQ ID NO: 29) in cDNA umgeschrieben.

Die Nukleinsäure codierend eine Ketolase aus *Haematococcus pluvialis* (Stamm 192.80) wurde mittels polymerase chain reaction (PCR) aus *Haematococcus pluvialis* unter Verwendung eines sense spezifischen Primers (PR2 SEQ ID NO: 30) und eines antisense spezifischen Primers (PR1 SEQ ID NO: 29) amplifiziert.

Die PCR-Bedingungen waren die folgenden:
Die PCR zur Amplifikation der cDNA, die für ein Ketolase Protein bestehend aus der gesamten Primärsequenz codiert, erfolgte in einem 50 ml Reaktionsansatz, in dem enthalten war:
   - 4 ml einer *Haematococcus pluvialis* cDNA (hergestellt wie oben beschrieben)
   - 0.25 mM dNTPs
   - 0.2 mM PR1 (SEQ ID NO: 29)
   - 0.2 mM PR2 (SEQ ID NO: 30)
   - 5 ml 10X PCR-Puffer (TAKARA)
   - 0.25 ml R Taq Polymerase (TAKARA)
   - 25.8 ml Aq. Dest.

Die PCR wurde unter folgenden Zyklusbedingungen durchgeführt:

| | | |
|---|---|---|
| 1X | 94_C 2 Minuten | Minuten |
| 35X | 94_C | 1 Minute |
| | 53_C | 2 Minuten |
| | 72_C | 3 Minuten |
| 1X | 72_C | 10 Minuten |

1X 35X 94_C 1 Minute 53_C 2 Minuten 72_C 3 Minuten 1X 72_C 10 Minuten

Die PCR-Amplifikation mit SEQ ID NO: 29 und SEQ ID NO: 30 resultierte in einem 1155 Bp-Fragment, das für ein Protein bestehend aus der gesamten Primärsequenz codiert (SEQ ID NO: 22). Unter Verwendung von Standardmethoden wurde das Amplifikat in den PCR-Klonierungsvektor pGEM-Teasy (Promega) kloniert und der Klon pGKET02 erhalten.

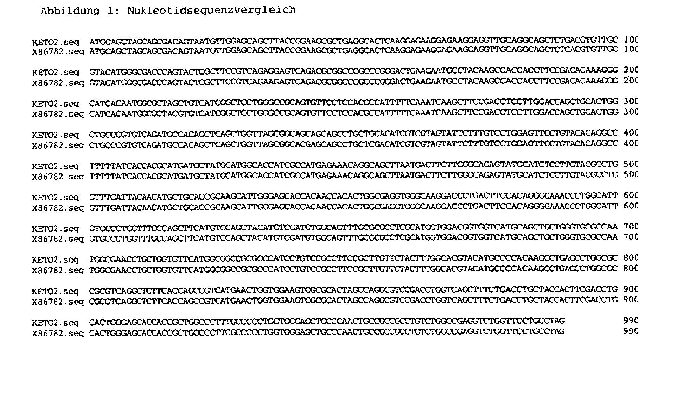

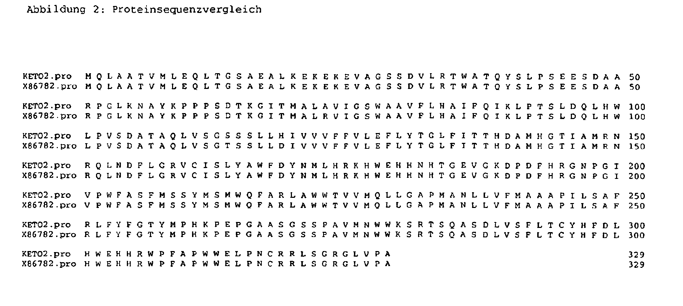

Sequenzierung des Klons pGKET02 mit dem T7- und dem SP6-Primer bestätigte eine Sequenz, die sich lediglich in den drei Codons 73, 114 und 119 in je einer Base von der publizierten Sequenz X86782 unterscheidet. Diese Nukleotidaustausche wurden in einem unabhängigem Amplifikationsexperiment reproduziert und repräsentieren somit die Nukleotidsequenz im verwendeten *Haematococcus pluvialis* Stamm 192.80 (Abbildung 1 und 2, Sequenzvergleiche).

Dieser Klon wurde daher für die Klonierung in den Expressionsvektor pJIT117 (Guerineau et al. 1988, Nucl. Acids Res. 16: 11380) verwendet. Die Klonierung erfolgte durch Isolierung des 1027 Bp SpHI-Fragmentes aus pGEM-Teasy und Ligierung in den SpHI geschnittenen Vektor pJIT117. Der Klon, der die *Haematococcus pluvialis* Ketolase in der korrekten Orientierung als N-terminale translationale Fusion mit dem rbcs Transitpeptid enthält, heißt pJKET02.

### Beispiel I.2:

### Amplifikation einer cDNA, die die Ketolase aus Haematococcus pluvialis Flotow em. Wille mit einem um 14 Aminosäuren verkürztem N-terminus codiert

Die cDNA, die für die Ketolase aus *Haematococcus pluvialis* (Stamm 192.80) mit einem um 14 Aminosäuren verkürztem N-Terminus codiert, wurde mittels PCR aus *Haematococcus pluvialis* Suspensionskultur (Stamm 192.80 der "Sammlung von AIgenkulturen der Universität Göttingen") amplifiziert.

Die Präparation von Total-RNA aus einer Suspensionskultur von *Haematococcus pluvialis* (Stamm 192.80) erfolgte wie in Beispiel 1 beschrieben.

Die cDNA-Synthese erfolgte wie unter Beispiel 1 beschrieben.

Die Nukleinsäure kodierend eine Ketolase aus *Haematococcus pluvialis* (Stamm 192.80) mit einem um 14 Aminosäuren verkürztem N-Terminus wurde mittels polymerase chain reaction (PCR) aus *Haematococcus pluvialis* unter Verwendung eines sense spezifischen Primers (PR3 SEQ ID NO: 31) und eines antisense spezifischen Primers (PR1 SEQ ID NO: 29) amplifiziert.

Die PCR-Bedingungen waren die folgenden:

Die PCR zur Amplifikation der cDNA, die für ein Ketolase Protein mit um 14 Aminosäuren verkürztem N-Terminus codiert, erfolgte in einem 50 ml Reaktionsansatz, in dem enthalten war:
- 4 ml einer *Haematococcus pluvialis* cDNA (hergestellt wie oben beschrieben)
- 0.25 mM dNTPs
- 0.2 mM PR1 (SEQ ID NO: 29)
- 0.2 mM PR3 (SEQ ID NO: 31)
- 5 ml 10X PCR-Puffer (TAKARA)
- 0.25 ml R Taq Polymerase (TAKARA)
- 25.8 ml Aq. Dest.

Die PCR wurde unter folgenden Zyklusbedingungen durchgeführt:

| | | |
|---|---|---|
| 1X | 94_C | 2 Minuten |
| 35X | 94_C | 1 Minute |
| | 53_C | 2 Minuten |
| | 72_C | 3 Minuten |
| 1X | 72_C 1 | 0 Minuten |

Die PCR-Amplifikation mit SEQ ID NO: 29 und SEQ ID NO: 31 resultierte in einem 1111 Bp Fragment, das für ein Ketolase Protein codiert, bei dem N-terminalen Aminosäuren (Position 2-16) durch eine einzige Aminosäure (Leucin) ersetzt sind.

Das Amplifikat wurde unter Verwendung von Standardmethoden in den PCR-Klonierungsvektor pGEM-Teasy (Promega) kloniert. Sequenzierungen mit mit den Primern T7- und SP6 bestätigten eine zur Sequenz SEQ ID NO: 22 identische Sequenz, wobei die 5'Region (Position 1-53) der SEQ ID NO: 22 im Amplifikat SEQ ID NO: 24 durch eine in der Sequenz abweichende Nonamersequenz ersetzt wurde. Dieser Klon wurde daher für die Klonierung in den Expressionsvektor pJIT117 (Guerineau et al. 1988, Nucl. Acids Res. 16: 11380) verwendet.

Die Klonierung erfolgte durch Isolierung des 985 Bp SpHI Fragmentes aus pGEM-Teasy und Ligierung mit dem SpHI geschnittenen Vektor pJIT117. Der Klon, der die *Haematococcus pluvialis* Ketolase mit einem um 14 Aminosäuren verkürztem N-Terminus in der korrekten Orientierung als N-terminale translationale Fusion mit dem rbcs Transitpeptid enthält, heisst pJKETO3.

### Beispiel I.3:

Amplifikation einer cDNA, die die Ketolase aus *Haematococcus pluvialis* Flotow em. Wille (Stamm 192.80 der "Sammlung von Algenkulturen der Universität Göttingen") bestehend aus der gesamten Primärsequenz und fusioniertem C-terminalem myc-Tag codiert.

Die cDNA, die für die Ketolase aus *Haematococcus pluvialis* (Stamm 192.80) bestehend aus der gesamten Primärsequenz und fusioniertem C-terminalem myc-Tag codiert, wurde mittels PCR unter Verwendung des Plasmids pGKETO2 (in Beispiel 1 beschrieben) und des Primers PR15 (SEO ID NO: 32) hergestellt. Der Primer PR15 setzt sich zusammen aus einer antisense spezifischen 3'Region (Nucleotide 40 bis 59) und einer myc-Tag codierenden 5'Region (Nucleotide 1 bis 39).

Die Denaturierung (5 min bei 95_C) und Annealing (langsame Abkühlung bei Raumtemperatur auf 40_C) von pGKETO2 und PR15 erfolgte in einem 11.5 ml Reaktionsansatz, in dem enthalten war:
- 1 mg pGKETO2 PlasmidDNA
- 0.1 mg PR15 (SEQ ID NO: 32)

Das Auffüllen der 3'Enden (30 min bei 30_C) erfolgte in einem 20 ml Reaktionsansatz, in dem enthalten war:
- 11.5 ml pGKETO2/PR15-Annealingsreaktion (hergestellt wie oben beschrieben)
- 50 mM dNTPs
- 2 ml 1X Klenow Puffer
- 2U Klenow Enzym

Die Nukleinsäure kodierend eine Ketolase aus *Haematococcus pluvialis* (Stamm 192.80) bestehend aus der gesamten Primärsequenz und fusioniertem C-terminalem myc-Tag wurde mittels polymerase chain reaction (PCR) aus *Haematococcus pluvialis* unter Verwendung eines sense spezifischen Primers (PR2 SEQ ID NO: 30) und eines antisense spezifischen Primers (PR15 SEQ ID NO: 32) amplifiziert.

Die PCR-Bedingungen waren die folgenden:
Die PCR zur Amplifikation der cDNA, die für ein Ketolase Protein mit fusioniertem C-terminalem myc-Tag codiert, erfolgte in einem 50 ml Reaktionsansatz, in dem enthalten war:
   - 1 ml einer Annealingsreaktion (hergestellt wie oben beschrieben)
   - 0.25 mM dNTPs
   - 0.2 mM PR15 (SEO ID NO: 32)
   - 0.2 mM PR2 (SEQ ID NO: 30)
   - 5 ml 10X PCR-Puffer (TAKARA)
   - 0.25 ml R Taq Polymerase (TAKARA)
   - 28.8 ml Aq. Dest.

Die PCR wurde unter folgenden Zyklusbedingungen durchgeführt:

| | | |
|---|---|---|
| 1X | 94_C | 2 Minuten |
| 35X | 94_C | 1 Minute |
| | 53_C | 1 Minute |
| | 72_C | 1 Minute |
| 1X | 72_C | 10 Minuten |

Die PCR-Amplifikation mit SEQ ID NO:32 und SEQ ID NO:30 resultierte in einem 1032 Bp-Fragment, das für ein Protein codiert, bestehend aus der gesamten Primärsequenz der Ketolase aus *Haematococcus pluvialis* als zweifache translationale Fusion mit dem rbcS Transitpeptide am N-Terminus und dem myc-Tag am C-Terminus.

Das Amplifikat wurde unter Verwendung von Standardmethoden in den PCR-Klonierungsvektor pGEM-Teasy (Promega) kloniert. Sequenzierungen mit mit den Primern T7- und SP6 bestätigten eine zur Sequenz SEQ ID NO: 22 identische Sequenz, wobei die 3'Region (Position 993 bis 1155) der SEQ ID NO: 22 im Amplifikat SEQ ID NO: 26 durch eine in der abweichende Sequenz aus 39 Bp ersetzt wurde. Dieser Klon wurde daher für die Klonierung in den Expressionsvektor pJIT117 (Guerineau et al. 1988, Nucl. Acids Res. 16: 11380) verwendet.

Die Klonierung erfolgte durch Isolierung des 1038 Bp EcoRl-SpHI Fragmentes aus pGEM-Teasy und Ligierung mit dem EcoRl-SpHI geschnittenen Vektor pJIT117. Durch die Ligation entsteht eine translationale Fusion zwischen dem C-Terminus der rbcS Transitpeptidsequenz und dem N-Terminus der Ketolase Sequenz. Der Klon, der die *Haematococcus pluvialis* Ketolase mit fusioniertem C-terminalem myc-Tag in der korrekten Orientierung als translationale N-terminale Fusion mit dem rbcs Transitpeptid enthält, heisst pJKET04.

### Beispiel I.4:

### Amplifikation einer DNA, die die gesamte Primärsequenz der Ketolase aus Nostoc sp. PCC 7120 codiert

Die DNA, die für die Ketolase aus *Nostoc PCC 7120* kodiert, wurde mittels PCR aus *Nostoc PCC 7120* (Stamm der "Pasteur Culture Collection of Cyanobacterium") amplifiziert.

### Für die Präparation von genomischer DNA aus einer Suspensionskultur von Nostoc

*PCC 7120,* die 1 Woche mit Dauerlicht und konstantem Schütteln (150 rpm) at 25°C in *BG* 11-Medium (1.5 g/l NaNO3, 0.04 g/l K2P04x3H2O, 0.075 g/l MgSO4xH2O, 0.036 g/l CaCl2x2H2O, 0.006 g/l citric acid, 0.006 g/l Ferric ammonium citrate, 0.001 g/l ED-TA disodium magnesium, 0.04 g/l Na2CO3, 1 ml trace metal mix A5+Co (2.86 g/l H3BO3, 1.81 g/l MnCl2x4H2o, 0.222 g/l ZnSO4x7H2o,0.39 g/l NaMoO4X2H2o, 0.079 g/l CuSO4x5H2O, 0.0494 g/l Co(NO3)2x6H2O) gewachsen war, wurden die Zellen durch Zentrifugation geerntet, in flüssigem Stickstoff eingefroren und im Mörser pulverisiert.

Protokoll für DNA Isolation aus Nostoc PCC7120:

Aus einer 10 ml Flüssigkultur wurden die Bakterienzellen durch 10minütige Zentrifugation bei
8 000 rpm pelletiert. Anschließend wurden die Bakterienzellen in flüssigem Stickstoff mit einem Mörser zerstoßen und gemahlen. Das Zellmaterial wurde in 1 ml 10mM Tris HCI (pH 7.5) resuspendiert und in ein Eppendorf Reaktionsgefäß (2ml Volumen) überführt. Nach Zugabe von
100 µl Proteinase K (Konzentration: 20 mg/ml) wurde die Zellsuspension für 3 Stunden bei 37°C inkubiert. Anschließend wurde die Suspension mit 500 µl Phenol extrahiert. Nach 5minütiger Zentrifugation bei 13 000 upm wurde die obere, wässrige Phase in ein neues 2 ml-Eppendorf Reaktionsgefäß überführt. Die Extraktion mit Phenol wurde 3mal wiederholt. Die DNA wurde durch Zugabe von 1/10 Volumen 3 M Natriumacetat (pH 5.2) und 0.6 Volumen Isopropanol gefällt und anschließend mit 70% Ethanol gewaschen. Das DNA-Pellet wurde bei Raumtemperatur getrocknet, in 25 µl Wasser aufgenommen und unter Erhitzung auf 65°C gelöst.

Die Nukleinsäure, kodierend eine Ketolase aus *Nostoc PCC 7120,* wurde mittels "polymerase chain reaction" (PCR) aus *Nostoc PCC 7120* unter Verwendung eines sensespezifischen Primers (NOSTF, SEQ ID No. 87) und eines antisense-spezifischen Primers (NOSTG, SEQ ID NO. 88) amplifiziert.

Die PCR-Bedingungen waren die folgenden:
Die PCR zur Amplifikation der DNA, die für ein Ketolase Protein bestehend aus der gesamten Primärsequenz kodiert, erfolgte in einem 50 ul Reaktionsansatz, in dem enthalten war:
   - 1 ul einer *Nostoc PCC 7120* DNA (hergestellt wie oben beschrieben)
   - 0.25 mM dNTPs
   - 0.2 mM NOSTF (SEQ ID No. 87)
   - 0.2 mM NOSTG (SEQ ID No. 88)
   - 5 ul 10X PCR-Puffer (TAKARA)
   - 0.25 ul R Taq Polymerase (TAKARA)
   - 25.8 ul Aq. Dest.

Die PCR wurde unter folgenden Zyklusbedingungen durchgeführt:

| | | |
|---|---|---|
| 1X | 94°C | 2 Minuten |
| 35X | 94°C | 1 Minute |
| | 55°C | 1 Minuten |
| | 72°C | 3 Minuten |
| 1X | 72°C | 10 Minuten |

Die PCR-Amplifikation mit SEQ ID No. 87 und SEQ ID No. 88 resultierte in einem 805 Bp-Fragment, das für ein Protein bestehend aus der gesamten Primärsequenz kodiert (SEQ ID No. 89). Unter Verwendung von Standardmethoden wurde das Amplifikat in den PCR-Klonierungsvektor pGEM-T (Promega) kloniert und der Klon pNOSTF-G erhalten.

Sequenzierung des Klons pNOSTF-G mit dem M13F- und dem M13R-Primer bestätigte eine Sequenz, welche mit der DNA-Sequenz des Datenbankeintrages AP003592 identisch ist. Diese Nukleotidsequenz wurde in einem unabhängigem Amplifikationsexperiment reproduziert und repräsentiert somit die Nukleotidsequenz im verwendeten *Nostoc PCC 7120.*

Dieser Klon pNOSTF-G wurde daher für die Klonierung in den Expressionsvektor pJIT117 (Guerineau et al. 1988, Nucl. Acids Res. 16: 11380) verwendet. Die Klonierung erfolgte durch Isolierung des 1027 Bp Sphl-Fragmentes aus pGEM-T und Ligierung in den Sphl geschnittenen Vektor pJIT117. Der Klon, der die Ketolase von *Nostoc* in der korrekten Orientierung als N-terminale translationale Fusion mit dem rbcS Transitpeptid enthält, heisst pJNOST.

### Beispiel I.5:

### Herstellung von Expressionsvektoren zur konstitutiven Expression der Haematococcus pluvialis Ketolase in Tagetes erecta.

Die Expression der Ketolase aus *Haematococcus pluvialis* in *Tagetes erecta* erfolgte unter Kontrolle des konstitutiven Promoters d35S aus CaMV (Franck et al. 1980, Cell 21: 285-294). Die Expression erfolgte mit dem Transitpeptid rbcS aus Erbse (Anderson et al. 1986, Biochem J. 240:709-715).

Die Herstellung einer Expressionskassette für die Agrobacterium-vermittelte Transformation der Ketolase aus *Haematococcus pluvialis* in Tagetes erecta erfolgte unter der Verwendung des binären Vektors pSUN5 (WO02/00900).

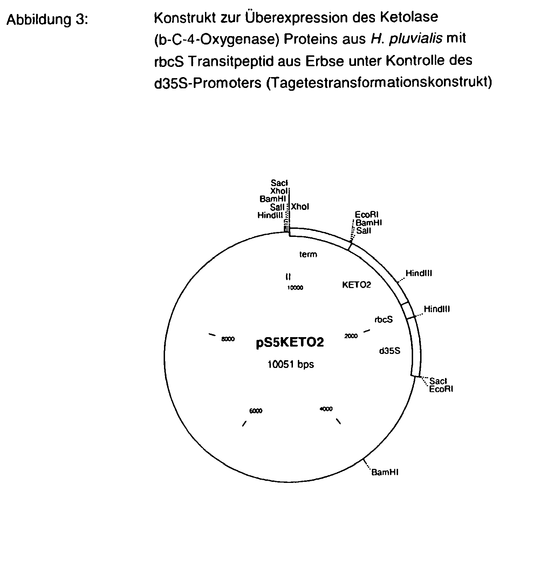

Zur Herstellung des Tagetes-Expressionsvektors pS5KETO2 wurde das 2.8 Kb Sacl-Xhol Fragment aus pJKET02 mit dem Sacl-Xhol geschnittenen Vektor pSUN5 ligiert (Abbildung 3, Konstruktkarte). In der Abbildung 3 beinhaltet Fragment *d35S* den duplizierten 35S Promoter (747 bp), Fragment *rbcS* das rbcS Transitpeptid aus Erbse

(204 bp), Fragment *KETO2* (1027 bp) die gesamte Primärsequenz codierend für die *Haematococcus pluvialis* Ketolase, Fragment *term* (761 bp) das Polyadenylierungssignal von CaMV.

### Beispiel I.5A:

### Herstellung von Expressionsvektoren zur blütenspezifischen Expression der Haematococcus pluvialis Ketolase in Tagetes erecta.

Die Expression der Ketolase aus *Haematococcus pluvialis* in *Tagetes erecta* erfolgte mit dem Transitpeptid rbcS aus Erbse (Anderson et al. 1986, Biochem J. 240:709-715). Die Expression erfolgte unter Kontrolle einer modifizierten Version AP3P des blütenspezifischen Promoters AP3 aus *Arabidopsis thaliana* (AL132971: Nukleotidregion 9298 bis 10200; Hill et al. (1998) Development 125: 1711-1721).

Das DNA Fragment, das die AP3 Promoterregion -902 bis +15 aus *Arabidopsis thaliana* beinhaltet, wurde mittels PCR unter Verwendung genomischer DNA (nach Standardmethoden aus *Arabidopsis thaliana* isoliert) sowie der Primer PR7 (SEQ ID NO: 33) und PR10 (SEQ ID NO: 36) hergestellt.

Die PCR-Bedingungen waren die folgenden:

Die PCR zur Amplifikation der DNA, die das AP3-Promoterfragment (-902 bis +15) beinhaltet, erfolgte in einem 50 ml Reaktionsansatz, in dem enthalten war:
- 100 ng genomischer DNA aus *A.thaliana*
- 0.25 mM dNTPs
- 0.2 mM PR7 (SEQ ID NO: 33)
- 0.2 mM PR10 (SEQ ID NO: 36)
- 5 ml 10X PCR-Puffer (Stratagene)
- 0.25 ml Pfu Polymerase (Stratagene)
- 28.8 ml Aq. Dest.

Die PCR wurde unter folgenden Zyklusbedingungen durchgeführt:

| | | |
|---|---|---|
| 1X | 94_C | 2 Minuten |
| 35X | 94_C | 1 Minute |
| | 50_C | 1 Minute |
| | 72_C | 1 Minute |
| 1X | 72_C | 10 Minuten |

Das 922 Bp Amplifikat wurde unter Verwendung von Standardmethoden in den PCR-Klonierungsvektor pCR 2.1 (Invitrogen) kloniert und das Plasmid pTAP3 erhalten.

Sequenzierung des Klons pTAP3 bestätigte eine Sequenz, die sich lediglich in durch eine Insertion (ein G in Position 9765 der Sequenz AL132971) und einen Basenaustausch (ein G statt ein A in Position 9726 der Sequenz AL132971) von der publizierten AP3 Sequenz (AL132971, Nukleotidregion 9298 bis 10200) unterscheidet. Diese Nukleotidunterschiede wurden in einem unabhängigen Amplifikationsexperiment reproduziert und repräsentieren somit die tatsächliche Nukleotidsequenz in den verwendeten *Arabidopsis thaliana* Pflanzen.

Die modifizierte Version AP3P wurde mittels rekombinanter PCR unter Verwendung des Plasmids pTAP3 hergestellt. Die Region 10200 bis 9771 wurde mit den Primern PR7 (SEQ ID NO: 33) und Primern PR9 (SEQ ID NO: 35) amplifiziert (Amplifikat A7/9), die Region 9526 bis 9285 wurde mit den PR8 (SEQ ID NO: 34) und PR10 (SEQ ID NO: 36) amplifiziert (Amplifikat A8/10).

Die PCR-Bedingungen waren die folgenden:

Die PCR-Reaktionen zur Amplifikation der DNA-Fragmente, die die Regionen Region 10200-9771 und Region 9526 bis 9285 des AP3 Promoters beinhalten, erfolgte in 50 al Reaktionsansätzen, in denen enthalten war:
- 100 ng AP3 Amplifikat (oben beschrieben)
- 0.25 mM dNTPs
- 0.2 mM sense Primer (PR7 SEQ ID NO: 33 bzw. PR8 SEQ ID NO: 34)
- 0.2 mM antisense Primer (PR9 SEQ. ID NO: 35 bzw. PR10 SEQ ID NO: 36)
- 5 ml 10X PCR-Puffer (Stratagene)
- 0.25 ml Pfu Taq Polymerase (Stratagene)
- 28.8 ml Aq. Dest.

Die PCR wurde unter folgenden Zyklusbedingungen durchgeführt:

| | | |
|---|---|---|
| 1 X | 94_C | 2 Minuten |
| 35X | 94_C | 1 Minute |
| | 50_C | 1 Minute |
| | 72_C | 1 Minute |
| 1X | 72_C | 10 Minuten |

Die rekombinante PCR beinhaltet Annealing der sich über eine Sequenz von 25 Nukleotiden überlappenden Amplifikate A7/9 und A8/10, Vervollständigung zu einem Doppelstrang und anschließende Amplifizierung. Dadurch entsteht eine modifizierte Version des AP3 Promoters, AP3P, in dem die Positionen 9670 bis 9526 deletiert sind.

Die Denaturierung (5 min bei 95_C) und Annealing (langsame Abkühlung bei Raumtemperatur auf 40_C) beider Amplifikate A7/9 und A8/10 erfolgte in einem 17.6 ml Reaktionsansatz, in dem enthalten war:
- 0.5 mg A7/9 Amplifikat
- 0.25 mg A8/10 Amplifikat

Das Auffüllen der 3'Enden (30 min bei 30_C) erfolgte in einem 20 ml Reaktionsansatz, in dem enthalten war:
- 17.6 m gA7/9 und A8/10-Annealingsreaktion (hergestellt wie oben beschrieben)
- 50 mM dNTPs
- 2 ml 1X Klenow Puffer
- 2U Klenow Enzym

Die Nukleinsäure codierend für die modifizierte Promoterversion AP3P wurde mittels PCR unter Verwendung eines sense spezifischen Primers (PR7 SEQ ID NO: 33) und eines antisense spezifischen Primers (PR10 SEQ ID NO: 36) amplifiziert.

Die PCR-Bedingungen waren die folgenden:

Die PCR zur Amplifikation des AP3P Fragmentes erfolgte in einem 50 ml Reaktionsansatz, in dem enthalten war:
- 1 ml Annealingsreaktion (hergestellt wie oben beschrieben)
- 0.25 mM dNTPs
- 0.2 mM PR7 (SEQ ID NO: 33)
- 0.2 mM PR10 (SEQ ID NO: 36)
- 5 ml 10X PCR-Puffer (Stratagene)
- 0.25 ml Pfu Taq Polymerase (Stratagene)
- 28.8 ml Aq. Dest.

Die PCR wurde unter folgenden Zyklusbedingungen durchgeführt:

| | | |
|---|---|---|
| 1X | 94_C | 2 Minuten |
| 35X | 94_C | 1 Minute |
| | 50_C | 1 Minute |
| | 72_C | 1 Minute |
| 1X | 72_C | 10 Minuten |

Die PCR-Amplifikation mit SEQ ID NO: 33 und SEQ ID NO: 36 resultierte in einem 778 Bp Fragment das für die modifizierte Promoterversion AP3P codiert. Das Amplifikat wurde in den Klonierungsvektor pCR2.1 (Invitrogen) kloniert. Sequenzierungen mit den Primern T7 und M13 bestätigten eine zur Sequenz AL132971, Region 10200 bis 9298 identische Sequenz, wobei die interne Region 9285 bis 9526 deletiert wurde. Diese Klon wurde daher für die Klonierung in den Expressionsvektor pJIT117 (Guerineau et al. 1988, Nucl. Acids Res. 16: 11380) verwendet.

Die Klonierung erfolgte durch Isolierung des 771 Bp SacI-HindIII Fragmentes aus pTAP3P und Ligierung in den SacI-HindIII geschnittenen Vektor pJIT117. Der Klon, der den Promoter AP3P anstelle des ursprünglichen Promoters d35S enthält, heisst pJAP3P.

Zur Herstellung einer Expressionskassette pJAP3PKET02 wurde das 1027 Bp SpHI-Fragment KET02 in den SpHI geschnittenen Vektor pJAP3P kloniert. Der Klon, der das Fragment KETO2 in der korrekten Orientierung als N-terminale Fusion mit dem rbcS Transitpeptid enthält, heisst pJAP3PKETO2.

Zur Herstellung einer Expressionskassetten pJAP3PKETO4 wurde das 1032 Bp SpHI-EcoRl Fragment KET04 (in Beispiel 3 beschrieben) in den SpHI-EcoRI geschnittenen

Vektor pJAP3P kloniert. Der Klon, der das Fragment KET04 in der korrekten Orientierung als N-terminale Fusion mit dem rbcS Transitpeptid enthält, heisst pJAP3PKET04.

Die Herstellung einer Expressionsvektors für die Agrobacterium-vermittelte Transformation der AP3P-kontrollierten Ketolase aus *Haematococcus pluvialis* in *Tagetes erec*ta erfolgte unter der Verwendung des binären Vektors pSUN5 (WO02/00900).

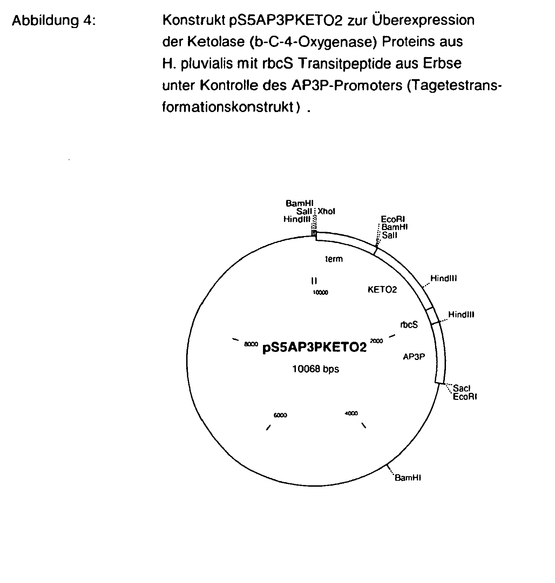

Zur Herstellung des Expressionsvektors pS5AP3PKETO2 wurde das 2.8 KB bp Sacl-Xhol Fragment aus pJAP3PKETO2 mit dem Sacl-Xhol geschnittenen Vektor pSUN5 ligiert (Abbildung 4, Konstruktkarte). In der Abbildung 4 beinhaltet Fragment *AP3P* den modifizierten AP3P Promoter (771 bp), Fragment *rbcS* das rbcS Transitpeptid aus Erbse (204 bp), Fragment *KETO2* (1027 bp) die gesamte Primärsequenz codierend für die *Haematococcus pluvialis* Ketolase, Fragment *term* (761 Bp) das Polyadenylierungssignal von CaMV

### Beispiel I.5.B:

### Herstellung von Expressionsvektoren zur konstitutiven Expression der Nostoc sp. PCC 7120 Ketolase in Tagetes erecta.

Die Expression der Ketolase aus *Nostoc* in *Tagetes erecta* erfolgte unter Kontrolle des konstitutiven Promoters FNR (Ferredoxin NADPH Oxidoreductase) aus *Arabidopsis thaliana.* Die Expression erfolgte mit dem Transitpeptid rbcS aus Erbse (Anderson et al. 1986, Biochem J. 240:709-715).

Das DNA Fragment, das die FNR Promotorregion -635 bis -1 aus *Arabidopsis thaliana* beinhaltet, wurde mittels PCR unter Verwendung genomischer DNA (nach Standardmethoden aus *Arabidopsis thaliana* isoliert) sowie der Primer FNR-1 (SEQ ID No.90) und FNR-2 (SEQ ID No. 91) hergestellt.

Die PCR-Bedingungen waren die folgenden:

Die PCR zur Amplifikation der DNA, die das FNR-Promotorfragment FNR1-2 (-635 bis -1) beinhaltet, erfolgte in einem 50 ul Reaktionsansatz, in dem enthalten war:
- 100 ng genomischer DNA aus *A.thaliana*
- 0.25 mM dNTPs
- 0.2 mM FNR-1 (SEQ ID No. 90)
- 0.2 mM FNR-2 (SEQ ID No. 91)
- 5 ul 10X PCR-Puffer (Stratagene)
- 0.25 ul Pfu Polymerase (Stratagene)
- 28.8 ul Aq. Dest.

Die PCR wurde unter folgenden Zyklusbedingungen durchgeführt:

| | | |
|---|---|---|
| 1X | 94°C | 2 Minuten |
| 35X | 94°C | 1 Minute |
| | 50°C | 1 Minute |
| | 72°C | 1 Minute |
| 1X | 72°C | 10 Minuten |

Das 653 bp Amplifikat wurde unter Verwendung von Standardmethoden in den PCR-Klonierungsvektor pCR 2.1 (Invitrogen) kloniert und das Plasmid pFNR erhalten.

Sequenzierung des Klons pFNR bestätigte eine Sequenz, die mit einem Sequenzabschnitt auf Chromosom 5 von Arabidopsis thaliana (Datenbankeintrag AB011474) von Position 70127 bis 69493 übereinstimmt. Das Gen beginnt bei Basenpaar 69492 und ist mit "Ferredoxin-NADP+ Reductase" annotiert.

Dieser Klon heisst pFNR und wurde daher für die Klonierung in den Expressionsvektor pJIT117 (Guerineau et al. 1988, Nucl. Acids Res. 16: 11380) verwendet.

Die Klonierung erfolgte durch Isolierung des 635 bp SacI-HindIII Fragmentes aus pFNR und Ligierung in den SacI-HindIII geschnittenen Vektor pJIT117. Der Klon, der den Promoter FNR anstelle des ursprünglichen Promoters d35S enthält, heisst pJITFNR.

Zur Herstellung einer Expressionskassette pJFNRNOST wurde das 805 bp SpHI-Fragment NOSTF-G (in Beispiel 1 beschrieben) in den SpHI geschnittenen Vektor pJITFNR kloniert. Der Klon, der das Fragment NOSTF-G in der korrekten Orientierung als N-terminale Fusion mit dem rbcS Transitpeptid enthält, heisst pJFNRNOST.

Die Herstellung einer Expressionskassette für die Agrobacterium-vermittelte Transformation des Expressionsvektor mit der Ketolase aus *Nostoc* in Tagetes erecta erfolgte unter der Verwendung des binären Vektors pSUNS (WO02/00900).

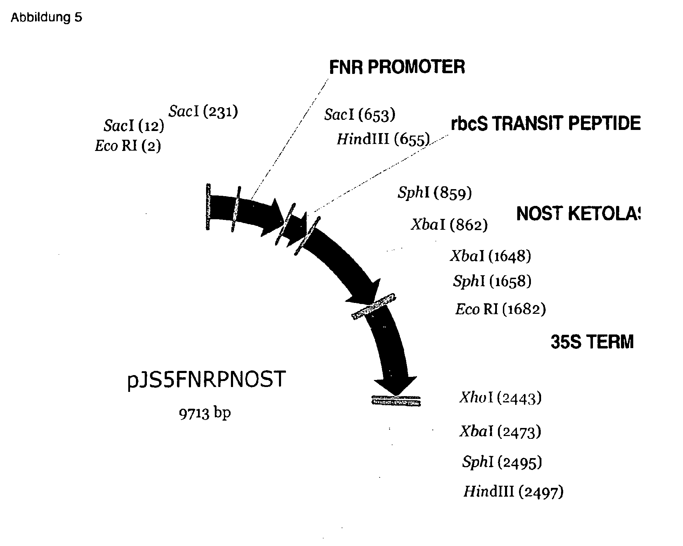

Zur Herstellung des Tagetes-Expressionsvektors pS5FNRNOST wurde das 2.4 Kb Sacl-Xhol Fragment (partielle Sacl Hydrolyse) aus pJFNRNOST mit dem Sacl-Xhol geschnittenen Vektor pSUN5 ligiert (Abbildung 5, Konstruktkarte). In der Abbildung 5 beinhaltet Fragment *FNR Promotorden* duplizierten FNR Promotor (655 bp), Fragment *rbcs Transit Peptid das* rbcS Transitpeptid aus Erbse (204 bp), Fragment *Nost Ketolase* (799 bp) die gesamte Primärsequenz, kodierend für die *Nostoc* Ketolase, Fragment *35S Terminator* (761 bp) das Polyadenylierungssignal von CaMV.

### Beispiel I.5C:

### Herstellung von Expressionsvektoren zur blütenspezifischen Expression der Nostoc sp. PCC 7120 Ketolase in Tagetes erecta.

Die Expression der Ketolase aus *Nostoc* in Tagetes erecta erfolgte mit dem Transitpeptid rbcS aus Erbse (Anderson et al. 1986, Biochem J. 240:709-715). Die Expression erfolgte unter Kontrolle einer modifizierten Version AP3P des blütenspezifischen Promoters AP3 aus Arabidopsis thaliana (AL132971: Nukleotidregion 9298-10200; Hill et al. (1998) Development 125: 1711-1721).

Das DNA Fragment, das die AP3 Promoterregion -902 bis +15 aus Arabidopsis thaliana beinhaltet, wurde mittels PCR unter Verwendung genomischer DNA (nach Standardmethoden aus Arabidopsis thaliana isoliert) sowie der Primer AP3-1 (SEQ ID No.93) und AP3-2 (SEQ ID No. 94) hergestellt.

Die PCR-Bedingungen waren die folgenden:

Die PCR zur Amplifikation der DNA, die das AP3-Promoterfragment (-902 bis +15) beinhaltet, erfolgte in einem 50 µl Reaktionsansatz, in dem enthalten war:
- 100 ng genomischer DNA aus A.thaliana
- 0.25 mM dNTPs
- 0.2 mM AP3-1 (SEQ ID No. 93)
- 0.2 mM AP3-2 (SEQ ID No. 94)
- 5 ul 10X PCR-Puffer (Stratagene)
- 0.25 ul Pfu Polymerase (Stratagene)
- 28.8 ul Aq. Dest.

Die PCR wurde unter folgenden Zyklusbedingungen durchgeführt:

| | | |
|---|---|---|
| 1X | 94°C | 2 Minuten |
| 35X | 94°C | 1 Minute |
| | 50°C | 1 Minute |
| | 72°C | 1 Minute |
| 1X | 72°C | 10 Minuten |

Das 929 Bp Amplifikat wurde unter Verwendung von Standardmethoden in den PCR-Klonierungsvektor pCR 2.1 (Invitrogen) kloniert und das Plasmid pAP3 erhalten.

Sequenzierung des Klons pAP3 bestätigte eine Sequenz, die sich lediglich in durch eine Insertion (ein G in Position 9765 der Sequenz AL132971) und einen Basenaustausch (ein G statt ein A in Position 9726 der Sequenz AL132971) von der publizierten AP3 Sequenz (AL132971, Nukleotidregion 9298-10200) unterscheidet. Diese Nukleotidunterschiede wurden in einem unabhängigen Amplifikationsexperiment reproduziert und repräsentieren somit die tatsächliche Nukleotidsequenz in den verwendeten Arabidopsis thaliana Pflanzen.

Die modifizierte Version AP3P wurde mittels rekombinanter PCR unter Verwendung des Plasmids pAP3 hergestellt. Die Region 10200 - 9771 wurde mit den Primern AP3-1 (SEQ ID No. 93) und Primern AP3-4 (SEQ ID No. 96) amplifiziert (Amplifikat A1/4), die Region 9526-9285 wurde mit den AP3-3 (SEQ ID No. 95) und AP3-2 (SEQ ID No. 94) amplifiziert (Amplifikat A2/3).

Die PCR-Bedingungen waren die folgenden:

Die PCR-Reaktionen zur Amplifikation der DNA-Fragmente, die die Regionen Region 10200 - 9771 und Region 9526-9285 des AP3 Promoters beinhalten, erfolgte in 50 ul Reaktionsansätzen, in denen enthalten war:
- 100 ng AP3 Amplifikat (oben beschrieben)
- 0.25 mM dNTPs
- 0.2 mM sense Primer (AP3-1 SEQ ID No. 93 bzw. AP3-3 SEQ ID No. 95)
- 0.2 mM antisense Primer (AP3-4 SEQ ID No. 96 bzw. AP3-2 SEQ ID No. 94)
- 5 ul 10X PCR-Puffer (Stratagene)
- 0.25 ul Pfu Taq Polymerase (Stratagene)
- 28.8 ul Aq. Dest.

Die PCR wurde unter folgenden Zyklusbedingungen durchgeführt:

| | | |
|---|---|---|
| 1X | 94°C | 2 Minuten |
| 35X | 94°C | 1 Minute |
| | 50°C | 1 Minute |
| | 72°C | 1 Minute |
| 1X | 72°C | 10 Minuten |

Die rekombinante PCR beinhaltet Annealing der sich über eine Sequenz von 25 Nukleotiden überlappenden Amplifikate A1/4 und A2/3, Vervollständigung zu einem Doppelstrang und anschließende Amplifizierung. Dadurch entsteht eine modifizierte Version des AP3 Promoters, AP3P, in dem die Positionen 9670 - 9526 deletiert sind. Die Denaturierung (5 min bei 95°C) und Annealing (langsame Abkühlung bei Raumtemperatur auf 40°C) beider Amplifikate A1/4 und A2/3 erfolgte in einem 17.6 ul Reaktionsansatz, in dem enthalten war:
- 0.5 ug A1/4 Amplifikat
- 0.25 ug A2/3 Amplifikat

Das Auffüllen der 3'-Enden (30 min bei 30°C) erfolgte in einem 20 ul Reaktionsansatz, in dem enthalten war:
- 17.6 ul A1/4 und A2/3-Annealingsreaktion (hergestellt wie oben beschrieben)
- 50 uM dNTPs
- 2 ul 1X Klenow Puffer
- 2U Klenow Enzym

Die Nukleinsäure kodierend für die modifizierte Promoterversion AP3P wurde mittels PCR unter Verwendung eines sense spezifischen Primers (AP3-1 SEQ ID No. 93) und eines antisense spezifischen Primers (AP3-2 SEQ ID No. 94) amplifiziert.

Die PCR-Bedingungen waren die folgenden:

Die PCR zur Amplifikation des AP3P Fragmentes erfolgte in einem 50 ul Reaktionsansatz, in dem enthalten war:
- 1 ul Annealingsreaktion (hergestellt wie oben beschrieben)
- 0.25 mM dNTPs
- 0.2 mM AP3-1 (SEQ ID No. 93)
- 0.2 mM AP3-2 (SEQ ID No. 94)
- 5 ul 10X PCR-Puffer (Stratagene)
- 0.25 ul Pfu Taq Polymerase (Stratagene)
- 28.8 ul Aq. Dest.

Die PCR wurde unter folgenden Zyklusbedingungen durchgeführt:

| | | |
|---|---|---|
| 1X | 94°C | 2 Minuten |
| 35X | 94°C | 1 Minute |
| | 50°C | 1 Minute |
| | 72°C | 1 Minute |
| 1X | 72°C | 10 Minuten |

Die PCR-Amplifikation mit SEQ ID No. 93 (AP3-1) und SEQ ID No. 94 (AP3-2) resultierte in einem 783 Bp Fragment, das für die modifizierte Promoterversion AP3P kodiert. Das Amplifikat wurde in den Klonierungsvektor pCR2.1 (Invitrogen) kloniert und das Plasmid pAP3P erhalten. Sequenzierungen mit den Primern T7 und M13 bestätigten eine zur Sequenz AL132971, Region 10200-9298 identische Sequenz, wobei die interne Region 9285 - 9526 deletiert wurde. Diese Klon wurde daher für die Klonierung in den Expressionsvektor pJIT117 (Guerineau et al. 1988, Nucl. Acids Res. 16: 11380) verwendet.

Die Klonierung erfolgte durch Isolierung des 783 Bp SacI-HindIII Fragmentes aus pAP3P und Ligierung in den SacI-HindIII geschnittenen Vektor pJIT117. Der Klon, der den Promoter AP3P anstelle des ursprünglichen Promoters d35S enthält, heisst pJl-TAP3P.Zur Herstellung einer Expressionskassette pJAP3NOST wurde das 805 Bp SpHI-Fragment NOSTF-G (in Beispiel 1 beschrieben) in den SpHI geschnittenen Vektor pJITAP3P kloniert. Der Klon, der das Fragment NOSTF-G in der korrekten Orientierung als N-terminale Fusion mit dem rbcS Transitpeptid enthält, heisst pJAP3PNOST.

Die Herstellung einer Expressionsvektors für die Agrobacterium-vermittelte Transformation der AP3P-kontrollierten Ketolase aus Nostoc in Tagetes erecta erfolgte unter der Verwendung des binären Vektors pSUN5 (WO02/00900).

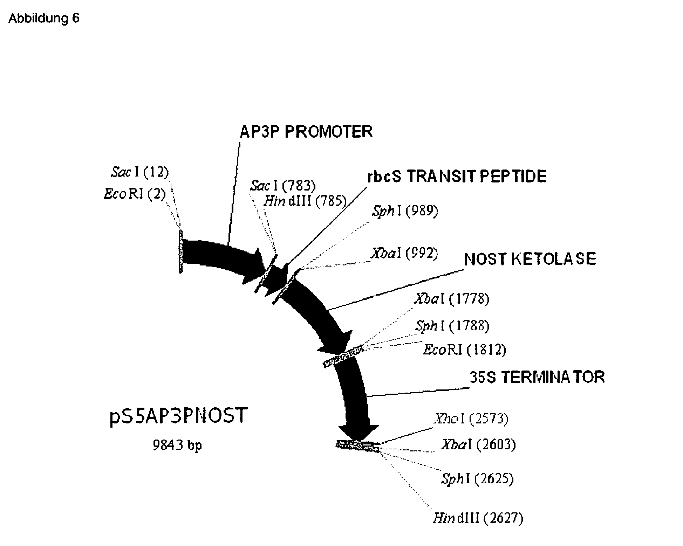

Zur Herstellung des Expressionsvektors pS5AP3PNOST wurde das 2.6 KB bp SacI-XhoI (partielle SacI Hydrolyse) Fragment aus pS5AP3PNOST mit dem SacI-XhoI geschnittenen Vektor pSUN5 ligiert (Abbildung 6, Konstruktkarte). In der Abbildung 6 beinhaltet Fragment AP3P den modifizierten AP3P Promoter (783 bp), Fragment rbcS das rbcS Transitpeptid aus Erbse (207 bp), Fragment NOSTF-G (792 bp) die gesamte Primärsequenz codierend für die Nostoc Ketolase, Fragment term (795 bp) das Polyadenylierungssignal von CaMV.

### Beispiel I.6:

### Herstellung transgener Tagetes Pflanzen

Tagetessamen werden sterilisiert und auf Keimungsmedium (MS-Medium; Murashige and Skoog, Physiol. Plant. 15(1962), 473-497) pH 5,8, 2 % Saccharose) aufgelegt. Die Keimung erfolgt in einem Temperatur/Licht/Zeitintervall von 18 bis 28_C/20-200 mE/3 bis 16 Wochen, bevorzugt jedoch bei 21_C, 20 bis 70 mE, für 4 bis 8 Wochen.

Alle Blätter der sich bis dahin entwickelten in vitro Pflanzen werden geerntet und quer zur Mittelrippe geschnitten. Die dadurch entstehenden Blattexplantate mit einer Größe von 10 bis 60 mm² werden im Verlaufe der Präparation in flüssigem MS-Medium bei Raumtemperatur für maximal 2 h aufbewahrt.

Ein beliebiger Agrobakterium tumefaciens Stamm, bevorzugt aber ein supervirulenter Stamm, wie z.B. EHA105 mit einem entsprechenden Binärplasmid, das ein Selektionsmarkergen (bevorzugt *bar* oder *pat*) sowie ein oder mehrere Trait- oder Reportergene tragen kann wird (beispielsweise pS5KETO2 und pS5AP3PKET02), über Nacht angezogen und für die Co-Kultivierung mit dem Blattmaterial verwendet. Die Anzucht des Bakterienstammes kann wie folgt erfolgen: Eine Einzelkolonie des entsprechenden Stammes wird in YEB (0,1 % Hefeextrakt, 0,5 % Rindfleischextrakt, 0,5 % Pepton, 0,5 % Saccharose, 0,5 % Magnesiumsulfat x 7 H₂0) mit 25 mg/l Kanamycin angeimpft und bei 28_C für 16 bis 20 h angezogen. Anschließend wird die Bakteriensuspension durch Zentrifugation bei 6000 g für 10 min geerntet und derart in flüssigem MS Medium resuspendiert, dass eine OD₆₀₀ von ca. 0,1 bis 0,8 entstand. Diese Suspension wird für die C-Kultivierung mit dem Blattmaterial verwendet.

Unmittelbar vor der Co-Kultivierung wird das MS-Medium, in dem die Blätter aufbewahrt worden sind, durch die Bakteriensuspension ersetzt. Die Inkubation der Blättchen in der Agrobakteriensuspension erfolgte für 30 min unter leichtem Schütteln bei Raumtemperatur. Anschließend werden die infizierten Explantate auf ein mit Agar (z.B. 0,8 % Plant Agar (Duchefa, NL) verfestigtes MS-Medium mit Wachstumsregulatoren, wie beispielsweise 3 mg/l Benzylaminopurin (BAP) sowie 1 mg/l Indolylessigsäure (IAA) aufgelegt. Die Orientierung der Blätter auf dem Medium ist bedeutungslos. Die Kultivierung der Explantate findet für 1 bis 8 Tage, bevorzugt aber für 6 Tage statt, dabei können folgende Bedingungen angewendet werden: Lichtintensität: 30 bis 80 mMol/m² x sec, Temperatur: 22 bis 24°C, hell/dunkel Wechsel von 16/8 Stunden. Anschließend werden die co-kultivierten Explantate auf frisches MS-Medium, bevorzugt mit den gleichen Wachstumsregulatoren übertragen, wobei dieses zweite Medium zusätzlich ein Antibiotikum zur Unterdrückung des Bakterienwachstums enthält. Timentin in einer Konzentration von 200 bis 500 mg/l ist für diesen Zweck sehr geeignet. Als zweite selektive Komponente wird eine für die Selektion des Transformationserfolges eingesetzt. Phosphinothricin in einer Konzentration von 1 bis 5 mg/l selektiert sehr effizient, aber auch andere selektive Komponenten gemäß des zu verwendenden Verfahrens sind denkbar.

Nach jeweils ein bis drei Wochen erfolgt der Transfer der Explantate auf frisches Medium bis sich Sprossknospen und kleine Sprosse entwickeln, die dann auf das gleiche Basalmedium einschließlich Timentin und PPT oder alternative Komponenten mit Wachstumsregulatoren, nämlich z.B. 0,5 mg/l Indolylbuttersäure (IBA) und 0,5 mg/l Gibberillinsäure GA₃, zur Bewurzelung übertragen werden. Bewurzelte Sprosse können ins Gewächshaus überführt werden.

Zusätzlich zu der beschriebenen Methode sind folgende vorteilhafte Modifikationen möglich:
- Bevor die Explantate mit den Bakterien infiziert werden, können sie für 1 bis 12 Tage, bevorzugt 3 bis 4, auf das oben beschriebene Medium für die Co-Kultur vorinkubiert werden. Anschließend erfolgt die Infektion, Co-Kultur und selektive Regeneration wie oben beschrieben.
- Der pH Wert für die Regeneration (normalerweise 5,8) kann auf pH 5,2 gesenkt werden. Dadurch wird die Kontrolle des Agrobakterienwachstums verbessert.
- Die Zugabe von AgNO₃ (3 - 10 mg/l) zum Regenerationsmedium verbessert den Zustand der Kultur einschließlich der Regeneration selbst.
- Komponenten, die die Phenolbildung reduzieren und dem Fachmann bekannt sind, wie z.B. Zitronensäure, Ascorbinsäure, PVP u.v.a.m., wirken sich positiv auf die Kultur aus.
- Für das gesamte Verfahren kann auch flüssiges Kulturmedium Verwendung finden. Die Kultur kann auch auf handelsüblichen Trägern, die auf dem flüssigen Medium positioniert werden inkubiert werden.

Gemäß der oben beschriebenen Transformationsmethode wurden mit folgenden Expressionskonstrukten folgende Linien erhalten:
Mit pS5KETO2 wurde beispielsweise erhalten: cs18-1 und cs18-2, mit
pS5AP3PKET02 wurde beispielsweise erhalten: cs19-1, cs19-2 und cs19-3.
Mit pS5FNRNOST wurde beispielsweise erhalten: ms 103-1, ms103-2, ms103-3, mit
pS5AP3NOST wurde beispielsweise erhalten: ms 104-1, ms104-2, ms104-3.

### Beispiel 1.8

### Charakterisierung der transgenen Pflanzenblüten

### Beispiel I.8.1

### Trennung von Carotinoidestern in Blütenblättern transgener Pflanzen

Allgemeine Arbeitsvorschrift:

Die Blütenblätter der transgenen Pflanzen werden in flüssigem Stickstoff gemörsert und das Petalenpulver (etwa 40 mg) mit 100 % Aceton extrahiert (dreimal je 500 ml). Das Lösungsmittel wird evaporiert und die Carotinoide in 100 bis 200 ml Petrolether/Aceton (5:1, v/v) resuspendiert.

Die Carotinoide werden in konzentrierter Form mittels Dünnschicht-Chromatographie (TLC) auf Silica60 F254- Platten (Merck) in einem organischen Laufmittel (Petrolether/Aceton; 5:1) entsprechend ihrer Phobizität aufgetrennt. Gelbe (Xanthophyllester), rote (Ketocarotinoidester) und orange Banden (Mischung aus Xanthophyll- und Ketocarotinoidestern) auf der TLC werden ausgekratzt.

Die an Silica gebundenen Carotinoide werden dreimal mit 500 ml Aceton eluiert, das Lösungsmittel evaporiert und die Carotinoide mittels HPLC aufgetrennt und identifiziert.

Mittels einer C30-reverse phase-Säule kann zwischen Mono- und Diestern der Carotinoide unterschieden werden. HPLC-Laufbedingungen waren nahezu identisch mit einer publizierten Methode (Frazer et al.(2000), Plant Journal 24(4): 551-558). Eine Identifizierung der Carotinoide ist aufgrund der UV-VIS-Spektren möglich.

### Beispiel 1.9

### Enzymatische Hydrolyse von Carotinoidestern und Identifizierung der Carotinoide

### Allgemeine Arbeitsvorschrift

Gemörsertes Petalenmaterial (50 bis 100 mg Frischgewicht) wird mit 100 % Aceton (dreimal 500 ml; jeweils etwa 15 Minuten schütteln) extrahiert. Das Lösungsmittel wird evaporiert. Carotinoide werden anschließend in 400 ml Aceton aufgenommen (Absorption bei 475 nm zwischen 0.75 und 1,25) und 5 min im Ultraschall-Bad behandelt. Der Carotinoid-Extrakt wird mit 300 ml 50 mM Tris-HCl-Puffer (pH 7,0) gemischt und 5 bis 10 Minuten bei 37C inkubiert. Danach erfolgt die Zugabe von 100 bis 200 ml Cholesterol-Esterase (Stammlösung: 6,8 units/ml einer Cholesterol-Esterase von Pseudomonas spec.). Nach 8 bis 12 Stunden wird nochmals 100 bis 200 ml Enzym zugegeben; Hydrolyse der Ester erfolgt innerhalb von 24 Stunden bei Inkubation bei 37C. Nach Zugabe 0.35 g Na2S04x10H20 und 500 ml Petrolether wird gut gemischt und zentrifugiert (3 Minuten; 4500 g). Petrolether-Phase wird abgezogen und nochmals mit 0,35 g Na2S04x10H20 (anhydrous) gemischt. Zentrifugation für 1 Minute bei 10000 g. Petrolether wird evaporiert und freie Carotinoide werden in 100 bis 120 ml Aceton aufgenommen. Mittels HPLC und C30-reverse phase-Säule können freie Carotinoide aufgrund von Retentionszeit und UV-VIS-Spektren identifiziert werden.

Beispiel I.10:

### Herstellung eines Klonierungsvektors zur Herstellung von Inverted-Repeat-Expressionskassetten für die blütenspezifischen Expression von Epsilon-cyclase dsRNAs in Tagetes erecta

Die Expression von Inverted-Repeat Transkripten bestehend aus Fragmenten der Epsilon-Cyclase in *Tagetes erecta* erfolgte unter Kontrolle einer modifizierten Version AP3P des blütenspezifischen Promoters AP3 aus *Arabidopsis thaliana* (AL132971: Nukleotidregion 9298 bis 10200; Hill et al. (1998) Development 125: 1711 bis 1721).

Das Inverted-Repeat Transkript enthält jeweils ein Fragment in korrekter Orientierung (Sense-Fragment) und ein sequenzidentisches Fragment in entgegengesetzter Orientierung (Antisense-Fragment), die durch ein funktionelles Intron, das PIV2 Intron des ST-LH1 Genes aus Kartoffel (Vancanneyt G. et al.(1990) Mol Gen Genet 220: 245-50) mit einander verbunden sind.

Die cDNA, die für den AP3 Promoter (-902 bis +15) aus *Arabidopsis thaliana* codiert, wurde mittels PCR unter Verwendung genomischer DNA (nach Standardmethode aus *Arabidopsis thaliana* isoliert) und der Primer PR7 (SEQ ID NO: 49) und PR10 (SEQ ID NO: 52) hergestellt.

Die PCR-Bedingungen waren die folgenden:

Die PCR zur Amplifikation der DNA, die das AP3-Promoterfragment (-902 bis +15) codiert, erfolgte in einem 50 ml Reaktionsansatz, in dem enthalten war:
- 1 ml genomischer DNA aus *A. thaliana* (1:100 verd hergestellt wie oben beschrieben)
- 0.25 mM dNTPs
- 0.2 mM PR7 (SEQ ID NO: 49)
- 0.2 mM PR10 (SEQ ID NO: 52)
- 5 ml 10X PCR-Puffer (Stratagene)
- 0.25 ml Pfu Polymerase (Stratagene)
- 28.8 ml Aq. Dest.

Die PCR wurde unter folgenden Zyklusbedingungen durchgeführt:

| | | |
|---|---|---|
| 1X | 94_C | 2 Minuten |
| 35X | 94_C | 1 Minute |
| | 50_C | 1 Minute |
| | 72_C | 1 Minute |
| 1X | 72_C | 10 Minuten |

Das 922 Bp Amplifikat wurde unter Verwendung von Standardmethoden in den PCR-Klonierungsvektor pCR 2.1 (Invitrogen) kloniert und das Plasmid pTAP3 erhalten. Sequenzierung des Klons pTAP3 bestätigte eine Sequenz, die sich lediglich in durch eine Insertion (ein G in Position 9765 der Sequenz AL132971) und einen Basenaustausch (ein G statt ein A in Position 9726 der Sequenz AL132971) von der publizierten AP3 Sequenz (AL132971, Nukleotidregion 9298 bis 10200) unterscheidet (Position 33: T statt G, Position 55: T statt G). Diese Nukleotidunterschiede wurden in einem unabhängigen Amplifikationsexperiment reproduziert und repräsentieren somit die Nukleotidsequenz in der verwendeten *Arabidopsis thaliana* Pflanze.

Die modifizierte Version AP3P wurde mittels rekombinanter PCR unter Verwendung des Plasmids pTAP3 hergestellt. Die Region 10200 bis 9771 wurde mit den Primern PR7 (SEQ ID NO: 49) und Primern PR9 (SEQ ID NO: 51) amplifiziert (Amplifikat A7/9), die Region 9526 bis 9285 wurde mit den PR8 (SEQ ID NO: 50) und PR10 (SEQ ID NO: 52) amplifiziert (Amplifikat A8/10).

Die PCR-Bedingungen waren die folgenden:

Die PCR-Reaktionen zur Amplifikation der DNA-Fragmente, die für die Regionen Region 10200 bis 9771 und 9526 bis 9285 des AP3 Promoters codieren, erfolgte in 50 ml Reaktionsansätzen, in denen enthalten war:
- 100 ng AP3 Amplifikat (oben beschrieben)
- 0.25 mM dNTPs
- 0.2 mM PR7 (SEQ ID NO: 49) bzw. PR8 (SEQ ID NO: 50)
- 0.2 mM PR9 (SEQ ID NO: 51) bzw. PR10 (SEQ ID NO: 52)
- 5 ml 10X PCR-Puffer (Stratagene)
- 0.25 ml Pfu Taq Polymerase (Stratagene)
- 28.8 ml Aq. Dest.

Die PCR wurde unter folgenden Zyklusbedingungen durchgeführt:

| | | |
|---|---|---|
| 1X | 94_C | 2 Minuten |
| 35X | 94_C | 1 Minute |
| | 50_C | 2 Minuten |
| | 72_C | 3 Minuten |
| 1X | 72_C | 10 Minuten |

Die rekombinante PCR beinhaltet Annealing der sich über eine Sequenz von 25 Nukleotiden überlappenden Amplifikate A7/9 und A8/10, Vervollständigung zu einem Doppelstrang und anschließende Amplifizierung. Dadurch entsteht eine modifizierte Version des AP3 Promoters, AP3P, in dem die Positionen 9670 bis 9526 deletiert sind. Die Denaturierung (5 min bei 95_C) und Annealing (langsame Abkühlung bei Raumtemperatur auf 40_C) beider Amplifikate A7/9 und A8/10 erfolgte in einem 17.6 ml Reaktionsansatz, in dem enthalten war:
- 0.5 mg A7/9
- 0.25 mg A8/10

Das Auffüllen der 3'Enden (30 min bei 30_C) erfolgte in einem 20 ml Reaktionsansatz, in dem enthalten war:
- 17.6 ml A7/9 und A8/10-Annealingsreaktion (hergestellt wie oben beschrieben)
- 50 mM dNTPs
- 2 ml 1X Klenow Puffer
- 2U Klenow Enzym

Die Nukleinsäure codierend für die modifizierte Promoterversion AP3P wurde mittels PCR unter Verwendung eines sense spezifischen Primers (PR7 SEQ ID NO: 49) und eines antisense spezifischen Primers (PR10 SEQ ID NO: 52) amplifiziert.

Die PCR-Bedingungen waren die folgenden:

Die PCR zur Amplifikation des AP3P Fragmentes erfolgte in einem 50 ml Reaktionsansatz, in dem enthalten war:
- 1 ml Annealingsreaktion (hergestellt wie oben beschrieben)
- 0.25 mM dNTPs
- 0.2 mM PR7 (SEQ ID NO: 49)
- 0.2 mM PR10 (SEQ ID NO: 52)
- 5 ml 10X PCR-Puffer (Stratagene)
- 0.25 ml Pfu Taq Polymerase (Stratagene)
- 28.8 ml Aq. Dest.

Die PCR wurde unter folgenden Zyklusbedingungen durchgeführt:

| | | |
|---|---|---|
| 1X | 94_C | 2 Minuten |
| 35X | 94_C | 1 Minute |
| | 50_C | 1 Minuten |
| | 72_C | 1 Minuten |
| 1X | 72_C | 10 Minuten |

Die PCR-Amplifikation mit PR7, SEQ ID NO: 49 und PR10 SEQ ID NO: 52 resultierte in einem 778 Bp Fragment das für die modifizierte Promoterversion AP3P codiert. Das Amplifikat wurde in den Klonierungsvektor pCR2.1 (Invitrogen) kloniert. Sequenzierungen mit den Primern T7 und M13 bestätigten eine zur Sequenz AL132971, Region 10200 bis 9298 identische Sequenz, wobei die interne Region 9285 bis 9526 deletiert wurde. Diese Klon wurde daher für die Klonierung in den Expressionsvektor pJIT117 (Guerineau et al. 1988, Nucl. Acids Res. 16: 11380) verwendet.

Die Klonierung erfolgte durch Isolierung des 771 Bp SacI-HindIII Fragmentes aus pTAP3P und Ligierung in den SacI-HindIII geschnittenen Vektor pJIT117. Der Klon, der den Promoter AP3P anstelle des ursprünglichen Promoters d35S enthält, heisst pJAP3P.

Ein DNA-Fragment, das das PIV2 Intron des Gens ST-LS1 enthält wurde mittels PCR unter Verwendung von Plasmid-DNA p35SGUS INT (Vancanneyt G. et al.(1990) Mol Gen Genet 220: 245-50)sowie der Primer PR40 (Seq ID NO: 54) und Primer PR41 (Seq ID NO: 55) hergestellt.

Die PCR-Bedingungen waren die folgenden:

Die PCR zur Amplifikation der Sequenz des Intron PIV2 des Gens ST-LS1, erfolgte in einem 50 ml Reaktionsansatz, in dem enthalten war:
- 1 ml p35SGUS INT
- 0.25 mM dNTPs
- 0.2 mM PR40 (SEQ ID NO: 54)
- 0.2 mM PR41 (SEQ ID NO: 55)
- 5 ml 10X PCR-Puffer (TAKARA)
- 0.25 ml R Taq Polymerase (TAKARA)
- 28.8 ml Aq. Dest.

Die PCR wurde unter folgenden Zyklusbedingungen durchgeführt:

| | | |
|---|---|---|
| 1X | 94_C | 2 Minuten |
| 35X | 94_C | 1 Minute |
| | 53_C | 1 Minuten |
| | 72_C | 1 Minuten |
| 1X | 72_C | 10 Minuten |

Die PCR-Amplifikation mit PR40 und PR41 resultierte in einem 206 Bp-Fragment. Unter Verwendung von Standardmethoden wurde das Amplifikat in den PCR-Klonierungsvektor pBluntII (Invitrogen) kloniert und der Klon pBluntII-40-41 erhalten. Sequenzierungen dieses Klons mit dem Primer SP6 bestätigte eine Sequenz, die identisch ist mit der entsprechenden Sequenz aus dem Vektor p35SGUS INT.

Dieser Klon wurde daher für die Klonierung in den Vektor pJAP3P (oben beschrieben).

Die Klonierung erfolgte durch Isolierung des 206 Bp SalI-BamHI Fragmentes aus pBluntll-40-41 und Ligierung mit dem SalI-BamHI geschnittenen Vektor pJAP3P. Der Klon, der das Intron PIV2 des Gens ST-LS1 in der korrekten Orientierung anschließend an das 3'Ende des rbcs Transitpeptides enthält, heisst pJAI1 und ist geeignet, Expressionskassetten für die blütenspezifische Expression von Inverted-Repeat Transkripten herzustellen.

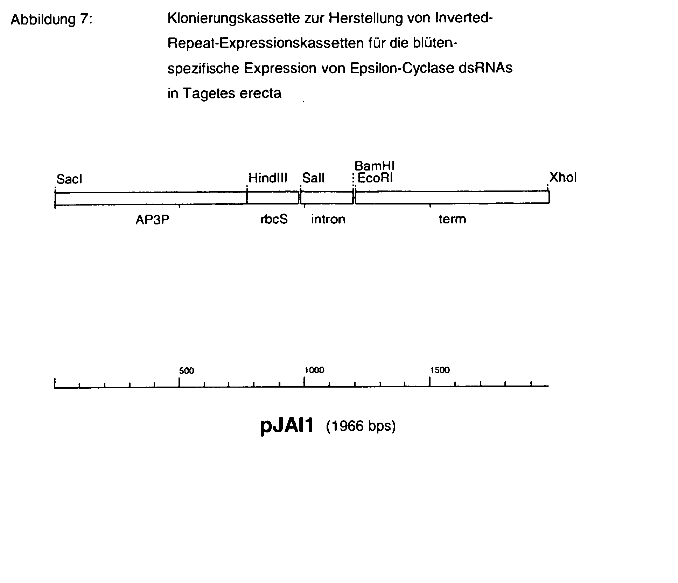

In der Abbildung 7 beinhaltet Fragment *AP3P* den modifizierten AP3P Promoter (771 bp), Fragment *rbcs* das rbcS Transitpeptid aus Erbse (204 bp), Fragment *intron* das Intron PIV2 des Kartoffel-Gens ST-LS1, und Fragment *term* (761 Bp) das Polyadenylierungssignal von CaMV.

### Beispiel I.11

### Herstellung von Inverted-Repeat-Expressionskassetten für die blütenspezifische Expression von Epsilon-cyclase dsRNAs in Tagetes erecta (gerichtet gegen die 5'Region der Epsilon-Cyclase cDNA)

Die Nukleinsäure, die die 5'terminale 435bp Region der Epsilon-Cyclase cDNA (Genbank accession NO: AF251016) enthält, wurde mittels polymerase chain reaction (PCR) aus *Tagetes erecta* cDNA unter Verwendung eines sense spezifischen Primers (PR42 SEQ ID NO: 56) und eines antisense spezifischen Primers (PR43 SEQ ID NO: 57) amplifiziert. Die 5'terminale 435 bp Region der Epsilon-Cyclase cDNA aus *Tagetes erecta* setzt sich zusammen aus 138 bp 5'Nicht-translatierter Sequenz (5'UTR) und 297 bp der dem N-Terminus entsprechenden kodierenden Region.

Für die Präparation von Total-RNA aus Blüten von Tagetes wurden 100mg der gefrorenen, pulverisierten Blüten in ein Reaktionsgefäß überführt und in 0.8 ml Trizol-Puffer (LifeTechnologies) aufgenommen. Die Suspension wurde mit 0.2 ml Chloroform extrahiert. Nach 15 minütiger Zentrifugation bei 12000 g wurde der wässrige Überstand abgenommen und in ein neues Reaktionsgefäß überführt und mit einem Volumen Ethanol extrahiert. Die RNA wurde mit einem Volumen Isopropanol gefällt, mit 75% Ethanol gewaschen und das Pellet in DEPC Wasser (über Nacht Inkubation von Wasser mit 1/1000 Volumen Diethylpyrocarbonat bei Raumtemperatur, anschließend autoklaviert) gelöst. Die RNA-Konzentration wurde photometrisch bestimmt. Für die cDNA-Synthese wurden 2.5 ug Gesamt-RNA für 10 min bei 60_C denaturiert, für 2 min auf Eis abgekühlt und mittels eines cDNA-Kits (Ready-to-go-you-prime-beads, Pharmacia Biotech) nach Herstellerangaben unter Verwendung eines antisense spezifischen Primers (PR17 SEQ ID NO: 53) in cDNA umgeschrieben.

Die Bedingungen der anschließenden PCR-Reaktionen waren die folgenden:

Die PCR zur Amplifikation des PR42-PR43 DNA-Fragmentes, das die 5'terminale 435bp Region der Epsilon-Cyclase enthält, erfolgte in einem 50 ml Reaktionsansatz, in dem enthalten war:
- 1 ml cDNA (hergestellt wie oben beschrieben)
- 0.25 mM dNTPs
- 0.2 mM PR42 (SEQ ID NO: 56)
- 0.2 mM PR43 (SEQ ID NO: 57)
- 5 ml 10X PCR-Puffer (TAKARA)
- 0.25 ml R Taq Polymerase (TAKARA)
- 28.8 ml Aq. Dest.

Die PCR zur Amplifikation des PR44-PR45 DNA-Fragmentes, das die 5'terminale 435 bp Region der Epsilon-Cyclase enthält, erfolgte in einem 50 ml Reaktionsansatz, in dem enthalten war:
- 1 ml cDNA (hergestellt wie oben beschrieben)
- 0.25 mM dNTPs
- 0.2 mM PR44 (SEQ ID NO: 58)
- 0.2 mM PR45 (SEQ ID NO: 59)
- 5 ml 10X PCR-Puffer (TAKARA)
- 0.25 ml R Taq Polymerase (TAKARA)
- 28.8 ml Aq. Dest.

Die PCR-Reaktionen wurden unter folgenden Zyklusbedingungen durchgeführt:

| | | |
|---|---|---|
| 1X | 94_C | 2 Minuten |
| 35X | 94_C | 1 Minute |
| | 58_C | 1 Minuten |
| | 72_C | 1 Minuten |
| 1X | 72_C | 10 Minuten |

Die PCR-Amplifikation mit Primer PR42 und PR43 resultierte in einem 443 Bp-Fragment, die PCR-Amplifikation mit Primer PR44 und PR45 resultierte in einem 444 Bp-Fragment.

Die beiden Amplifikate, das PR42-PR43 (HindIII-SalI sense) Fragment und das PR44-PR45 (EcoRI-BamHI antisense) Fragment, wurden unter Verwendung von Standardmethoden in den PCR-Klonierungsvektor pCR-BluntII (Invitrogen) kloniert. Sequenzierungen mit dem Primer SP6 bestätigten jeweils eine zur publizierten Sequenz AF251016 (SEQ ID NO: 38 ) identische Sequenz abgesehen von den eingeführten Restriktionsstellen. Diese Klone wurde daher für die Herstellung eines Inverted-Repeat Konstrukts in dem Klonierungsvektor pJAI1 (siehe Beispiel 1.10) verwendet.

Der erste Klonierungsschritt erfolgte durch Isolierung des 444 Bp PR44-PR45 BamHI-EcoRI Fragmentes aus dem Klonierungsvektor pCR-BluntII (Invitrogen) und Ligierung mit dem BamHI-EcoRI geschnittenen Vektor pJAI1. Der Klon, der 5'terminale Region der Epsilon-Cyclase in der antisense Orientierung enthält, heisst pJAI2. Durch die Ligation entsteht eine transkriptionelle Fusion zwischen dem antisense Fragment der 5'terminalen Region der Epsilon-Cyclase und dem Polyadenylierungssignal aus CaMV.

Der zweite Klonierungsschritt erfolgte durch Isolierung des 443 Bp PR42-PR43 HindIII-SalI Fragmentes aus dem Klonierungsvektor pCR-Bluntll (Invitrogen) und Ligierung mit dem HindIII-SalI geschnittenen Vektor pJAI2. Der Klon, der 435 bp 5'terminale Region der Epsilon-Cyclase cDNA in der sense Orientierung enthält, heisst pJAI3. Durch die Ligation entsteht eine transkriptionelle Fusion zwischen dem AP3P und dem sense Fragment der 5'terminalen Region der Epsilon-Cyclase.

Für die Herstellung einer Inverted-Repeat Expressionskassette unter Kontrolle des CHRC-Promoters wurde ein CHRC-Promoterfragment unter Verwendung genomischer DNA aus Petunie (nach Standardmethoden hergestellt) sowie der Primer PRCHRC5 (SEQ ID NO: 76) und PRCHRC3 (SEQ ID NO: 77) amplifiziert. Das Amplifikat wurde in den Klonierungsvektor pCR2.1 (Invitrogen) kloniert. Sequenzierungen des resultierenden Klons pCR2.1-CHRC mit den Primern M13 und T7 bestätigten eine zur Sequenz AF099501 identische Sequenz. Dieser Klon wurde daher für die Klonierung in den Expressionsvektor pJAI3 verwendet.

Die Klonierung erfolgte durch Isolierung des 1537 bp SacI-HindIII Fragments aus pCR2.1-CHRC und Ligierung in den SacI-HindIII geschnittenen Vektor pJAI3. Der Klon, der den Promoter CHRC anstelle des ursprünglichen Promoters AP3P enthält heisst pJCI3.

Die Herstellung der Expressionsvektoren für die Agrobacterium-vermittelte Transformation der AP3P- bzw. CHRC-kontrollierten Inverted-Repeat Transkripts in *Tagetes erec*ta erfolgte unter der Verwendung des binären Vektors pSUN5 (WO02/00900).

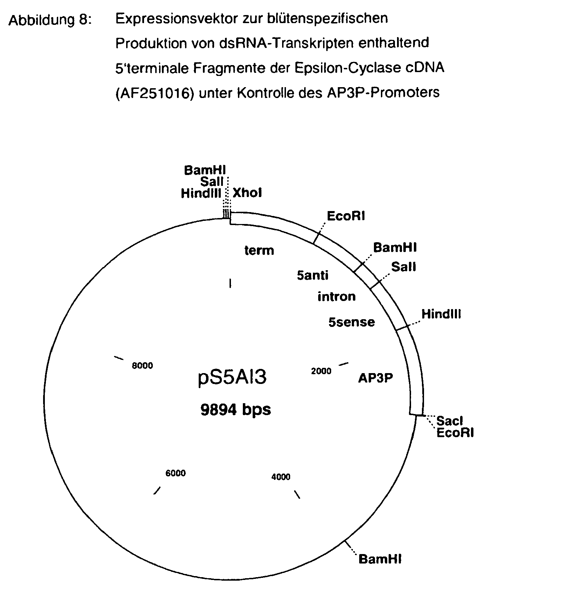

Zur Herstellung des Expressionsvektors pS5AI3 wurde das 2622 bp SacI-XhoI Fragment aus pJAI3 mit dem SacI-XhoI geschnittenen Vektor pSUN5 ligiert (Abbildung 8, Konstruktkarte).

In der Abbildung 8 beinhaltet Fragment *AP3P* den modifizierten AP3P Promoter (771 bp), Fragment *5sense* die 5'Region der Epsilon-Cyclase aus *Tagetes erecta* (435 bp) in Sense-Orientierung, Fragment *intron* das Intron PIV2 des Kartoffel-Gens ST-LS1, Fragment *5anti* die 5'Region der Epsilon- cyclase aus *Tagetes erecta* (435 bp) in antisense Orientierung, und Fragment *term* (761 Bp) das Polyadenylierungssignal von CaMV.

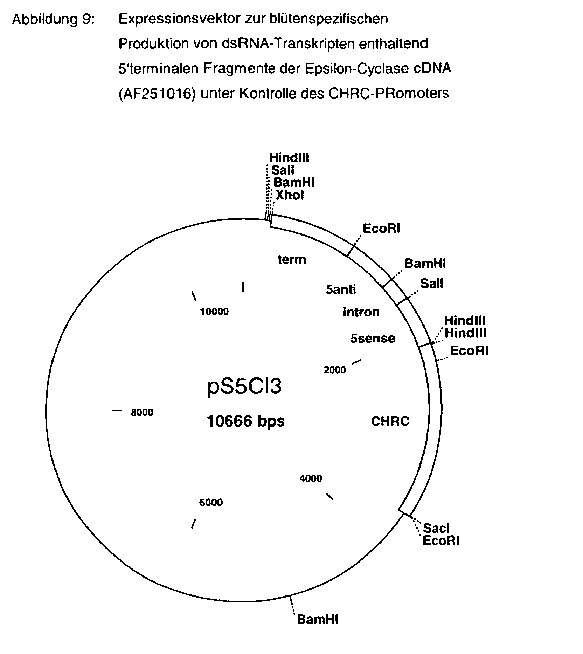

Zur Herstellung des Expressionsvektors pS5CI3 wurde das 3394 bp SacI-XhoI Fragment aus pJCI3 mit dem SacI-XhoI geschnittenen Vektor pSUN5 ligiert (Abbildung 9, Konstruktkarte).

In der Abbildung 9 beinhaltet Fragment *CHRC* den Promoter (1537 bp), Fragment *5sense* die 5'Region der Epsilon-Cyclase aus *Tagetes erecta* (435 bp) in Sense-Orientierung, Fragment *intron* das Intron PIV2 des Kartoffel-Gens ST-LS1, Fragment *5anti* die 5'Region der Epsilon-Cyclase aus *Tagetes erecta* (435 bp) in Antisense-Orientierung, und Fragment *term* (761 Bp) das Polyadenylierungssignal von CaMV.

### Beispiel I.12

### Herstellung einer Inverted-Repeat-Expressionskassette für die blütenspezifische Expression von Epsilon-cyclase dsRNAs in Tagetes erecta (gerichtet gegen die 3'Region der Epsilon-Cyclase cDNA)

Die Nukleinsäure, die die 3'terminale Region (384 bp) der Epsilon-Cyclase cDNA (Genbank accession NO: AF251016) enthält wurde mittels polymerase chain reaction (PCR) aus *Tagetes erecta* cDNA unter Verwendung eines sense spezifischen Primers (PR46 SEQ ID NO: 60) und eines antisense spezifischen Primers (PR47 SEQ ID NO: *61)* amplifiziert. Die 3'terminale Region (384 bp) der Epsilon-Cyclase cDNA aus *Tagetes erecta* setzt sich zusammen aus 140 bp 3'-Nicht-translatierter Sequenz (3'UTR) und 244 bp der dem C-Terminus entsprechenden kodierenden Region.

Die Präparation von Total-RNA aus Blüten von Tagetes erfolgte wie unter Beispiel I.11 beschrieben.

Die cDNA Synthese erfolgte wie unter Beispiel I.11 unter Verwendung des antisense spezifischen Primers PR17 (SEO ID NO: 53) beschrieben.

Die Bedingungen der anschließenden PCR-Reaktionen waren die folgenden:

Die PCR zur Amplifikation des PR46-PR457 DNA-Fragmentes, das die 3'terminale 384 bp Region der Epsilon-Cyclase enthält, erfolgte in einem 50 ml Reaktionsansatz, in dem enthalten war:
- 1 ml cDNA (hergestellt wie oben beschrieben)
- 0.25 mM dNTPs
- 0.2 mM PR46 (SEQ ID NO: 60)
- 0.2 mM PR47 (SEQ ID NO: 61)
- 5 ml 10X PCR-Puffer (TAKARA)
- 0.25 ml R Taq Polymerase (TAKARA)
- 28.8 ml Aq. Dest.

Die PCR zur Amplifikation des PR48-PR49 DNA-Fragmentes, das die 5'terminale 384 bp Region der Epsilon-Cyclase enthält, erfolgte in einem 50 ml Reaktionsansatz, in dem enthalten war:
- 1 ml cDNA (hergestellt wie oben beschrieben)
- 0.25 mM dNTPs
- 0.2 mM PR48 (SEQ ID NO: 62)
- 0.2 mM PR49 (SEQ ID NO: 63)
- 5 ml 10X PCR-Puffer (TAKARA)
- 0.25 ml R Taq Polymerase (TAKARA)
- 28.8 ml Aq. Dest.

Die PCR-Reaktionen wurden unter folgenden Zyklusbedingungen durchgeführt:

| | | |
|---|---|---|
| 1X | 94_C | 2 Minuten |
| 35X | 94_C | 1 Minute |
| | 58_C | 1 Minuten |
| | 72_C | 1 Minuten |
| 1X | 72_C | 10 Minuten |

Die PCR-Amplifikation mit SEQ ID NO: 60 und SEQ ID NO: 61 resultierte in einem 392 Bp-Fragment, die PCR-Amplifikation mit SEQ ID NO: 62 und SEQ ID NO: 63 resultierte in einem 396 Bp-Fragment.

Die beiden Amplifikate, das PR46-PR47 Fragment und das PR48-PR49 Fragment, wurden unter Verwendung von Standardmethoden in den PCR-Klonierungsvektor pCR-Bluntll (Invitrogen) kloniert. Sequenzierungen mit dem Primer SP6 bestätigten jeweils eine zur publizierten Sequenz AF251016 (SEQ ID NO: 38) identische Sequenz abgesehen von den eingeführten Restriktionsstellen. Diese Klone wurde daher für die Herstellung eines Inverted-Repeat Konstrukts in dem Klonierungsvektor pJAI1 (siehe Beispiel I.10) verwendet.

Der erste Klonierungsschritt erfolgte durch Isolierung des 396 Bp PR48-PR49 BamHI-EcoRI Fragmentes aus dem Klonierungsvektor pCR-Bluntll (Invitrogen) und Ligierung mit dem BamHI-EcoRI geschnittenen Vektor pJAI1. Der Klon, der 3'terminale Region der Epsilon-Cyclase in der antisense Orientierung enthält, heisst pJAI4. Durch die Ligation entsteht eine transkriptionelle Fusion zwischen dem Antisense-Fragment der 3'terminale Region der Epsilon-Cyclase und dem Polyadenylierungssignal aus CaMV.

Der zweite Klonierungsschritt erfolgte durch Isolierung des 392 Bp PR46-PR47 HindIII-SalI Fragmentes aus dem Klonierungsvektor pCR-Bluntll (Invitrogen) und Ligierung mit dem HindIII-SalI geschnittenen Vektor pJAI4. Der Klon, der 392 bp 3'terminale Region der Epsilon-Cyclase cDNA in der sense Orientierung enthält, heisst pJAI5. Durch die Ligation entsteht eine transkriptionelle Fusion zwischen dem AP3P und dem Sense-Fragment 3'terminale Region der Epsilon-Cyclase.

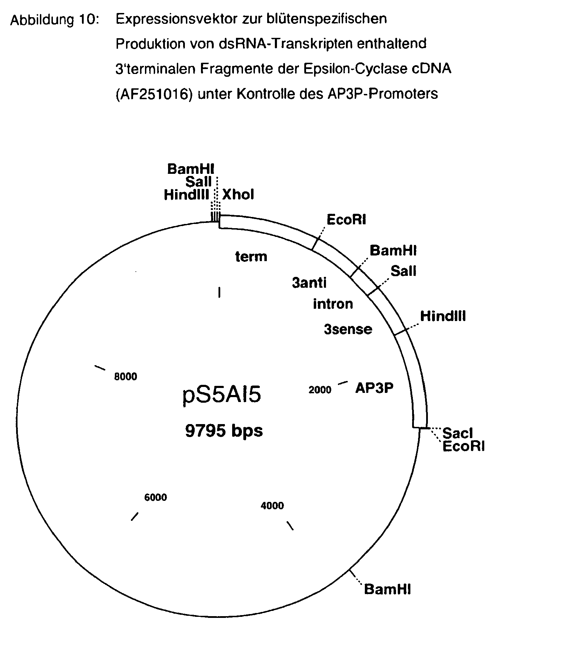

Die Herstellung eines Expressionsvektors für die Agrobacterium-vermittelte Transformation des AP3P-kontrollierten Inverted-Repeat Transkripts in Tagetes erecta erfolgte unter der Verwendung des binären Vektors pSUN5 (WO02/00900). Zur Herstellung des Expressionsvektors pS5AI5 wurde das 2523 bp SacI-XhoI Fragment aus pJAI5 mit dem SacI-XhoI geschnittenen Vektor pSUN5 ligiert (Abbildung 10, Konstruktkarte).

In der Abbildung 10 beinhaltet Fragment *AP3P* den modifizierten AP3P Promoter (771 bp), Fragment *3sense* die 3'region der Epsilon cyclase aus Tagetes erecta (435 bp) in sense Orientierung, Fragment *intron* das Intron IV2 des Kartoffel-Gens ST-LS1, Fragment *3anti* die 3'region der Epsilon cyclase aus Tagetes erecta (435 bp) in antisense Orientierung, und Fragment *term* (761 Bp) das Polyadenylierungssignal von CaMV.

### Beispiel 1.13

### Klonierung des Epsilon-Cyclase Promoters

Ein 199 bp Fragment bzw. das 312 bp Fragment des Epsilon-Cyclase Promoters wurde durch zwei unabhängige Klonierungsstrategien, Inverse PCR (adaptiert Long et al. Proc. Natl. Acad. Sci USA 90: 10370) und TAIL-PCR (Liu Y-G. et al. (1995) Plant J. 8: 457-463) unter Verwendung genomischer DNA (nach Standardmethode aus *Tagetes erecta,* Linie Orangenprinz, isoliert) isoliert.

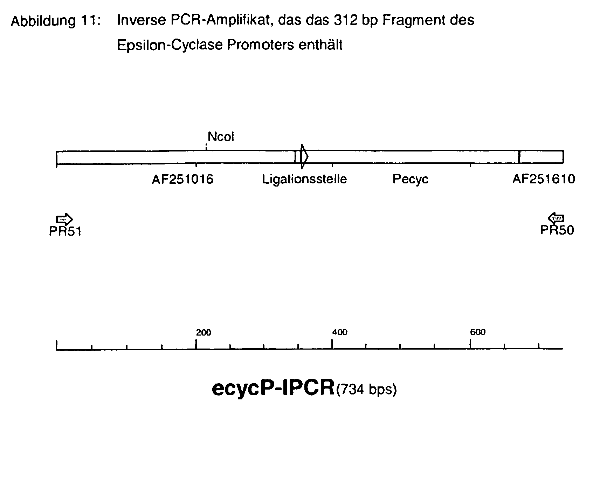

Für den Inverse PCR-Ansatz wurden 2 ug genomische DNA in einem 25 ul Reaktionsansatz mit EcoRV und Rsal verdaut, anschließend auf 300 ml verdünnt und über Nacht bei 16_C mit 3U Ligase religiert. Unter Verwendung der Primer PR50 (SEQ ID NO: 64) und PR51 (SEQ ID NO: 65) wurde durch PCR Amplifikation ein Fragment hergestellt, das, jeweils in Sense-Orientierung, 354 bp der Epsilon-Cyclase cDNA (Genbank Accession AF251016), ligiert an 300 bp des Epsilon-Cyclase Promoters sowie 70 bp des 5'terminalen Bereichs der cDNA Epsilon-Cyclase enthält (siehe Abbildung 11).

Die Bedingungen der PCR-Reaktionen waren die folgenden:

Die PCR zur Amplifikation des PR50-PR51 DNA-Fragmentes, das unter anderem das 312 bp Promoterfragment der Epsilon-Cyclase enthält, erfolgte in einem 50 ml Reaktionsansatz, in dem enthalten war:
- 1 ml Ligationsansatz (hergestellt wie oben beschrieben)
- 0.25 mM dNTPs
- 0.2 mM PR50 (SEQ ID NO: 64)
- 0.2 mM PR51 (SEQ ID NO: 65)
- 5 ml 10X PCR-Puffer (TAKARA)
- 0.25 ml R Taq Polymerase (TAKARA)
- 28.8 ml Aq. Dest.

Die PCR-Reaktionen wurden unter folgenden Zyklusbedingungen durchgeführt:

| | | |
|---|---|---|
| 1X | 94_C | 2 Minuten |
| 35X | 94_C | 1 Minute |
| | 53_C | 1 Minute |
| | 72_C | 1 Minute |
| 1X | 72_C | 10 Minuten |

Die PCR-Amplifikation mit Primer PR50 und PR51 resultierte in einem 734 Bp-Fragment, das unter anderem das 312 bp Promoterfragment der Epsilon-Cyclase enthält (Abbildung 11).

Das Amplifikat, wurde unter Verwendung von Standardmethoden in den PCR-Klonierungsvektor pCR2.1 (Invitrogen) kloniert. Sequenzierungen mit den Primern M13 und T7 ergaben die Sequenz SEQ ID NO: 45. Diese Sequenz wurde in einem unabhängigen Amplifikationsexperiment reproduziert und repräsentiert somit die Nukleotid-sequenz in der verwendeten *Tagetes erecta* Linie Orangenprinz.

Für den TAIL-PCR Ansatz wurden drei sukzessive PCR-Reaktionen mit jeweils unterschiedlichen gen-spezifischen Primern (nested primers) durchgeführt.

Die TAIL1-PCR erfolgte in einem 20 ml Reaktionsansatz, in dem enthalten war:
- 1 ng genomische DNA (hergestellt wie oben beschrieben)
- 0.2 mM jedes dNTPs
- 0.2 mM PR60 (SEQ ID NO: 66)
- 0.2 mM AD1 (SEQ ID NO: 69)
- 2 ml 10X PCR-Puffer (TAKARA)
- 0.5 U R Taq Polymerase (TAKARA)
- mit Aq. Dest. auf 20 ml aufgefüllt
- AD1 stellte dabei zunächst eine Mischung aus Primern der Sequenzen (a/c/g/t)tcga(g/c)t(a/t)t(g/c)g(a/t)gtt dar.

Die PCR-Reaktion TAIL1 wurden unter folgenden Zyklusbedingungen durchgeführt:
1 X 93_C: 1 Minute, 95_C: 1 Minute
5X 94_C: 30 Sekunden, 62_C: 1 Minute, 72_C: 2.5 Minuten 1 X 94_C: 30 Sekunden, 25_C: 3 Minuten, ramp to 72_C in 3 Minuten,
72_C: 2.5 Minuten
15X 94_C: 10 Sekunden, 68_C: 1 Minute, 72_C: 2.5 Minuten;
94_C: 10 Sekunden, 68_C: 1 Minute, 72_C: 2.5 Minuten;
94_C: 10 Sekunden, 29_C: 1 Minute, 72_C: 2.5 Minuten
1X 72_C: 5 Minuten

Die TAIL2-PCR erfolgte in einem 21 ml Reaktionsansatz, in dem enthalten war:
- 1 ml einer 1:50 Verdünnung des TAIL1-Reaktionsansatzes (hergestellt wie oben beschrieben)
- 0.8 mM dNTP
- 0.2 mM PR61 (SEQ ID NO: 67)
- 0.2 mM AD1 (SEQ ID NO: 69)
- 2 ml 10X PCR-Puffer (TAKARA)
- 0.5 U R Taq Polymerase (TAKARA)
- mit Aq. Dest. auf 21 ml aufgefüllt

Die PCR-Reaktion TAIL2 wurde unter folgenden Zyklusbedingungen durchgeführt:
12X 94_C: 10 Sekunden, 64_C: 1 Minute, 72_C: 2.5 Minuten;
94_C: 10 Sekunden, 64_C: 1 Minute, 72_C: 2.5 Minuten;
94_C: 10 Sekunden, 29_C: 1 Minute, 72_C: 2.5 Minuten
1 X 72_C: 5 Minuten

Die TAIL3-PCR erfolgte in einem 100 ml Reaktionsansatz, in dem enthalten war:
- 1 ml einer 1:10 Verdünnung des TAIL2-Reaktionsansatzes (hergestellt wie oben beschrieben)
- 0.8 mM dNTP
- 0.2 mM PR63 (SEQ ID NO: 68)
- 0.2 mM AD1 (SEQ ID NO: 69)
- 10 ml 10X PCR-Puffer (TAKARA)
- 0.5 U R Taq Polymerase (TAKARA)
- mit Aq. Dest. auf 100 ml aufgefüllt

Die PCR-Reaktion TAIL3 wurde unter folgenden Zyklusbedingungen durchgeführt:
20X 94_C: 15 Sekunden, 29_C: 30 Sekunden, 72_C: 2 Minuten
1X 72_C: 5 Minuten

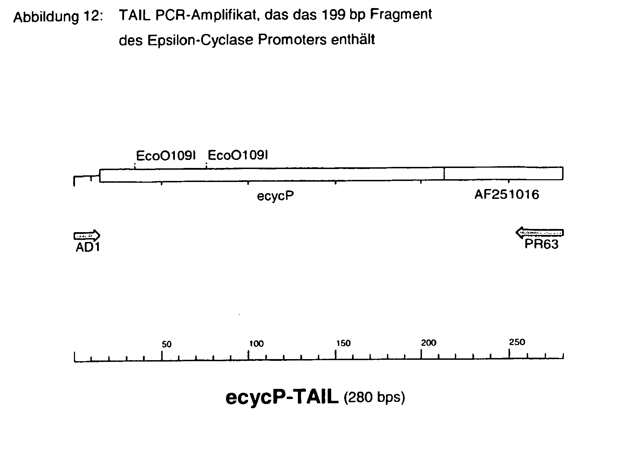

Die PCR-Amplifikation mit Primer PR63 und AD1 resultierte in einem 280 Bp-Fragment, das unter anderem das 199 bp Promoterfragment der Epsilon-Cyclase enthält (Abbildung 12).

Das Amplifikat, wurde unter Verwendung von Standardmethoden in den PCR-Klonierungsvektor pCR2.1 (Invitrogen) kloniert. Sequenzierungen mit den Primern M13 und T7 ergaben die Sequenz SEQ ID NO: 46. Diese Sequenz ist identisch mit der Sequenz SEQ ID NO: 45, die mit der IPCR Strategie isoliert wurde und repräsentiert somit die Nukleotidsequenz in der verwendeten *Tagetes erecta* Linie Orangenprinz.

Der pCR2.1-Klon, der das 312 bp Fragment (SEQ ID NO: 45) des Epsilon-Cyclase Promoters, das durch die IPCR-Strategie isoliert wurde, enthält, heisst pTA-ecycP und wurde für die Herstellung der IR Konstrukte verwendet.

### Beispiel I.14

### Herstellung einer Inverted-Repeat-Expressionskassette für die blütenspezifische Expression von Epsilon-cyclase dsRNAs in Tagetes erecta (gerichtet gegen die Promoterregion der Epsilon-Cyclase cDNA).

Die Expression von Inverted-Repeat Transkripten bestehend aus Promoterfragmenten der Epsilon-cyclase in *Tagetes erecta* erfolgte unter Kontrolle einer modifizierten Version AP3P des blütenspezifischen Promoters AP3 aus *Arabidopsis* (siehe Beispiel I.10) oder des blütenspezifischen Promoters CHRC (Genbank accession NO: AF099501). Das Inverted-Repeat Transkript enthält jeweils ein Epsilon-Cyclase-Promoterfragment in korrekter Orientierung (Sense-Fragment) und ein sequenzidentisches Epsilon-Cyclase-Promoterfragment in entgegengesetzter Orientierung (Antisense-Fragment), die durch ein funktionelles Intron (siehe Beispiel 1.10) mit einander verbunden sind.

Die Promoterfragmente wurde mittels PCR unter Verwendung von Plasmid-DNA (Klon pTA-ecycP, siehe Beispiel 1.13 ) und der Primer PR124 (SEQ ID NO: 70) und PR126 (SEQ ID NO: 72) bzw. der Primer PR125 (SEQ ID NO: 71) und PR127 (SEQ ID NO: 73) hergestellt.

Die Bedingungen der PCR-Reaktionen waren die folgenden:

Die PCR zur Amplifikation des PR124-PR126 DNA-Fragmentes, das das Promoterfragment der Epsilon-Cyclase enthält, erfolgte in einem 50 ml Reaktionsansatz, in dem enthalten war:
- 1 ml cDNA (hergestellt wie oben beschrieben)
- 0.25 mM dNTPs
- 0.2 mM PR124 (SEQ ID NO: 70)
- 0.2 mM PR126 (SEQ ID NO: 72)
- 5 ml 10X PCR-Puffer (TAKARA)
- 0.25 ml R Taq Polymerase (TAKARA)
- 28.8 uml Aq. Dest.

Die PCR zur Amplifikation des PR125-PR127 DNA-Fragmentes, das das 312bp Promoterfragment der Epsilon-Cyclase enthält, erfolgte in einem 50 ml Reaktionsansatz, in dem enthalten war:
- 1 ml cDNA (hergestellt wie oben beschrieben)
- 0.25 mM dNTPs
- 0.2 mM PR125 (SEQ ID NO: 71)
- 0.2 mM PR127 (SEQ ID NO: 73)
- 5 ml 10X PCR-Puffer (TAKARA)
- 0.25 ml R Taq Polymerase (TAKARA)
- 28.8 ml Aq. Dest.

Die PCR-Reaktionen wurden unter folgenden Zyklusbedingungen durchgeführt:

| | | |
|---|---|---|
| 1X | 94_C | 2 Minuten |
| 35X | 94_C | 1 Minute |
| | 53_C | 1 Minuten |
| | 72_C | 1 Minuten |
| 1X | 72_C | 10 Minuten |

Die PCR-Amplifikation mit Primer PR124 und PR126 resultierte in einem 358 Bp-Fragment, die PCR-Amplifikation mit Primer PR125 und PR127 resultierte in einem 361 Bp-Fragment.

Die beiden Amplifikate, das PR124-PR126 (HindIII-SalI sense) Fragment und das PR125-PR127 (EcoRI-BamHI antisense) Fragment, wurden unter Verwendung von Standardmethoden in den PCR-Klonierungsvektor pCR-Bluntll (Invitrogen) kloniert. Sequenzierungen mit dem Primer SP6 bestätigten jeweils eine Sequenz, die abgesehen von den eingeführten Restriktionsstellen identisch ist zu SEQ ID NO: 45. Diese Klone wurden daher für die Herstellung eines Inverted-Repeat Konstrukts in dem Klonierungsvektor pJAI1 (siehe Beispiel I.10) verwendet.

Der erste Klonierungsschritt erfolgte durch Isolierung des 358 Bp PR124-PR126 HindIII-SalI Fragmentes aus dem Klonierungsvektor pCR-Bluntll (Invitrogen) und Ligierung mit dem BamHI-EcoRI geschnittenen Vektor pJAI1. Der Klon, das Epsilon-Cyclase Promoterfragment in der sense Orientierung enthält, heisst cs43. Durch die Ligation wird das Sense-Fragment des Epsilon-Cyclase Promoters zwischen den AP3P Promoter und das Intron eingefügt.

Der zweite Klonierungsschritt erfolgte durch Isolierung des 361 Bp PR125-PR127 BamHI-EcoRI Fragmentes aus dem Klonierungsvektor pCR-BluntII (Invitrogen) und Ligierung mit BamHI-EcoRI geschnittenen Vektor cs43. Der Klon, der das Epsilon-Cyclase Promoterfragment in der antisense Orientierung enthält, heisst cs44. Durch die Ligation entsteht eine transkriptionelle Fusion zwischen dem Intron und dem Antisense-Fragment des Epsilon-Cyclase Promoters.

Für die Herstellung einer Inverted-Repeat Expressionskassette unter Kontrolle des CHRC-Promoters wurde ein CHRC-Promoterfragment unter Verwendung genomischer DNA aus Petunie (nach Standardmethoden hergestellt) sowie der Primer PRCHRC3' (SEQ ID NO: 77) und PRCHRC5' (SEQ ID NO: 76) amplifiziert. Das Amplifikat wurde in den Klonierungsvektor pCR2.1 (Invitrogen) kloniert. Sequenzierungen des resultierenden Klons pCR2.1-CHRC mit den Primern M13 und T7 bestätigten eine zur Sequenz AF099501 identische Sequenz. Dieser Klon wurde daher für die Klonierung in den Expressionsvektor cs44 verwendet.

Die Klonierung erfolgte durch Isolierung des 1537 bp SacI-HindIII Fragments aus pCR2.1-CHRC und Ligierung in den SacI-HindIII geschnittenen Vektor cs44. Der Klon, der den Promoter CHRC anstelle des ursprünglichen Promoters AP3P enthält heisst cs45.

Für die Herstellung einer Inverted-Repeat Expressionskassette unter Kontrolle zweier Promotoren, des CHRC-Promoter und des AP3P-Promoters, wurde der AP3P-Promoter in antisense Orientierung an den 3'Terminus des Epsilon-Cyclase antisense Fragmentes in cs45 kloniert. Das AP3P-Promoterfragments aus pJAI1 wurde unter Verwendung der Primer PR128 und PR129 amplifiziert. Das Amplifikat wurde in den Klonierungsvektor pCR2.1 (Invitrogen) kloniert. Die Sequenzierung mit den Primern M13 und T7 bestätigten eine zur Sequenz SEQ ID NO: 28 (AL132971) identische Sequenz. Dieser Klon pCR2.1-AP3PSX wurde für Herstellung einer Inverted-Repeat Expressionskassette unter Kontrolle zweier Promotoren verwendet.

Die Klonierung erfolgte durch Isolierung des 771 bp SalI-XhoI Fragments aus pCR2.1-AP3PSX und Ligierung in den XhoI geschnittenen Vektor cs45. Der Klon, der 3'seitig des Inverted Repeats, den Promoter AP3P in antisense Orientierung enthält heisst cs46.

Die Herstellung der Expressionsvektoren für die Agrobacterium-vermittelte Transformation des AP3P-kontrollierten Inverted-Repeat Transkripts in Tagetes erecta erfolgte unter der Verwendung des binären Vektors pSUN5 (WO 02/00900).

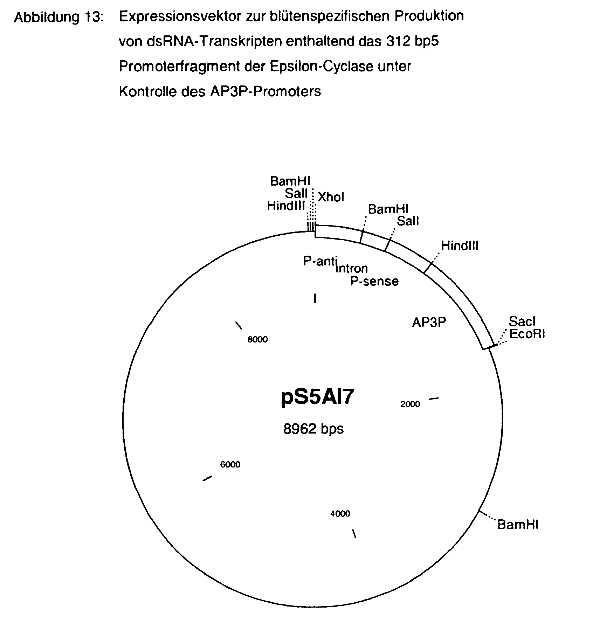

Zur Herstellung des Expressionsvektors pS5AI7 wurde das 1685bp SacI-XhoI Fragment aus cs44 mit dem SacI-XhoI geschnittenen Vektor pSUN5 ligiert (Abbildung 13, Konstruktkarte).In der Abbildung 13 beinhaltet Fragment *AP3P den* modifizierten AP3P Promoter (771 bp), Fragment *P-sense* das 312 bp Promoterfragment der Epsilon-Cyclase in sense Orientierung, Fragment *intron* das Intron IV2 des Kartoffel-Gens ST-LS1), und Fragment *P-anti* das 312 bp Promoterfragment der Epsilon- Cyclase in antisense Orientierung.

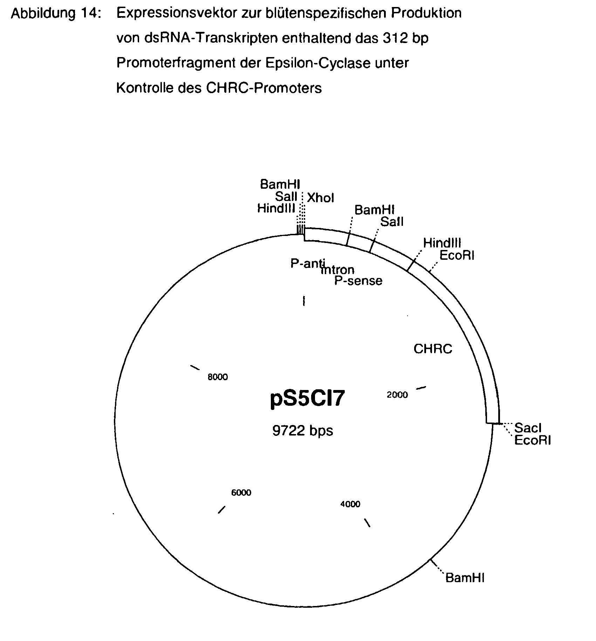

Zur Herstellung des Expressionsvektors pS5CI7 wurde das 2445bp SacI-XhoI Fragment aus cs45 mit dem SacI-XhoI geschnittenen Vektor pSUN5 ligiert (Abbildung 14, Konstruktkarte).

In der Abbildung 14 beinhaltet Fragment *CHRC* den CHRC-Promoter (1537 bp), Fragment *P-sense* das 312 bp Promoterfragment der Epsilon-Cyclase in sense Orientierung, Fragment *intron* das Intron IV2 des Kartoffel-Gens ST-LS1), und Fragment *P-anti* das 312 bp Promoterfragment der Epsilon- Cyclase in antisense Orientierung.

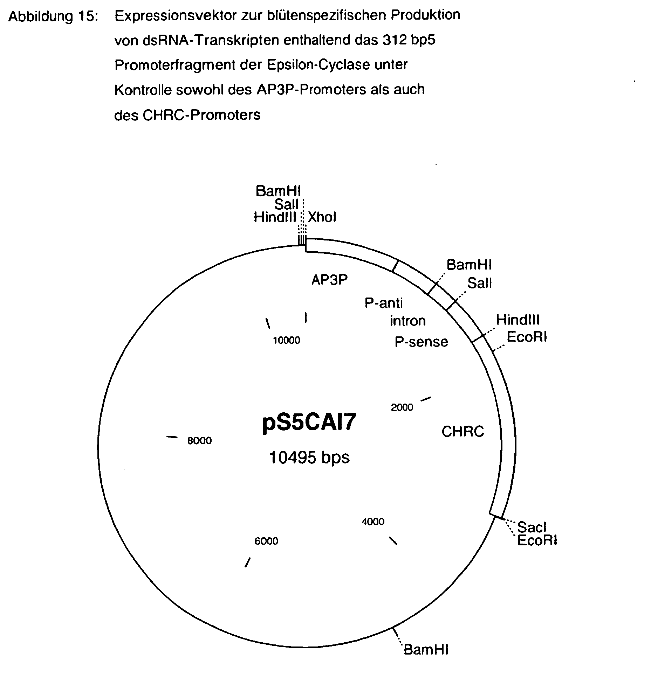

Zur Herstellung des Expressionsvektors pS5CAI7 wurde das 3219bp SacI-XhoI Fragment aus cs46 mit dem SacI-XhoI geschnittenen Vektor pSUN5 ligiert (Abbildung 15, Konstruktkarte)

In der Abbildung 15 beinhaltet Fragment *CHRC* den CHRC-Promoter (1537 bp), Fragment *P-sense* das 312 bp Promoterfragment der Epsilon-Cyclase in sense Orientierung, Fragment *intron* das Intron IV2 des Kartoffel-Gens ST-LS1), Fragment *P-anti das* 312 bp Promoterfragment der Epsilon-Cyclase in antisense Orientierung und das Fragment *AP3P* das 771 bp AP3P-Promoterfragment in antisense Orientierung.

### Beispiel I.15

### Herstellung transgener Tagetes Pflanzen mit reduzierter ε-Cyclase-Aktivität

Tagetessamen werden sterilisiert und auf Keimungsmedium (MS-Medium; Murashige and Skoog, Physiol. Plant. 15(1962), 473-497) pH 5,8, 2 % Saccharose) aufgelegt. Die

Keimung erfolgt in einem Temperatur/Licht/Zeitintervall von 18 bis 28_C / 20 bis 200 mE /3 bis 16 Wochen, bevorzugt jedoch bei 21_C, 20 bis 70 mE, für 4 bis 8 Wochen.

Alle Blätter der sich bis dahin entwickelten in vitro Pflanzen werden geerntet und quer zur Mittelrippe geschnitten. Die dadurch entstehenden Blattexplantate mit einer Größe von 10 bis 60 mm² werden im Verlaufe der Präparation in flüssigem MS-Medium bei Raumtemperatur für maximal 2 h aufbewahrt.

Der Agrobakterium tumefaciens Stamm EHA105 wurde mit dem Binärplasmid pS5AI3 transformiert. Die Anzucht des transformierten A. tumefaciens Stammes EHA105 erfolgte über Nacht unter folgenden Bedingungen: Eine Einzelkolonie wurde in YEB (0,1 % Hefeextrakt, 0,5 % Rindfleischextrakt, 0,5 % Pepton, 0,5 % Saccharose, 0,5 % Magnesiumsulfat x 7 H₂O) mit 25 mg/l Kanamycin angeimpft und bei 28_C für 16 bis 20 h angezogen. Anschließend wurde die Bakteriensuspension durch Zentrifugation bei 6000 g für 10 min geerntet und derart in flüssigem MS Medium resuspendiert, das eine OD₆₀₀ von ca. 0,1 bis 0,8 entstand. Diese Suspension wurde für die Co-Kultivierung mit dem Blattmaterial verwendet.

Unmittelbar vor der Co-Kultivierung wird das MS-Medium, in dem die Blätter aufbewahrt worden sind, durch die Bakteriensuspension ersetzt. Die Inkubation der Blättchen in der Agrobakteriensuspension erfolgte für 30 min unter leichtem Schütteln bei Raumtemperatur. Anschließend werden die infizierten Explantate auf ein mit Agar (z.B. 0,8 % Plant Agar (Duchefa, NL) verfestigtes MS-Medium mit Wachstumsregulatoren, wie beispielsweise 3 mg/l Benzylaminopurin (BAP) sowie 1 mg/l Indolylessigsäure (IAA) aufgelegt. Die Orientierung der Blätter auf dem Medium ist bedeutungslos. Die Kultivierung der Explantate findet für 1 bis 8 Tage, bevorzugt aber für 6 Tage statt, dabei können folgende Bedingungen angewendet werden: Lichtintensität: 30 bis 80 mMol/m² x sec, Temperatur: 22 bis 24°C, hell/dunkel Wechsel von 16/8 Stunden. Anschließend werden die co-kultivierten Explantate auf frisches MS-Medium, bevorzugt mit den gleichen Wachstumsregulatoren übertragen, wobei dieses zweite Medium zusätzlich ein Antibiotikum zur Unterdrückung des Bakterienwachstums enthält. Timentin in einer Konzentration von 200 bis 500 mg/l ist für diesen Zweck sehr geeignet. Als zweite selektive Komponente wird eine für die Selektion des Transformationserfolges eingesetzt. Phosphinothricin in einer Konzentration von 1 bis 5 mg/l selektiert sehr effizient, aber auch andere selektive Komponenten gemäß des zu verwendenden Verfahrens sind denkbar.

Nach jeweils ein bis drei Wochen erfolgt der Transfer der Explantate auf frisches Medium bis sich Sprossknospen und kleine Sprosse entwickeln, die dann auf das gleiche Basalmedium einschließlich Timentin und PPT oder alternative Komponenten mit Wachstumsregulatoren, nämlich z.B. 0,5 mg/l Indolylbuttersäure (IBA) und 0,5 mg/l Gibberillinsäure GA₃, zur Bewurzelung übertragen werden. Bewurzelte Sprosse können ins Gewächshaus überführt werden.

Zusätzlich zu der beschriebenen Methode sind folgende vorteilhafte Modifikationen möglich:
- Bevor die Explantate mit den Bakterien infiziert werden, können sie für 1 bis 12 Tage, bevorzugt 3 bis 4, auf das oben beschriebene Medium für die Co-Kultur vorinkubiert werden. Anschließend erfolgt die Infektion, Co-Kultur und selektive Regeneration wie oben beschrieben.
- Der pH Wert für die Regeneration (normalerweise 5,8) kann auf pH 5,2 gesenkt werden. Dadurch wird die Kontrolle des Agrobakterienwachstums verbessert.
- Die Zugabe von AgNO₃ (3 - 10 mg/l) zum Regenerationsmedium verbessert den Zustand der Kultur einschließlich der Regeneration selbst.
- Komponenten, die die Phenolbildung reduzieren und dem Fachmann bekannt sind, wie z.B. Zitronensäure, Ascorbinsäure, PVP u.v.a.m., wirken sich positiv auf die Kultur aus.
- Für das gesamte Verfahren kann auch flüssiges Kulturmedium Verwendung finden. Die Kultur kann auch auf handelsüblichen Trägern, die auf dem flüssigen Medium positioniert werden inkubiert werden.

Gemäß der oben beschriebenen Transformationsmethode wurden mit dem Expressionskonstrukt pS5A13 folgende Linien erhalten:
CS30-1, CS30-3 und CS30-4

### Beispiel I.16:

### Charakterisierung der transgenen Tagetes Pflanzen mit reduzierter ε-Cyclase-Aktivität

Das Blütenmaterial der transgenen Tagetes erecta Pflanzen aus Beispiel 1.15 wurde in flüssigem Stickstoff gemörsert und das Pulver (etwa 250 bis 500 mg) mit 100 % Aceton extrahiert (dreimal je 500 ml). Das Lösungsmittel wurde evaporiert und die Carotinoide in 100 ml Aceton resuspendiert.

Mittels einer C30-reverse phase-Säule konnten die individuellen Carotinoide quantifiziert werden. Die HPLC-Laufbedingungen waren nahezu identisch mit einer publizierten Methode (Frazer et al. (2000), Plant Journal 24(4): 551-558). Eine Identifizierung der Carotinoide war aufgrund der UV-VIS-Spektren möglich.

Tabelle 2 zeigt das Carotinoidprofil in Tagetespetalen der gemäß der vorstehend beschriebenen Beispiele hergestellten transgenen Tagetes- und Kontrolltagetespflanzen. Alle Carotinoidmengen sind in [ug/g] Frischgewicht angegeben, prozentuale Veränderungen gegenüber der Kontrollpflanze sind in Klammern angegeben.

Im Vergleich zur genetisch nicht veränderten Kontrollpflanze, weisen die genetisch veränderten Pflanzen mit reduzierter epsilon-Cyclase-Aktivität einen deutlich erhöhten Gehalt an Carotinoiden des "β-Carotin-Weges", wie beispielsweise β-Carotin und Zeaxanthin und einen deutlich reduzierten Gehalt an Carotinoiden des "α-Carotin-Weges", wie beispielsweise Lutein auf.

**Tabelle 2**

| Pflanze | Lutein | b-Carotin | Zeaxanthin | Violaxanthin | Gesamt-Carotinoide |
|---|---|---|---|---|---|
| Kontrolle | 260 | 4,8 | 2,7 | 36 | 304 |
| CS 30-1 | 35 (-86%) | 13 (+170%) | 4,4 (+62%) | 59 (+63%) | 111 (-63%) |
| Kontrolle | 456 | 6,4 | 6,9 | 58 | 527 |
| CS 30-3 | 62 (-86%) | 13 (+103%) | 8,9 (+29%) | 75 (+29%) | 159 (-70%) |
| CS 30-4 | 68 (-85%) | 9,1 (+42%) | 5,7 (-17%) | 61 (+5%) | 144 (-73%) |

### Beispiel II

### Herstellung astaxanthinhaltiger Pflanzenteile der Gattung Tagetes

Die Blütenkopfe oder die Petalen der gemäß Beispiel I.6 hergestellten astaxanthinhalligen Pflanzen der Gattung Tagetes werden abgetrennt und getrocknet. Anschließend werden die getrockneten Blütenköpfe oder Petalen durch Zerkleinerung in Pulverform überführt

### Beispiel IV

### Herstellung von extrudiertem Forelienfutter, enthaltend astaxanthinhaltige Pflanzen oder Pflanzenteilen der Gattung Tagetes

Die folgenden Komponenten werden in einem Doppeischneckenextruder extrudiert.

| ***Komponenten*** | ***(%)*** | **Einwaage f. 500 kg** ***Kg*** |
|---|---|---|
| Fischmehl | 30,00 | 150,00 |
| Sojavollfettbohnen | 20,00 | 100,00 |
| Weizenquellstärke | 18,00 | 90,00 |
| Vitamin-Prämix | 0,80 | 4,00 |
| Cholinchlorid (50%) | 0,20 | 1,00 |
| Weizenkleber | 20,00 | 100,00 |
| Sipernat 50S | 3,00 | 15,00 |
| Fischöl | 8,00 | 40,00 |

Die pulverförmigen, prozessierten astaxanthinhaltige Pflanzen oder Pflanzenteilen der Gattung Tagetes, beispielsweise hergestellt nach Beispiel II, werden vor der Extrusion als Komponente zugegeben.

Die Astaxanthin-wirkstoff-Dosierung liegt bei 10, 20 und 40 mg Astaxanthin pro kg Diät.

Nach Beendigung des Extrusionsprozesses wird das Extrudat getrocknet und gekühlt.

### Beispiel V

Orale Verabreichung astaxanthinhaltiger Pflanzen oder Pflanzenteilen der Gattung Tagetes an Forellen in einem Forellenstandardfutter - Prüfung der Bioverfügbarkeit.

Das Forellenfutter, enthaltend die erfindungsgemäßen Astaxanthinpigmentierstoffe, wird gemäß Beispiel IV hergestellt und an Forellen (durchschnittliche Lebendmasse von 180 g) oral verabreicht. Es werden 3 Konzentrationen getestet: 10, 20 und 40 mg Astaxanthin aus der erfindungsgemäßen Astaxanthinpigmentierung pro kg Diät.

Die Haltung der Forellen erfolgt wie nachstehend beschrieben:
- Die Forellen erhalten standardmäßig eine Adaptationsphage von 14 Tagen.
- Während des Fütterungsversuches werden 10 Forellen pro Becken in 80 l Wasser fassenden Durchfluß-Kunstofftanks gehalten. Die Wassertemperatur liegt bei 15°C. Das Wasser wird biologisch gereinigt und as werden täglich mindestens 10% der Gesamtwassermenge durch Frischwasser ersetzt.
- Die Beleuchtungsdauer liegt bei 12 Stunden pro Tag, um eine vorzeitige Geschlechtsreife der Tier zu vermeiden.
- Die Anzahl Becken pro Behandlung liegt bei 3. Dies ist äquivalent zu 30 Forellen pro Dosisstufe.
- Aufbewahrung der Diäten erfolgt bei -20°C, um Astaxanthinverluste zu ermelden. Das Futter wird portionsweise (wochenweise) aufgetaut und verabreicht.
- Die Versuchsdauer beträgt 8 Wochen.

Die Fütterung der Forellen erfolgt wie nachstehend beschrieben:
- Bei den verabreichten Versuchsdiäten handelt es sich um das gemäß Beispiel IV hergestellte extrudierte Forellenfutter, das zusätzlich noch öl-geccated wird.
- Während der Adaptationsphase wird extrudiertes mit Öl gecoatetes actaxanthin-freies Forellenstandardfutter gemäß Beispiel IV ohne Astaxanthin verabreicht.
- Als Negativkontrolle wird extrudiertes mit Öl gecoatetes astaxanthin-freies Forellenstandardfutter gemäß Beispiel IV ohne Astaxanthin während des gesamden Versuchszeitraumes verabreicht.
- Die Fütterung erfolgt 2x täglich von Hand bis zur Sättigung der Tiere.

Untersucht wird der Einfluß der erfindunggemäßen Astaxanthinpigmentierung sowohl auf Leistungsparameter der Fische, wie Futteraufnahme, Futterverwertung und Lebendmassezuwachs als auch auf die Bioeffizienz der Pigmentierung.

Statisch ausgewertet werden durchschnittlicher Futterverbrauch pro Fisch, Futteraufwand und Lebendmassezuwachs.

Die Pigmentierung der Fische wird durch remissionspektrophotometrische Messungen (Minolta-a-Wert = Rotwert am Filetanschnitt) und durch Bestimmung des Astaxanthingehalts (mg/kg) im Filet jeweils im Vergleich zur Negativkontrolle gemessen.

Die Minoltawerte a-Werte, welche den Rotanteil des Farbtons repräsentieren, nehmen mit kleiner werdender Steigung der Funktion dosisabhängig zu. Die Minolta b- Werte, die den Gelbanteil widerspiegeln liegen im negativen Bereich oder bewegen sich um Null. Dies bedeutet der Rotton der Forellenfilets weist eine Abhängigkeit zu der aufgenommenen Astaxanthinmenge auf.

Während der Versuches werden für die beobachteten Leistungsparameter sowohl zwischen als auch innerhalb der Behandlungen (Astaxanthinhaltiges Pulver, astaxanthinhaltiger Extrakt in flüssiger Form, synthetisches Astaxanthin, Negativkontrolle) keine statistisch gerichteten Unterschiede beobachtet.

Es zeigt sich, dass astaxanthinhaltige Pflanzen oder Pflanzenteile der Gattung Tagetes bei der Pigmentierung von Forellen als Vertreter der Salmoniden bioverfügbar sind und zudem zu keinen adversen Effekten auf die biologische Leistung der Forelle führen.

## Patentansprüche

1. Verwendung von astaxanthinhaltigen Pflanzen oder Pflanzenteilen der Gattung Tagetes zur oralen Verabreichung an Tiere.

2. Verwendung nach Anspruch 1, **dadurch gekennzeichnet, dass** die astaxanthinhaltigen Pflanzen oder Pflanzenteile der Gattung Tagetes zur Pigmentierung von Tieren und der entsprechenden Tierprodukte verwendet werden.

3. Verwendung nach Anspruch 1 oder 2, **dadurch gekennzeichnet, dass** die astaxanthinhaltigen Pflanzen oder Pflanzenteile der Gattung Tagetes Tierfutterzubereitungen beigemischt werden und die Tierfutterzubereitung an Tiere oral verabreicht werden.

4. Verwendung nach Anspruch 3, **dadurch gekennzeichnet, dass** die astaxanthinhaltigen Pflanzen oder Pflanzenteile der Gattung Tagetes vor der Beimischung zu Tierfutterzubereitungen in eine Form prozessiert werden, die eine Beimischung zu Tierfutterzubereitungen ermöglicht.

5. Verwendung nach Anspruch 1 oder 2, **dadurch gekennzeichnet, dass** die astaxanthinhaltigen Pflanzen oder Pflanzenteile der Gattung Tagetes direkt an Tiere oral verabreicht werden.

6. Verwendung nach Anspruch 5, **dadurch gekennzeichnet, dass** die astaxanthinhaltigen Pflanzen oder Pflanzenteile der Gattung Tagetes vor der Verabreichung in eine Form prozessiert werden, die eine direkte orale Verabreichung an Tiere ermöglicht.

7. Verwendung nach einem der Ansprüche 1 bis 6, **dadurch gekennzeichnet, dass** die Tiere ausgewählt sind aus der Gruppe Fische, Crustaceae, Galliformes und Anatridae.

8. Verwendung nach einem der Ansprüche 1 bis 7, **dadurch gekennzeichnet, dass** man als Pflanzenteile Blütenköpfe oder Petalen verwendet.

9. Verfahren zur Herstellung von Tierfutterzubereitungen durch Zusammenfügen von astaxanthinhaltigen Pflanzen oder Pflanzenteilen der Gattung Tagetes und üblichen Tierfutterkomponenten.

10. Verfahren nach Anspruch 9, **dadurch gekennzeichnet, dass** die astaxanthinhaltigen Pflanzen oder Pflanzenteile der Gattung Tagetes vor dem Zusammenfügen mit Tierfuttermitteln in eine Form prozessiert werden, die ein Zusammenfügen mit Tierfuttermittein ermöglicht.

11. Verfahren zur Pigmentierung von Tieren oder Tierprodukten durch orale Verabreichung von astaxanthinhaltigen Pflanzen oder Pflanzenteilen der Gattung Tagetes an Tiere.

12. Verfahren zur Herstellung von pigmentierten Tieren oder Tierprodukten durch orale Verabreichung von astaxanthinhaltigen Pflanzen oder Pflanzenteilen der Gattung Tagetes an Tiere.

13. Verfahren nach Anspruch 11 oder 12, **dadurch gekennzeichnet, dass** die astaxanthinhaltigen Pflanzen oder Pflanzenteile der Gattung Tagetes Tierfutterzubereitungen beigemischt werden und die Tierfutterzubereitung an Tiere oral verabreicht werden.

14. Verfahren nach Anspruch 13, **dadurch gekennzeichnet, dass** die astaxanthinhaltigen Pflanzen oder Pflanzenteile der Gattung Tagetes vor der Beimischung zu Tierfutterzubereitungen in eine Form prozessiert werden, die eine Beimischung zu Tierfutterzubereitungen ermöglicht.

15. Verfahren nach Anspruch 11 oder 12, **dadurch gekennzeichnet, dass** die astaxanthinhaltigen Pflanzen oder Pflanzenteile der Gattung Tagetes direkt an Tiere oral verabreicht werden.

16. Verfahren nach Anspruch 15, **dadurch gekennzeichnet, dass** die astaxanthinhaltigen Pflanzen oder Pflanzenteile der Gattung Tagetes vor der Verabreichung in eine Form prozessiert werden, die eine direkte orale Verabreichung an Tiere ermöglicht.

17. Verfahren nach einem der Ansprüche 11 bis 16 **dadurch gekennzeichnet, dass** die astaxanthinhaltigen Pflanzen der Gattung Tagetes durch genetische Veränderung in die Lage versetzt wurden, Astaxanthin herzustellen.

18. Verfahren nach einem der Ansprüche 11 bis 17, **dadurch gekennzeichnet, dass** die Tiere ausgewählt sind aus der Gruppe Fische, Crustaceae, Galliformes und Anatridae.

19. Verfahren nach einem der Ansprüche 11 bis 18, **dadurch gekennzeichnet, dass** die Tierprodukte ausgewählt sind aus der Gruppe Fleisch, Haut, Feder und Ei.

20. Verfahren nach einem der Ansprüche 11 bis 19, **dadurch gekennzeichnet, dass** man als Pflanzenteile Blütenköpfe oder Petalen verwendet.

21. Verwendung von astaxanthinhaltigen Pflanzen oder Pflanzenteilen der Gattung Tagetes als Tierfutter oder Tierfutterzusatz.

## Claims

1. The use of astaxanthin-containing plants or parts of plants of the genus Tagetes for oral administration to animals.

2. The use according to claim 1, wherein the astaxanthin-containing plants or parts of plants of the genus Tagetes are used for pigmenting animals and the corresponding animal products.

3. The use according to claim 1 or 2, wherein the astaxanthin-containing plants or parts of plants of the genus Tagetes are admixed to animal feed preparations and the animal feed preparation is orally administered to animals.

4. The use according to claim 3, wherein the astaxanthin-containing plants or parts of plants of the genus Tagetes, before the admixture to animal feed preparations, are processed into a form which makes possible admixture to animal feed preparations.

5. The use according to claim 1 or 2, wherein the astaxanthin-containing plants or parts of plants of the genus Tagetes are orally administered directly to animals.

6. The use according to claim 5, wherein the astaxanthin-containing plants or parts of plants of the genus Tagetes, before the administration, are processed into a form which makes possible direct oral administration to animals.

7. The use according to one of claims 1 to 6, wherein the animals are selected from the group consisting of fish, Crustaceae, Galliformes and Anatridae.

8. The use according to one of claims 1 to 7, wherein the plant parts used are flower heads or petals.

9. A method for producing animal feed preparations by combining astaxanthin-containing plants or parts of plants of the genus Tagetes and customary animal feed components.

10. The method according to claim 9, wherein the astaxanthin-containing plants or parts of plants of the genus Tagetes are, before the combination with animal feedstuffs, processed into a form which makes possible combination with animal feedstuffs.

11. A method for pigmenting animals or animal products by oral administration of astaxanthin-containing plants or parts of plants of the genus Tagetes to animals.

12. A method for producing pigmented animals or animal products by oral administration of astaxanthin-containing plants or parts of plants of the genus Tagetes to animals.

13. The method according to claim 11 or 12, wherein the astaxanthin-containing plants or parts of plants of the genus Tagetes are admixed to animal feed preparations and the animal feed preparation is orally administered to animals.

14. The method according to claim 13, wherein the astaxanthin-containing plants or parts of plants of the genus Tagetes, before admixture to animal feed preparations, are processed into a form which makes possible admixture to animal feed preparations.

15. The method according to claim 11 or 12, wherein the astaxanthin-containing plants or parts of plants of the genus Tagetes are orally administered directly to animals.

16. The method according to claim 15, wherein the astaxanthin-containing plants or parts of plants of the genus Tagetes, before administration, are processed into a form which makes possible direct oral administration to animals.

17. The method according to one of claims 11 to 16, wherein the astaxanthin-containing plants of the genus Tagetes have, by genetic manipulation, been made capable of producing astaxanthin.

18. The method according to one of claims 11 to 17, wherein the animals are selected from the group consisting of fish, Crustaceae, Galliformes and Anatridae.

19. The method according to one of claims 11 to 18, wherein the animal products are selected from the group consisting of meat, skin, feathers and egg.

20. The method according to one of claims 11 to 19, wherein the plant parts used are flower heads or petals.

21. The use of astaxanthin-containing plants or parts of plants of the genus Tagetes as animal feed or animal feed additive.

## Revendications

1. Utilisation de plantes ou de parties de plantes appartenant au genre *Tagetes,* contenant de l'astaxanthine, pour l'administration par voie orale à des animaux.

2. Utilisation selon la revendication 1, **caractérisée en ce que** les plantes ou parties de plantes appartenant au genre *Tagetes,* contenant de l'astaxanthine, sont utilisées pour la pigmentation d'animaux et des produits animaux correspondants.

3. Utilisation selon la revendication 1 ou 2, **caractérisée en ce qu'**on ajoute à des préparations d'aliments pour animaux les plantes ou parties de plantes appartenant au genre *Tagetes,* contenant de l'astaxanthine et on administre par voie orale à des animaux la préparation d'aliment pour animaux.

4. Utilisation selon la revendication 3, **caractérisée en ce qu'**avant l'addition à des préparations d'aliments pour animaux on met les plantes ou parties de plantes appartenant au genre *Tagetes,* contenant de l'astaxanthine, sous une forme qui permet une addition à des préparations d'aliments pour animaux.

5. Utilisation selon la revendication 1 ou 2, **caractérisée en ce qu'**on administre directement aux
animaux par voie orale les plantes ou parties de plantes appartenant au genre Tagetes, contenant de l'astaxanthine.

6. Utilisation selon la revendication 5, **caractérisée en ce qu'**avant l'administration on met les plantes ou parties de plantes appartenant au genre Tagetes, contenant de l'astaxanthine, sous une forme qui permet une administration directe par voie orale à des animaux.

7. Utilisation selon l'une quelconque des revendications 1 à 6, **caractérisée en ce que** les animaux sont choisis dans le groupe des poissons, crustacés, galliformes et anatidés.

8. Utilisation selon l'une quelconque des revendications 1 à 7, **caractérisée en ce qu'**on utilise comme parties de plantes les capitules ou les pétales.

9. Procédé pour la production de préparations d'aliments pour animaux par réunion de plantes ou de parties de plantes appartenant au genre *Tagetes,* contenant de l'astaxanthine, et de composants usuels d'aliments pour animaux.

10. Procédé selon la revendication 9, **caractérisé en ce qu'**avant la réunion avec les compositions d'aliments pour animaux on met les plantes ou parties de plantes appartenant au genre Tagetes, contenant de l'astaxanthine, sous une forme qui permet une réunion avec des compositions d'aliments pour animaux.

11. Procédé pour la pigmentation d'animaux ou de produits animaux par administration orale de plantes ou de parties de plantes appartenant au genre *Tagetes,* contenant de l'astaxanthine, à des animaux.

12. Procédé pour la production d'animaux ou de produits animaux pigmentés, par administration orale de plantes ou de parties de plantes appartenant au genre Tagetes, contenant de l'astaxanthine, à des animaux.

13. Procédé selon la revendication 11 ou 12, **caractérisé en ce qu'**on ajoute à des préparations d'aliments pour animaux les plantes ou parties de plantes appartenant au genre Tagetes, contenant de l'astaxanthine, et on administre la préparation d'aliment pour animaux par voie orale à des animaux.

14. Procédé selon la revendication 13, **caractérisé en ce qu'**avant l'addition à des préparations d'aliments pour animaux on met les plantes ou parties de plantes appartenant au genre *Tagetes,* contenant de l'astaxanthine, sous une forme qui permet une addition à des préparations d'aliments pour animaux.

15. Procédé selon la revendication 11 ou 12, **caractérisé en ce qu'**on administre directement aux animaux par voie orale les plantes ou parties de plantes appartenant au genre Tagetes, contenant de l'astaxanthine.

16. Procédé selon la revendication 15, **caractérisé en ce qu'**avant l'administration on met les plantes ou parties de plantes appartenant au genre *Tagetes,* contenant de l'astaxanthine, sous une forme qui permet une administration directe par voie orale à des animaux.

17. Procédé selon l'une quelconque des revendications 11 à 16, **caractérisé en ce que** par des modifications génétiques on a rendu les plantes appartenant au genre *Tagetes,* contenant de l'astaxanthine, aptes à produire de l'astaxanthine.

18. Procédé selon l'une quelconque des revendications 11 à 17, **caractérisé en ce que** les animaux sont choisis dans le groupe des poissons, crustacés, galliformes et anatidés.

19. Procédé selon l'une quelconque des revendications 11 à 18, **caractérisé en ce que** les produits animaux sont choisis dans le groupe constitué par la chair, la peau, les plumes et les oeufs.

20. Procédé selon l'une quelconque des revendications 11 à 19, **caractérisé en ce qu'**on utilise comme parties de plantes les capitules ou les pétales.

21. Utilisation de plantes ou de parties de plantes appartenant au genre *Tagetes,* contenant de l'astaxanthine, en tant qu'aliment pour animaux ou additif à des aliments pour animaux.
